# EUROPEAN PATENT SPECIFICATION

(11) **EP 3 212 225 B1**
(45) Date of publication and mention of the grant of the patent: **19.11.2025**
(21) Application number: 15855013.7
(22) Date of filing: 30.10.2015
(51) Int. Cl.: A61K 40/42, A61K 40/11, C07K 14/725, C07K 14/705, C07K 16/28, C07K 16/32, C12N 5/0783, A61K 40/32, A61K 40/33, A61K 40/36

(54) **METHODS AND COMPOSITIONS FOR MODIFIED T CELLS**
VERFAHREN UND ZUSAMMENSETZUNGEN FÜR MODIFIZIERTE T-ZELLEN
PROCÉDÉS ET COMPOSITIONS PERMETTANT L'OBTENTION DE LYMPHOCYTES T MODIFIÉS

(30) Priority: 31.10.2014 US 201462073144 P; 31.10.2014 US 201462073343 P; 31.10.2014 US 201462073467 P; 31.10.2014 US 201462073540 P; 31.10.2014 US 201462073681 P
(43) Date of publication of application: 06.09.2017
(60) Divisional application: 25207728.4
(73) Proprietor: The Trustees of the University of Pennsylvania, Philadelphia PA 19104-6283 (US)
(72) Inventor: ZHAO, Yangbing, Lumberton, New Jersey 08048 (US); LIU, Xiaojun, Swarthmore, Pennsylvania 19081 (US); JUNE, Carl H., Merion Station, Pennsylvania 19066 (US)
(74) Representative: Grünecker Patent- und Rechtsanwälte PartG mbB
(86) International application number: PCT/US2015/058339
(87) International publication number: WO 2016/070061

(56) References cited:
- WO-A1-2013/123061
- WO-A1-2014/138306
- WO-A2-2014/011988
- US-A1- 2012 294 874
- US-A1- 2013 274 203
- LING ZHANG ET AL: "Improving Adoptive T Cell Therapy by Targeting and Controlling IL-12 Expression to the Tumor Environment", MOLECULAR THERAPY : THE JOURNAL OF THE AMERICAN SOCIETY OF GENE THERAPY, vol. 19, no. 4, 1 February 2011 (2011-02-01), US, pages 751 - 759, XP055327746, ISSN: 1525-0016, DOI: 10.1038/mt.2010.313
- ROBBINS PAUL F ET AL: "Single and dual amino acid substitutions in TCR CDRs can enhance antigen-specific T cell functions", THE JOURNAL OF IMMUNOLOGY, AMERICAN ASSOCIATION OF IMMUNOLOGISTS, US, vol. 180, no. 9, 1 May 2008 (2008-05-01), pages 6116 - 6131, XP002571412, ISSN: 0022-1767

## Description

### BACKGROUND OF THE INVENTION

Adoptive cell transfer (ACT) using chimeric antigen receptor (CAR) modified T cells has been shown to be a promising strategy for the treatment of cancers (Louis et al., 2011, Blood 118:6050-6056; Kochenderfer et al., 2010, Blood 116:3875-3886 and Porter et al., 2011, N Engl J Med 365:725-733).

Integration associated safety concerns using lentiviral or retroviral vectors are a major concern for modification of cells used for ACT. Some advances are being made to avoid on-target or off-target unwanted side effects, such as RNA transfection of T cells with T cell receptor (TCR) or CAR RNA electroporation (Zhao, 2006, Mol Ther 13: 151-159; Mitchell et al., Smits et al., 2004, Leukemia 18:1898-1902). By minimizing dosage of both RNA and T cells, such methods efficiently permit the introduction of multiple genes. However, the major constraint for transient expression of CARs is the suboptimal effector activity and functionality of RNA transfected T cells. Multiple T cell infusions and/or significant use of low dose chemotherapy have been used to improve function (Barrett et al., 2013, Hum Gene Ther 24(8):717-27).

Various attempts have been made to improve effector activity and functionality while avoiding combination therapies and additional treatments. Increasing RNA during the transfection process posed a negative impact on T cell function, especially in vivo anti-tumor activities (Barrett et al., 2011, Hum Gene Ther 22:1575-1586). Additionally, alternative constructs fusing an anti-CD3 antigen antibody fragment to an anti-tumor antigen antibody fragment have also been tested in clinical trials for cancer treatments (Bargou et al., 2008, Science 321:974-977; Klinger et al., 2012, Blood 119:6226-6233.). Unfortunately, these constructs were severely limited in functionality by a short half-life, poor accessibility to target cell sites, and lack of proper long term signaling function.

Therefore a need exists for safer methods of modifying T cells, while generating T cells with maximal effector activity and functionality in vivo for T cell based adoptive immunotherapy.

Ling Zhang et al., Molecular Therapy : The Journal of the American Society of Gene Therapy, vol. 19, no. 4, published 1 February 2011, pages 751-759, describes improving adoptive T cell therapy by targeting and controlling IL-12 expression to the tumor environment.

Robbins Paul F. et al., The Journal of Immunology, American Association of Immunologists, US, vol. 180, no. 9, published 1 May 2008, pages 6116-6131, describes single and dual amino acid substitutions in TCR CDRs that can enhance antigen-specific T cell functions.

### SUMMARY OF THE INVENTION

The invention is defined in the appended claims. The references to methods of treatment by therapy in this description and examples are to be interpreted as references to modified T cells, pharmaceutical compositions and medicaments of the invention for use in those methods. As described herein, the present invention relates to compositions and methods for modifying T cells, as defined in the claims.

In one aspect, the invention includes a modified T cell comprising an exogenous nucleic acid encoding a T cell receptor (TCR) comprising affinity for an antigen on a target cell and a nucleic acid encoding a bispecific antibody, wherein the T cell expresses the TCR and bispecific antibody on a surface of the T cell, wherein the bispecific antibody comprises a bispecific antigen binding domain wherein the bispecific antigen binding domain comprises a first and a second single chain variable fragment (scFv) molecules, and wherein the first scFv molecule is specific for at least one antigen on a target cell and the second scFv molecule is specific for an antigen on an activating T cell, and wherein the activating T cell antigen is CD3.

In another aspect, the invention includes a method for generating a modified T cell comprising introducing a nucleic acid encoding a modified T cell receptor (TCR) comprising affinity for an antigen on a target cell into a T cell and a nucleic acid encoding a bispecific antibody, wherein the electroporated T cell is capable of expressing the TCR and bispecific antibody, preferably wherein the nucleic acid encoding the TCR comprises a nucleic acid encoding a TCR alpha and a TCR beta chain, wherein the bispecific antibody comprises a bispecific antigen binding domain wherein the bispecific antigen binding domain comprises a first and a second single chain variable fragment (scFv) molecules, and wherein the first scFv molecule is specific for at least one antigen on a target cell and the second scFv molecule is specific for an antigen on an activating T cell, and wherein the activating T cell antigen is CD3 .

In yet another aspect, the invention includes the modified T cell described herein for use in stimulating a T cell-mediated immune response to a target cell or tissue in a subject; optionally further comprising inducing lysis of the target cell or tissue; preferably wherein the induced lysis is antibody-dependent cell-mediated cytotoxicity (ADCC). The invention further provides the modified T cell of the invention for use in adoptive cell transfer therapy for preventing or treating an immune reaction that is adverse to the subject.

The invention further provides the modified T cell of the invention for use in treating an autoimmune disease or cancer.

In another aspect, the invention includes a pharmaceutical composition comprising the modified T cell of the invention and optionally a pharmaceutically acceptable carrier.

In various embodiments of the above aspects or any other aspect of the invention delineated herein, the TCR comprises at least one disulfide bond. In one embodiment, the TCR comprises at least one murine constant region. In another embodiment, the TCR has higher affinity for the target cell antigen than a for a wildtype TCR. In yet another embodiment, the TCR comprises TCR alpha and beta chains. In one embodiment, the TCR comprises a co-stimulatory signaling domain, such as-a a 4-1BB co-stimulatory signaling domain, at a C' terminal of at least one of the chains. In one embodiment, the beta chain comprises at least one N-deglycosylation. In one embodiment, the alpha chain comprises at least one N-deglycosylation.

In another embodiment, the target cell antigen is selected from the group consisting of a viral antigen, bacterial antigen, parasitic antigen, tumor cell associated antigen (TAA), disease cell associated antigen, and any fragment thereof.

In another embodiment, the bispecific antibody comprises a bispecific antigen binding domain selected from the group consisting of a synthetic antibody, human antibody, a humanized antibody, single chain variable fragment, single domain antibody, an antigen binding fragment thereof, and any combination thereof.

The bispecific antigen binding domain comprises a first and a second single chain variable fragment (scFv) molecules, such as the first scFv molecule is specific for at least one antigen on a target cell and the second scFv molecule is specific for an antigen on an activating T cell.

The activating T cell antigen is CD3, as defined in the appended claims.

In another embodiment, the T cell described herein further comprises an electroporated nucleic acid encoding a costimulatory molecule. In yet another embodiment, the T cell is obtained from the group consisting of peripheral blood mononuclear cells, cord blood cells, a purified population of T cells, and a T cell line.

In another embodiment, the co-stimulatory molecule is selected from the group consisting of CD3, CD27, CD28, CD83, CD86, CD127, 4-1BB, 4-1BBL, PD1 and PD1L. In yet another embodiment, the methods described herein further comprise electroporating a RNA encoding CD3 into the T cells. In one embodiment, the CD3 RNA is co-electroporated with the TCR nucleic acid.

In another embodiment, the nucleic acids comprise in vitro transcribed RNA or synthetic RNA. In yet another embodiment, the nucleic acid encoding the TCR comprises a nucleic acid encoding a TCR alpha and a TCR beta chain. In one embodiment, the step of introducing the nucleic acid comprises co-electroporating a RNA encoding the TCR alpha chain and a separate RNA encoding the TCR beta chain.

In another embodiment, the methods described herein further comprise expanding the T cell. In one embodiment, the expanding comprises culturing the T cell with a factor selected from the group consisting of flt3-L, IL-1, IL-2, IL-3 and c-kit ligand. In one embodiment, the expanding comprises electroporating the T cell with RNA encoding a chimeric membrane protein and culturing the electroporated T cell, such as a chimeric membrane protein comprising a single chain variable fragment (scFv) against CD3 and an intracellular domain comprising a fragment of an intracellular domain of CD28 and 4-1BB.

In another embodiment, the methods described herein further comprise cryopreserving the T cell. In one embodiment, the methods described herein further comprise thawing the cryopreserved T cell prior to introducing the nucleic acids into the T cell. In yet another embodiment, the methods described herein further comprise cryopreserving the T cells after introducing the TCR nucleic acid. In still another embodiment, the methods described herein further comprise expressing the bispecific antibody as a membrane protein. In still another embodiment, the methods described herein further comprise cryopreserving the bispecific antibody introduced T cells. In yet another embodiment, the methods described herein further comprise inducing lysis of the target cell or tissue. In one embodiment, the induced lysis is antibody-dependent cell-mediated cytotoxicity (ADCC).

In another embodiment, the condition is an autoimmune disease, such as an autoimmune disease selected from the group consisting of Acquired Immunodeficiency Syndrome (AIDS), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barre syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Meniere's disease, mixed connective tissue disease, multiple sclerosis, myasthenia gravis, pernacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjogren's syndrome, stiff-man syndrome, systemic lupus erythematosus, Takayasu arteritis, temporal arteritis/giant cell arteritis, ulcerative colitis, uveitis, vitiligo, Wegener's granulomatosis, and any combination thereof. In yet another embodiment, the condition is a cancer, such as a cancer selected from the group consisting of breast cancer, prostate cancer, ovarian cancer, cervical cancer, skin cancer, pancreatic cancer, colorectal cancer, renal cancer, liver cancer, brain cancer, lymphoma, leukemia, lung cancer, and any combination thereof.

Disclosed but not claimed is a modified T cell comprising a nucleic acid encoding a bispecific antibody comprising bispecificity for an antigen on a target cell and an antigen on the T cell and a nucleic acid encoding a chimeric ligand engineered activation receptor (CLEAR), wherein the T cell expresses the bispecific antibody and CLEAR.

Disclosed, but not claimed, is a method for generating a modified T cell comprising introducing a nucleic acid encoding a bispecific antibody and a nucleic acid encoding a chimeric ligand engineered activation receptor (CLEAR) into a T cell, wherein the T cell is capable of expressing the bispecific antibody and the CLEAR.

Disclosed, but not claimed, is a method for stimulating a T cell-mediated immune response to a target cell or tissue in a subject comprising administering to a subject an effective amount of a modified T cell comprising a nucleic acid encoding chimeric ligand engineered activation receptor (CLEAR) and a nucleic acid encoding a bispecific antibody with bispecificity for an antigen on the target cell and the CLEAR on the T cell, wherein the modified T cells express the CLEAR and bispecific antibody.

Disclosed, but not claimed, is a method for adoptive cell transfer therapy comprising administering a population of modified T cells to a subject in need thereof to prevent or treat an immune reaction that is adverse to the subject, wherein the modified T cells comprise a nucleic acid encoding a chimeric ligand engineered activation receptor (CLEAR) and a nucleic acid encoding a bispecific antibody with bispecificity for an antigen on the target cell and the CLEAR on the T cell.

Disclosed, but not claimed, is a method of treating a disease or condition associated with enhanced immunity in a subject comprising administering a population of modified T cells to a subject in need thereof, wherein the modified T cells comprise a nucleic acid encoding chimeric ligand engineered activation receptor (CLEAR) and a nucleic acid encoding a bispecific antibody with bispecificity for an antigen on the target cell and the CLEAR on the T cell.

In still another aspect, the invention includes a method of treating a condition in a subject, comprising administering to the subject a therapeutically effective amount of a pharmaceutical composition comprising the modified T cell described herein.

In still another aspect, the invention includes a pharmaceutical composition comprising the modified T cell described herein and optionally a pharmaceutically acceptable carrier.

In various described, but not claimed, disclosures, the CLEAR comprises an intracellular activation domain and an extracellular domain. In one disclosure, the intracellular activation domain comprises a portion of an intracellular activation domain of CD3 zeta. In one disclosure, the extracellular domain is selected from the group consisting of an antigen binding domain of an antibody, a ligand binding domain of a receptor, an antigen, and a ligand. In one disclosure, the extracellular domain is selected from the group consisting of CD27, CD28, CD70, CD80, PD1, and PD-L1. In one disclosure, the extracellular domain is capable of binding to a tumor antigen.

In another disclosure, the CLEAR further comprises a co-stimulatory domain. In one disclosure, the co-stimulatory domain is selected from the group consisting of CD4, CD8, and 4-1BB.

In another embodiment, the target cell antigen is selected from the group consisting of a viral antigen, bacterial antigen, parasitic antigen, tumor cell associated antigen (TAA), disease cell associated antigen, and any fragment thereof.

In another embodiment, the bispecific antibody comprises a bispecific antigen binding domain selected from the group consisting of a synthetic antibody, human antibody, a humanized antibody, single chain variable fragment, single domain antibody, an antigen binding fragment thereof, and any combination thereof. In yet another embodiment, the bispecific antigen binding domain comprises a first and a second single chain variable fragment (scFv) molecules, such as the first scFv molecule is specific for at least one antigen on a target cell and the second scFv molecule is specific for the antigen on the T cell. In still another disclosure, the bispecific antibody comprises bispecificity for an antigen on the target cell and the CLEAR on the T cell.

In another embodiment, the T cell described herein further comprises a nucleic acid encoding a costimulatory molecule, such as a co-stimulatory molecule selected from the group consisting of CD3, CD27, CD28, CD83, CD86, CD127, 4-1BB, 4-1BBL, PD1 and PD1L. In still another embodiment, the T cell described herein is obtained from the group consisting of peripheral blood mononuclear cells, cord blood cells, a purified population of T cells, and a T cell line.

In another embodiment, at least one of the nucleic acids is introduced by a method selected from the group consisting of transducing the T cell, transfecting the T cell, and electroporating the T cell. In yet another embodiment, at least one of the nucleic acids comprise in vitro transcribed RNA or synthetic RNA.

In another embodiment, the methods described herein further comprise expanding the T cell. In one embodiment, the expanding comprises culturing the T cell with a factor selected from the group consisting of flt3-L, IL-1, IL-2, IL-3 and c-kit ligand. In one embodiment, the expanding comprises electroporating the T cell with RNA encoding a chimeric membrane protein, such as a chimeric membrane protein comprising a single chain variable fragment (scFv) against CD3 and an intracellular domain comprising a fragment of an intracellular domain of CD28 and 4-1BB, and culturing the electroporated T cell.

In another embodiment, the methods described herein further comprise cryopreserving the T cell. In one embodiment, the methods described herein further comprise thawing the cryopreserved T cell prior to introducing the nucleic acids into the T cell. In yet another disclosure, the methods described herein, but not claimed, further comprise cryopreserving the T cells after introducing the CLEAR nucleic acid. In still another embodiment, the methods described herein further comprise expressing the bispecific antibody as a membrane protein. In still another embodiment, the methods described herein further comprise cryopreserving the bispecific antibody introduced T cells. In yet another embodiment, the methods described herein further comprise inducing lysis of the target cell or tissue. In one embodiment, the induced lysis is antibody-dependent cell-mediated cytotoxicity (ADCC).

In another embodiment, the condition is an autoimmune disease, such as an autoimmune disease selected from the group consisting of Acquired Immunodeficiency Syndrome (AIDS), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barre syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Meniere's disease, mixed connective tissue disease, multiple sclerosis, myasthenia gravis, pernacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjogren's syndrome, stiff-man syndrome, systemic lupus erythematosus, Takayasu arteritis, temporal arteritis/giant cell arteritis, ulcerative colitis, uveitis, vitiligo, Wegener's granulomatosis, and any combination thereof. In yet another embodiment, the condition is a cancer, such as a cancer selected from the group consisting of breast cancer, prostate cancer, ovarian cancer, cervical cancer, skin cancer, pancreatic cancer, colorectal cancer, renal cancer, liver cancer, brain cancer, lymphoma, leukemia, lung cancer, and any combination thereof.

### BRIEF DESCRIPTION OF THE DRAWINGS

The invention is defined in the appended claims. The references to methods of treatment by therapy in this description and examples are to be interpreted as references to modified T cells, pharmaceutical compositions and medicaments of the invention for use in those methods. The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.
Figure 1 is panel of graphs showing transgene expression in T cells co-electroporated with TCR and BiTEs. T cells were co-electroporated with CD19.CD3 (upper panel) or 4D5.CD3 (ErbB2) (middle panel) BiTEs with or without CD3zeta and epsilon. Eighteen hours post electroporation, T cells were stained for TCR vb13.1 and mIgG Fab (or Her2-Fc). Lower panel shows TCR (vb13.1) expression 3 days after electroporation.
Figure 2 is a panel of graphs showing CD107a up-regulated in T cells stimulated with tumors. T cells were co-electroporated with RNA as shown and stimulated with tumor cell lines having single or double positivity for CD19 and NY-ESO-1 (ESO). CD107a up-regulation was assessed after 4 hours.
Figure 3 is a panel of graphs showing CD107a up-regulated in T cells stimulated with tumors. T cells were co-electroporated with RNA as shown and stimulated with tumor cell lines having single or double positivity for CD19 and NY-ESO-1 (ESO). CD107a up-regulation was assessed after 4 hours.
Figure 4 is a graph showing IFN-gamma production in RNA electroporated T cells stimulated with tumor cell lines.
Figure 5 is a graph showing IL-2 production in RNA electroporated T cells stimulated with tumor cell lines.
Figure 6 is a panel of graphs showing CD107a up-regulation in T cells expressing NY-ESO-1 TCR (1G4) and mesothelin BiTEs (ss1.CD3) and stimulated by tumor cells.
Figure 7 is a panel of images showing tumor growth in mice intravenously injected with Naml6-ESO-CBG cells (2x10⁶ cells). Five days after tumor cell injection, mice were treated with RNA electroporated T cells as indicated (5 mice/group). Tumor growth was imaged with bioluminescent imaging.
Figure 8 is a graph showing T cells expressing bispecific RNA to target tumor-associated antigens with enhanced T cell function.
Figure 9 is a panel of graphs showing TCR on T cells recognized both cognate and MHC/peptide and surface tumor antigens without HLA restriction by combining bi-specific antibodies in the T cells.
Figure 10, comprising Figure 10A and 10B, shows a list of the constructs of bi-specific antibody RNAs and TCR RNAs electroporated into T cells.
Figure 11 is a panel of graphs showing T cells recognized both cognate antigen (HLA-A2/NY-ESO-1) and CD19 or Her2 by a modified NY-ESO-1 TCR and bi-specific antibodies.
Figure 12 is a diagram illustrating engagement of an enhanced chimeric ligand engineered activation receptor (CLEAR) between a T cell and target tumor cell and a bi-specific antibody on another T cell engaging the same target tumor cell.
Figure 13, comprising Figure 13A and 13B, is a list of the constructs of CLEARs and bi-specific antibodies generated in the invention.
Figure 14 is a panel of graphs showing CD27 express in T cells or K562 cells eighteen hours after electroporation with CD27-BBZ or CD27-Z CLEARs RNA (upper panel) and CD70 expression in cells lines (lower panel).
Figure 15 is a panel of graphs showing CD107a up-regulation in CD27 CLEARs RNA electroporated T cells stimulated with CD70 positive tumor cell lines.
Figure 16 is a panel of graphs showing PD1 expression in PD1 CLEARs RNA electroporated T cells (upper panel) and CD107a up-regulation in the T cells after stimulation with PD-L1 positive tumor cells, Nalm6-PD-L1.
Figure 17 is a graph showing cytokine production in PD1 CLEARs RNA electroporated T cells stimulated with PD-L1 positive tumor cells, Nalm6-PD-L1.
Figure 18 is a panel of graphs showing transgene expression in T cells co-electroporated with PD1-Z and aPD-ameso bi-specific antibody RNA.
Figure 19 is a panel of graphs showing CD107a up-regulation in PD1-Z and aPD-ameso bi-specific antibody RNA co-electroporated T cells stimulated with MESO positive tumor cells (K562-meso, SK-OV3 and PC3), or MESO/PD-L1 double positive tumor cells (PC3-PDL1).
Figure 20 is a panel of graphs showing cytokine production of PD1-Z and aPD-ameso bi-specific antibody RNA co-electroporated T cells stimulated with MESO positive tumor cells (K562-meso and SK-OV3).
Figure 21 is a panel of graphs showing transgene expression in T cells co-electroporated with CD27-Z and aCD27-aErbB2 bi-specific antibody RNAs (upper panel) or CD19 bi-specific antibody RNA (lower panel).
Figure 22 is a panel of graphs showing CD107a up-regulation in CD27-Z and aCD27-aErbB2 bi-specific antibody RNAs electroporated T cells stimulated with tumor cell lines expressing CD70 and/or ErbB2.
Figure 23 is a panel of graphs showing CD107a up-regulation in CD27-Z and aCD27-aCD19 bi-specific antibody RNAs electroporated T cells stimulated with tumor cell lines expressing CD70 and/or CD19.
Figure 24 is a panel of graphs showing T cells electroporated with RNA for PD1-Z and anti-PD1/anti-ErbB2 bis-specific antibodies, as indicated. Eighteen hours later, the electroporated T cells were stained for anti-PD1 and anti-mIgG Fab.
Figure 25 is a panel of graphs showing tumor cell lines (upper panel) and ErbB2 or Pd-L1 RNA electroporated K562 cells (lower panel, 18 hrs post electroporation). Cells were stained for PD-L1 and ErbB2 expression.
Figure 26 is a panel of graphs showing electroporated T cells were stimulated with tumor lines for 4 hrs. CD107a up-regulation was detected by flow cytometry.
Figure 27 is a panel of graphs showing electroporated T cells were stimulated with tumor lines for 4 hrs. CD107a up-regulation was detected by flow cytometry.
Figure 28 is an image showing illustrations of the affinity antibody mimetic constructs.

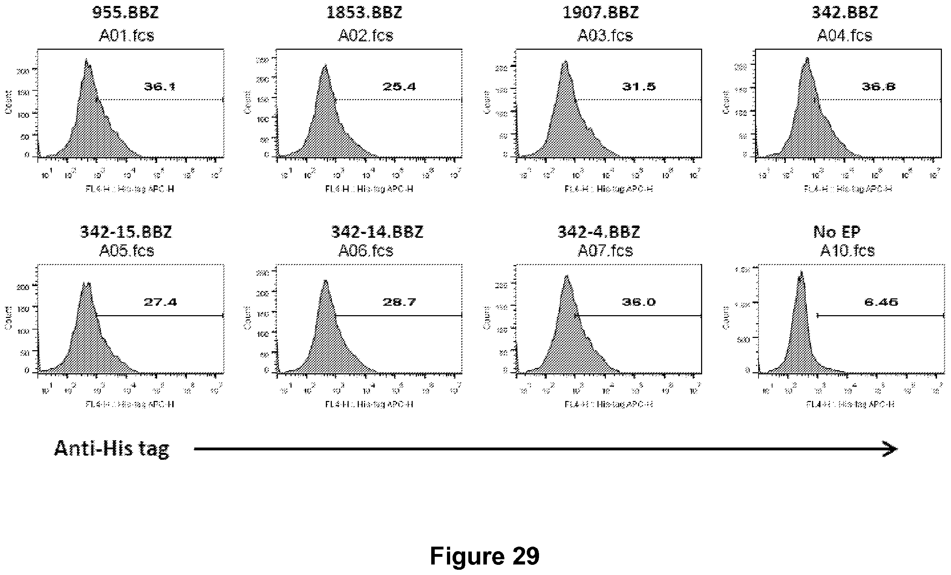

Figure 29 is a panel of graphs showing expression of affinity antibody mimetic redirected T cells (ARTs) by staining with anti-His antibody. Ten micrograms of RNA encoding ARTs against either EGFR (955.BBZ, 1853.BBZ or 1970.BBZ) or ErbB2 (342.BBZ, 432-15.BBZ, 342-14.BBZ or 342-4.BBZ) was electroporated T cells and cultured in R10 overnight. One hundred microliters of electroporated T cells were stained by an anti-His tag antibody for flow cytometry detection of ART expression (lower panel) and showed that T cells electroporated with all the ART RNA were stained positive, comparing with the T cells that were no electroporated (No EP).

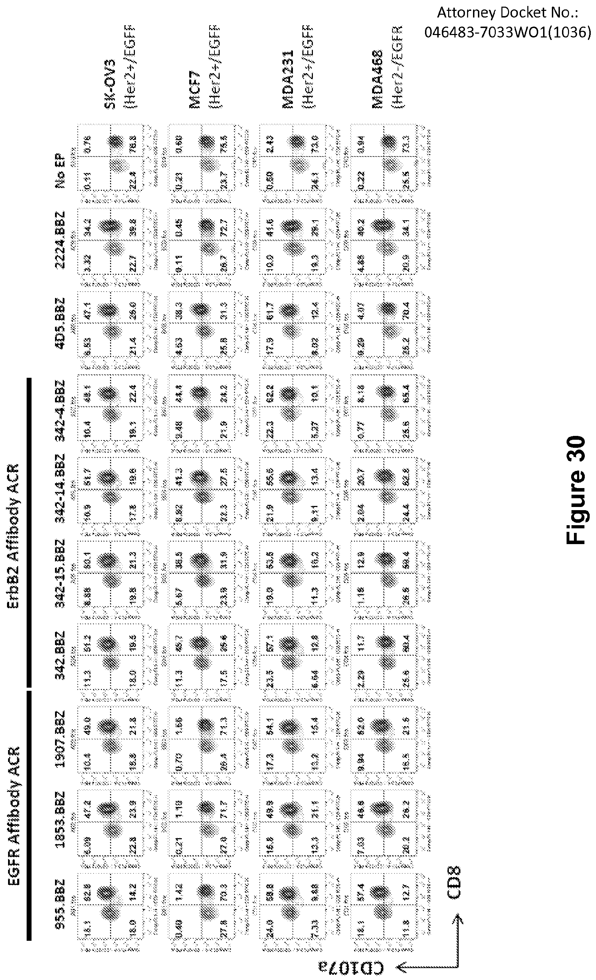

Figure 30 is a panel of graphs showing specific CD107a up-regulation of EGFR and ErbB2 ARTs. Electroporated T cells as shown in Figure 29 were stimulated with 4 different tumor cell lines that express EGFR and/or ErbB2 as indicated below the name of each tumor. After 4h incubation, CD107a upregulation was measured by staining the cell with CD107a-PE, CD3-APC and CD8-FITC. As shown in the Figure 29, all the EGFR ARTs only strongly reactive to the tumor lines that are EGFR positive (SK-OV3, MDA231 and MDA468), but not EGFR negative tumor MCF7. While all the ErbB2 ARTs strongly reactive to the tumor lines that are ErbB2 positive (SK-OV3, MDA231 and MCF7), but not ErbB2negative tumor MDA468. 4D5.BBZ and 2224.BBZ were CARs against ErbB2 and EGFR respectively.

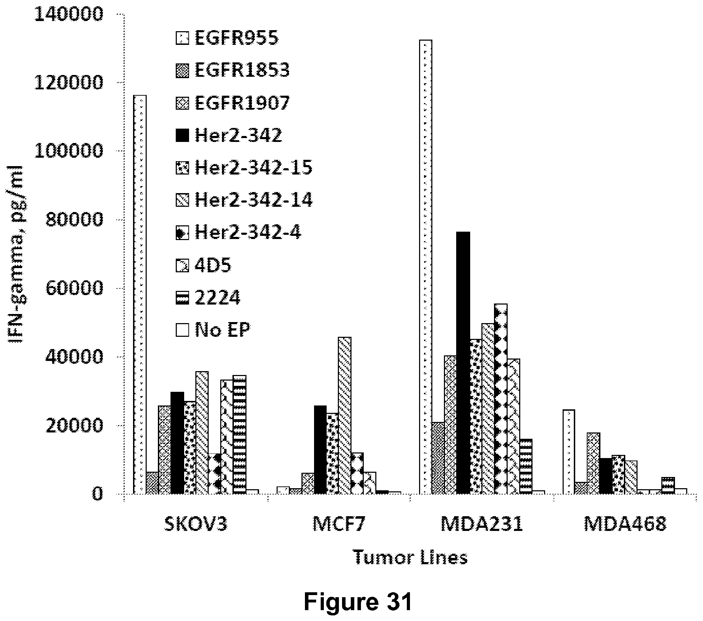

Figure 31 is a graph showing specific IFN-gamma production of EGFR and ErbB2 ARTs. Electroporated T cells as shown in Figure 29 were stimulated with 4 different tumor cell lines that express EGFR and/or ErbB2 as indicated below the name of each tumor. After overnight incubation, supernatant was used to measure INF-gamma production by ELISA. As shown in the figure, IFN-gamma could be detected at different levels for all the EGFR ARTs stimulated by the tumor lines that are EGFR positive (SK-OV3, MDA231 and MDA468), but not EGFR negative tumor MCF7. While all the ErbB2 ARTs strongly reactive to the tumor lines that are ErbB2 positive (SK-OV3, MDA231 and MCF7), but not ErbB2negative tumor MDA468. 4D5.BBZ and 2224.BBZ were CARs against ErbB2 and EGFR respectively.

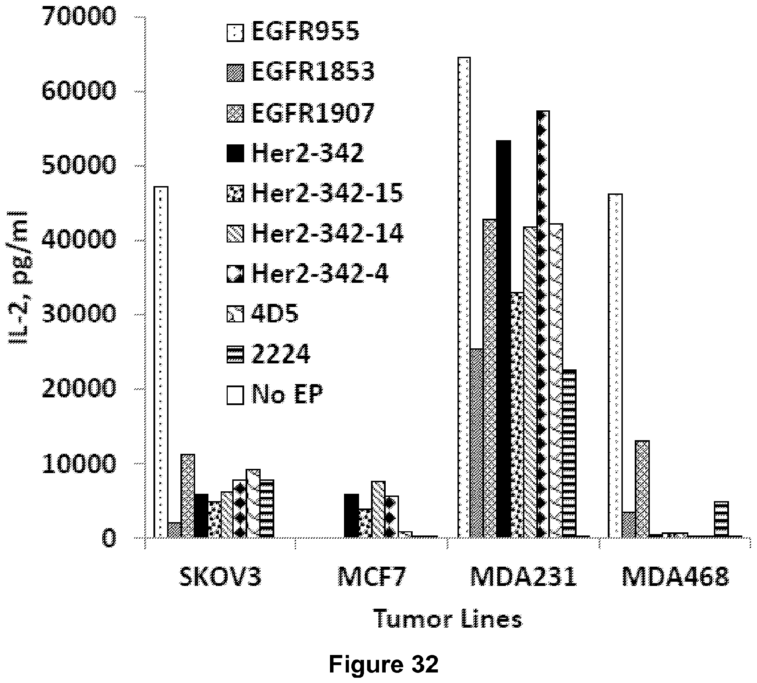

Figure 32 is a graph showing specific IL-2 production of EGFR and ErbB2 ARTs. Electroporated T cells as shown in Figure 29 were stimulated with four different tumor cell lines that express EGFR and/or ErbB2 as indicated below the name of each tumor. After overnight incubation, supernatant was used to measure IL-2 production by ELISA. As shown in the figure, IL-2 could be detected at different levels for all the EGFR ARTs stimulated by the tumor lines that are EGFR positive (SK-OV3, MDA231 and MDA468), but not EGFR negative tumor MCF7. While all the ErbB2 ARTs strongly reactive to the tumor lines that are ErbB2 positive (SK-OV3, MDA231 and MCF7), but not ErbB2negative tumor MDA468. 4D5.BBZ and 2224.BBZ were CARs against ErbB2 and EGFR respectively.
Figure 33 is a graph showing specific lytic activities of EGFR and ErbB2 ARTs. In a separate experiment to test the killing ability of two EGFR and two ErbB2 ARTs, comparing to their relevant CARs, against tumor cell line SK-OV3 that is positive for both EGFR and ErbB2. As shown in the figure, ART T cells killed the tumor cells as efficiently as relevant CAR T cells.

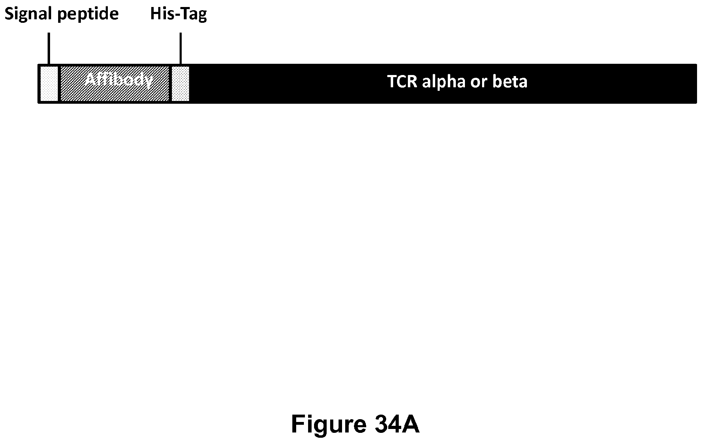

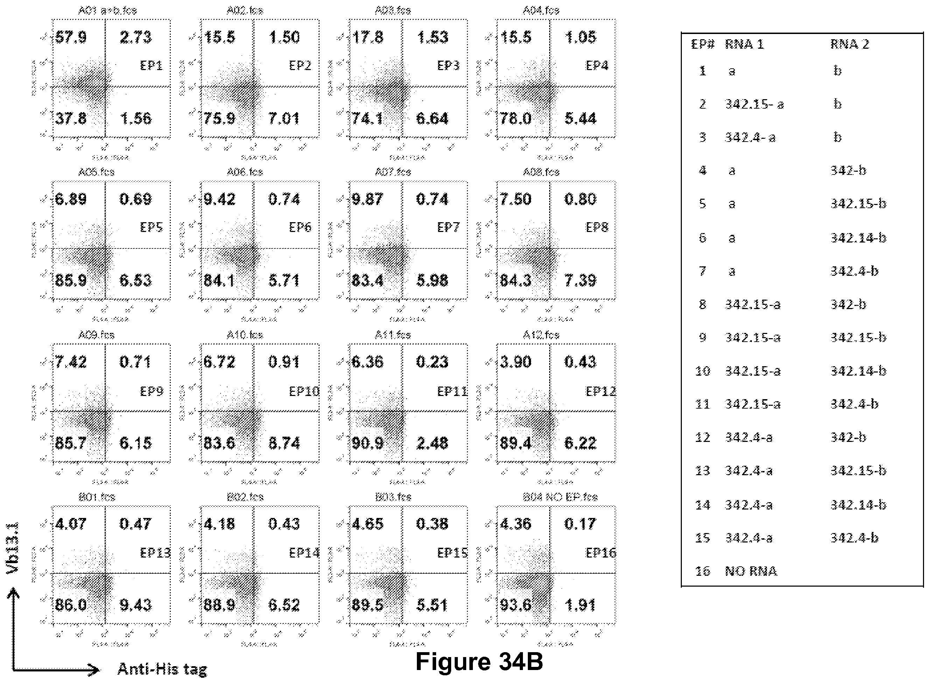

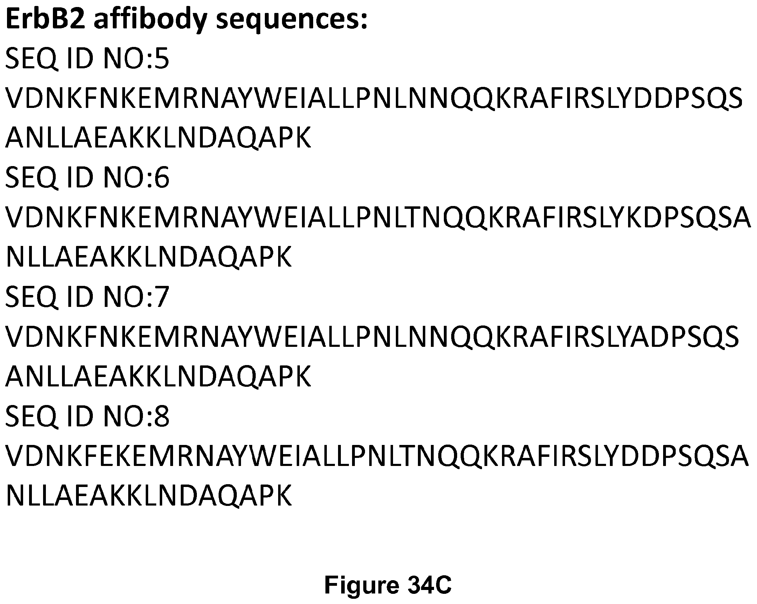

Figure 34, comprising Figures 34A-34C, is a panel of images showing affinity antibody mimetic modified TCR. Figure 34A is a diagrammatic sketch of affinity antibody mimetic modified TCR (Affi-TCR) by adding affinity antibody mimetic and His -tag sequence to the N' of either alpha or beta chain of a TCR. Figure 34B is a panel of graphs showing Vb13.1 TCR and His-tag detection of affinity antibody mimetic redirected TCR ( Affi-TCR) RNA electroporated T cells. T cells were co-electroporated with NY-ESO-1 (1G4) TCR alpha (a) and beta (b), or their ErbB2 affinity antibody mimetic (342, 342.15, 342 or 342.4) modification . After overnight, vb13.1 and His-Tag were detected by flow cytometry. Figure 34C shows the ErbB2 affinity antibody mimetic sequences.
Figure 35 is a panel of graphs showing CD107a up-regulation of Affi-TCR RNA electroporated T cells. T cells were co-electroporated with TCR alpha (a) and beta (b), or their ErbB2 affinity antibody mimetic (342, 342.15, 342 or 342.4) modification as indicated in Figure 34B and stimulated with tumor line Nalm-6-ESO (NY-eso-1+, ErbB2-), A549-ESO (NY-eso-1+, ErbB2+), SK-OV3 (NY-eso-1-, ErbB2+), A549 (NY-eso-1-, ErbB2+), or Nalm6 (NY-eso-1-, ErbB2-), for CD107a assay. The results show that T cells with ErbB2 Affi-TCR could specifically recognize both Ny-ESO-1 and ErbB2 positive tumors.

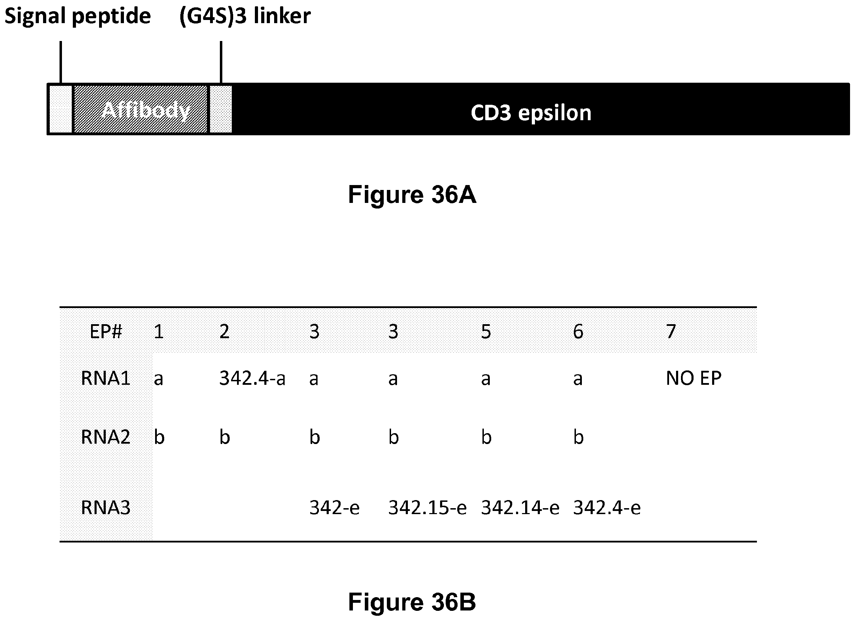

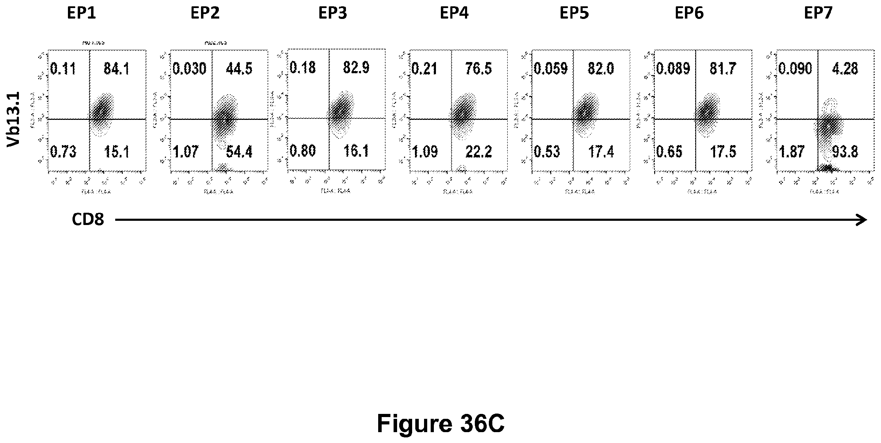

Figure 36, comprising Figures 36A-36C, is a panel of images showing affinity antibody mimetic modified CD3 epsilon. Figure 36A is a diagrammatic sketch of affinity antibody mimetic modified CD3 epsilon by adding affinity antibody mimetic and G4S linker to the N' of either CD3 epsilon. Figure 36B is a table showing electroporation of T cells. T cells were co-electroporated with NY-ESO-1 (1G4) TCR alpha (a) and beta (b), or together with ErbB2 affinity antibody mimetic (342, 342.15, 342 or 342.4) modified CD3 epsilon (e). Figure 36C is a panel of graphs showing the maintainenance of TCR expression and dual tageting by co-delivering affinity antibody mimetic modified CD3 epsilon. After overnight of the T cells of Figure 36B, vb13.1 expression was detected by flow cytometry.
Figure 37 is a panel of graphs showing CD107a up-regulation of Affi-TCR RNA electroporated T cells. T cells were co-electroporated with NY-ESO-1 (1G4) TCR alpha (a) and beta (b), or together with ErbB2 affinity antibody mimetic (342, 342.15, 342 or 342.4) modified CD3 epsilon (e) as indicated in Figure 36B and stimulated with tumor line Nalm-6-ESO (NY-eso-1+, ErbB2-), Nalm6 (NY-eso-1-, ErbB2-), SK-OV3 (NY-eso-1-, ErbB2+) or MDA231 (NY-eso-1-, ErbB2+), for CD107a assay.

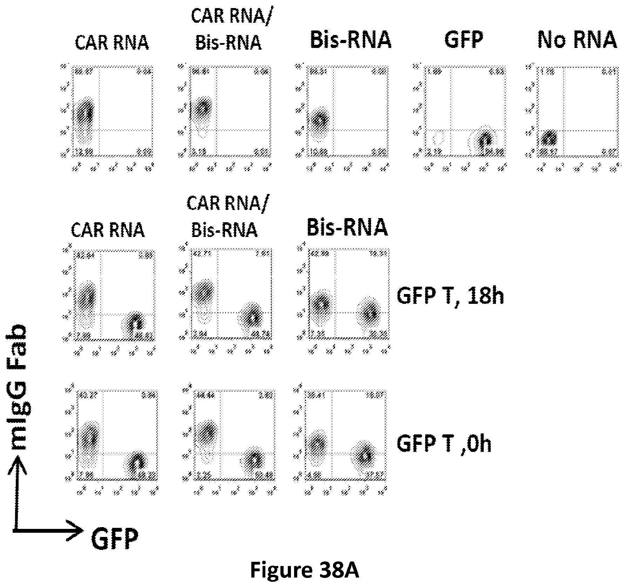

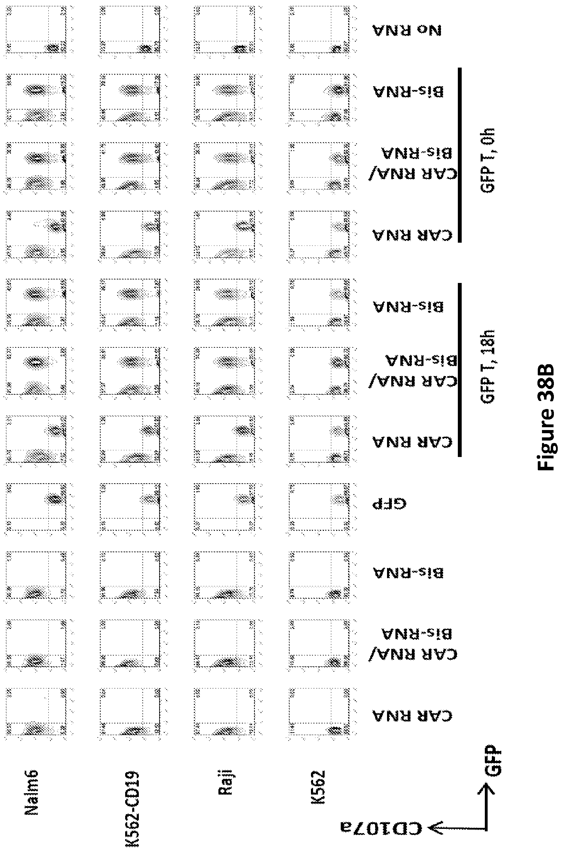

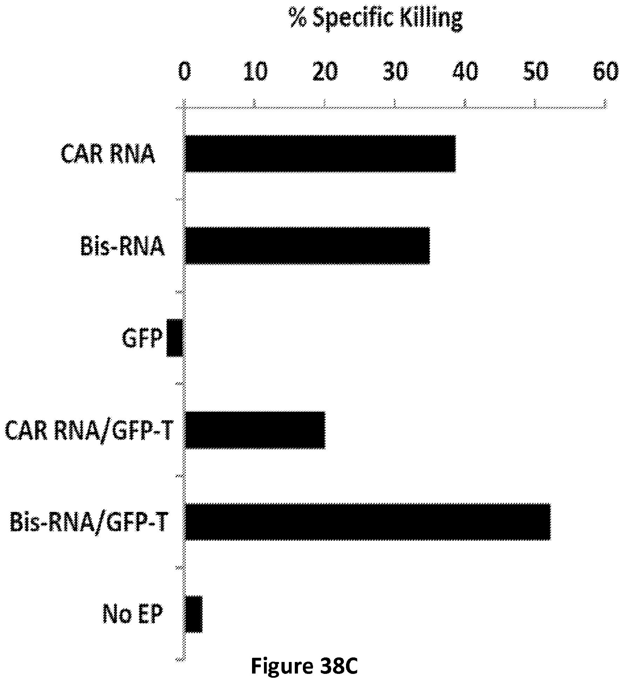

Figure 38, comprising Figures 38A-38C, demonstrates that bi-specifc antibodies can be secreted by RNA encoding a bispecific antibody (Bis-RNA) electroporated into T cells. T cells were electroporated with RNA encoding CD19 CAR (CAR RNA), Blinatumomab Bis-RNA (Bis-RNA), GFP or co-electroporated with both CD19 CAR and Blinatumomab Bis-RNA (CAR RNA/Bis-RNA). Eighteen hours after electroporation, the T cells were stained with a goat anti-mouse IgG Fab (mIgG Fab) to detect either the CD19 CAR expressed on the T cells, or bi-specific antibody engaged on the T cell surface via binding to CD3 (Figure 38A, upper panel shows both mIgG Fab staining and GFP expression of the electroporated T cells). Immediately after electroporation, aliquots of the T cells with either CAR RNA or Bis-RNA were mixed with an equal number of GFP RNA T cells and were co-cultured for 18 hours. mIgG Fab and GFP (Figure 38A, middle panel) were then assessed. Eighteen hours after electroporation, aliquots of the T cells with either CAR RNA or Bis-RNA were mixed with an equal number of GFP RNA T cells and subjected to staining for detection of mIgG Fab and GFP (Figure 38A, lower panel). Eighteen hours after electroporation, the T cells electroporated with different RNAs, or the T cells electroporated with CAR RNA or Bis-RNA were mixed with GFP RNA electroporated T cells for 18 h (GFP T, 18h) or 0 h (GFP T, 0h) and then stimulated with cell lines that express CD19 (Nalm6, K562-CD19 and Raji) or K562 cells that are CD19 negative. The mixtures were then assessed for CD107a staining (Figure 38B). Eighteen hours after electroporation, T cells electroporated with either CD19 CAR RNA (CAR RNA) or Bis-RNA alone or mixed with an equal number of GFP RNA electroporated T cells (GFP-T) were analyzed for lytic activity after a four hour flow based cytotoxic T lymphocyte assay at an effector:target ratio of 5:1 (Figure 38C).

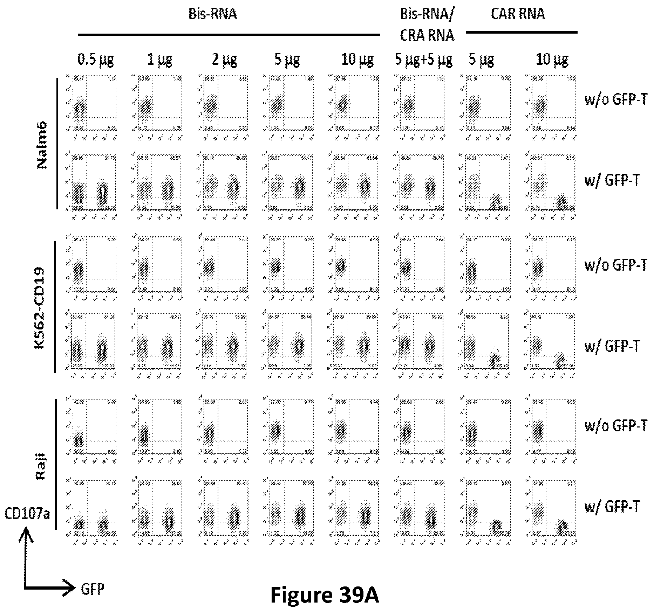

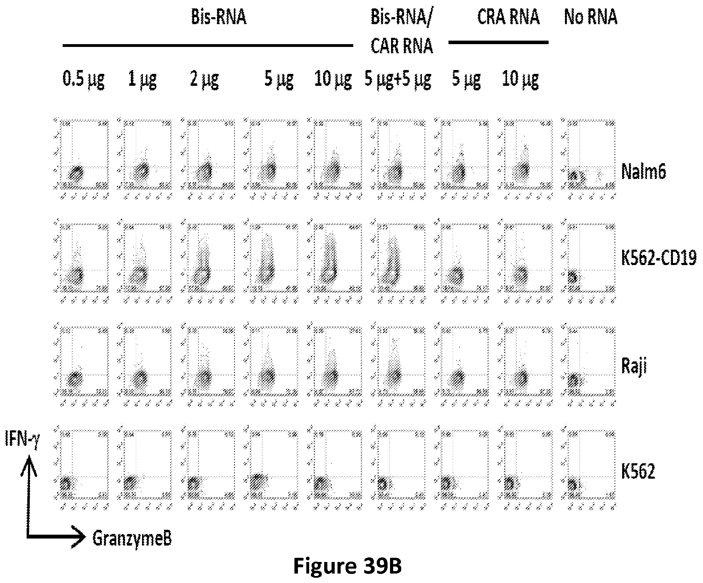

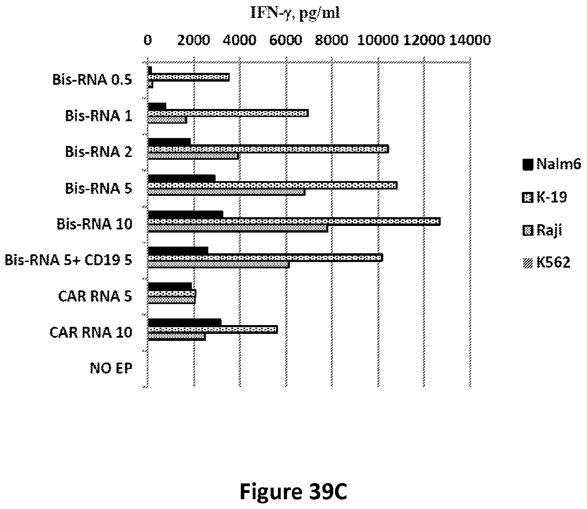

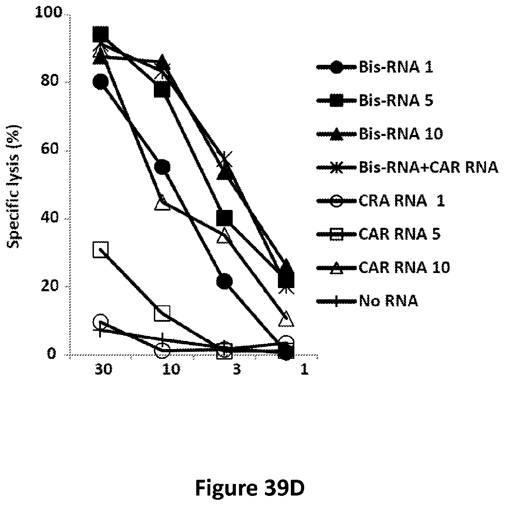

Figure 39, comprising Figures 39A-39D, highlights the increase in T cell activation and tumor killing ability of Bis-RNA electroporated T cells. T cells were electroporated with a different amount (µg RNA /0.1 ml T cells) of either Blinatumomab Bis-RNA (Bis-RNA) or CD19 CAR RNA (CAR RNA) as indicated. Eighteen hours after electroporation, the T cells with either Bis-RNA or CAR RNA were stimulated with CD19 positive cell lines with or without adding an equal number of GFP RNA electroporated T cells (GFP-T) and assessed for CD107a expression (Figure 39A). Eighteen hours after electroporation of the T cells described above, the electroporated T cells were subjected to intracellular staining of IFN-gamma and Granzyme B (Figure 39B). ELISA for IFN-gamma was performed after an overnight stimulation of the T cells with CD19 positive cells (Nalm6, K562-CD19 and Raji) or CD19 negative (K562) cell lines (Figure 39C). T cells were electroporated with either Blinatumomab Bis-RNA (Bis-RNA) or CD19bbz CAR RNA (CAR RNA at RNA doses of 1, 5 or 10 µg/0.1 ml of T cells, or co-electroporated with 5 µg of each Bis-RNA and CAR RNA (Bis-RNA+CAR RNA). Eighteen hours after electroporation, lytic activity was assessed with a four hour flow based cytotoxic T lymphocyte assay at the indicated effector:target ratios (Figure 39D).

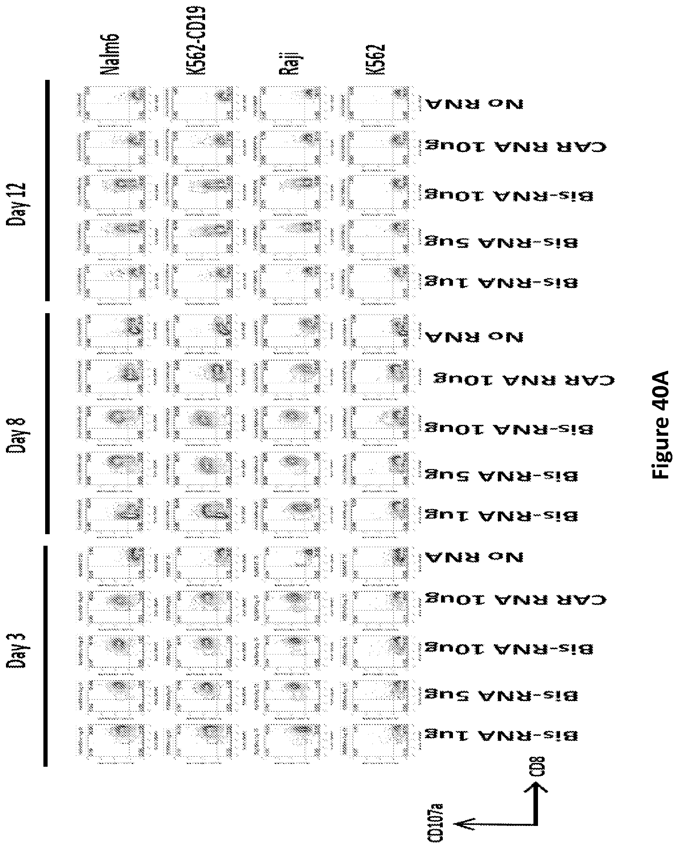

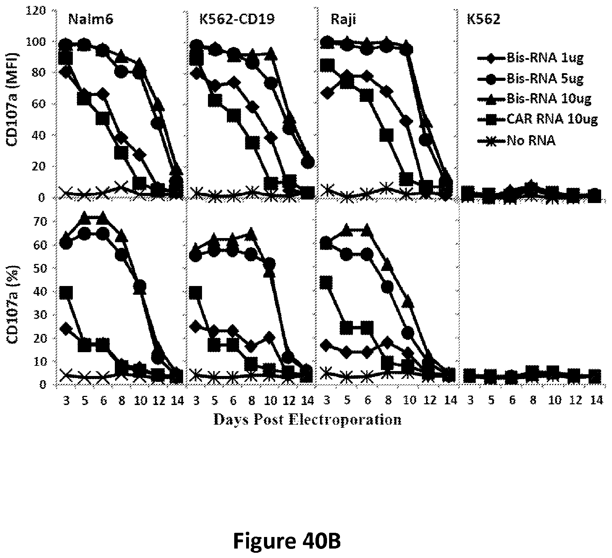

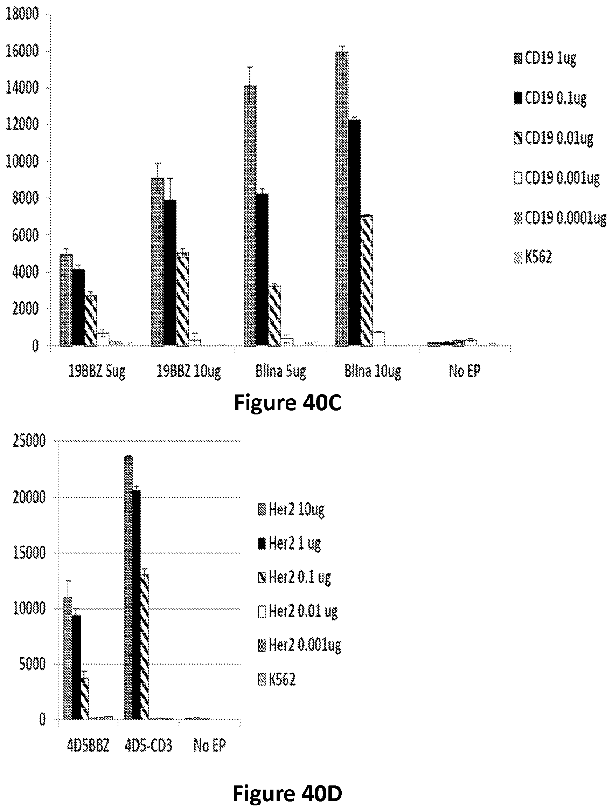

Figure 40, comprising Figures 40A-40D, demonstrates the prolonged tumor reactivity of Bis-RNA electroporated T cells and the sensitivity of CAR RNA T cells and Bis-RNA T cells. T cells were electroporated with different amounts of Blimatumomab Bis-RNA at 1, 5 and 10 µg/0.1 ml of T cells and compared with T cells electroporated with CD19BBZ CAR RNA at a dose of 10 µg of RNA. CD107a staining was conducted on different days after electroporartion and the results were plotted either as the dot plot for CD107a/CD8 expression at day 3, day 8 and day 12 (Figure 40A), the value of mean fluorescence intensity (MFI) (Figure 40B, upper panel), or the percentage of CD107a expressing cells (Figure 40B, lower panel) days post electroporation. T cells electroporated with either 5 or 10 µg of CD19BBZ (19BBZ) or Blinatumomab (Blina) RNA were stimulated with K562 cells and electroporated with decreasing amounts of CD19 mRNA. The cells were co-cultured for 18 hours and supernatant was subjected to IFN-gamma ELISA (Figure 40C). T cells electroporated with either 10 µg 4D5BBZ or 4D5-CD3 RNA were stimulated with K562 cells and electroporated with decreasing amounts of ErbB2 (Her2) mRNA. The cells were co-cultured for 18 hours and supernatant was subjected to IFN-gamma ELISA (Figure 40D).

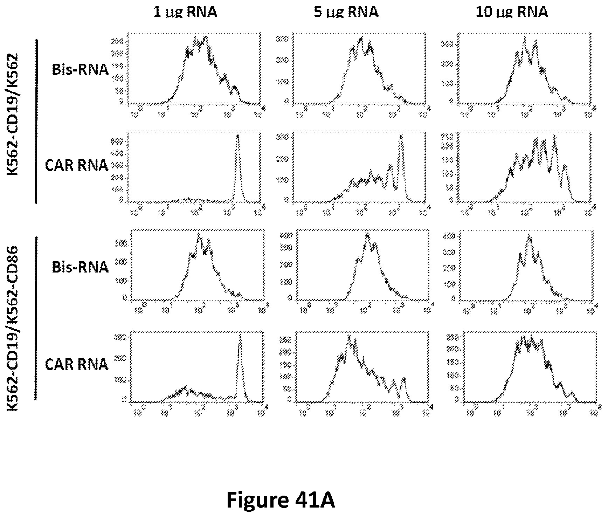

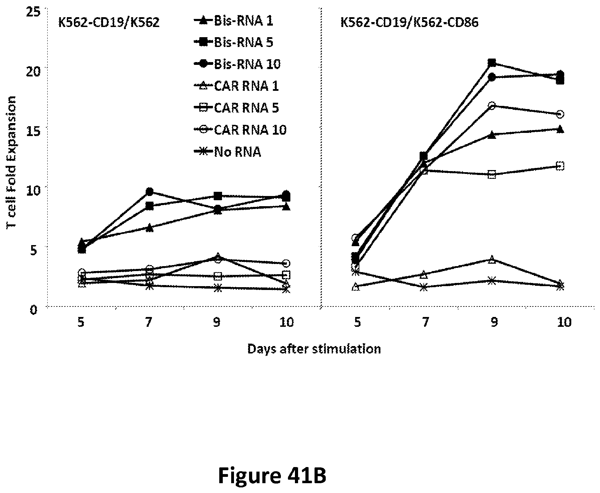

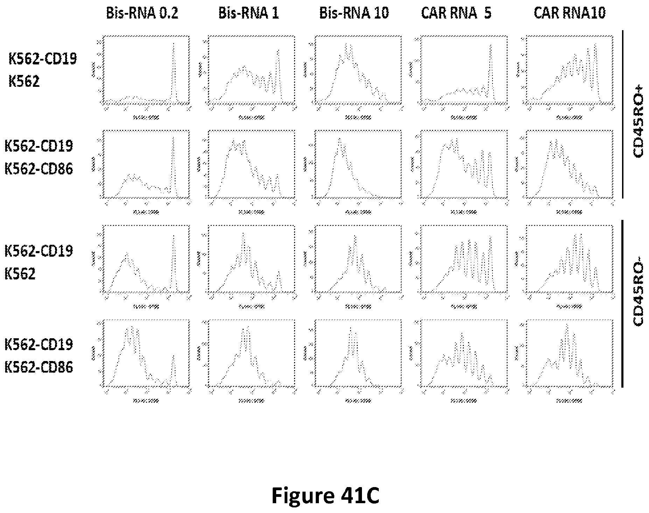

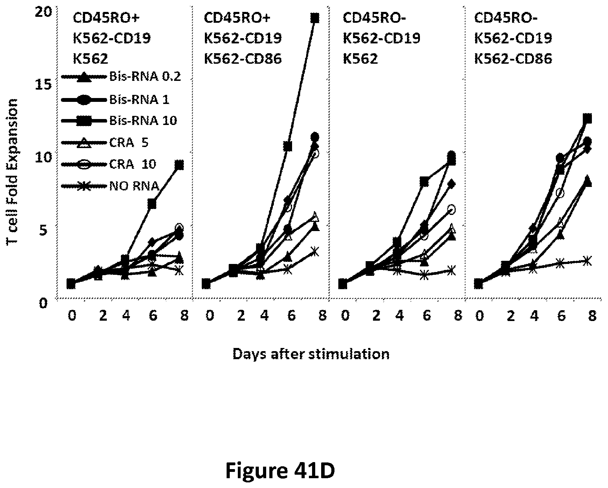

Figure 41, comprising Figures 41A-41D, highlights the Bis-RNA electroporated T cells decreased dependence on co-stimulation and enhanced division and proliferation. CFSE labeled resting CD4+ T cells were electroporated with Blinatumomab Bis-RNA or CD19BBZ RNA (CAR RNA) at different RNA doses and stimulated with either irradiated K562-CD19 cells (mixed with an equal number of irradiated K562 cells as a control for K562-CD86 cells), or irradiated K562-CD19 cells (mixed with an equal number of irradiated K562-CD86 cells). CFSE staining at day 6 (Figure 41A) and the number of T cells at different days after stimulation (Figure 41B) were monitored. CFSE labeled CD45RO+ (memory cells) or CD45RO- (Naive cells) resting CD4 T cells were electroporated with Blinatumomab Bis-RNA or CD19BBZ RNA (CAR RNA) at different RNA doses and stimulated with either irradiated K562- CD19 cels (mixed with an equal number of irradiated K562 cells as a control for K562-CD86 cells), or irradiated K562-CD19 cells (mixed with an equal number of irradiated K562-CD86 cells). CFSE staining at day 6 (Figure 41C) and the number of T cells at different days after stimulation (Figure 41D) were monitored

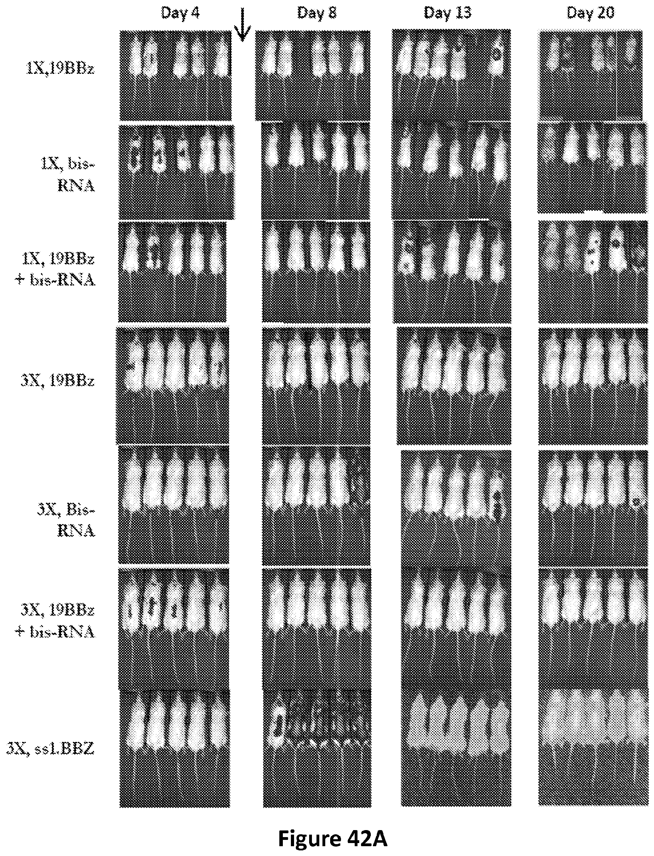

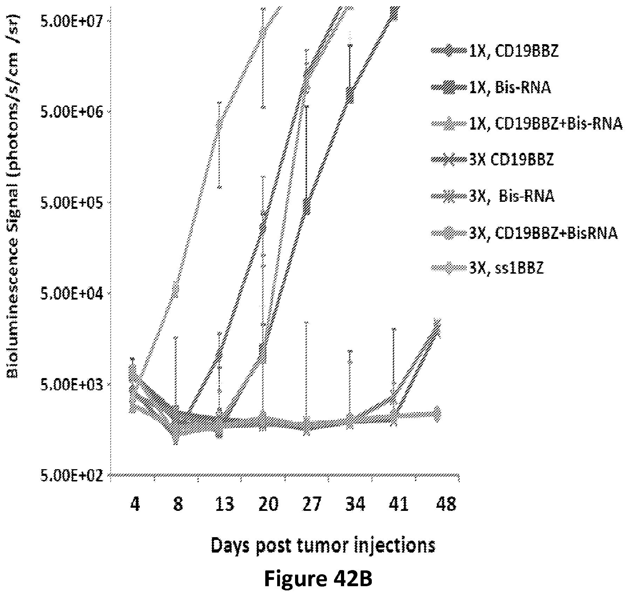

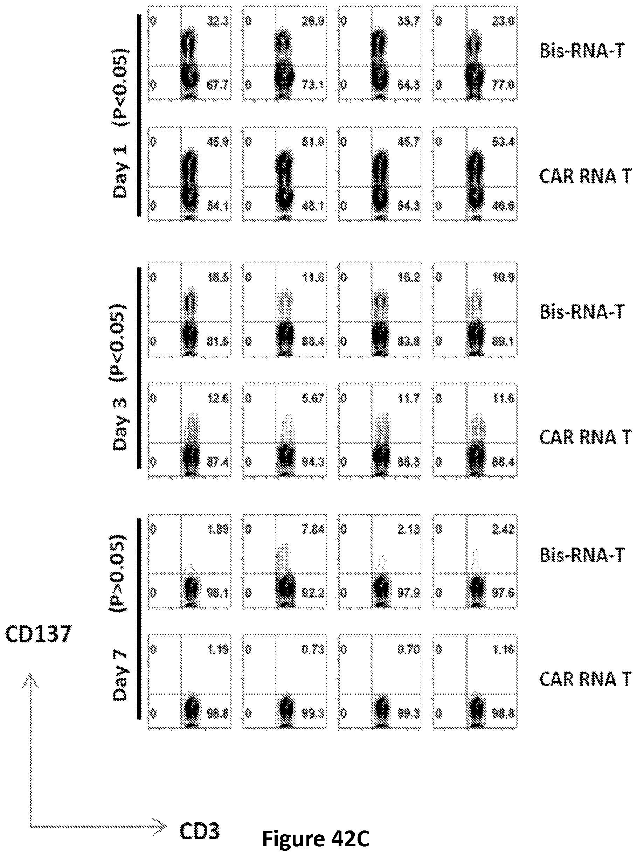

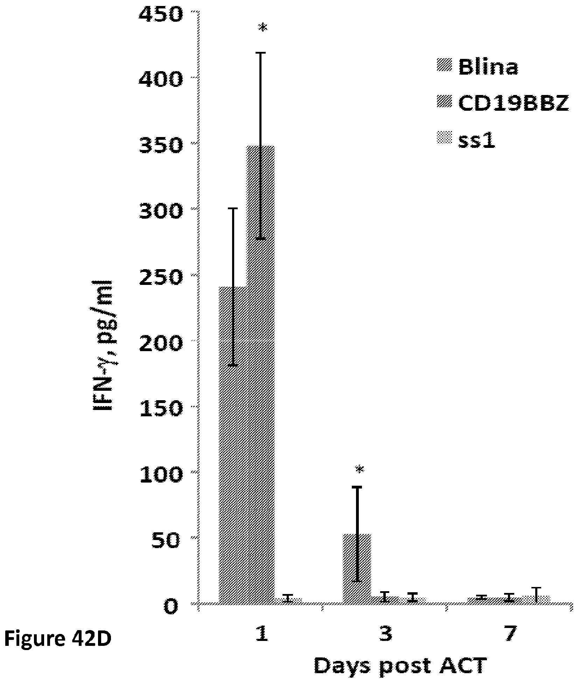

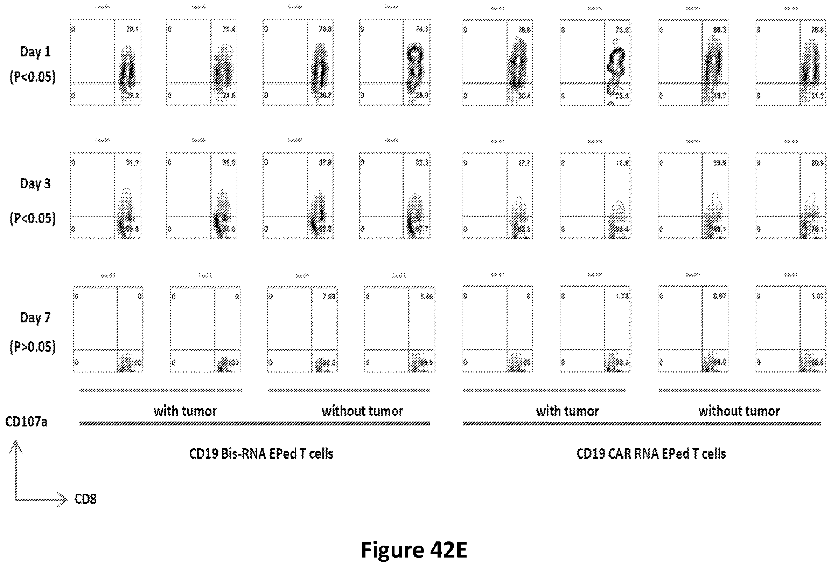

Figure 42, comprising Figures 42A-42E, displays the enhanced anti-tumor activity of Bis-RNA T cells in a Nalm-6 xenograft model. Leukemia was established after intravenous injection of Nalm6 cells (1x10⁶, i.v.) in NOD/scid/yc(-/-) (NSG) mice (n = 5). Seven days after tumor cell injuction, mice were randomized and treated with either Blinatumomab Bis-RNA or CD19BBZ RNA electroporated T cells. The mice treated with anti-mesothelin RNA CAR T cells (ss1BBZ) were served as a control. T cells were injected intravenously either as a single injection with 25e⁶ (1X) or injected once a week for three weeks (3X) with T cells at a dosage of 20e⁶ for the 1^{st} injection and 5e⁶ for the 2^{nd} and 3^{rd} injections. Animals were imaged at the indicated time points post injection (Figure 42A). BLI data for the experiment are plotted with total photon flux - SE indicated on the y-axis; 1e⁵ p/sec/ cm2/sr represents mice with no luciferase-containing cells (Figure 42B). NSG mice with established leukemia as described above, or NSG mice without leukemia were injected with 20e⁶ T cells electroporated with either CD19BBZ or Bis-RNA, or control ss1BBZ RNA. Bone marrow cells and splenocytes were harvested from two mice of each group at day 1, 3 and 7 after T cell injections. T cells were purified from each mouse by depleting mouse cells from pooled bone marrow cells and splenocytes. T cell function was evaluated through CD137 expression (* indicates p<0.05) (Figure 42C), cytokine production (via ELISA) (Figure 42D), and CD107a expression (Figure 42E) after being stimulated with a CD19 positive cell line, K562-CD19, for 18 hours (CD137 and IFN-gamma secretion) and 4 hours (CD107a), respectively.

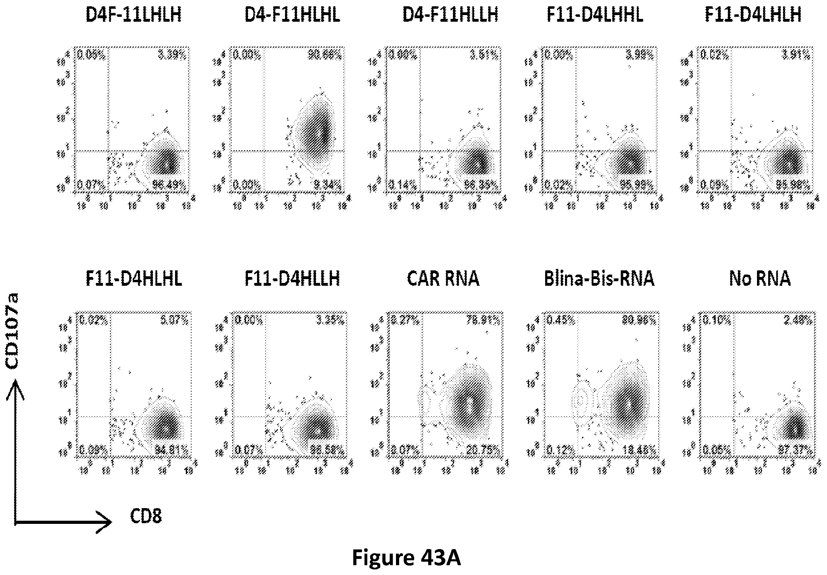

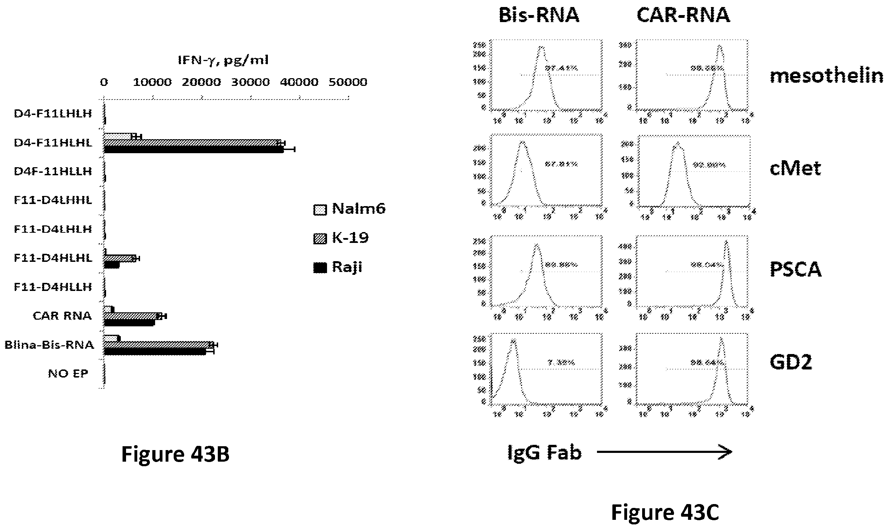

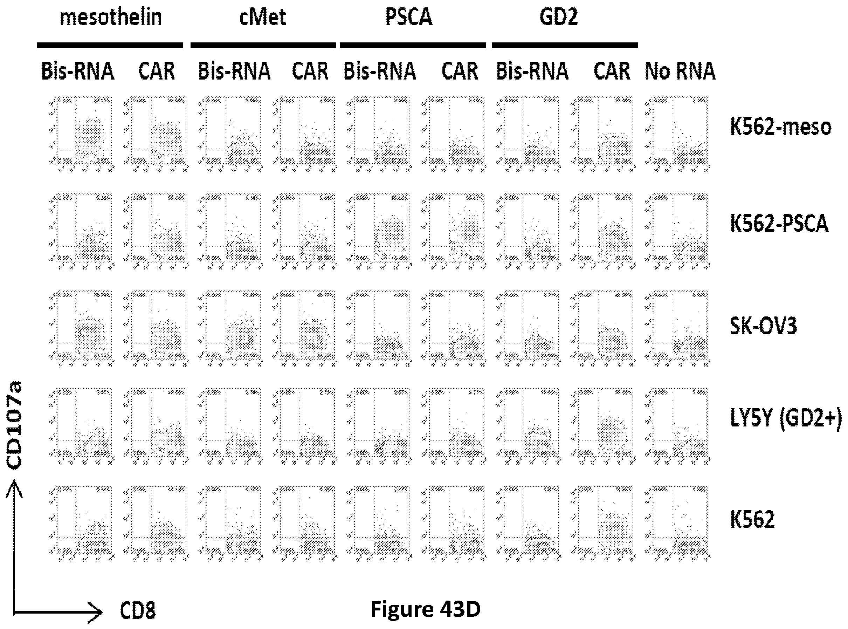

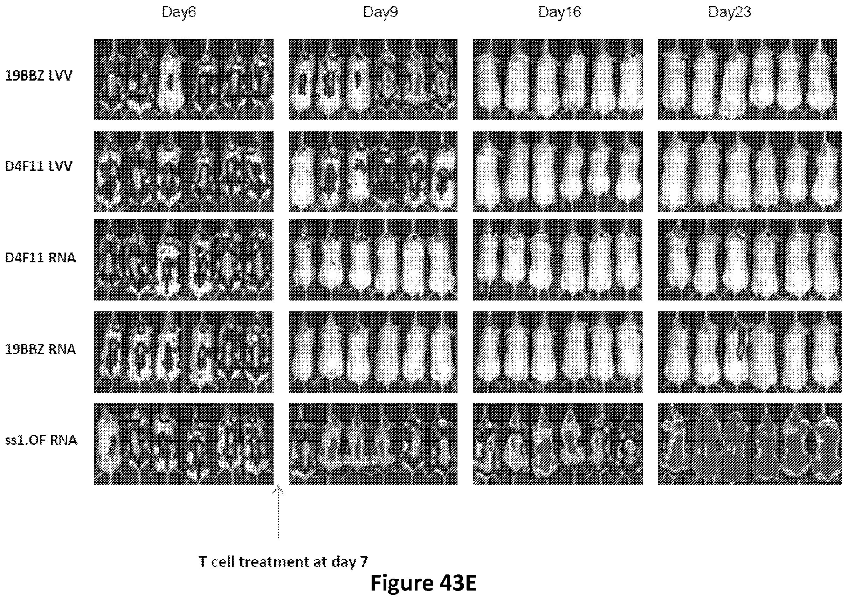

Figure 43, comprising Figures 43A-43E, illustrates different Bis-RNAs against different tumor antigens. T cells were electroporated with one of seven different constructs for Bis-RNA encoding a fully human CD19-CD3 using single chain variable fragments (scFvs) from fully human anti-CD19 (21D4) antibody and a fully human anti-CD3 scFv (28F11) antibody, respectively. CD107a up-regulation was monitored by flow cytometery after the electroporated T cells were stimulated by the CD19 positive cell line, K562-CD19, for 4 hours (Figure 43A). IFN-gamma production was detected by ELISA after the electroporated T cells were stimulated by the CD19 positive cell lines, Nalm6, K562-CD19 and Raji, for 18 hours. CD19BBZ CAR RNA (CAR RNA), Blinatumomab Bis-RNA (Blina-Bis-RNA), and no electroporation (No EP) were used as controls (Figure 43B). Figure 43C shows the detection of scFv on T cells electroporated with Bis-RNA or CAR RNA against mesothelin, cMet, PSCA and GD2 using anti-mouse IgG Fab antibody (for scFv against mesothelin and GD2) or anti-human IgG Fab (for cMet and PSCA). T cells electroporated with Bis-RNA or CAR RNA against mesothelin, cMet, PSCA and GD2 Bis-RNA or CAR RNA (CAR) were stimulated with cell lines expressing mesothelin (K562-meso, SK-OV3)), PSCA (K562-PSCA), cMet (SK-OV3) or GD2 (LY5Y) for 4 hours and CD107a up-regulation was monitored by flow cytomtry (Figure 43D). NSG mice (n=6) were injected with 1e⁶ Nalm6-CBG (i.v.) and 7 days later were injected with 5e⁶ T cells lentivirally transduced with CD19BBZ or D4F11, or 20e⁶ T cells electroporated with CD19BBZ or D4F11 RNA. The mice treated with T cells electroporated with ss1BBZ CAR RNA served as controls. At day 16 and 20 post tumor injection, the mice injected with RNA electroporated T cells were adminstered Cytoxan interperitoneally (40mg/kg, I.P.). The following day, the mice were injected with 5e⁶ T cells electroporated with RNAs, as described. One day prior to T cell injection and 2 days after each subsequent injection, bioluminescence was measured as described for Figure 42 (Figure 43E).

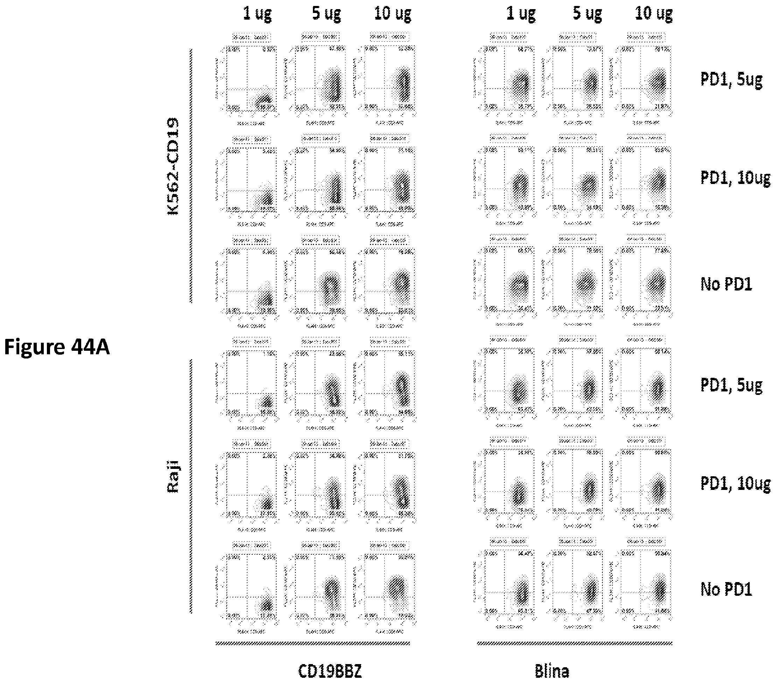

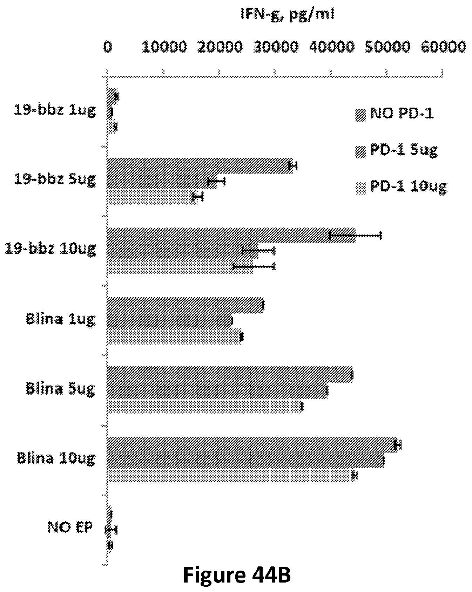

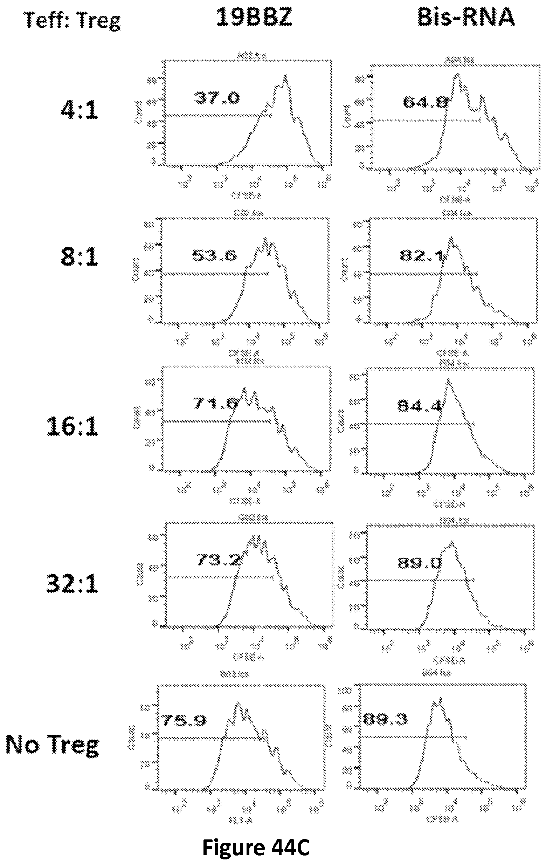

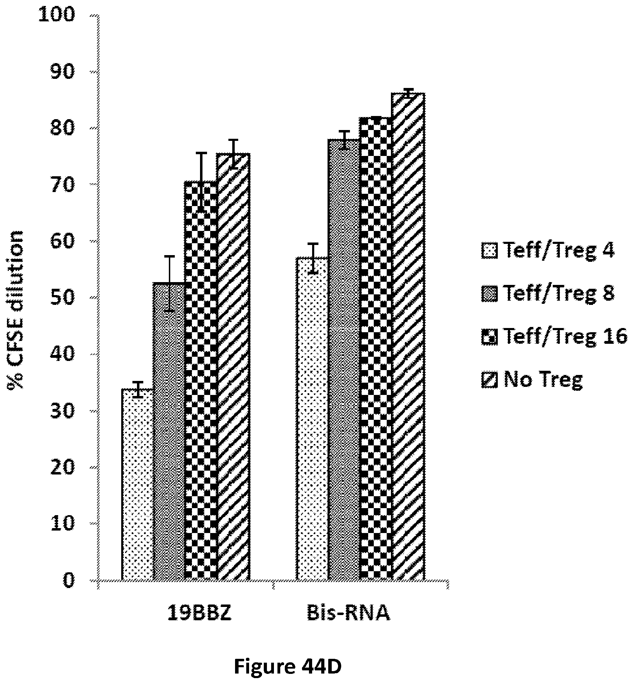

Figure 44, comprising Figures 44A-44D, shows that Bis-RNA electroporated T cells were more resistant to programmed cell death 1 (PD1) and regulatory T cell (Treg) induced suppression. T cells were co-electroporated with different amounts of CD19BBZ RNA, or CD19- CD3 Bis-RNA and 5 ug or 10 ug of PD1 RNA were stimulated with CD19 positive cell lines, K652- CD19 or Raji. CD107a expression was measured by flow cytometry (Figure 44A) and IFN-gamma production production was detected by ELISA (Figure 44B). Tregs purified from fresh resting CD4 T cells were added to CFSE labeled Blinatumomab Bis-RNA or CD19BBZ RNA (19BBZ) electroporated resting CD4+ T cells at different T effector:Treg ratios and stimulated with K562-CD19 cells. Six days after the stimulation, the cells were stained with anti-CD3. CFSE staining was monitored by flow cytometry (Figures 44C-D).

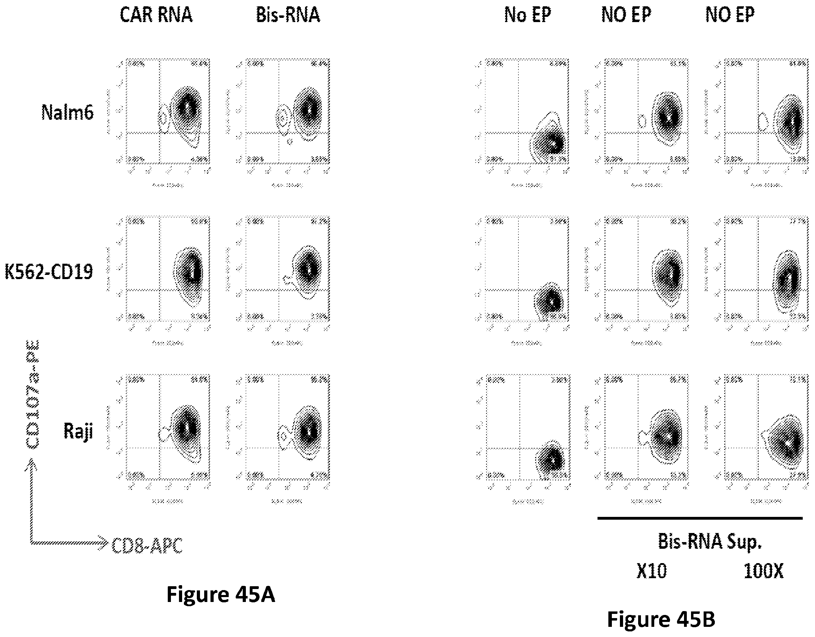

Figure 45, comprising Figures 45A-45B, shows the expression of T cells pulsed with supernatant from Bis-RNA electroporated T cells. T cells were electroporated with CD19BBZ RNA (CAR RNA) or Bis- RNA for Blinatumomab (Blina-Bis-RNA) or D4F11 RNA (D4F11-Bis-RNA). Eighteen hours after electroporation, supernatant was harvested from Bis-RNA electroporated T cells and diluted (as indicated 10X or 100X) supernatant was added to non-electroporated T cells co-cultured with CD19 positive cell lines, Nalm6, K562-CD19 or Raji. T cells electroporated with CAR RNA or Bis-RNA were controls. CD107a up-regulation was monitored after 4 hours of stimulation.
Figure 46 is a graph showing Bis-RNA electroporated T cells have significantly enhanced lytic activity. T cells were electroporated with either Blinatumomab Bis-RNA (Bis-RNA) or CD19bbz CAR RNA at RNA doses of 1, 5 or 10µg/0.1 ml of T cells. After 18 hours, lytic activity was measured using a four hour flow based cytotoxic T lymphocyte assay at a 30:1 T effector:target ratio.
Figure 47 is a panel of graphs showing CD107a upregulation in Bis-RNA electroporated T cells. T cells were electroporated with either 10 µg 4D5BBZ or 4D5-CD3 RNA and stimulated with a K562 cell line electroporated with decreasing amounts of ErbB2 (Her2) mRNA as indicated by co-culturing them for 4 hours. CD107a upregulation was detected by flow cytometry.

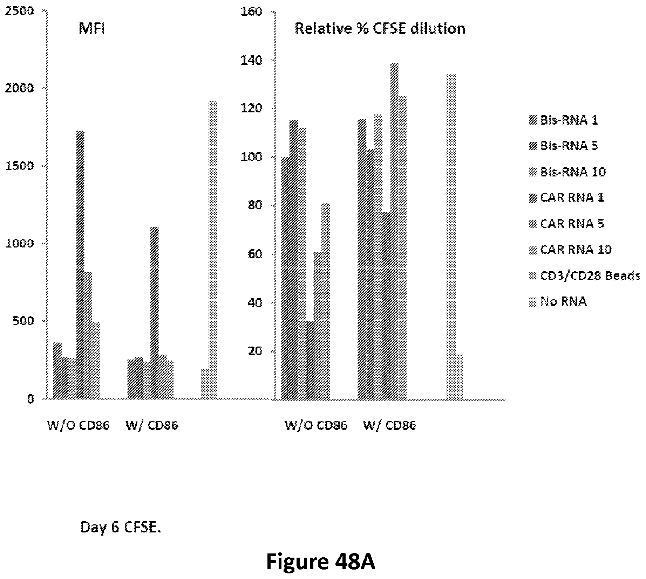

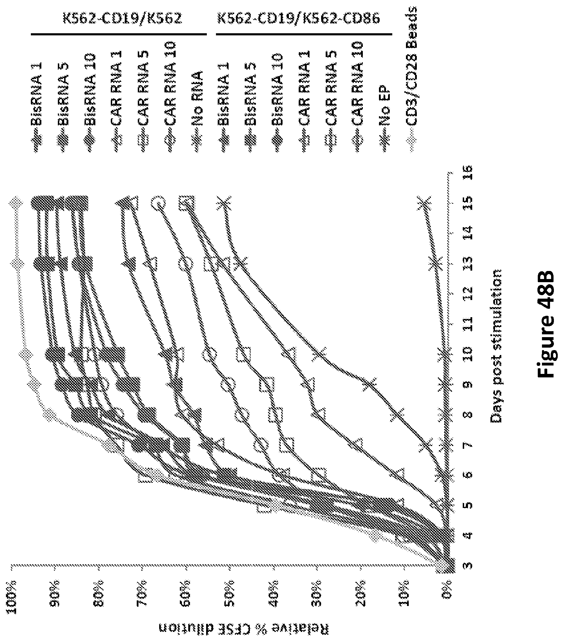

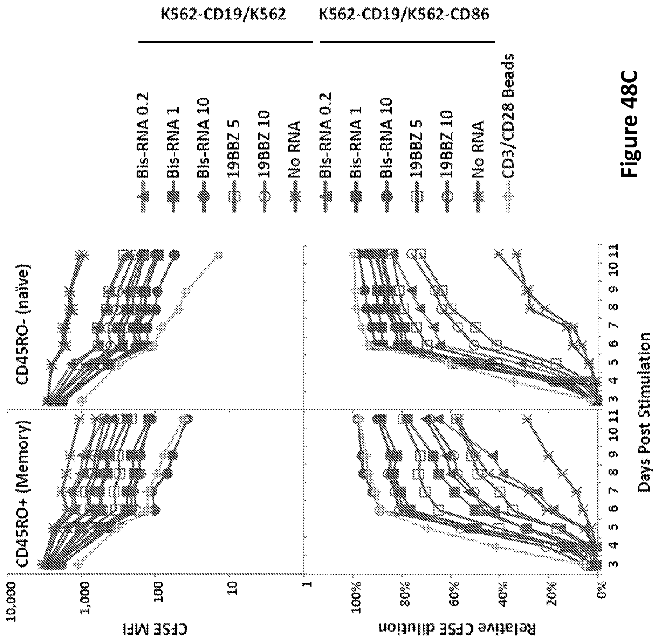

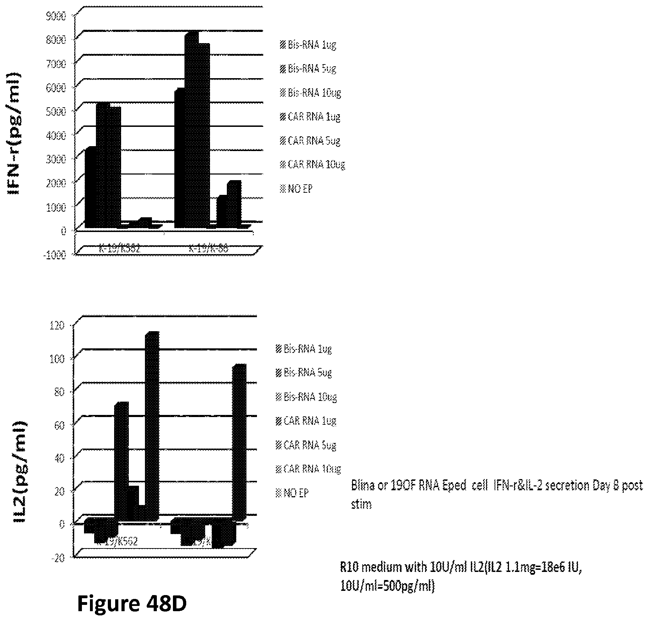

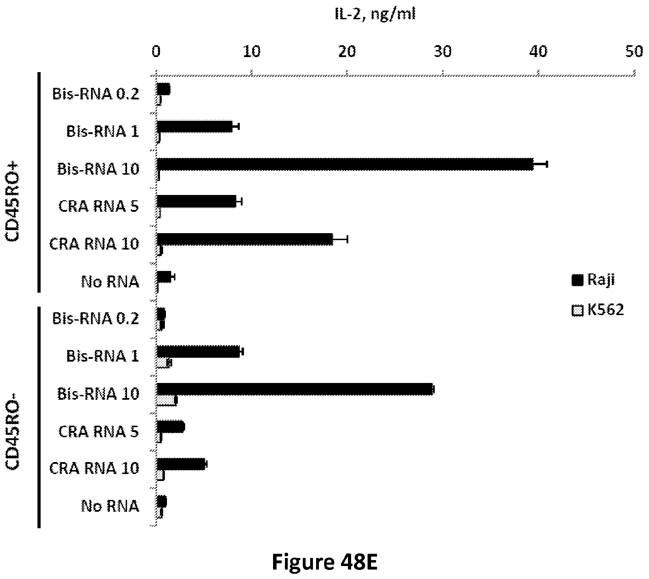

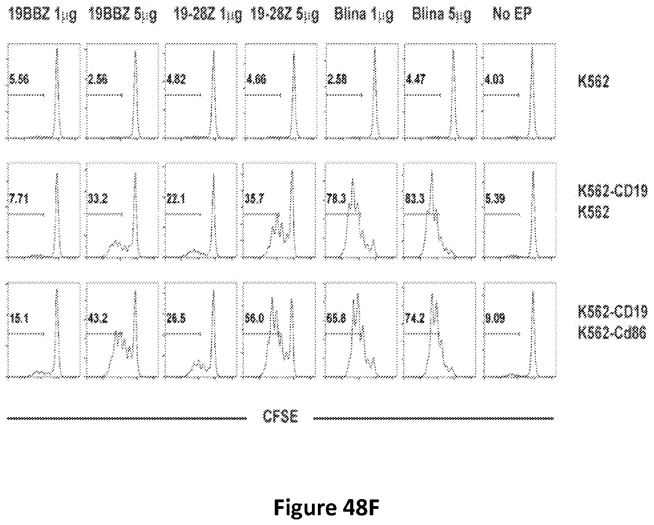

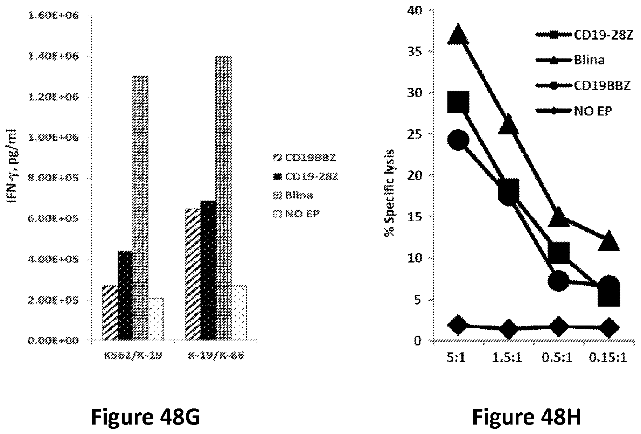

Figure 48, comprising Figures 48A-48H, show Bis-RNA electroporated T cells. Figure 48A is a panel of graphs showing CFSE labeling of resting CD4+ T cells that were electroporated with Blinatumomab Bis-RNA or CD19BBZ RNA (CAR RNA) at different RNA doses and stimulated with either irradiated K562-CD19 cells (mixed with an equal number of irradiated K562 cells as a control for K562-CD86 cells)(W/O CD86), or irradiated K562-CD19 cells (mixed with an equal number of irradiated K562-CD86 cells) (W/ CD86). CFSE dilution at day 6 was shown. Left panel shows mean fluorescence intensity (MFI) for CFSE labeling (higher MFI indicates less T cell division and proliferation). Right panel shows the relative percentage of CFSE dilution of T cells electroporated with 1 µg Bis-RNA. CD3/CD28 bead stimulated T cells and T cells without electroporation were used as positive and negative controls, respectively. Figure 48B is a graph showing CFSE labeling of resting CD4+ T cells that were electroporated with Blinatumomab Bis-RNA or CD19BBZ RNA (CAR RNA) at different RNA doses and stimulated with either irradiated K562-CD19 cells (mixed with an equal number of irradiated K562 cells as a control for K562-CD86 cells)(K562-CD19/K562), or irradiated K562-CD19 cells (mixed with an equal number of irradiated K562-CD86 cells) (K562-CD19/K562-CD86). Relative CFSE dilution starting at day 3 was shown (T cells electroporated with 1 µg Bis-RNA and stimulated with K562-CD19/K562 cells at day 6 was set as 50%). Figure 48C is a panel of graphs showing CFSE labeling of CD45RO+ (memory cells) or CD45RO- (Naive cells) resting CD4 T cells that were electroporated with Blinatumomab Bis-RNA or CD19BBZ RNA (CAR RNA) at different RNA doses and stimulated with either irradiated K562-CD19 cells (mixed with an equal number of irradiated K562 cells as a control for K562-CD86 cells)(K562-CD19/K562), or irradiated K562-CD19 cells (mixed with an equal number of irradiated K562-CD86 cells) (K562-CD19/K562-CD86). CFSE dilution starting at day 3 was shown as MFI of CFSE or Relative CFSE dilution (CD45RO+ T cells electroporated with 1 µg Bis-RNA and stimulated with K562-CD19/K562 cells at day 6 was set as 50%). Figure 48D is a panel of graphs showing CFSE labeling of resting CD4+ T cells that were electroporated with Blinatumomab Bis-RNA or CD19BBZ RNA (CAR RNA) at different RNA doses and stimulated with either irradiated K562-CD19 cells (mixed with an equal number of irradiated K562 cells as a control for K562-CD86 cells)(K19/K562), or irradiated K562-CD19 cells (mixed with an equal number of irradiated K562-CD86 cells) (K19/K86). Eight days after stimulation, supernatant from the cultures was subjected to ELISA for IFN-gamma and IL-2 detection. Figure 48E is a graph showing CFSE labeling of CD45RO+ (memory cells) or CD45RO- (Naive cells) resting CD4 T cells that were electroporated with Blinatumomab Bis-RNA or CD19BBZ RNA (CAR RNA) at different RNA doses and stimulated with Raji (CD19+) or K562 (Cd19-) cells for 18 hours. IL-2 secretion was assayed by ELISA. Figure 48F is a graph showing T cells that were CFSE labeled and electroporated with either 1 or 5 µg CD19BBZ (19BBZ), or CD19-28Z (19-28Z) or Blinatumomab (Blina) RNA were stimulated irradiated K562, or 1:1 mixture of K562 with K562-CD19 or 1:1 mixture of K562-CD19 and K562-CD86. Six days later, the T cells were subjected to flow cytometry analysis for CFSE dilution. Figure 48G is a graph showing total viability at day 6 of 5x10⁵ T cells that were electroporated with 5 µg CD19BBZ (19BBZ), or CD19-28Z (19-28Z) or Blinatumomab (Blina) RNA and stimulated with 1:1 mixture of K562 cells with K562-CD19 cells or 1:1 mixture of K562-CD19 cells and K562-CD86 cells. Figure 48H is a graph showing T cells that were electroporated with 5 µg CD19BBZ (19BBZ), or CD19-28Z (19-28Z) or Blinatumomab (Blina) RNA and analyzed in a flow based 4 hour cytotoxic T lymphocyte assay.

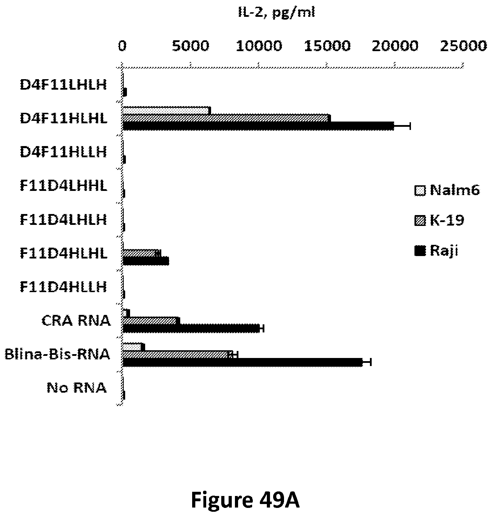

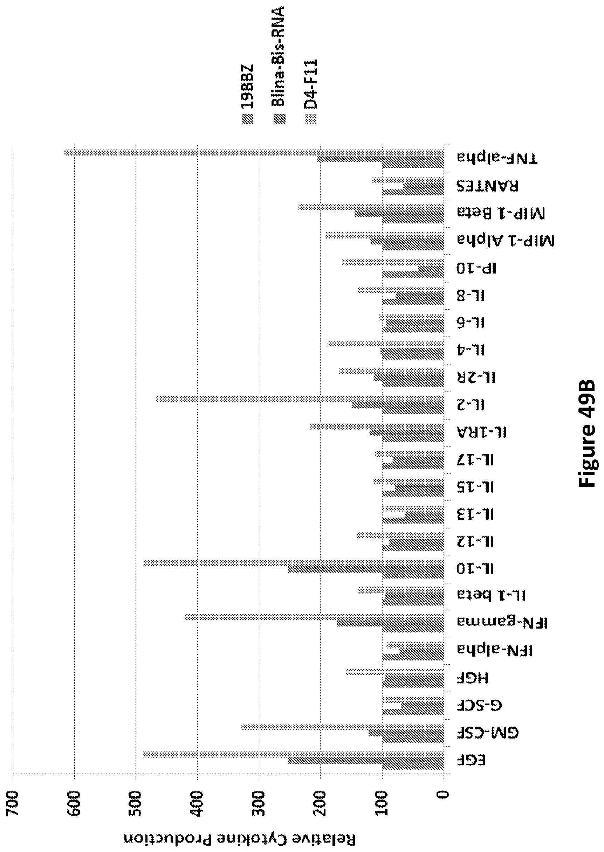

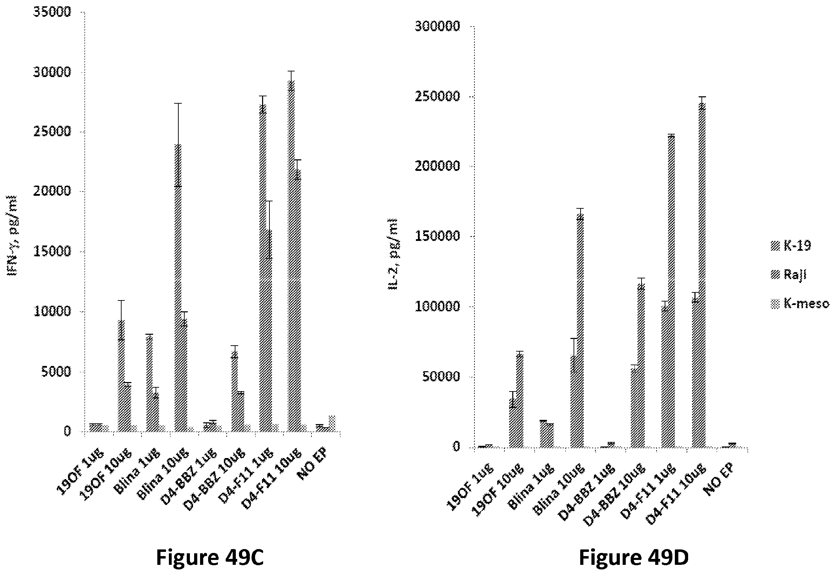

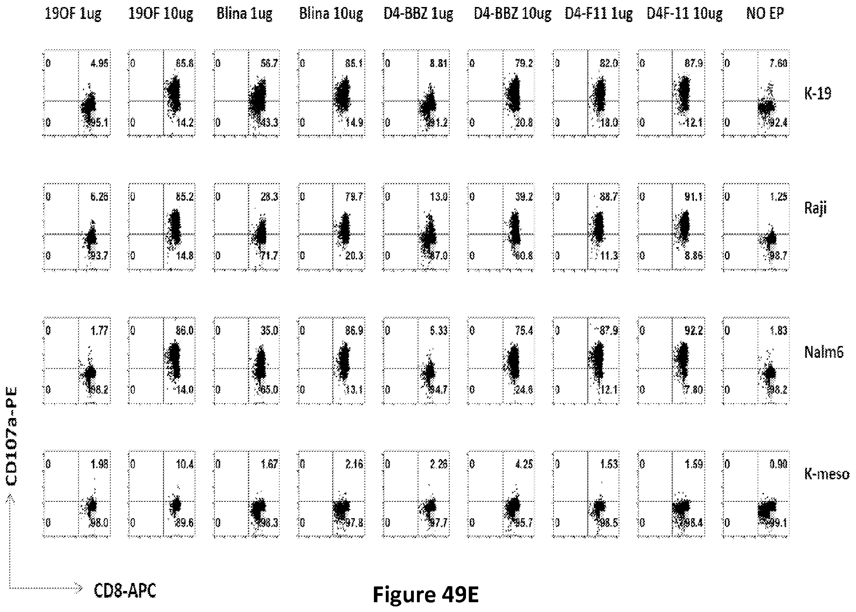

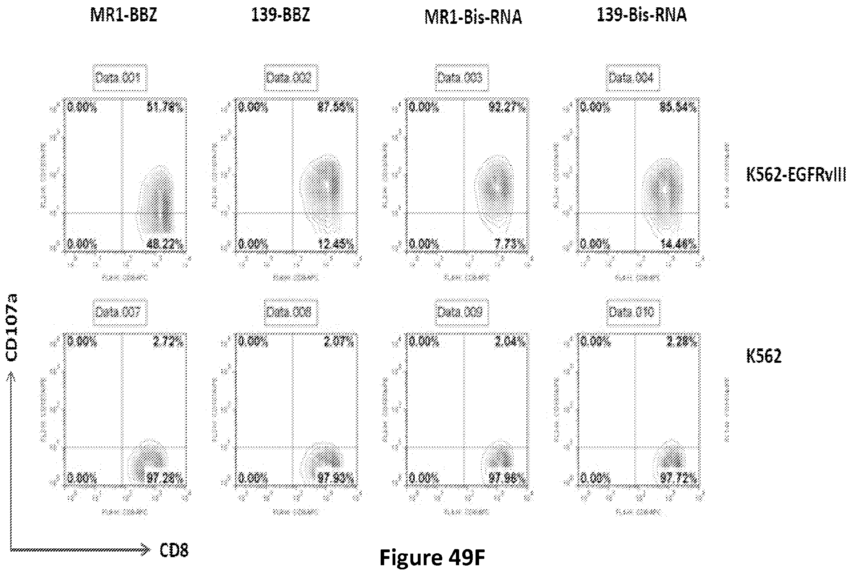

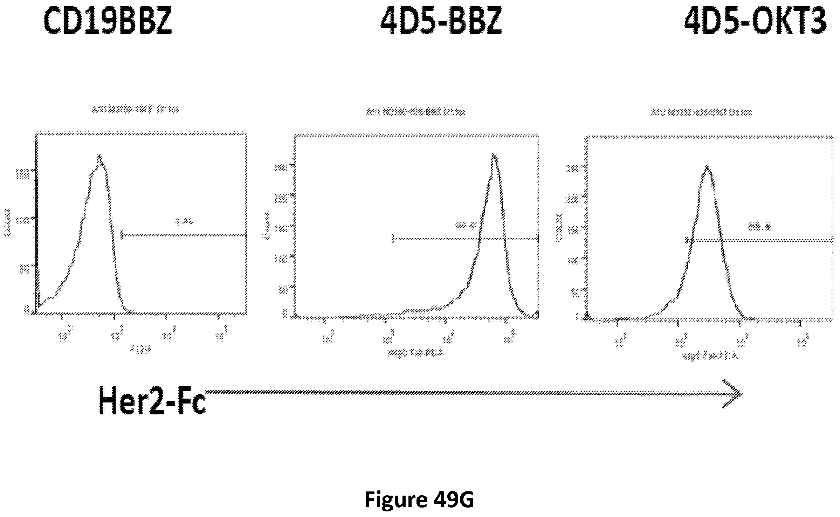

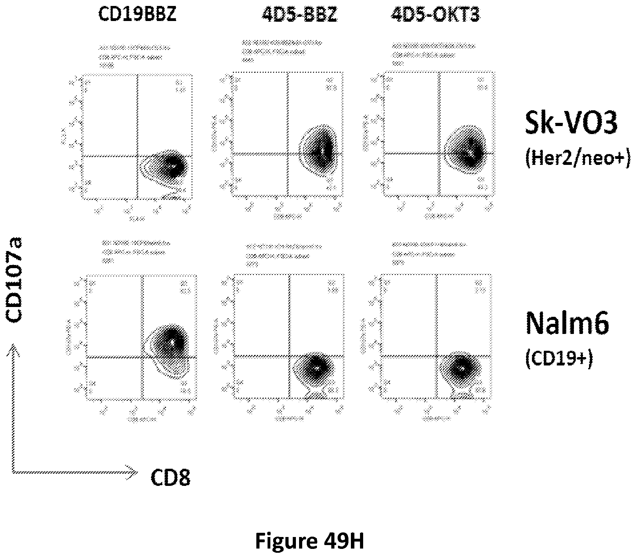

Figure 49, comprising Figures 49A-49H, shows the level of expression of T cells electroporated with various constructs of Bis-RNA. Figure 49A is a graph showing T cells electroporated with one of seven different Bis-RNA constructs encoding a fully human CD19-CD3 using scFvs from fully human anti-CD19 antibody (21D4) and a fully human anti-CD3 scFv antibody (28F11), respectively. IL-2 production was detected by ELISA after the electroporated T cells were stimulated with CD19 positive cell lines, Nalm6, K562-CD19 and Raji, for 18 hours. CD19BBZ CAR RNA (CAR RNA), Blinatumomab Bis-RNA (Blina-Bis-RNA) and no electroporation (No EP) were used as controls. Figure 49B is a graph showing T cells electroporated with CD19BBZ RNA (CAR RNA) or Blinatumomab Bis-RNA (Blina-Bis-RNA) or fully human CD19-CD3 Bis-RNA (D4F11). Four hours after electroporation, the T cells were stimulated with K562-CD19 for 18 hours and the supernatant was subjected to Luminex for multiple cytokine/chemokine detection. Data was normalized by setting CAR RNA T cells at 100%. Figure 49C is a graph showing T cells electroporated with either CD19BBZ, Blinatumomab Bis- RNA, D4BBZ or D4F11 Bis-RNA at RNA doses of 1 ug or 10 ug. The cells were stimulated with CD19 positive cell lines, K562-CD19 or Raji, and cytokine production (IFN-gamma and IL-2) was detected by ELISA. Figure 49D is a graph showing T cells electroporated with either CD19BBZ, Blinatumomab Bis- RNA, D4BBZ or D4F11 Bis-RNA at RNA doses of 1, 5 or 10 ug. The cells were stimulated with CD19 positive cell lines, K562-CD19, Raji or Nalm6, and a CD19 negative line K562-meso as a control. CD107a expression was detected by flow cytometry. Figure 49E is a graph showing CD107a upregulation of T cells electroporated with RNA encoding EGFRviii CAR (MR1-BBZ or 139-BBZ) or EGFRviii Bis-RNA (MR1-Bis-RNA or 139-Bis-RNA) and stimulated with EGFRviii RNA electroporated K562 cells or K562 cells. Figure 49F is a graph showing CD107a upregulation of T cells electroporated with RNA encoding a CD19 CAR (CD19BBZ) or ErBB2 CAR (4D5-BBZ) or ErBB2 Bis-RNA (4D5-OKT3). The upper panel shows the staining of T cells with a ErBB2 fusion protein (Her2-Fc) 18 hours post electroporation. The lower panel shows CD107a staining of T cells stimulated with either a ErBB2 positive/CD19 negative tumor cell line, (SK-OV3) or CD19 positive/ErBB2 negative tumor cell line (Nalm6). Figure 49G is a panel of graphs showing ErBB2 fusion protein (Her2-Fc) expression. Figure 49H is a panel of graphs showing CD107a expression in CD8+ cells.
Figure 50 is a graph showing T cells that were co-electroporated with different amounts of CD19BBZ RNA, or CD19-CD3 Bis-RNA and 5 ug or 10 ug of PD1 RNA. The cells were stimulated with a CD19 positive cell line Raji, and IFN-gamma secretion was detected by ELISA. Data was plotted as a percentage of cytokine production of T cells relative to cells without co-introducion of PD1.

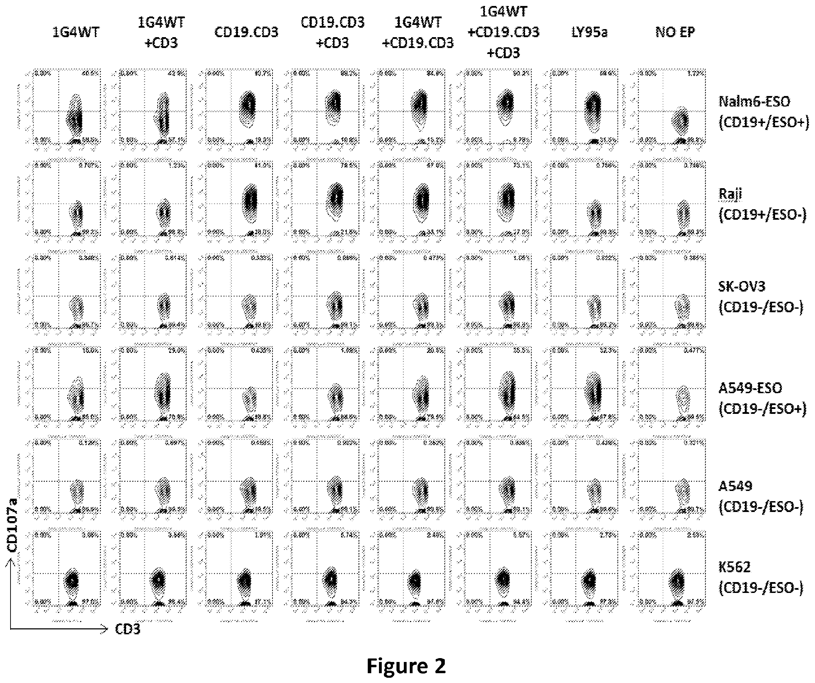

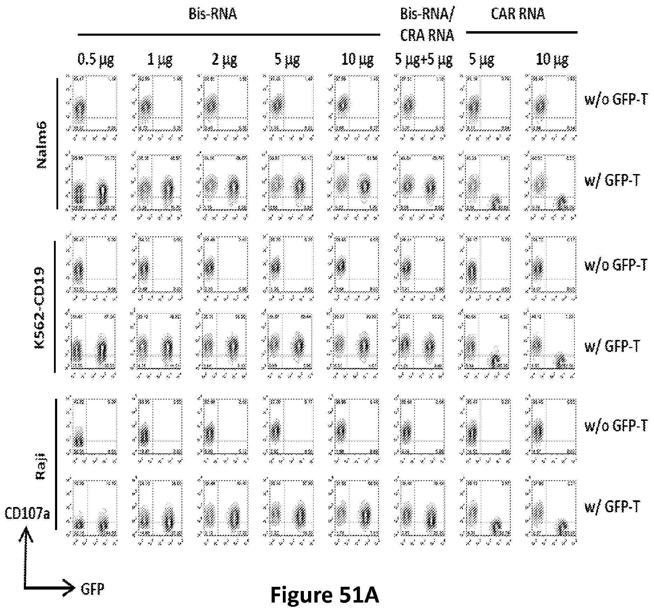

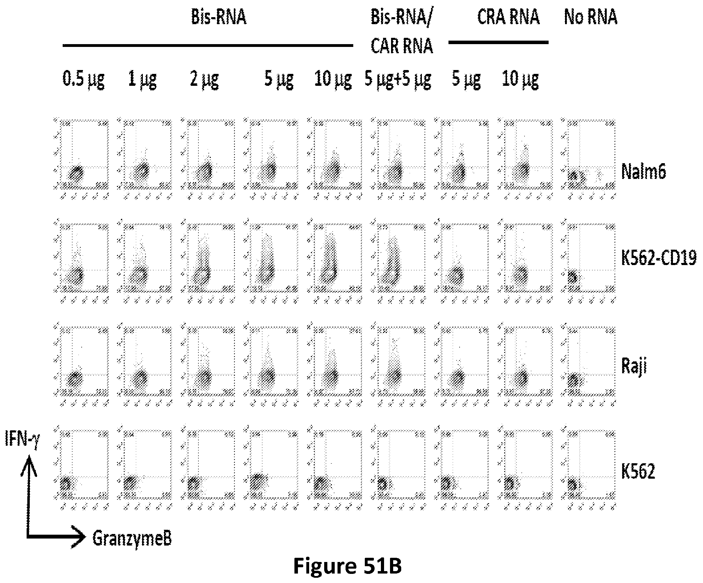

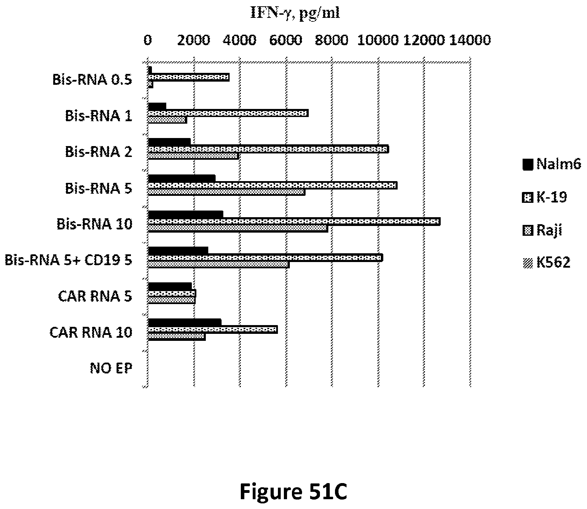

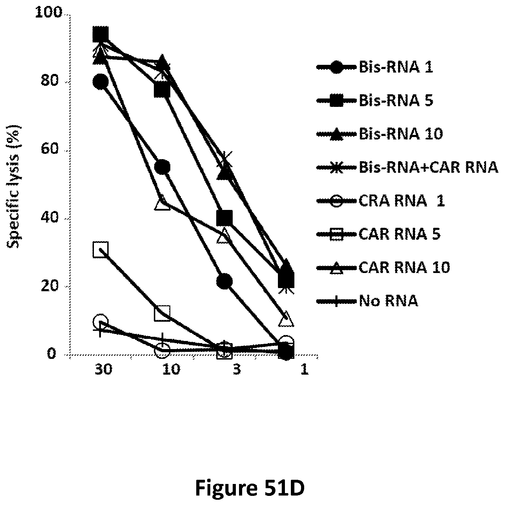

Figure 51, comprising Figures 51A-51D, is a panel of graphs characterizing co-electroporated T cells. Figure 51A is a panel of flow graphs showing the increase of T cell activation and tumor killing ability of Bis-RNA electroporated T cells. T cells were electroporated with different amounts (µg RNA /0.1 ml T cells) of either Blinatumomab Bis-RNA (Bis-RNA) or CD19 CAR RNA (CAR RNA), as indicated. Eighteen hours after electroporation, the T cells with either Bis-RNA or CAR RNA were stimulated with CD19 positive cell lines with or without adding an equal number of GFP RNA electroporated T cells (GFP-T) and assessed for CD107a expression. Figure 51B is a panel of graphs showing IFN-gamma and Granzyme B expression after Bis-RNA electroporation. Eighteen hours after electroporation of the T cells described above, the electroporated T cells were subjected to intracellular staining of IFN-gamma and Granzyme B (Figure 2B). Figure 51C is a graph showing ELISA detection of IFN-gamma. ELISA for IFN-gamma was performed after overnight stimulation of the T cells with CD19 positive cells (Nalm6, K562-CD19 and Raji) or CD19 negative (K562) cell lines. Figure 51D is a graph showing specificity of T cells after electroporation and expression of Bis-RNA. T cells were electroporated with either Blinatumomab Bis-RNA (Bis-RNA) or CD19bbz CAR RNA (CAR RNA at RNA doses of 1, 5 or 10 µg/0.1 ml of T cells, or co-electroporated with 5 µg of each Bis-RNA and CAR RNA (Bis-RNA+CAR RNA). Eighteen hours after electroporation, lytic activity was assessed with a four hour flow-based cytotoxic T lymphocyte assay at the indicated effector:target ratios.
Figure 52 is a table listing the soluble fusion-proteins RNAs electroporated into T cells.
Figure 53 is an illustration showing construction of bi-specific antibodies using anti-PD-L1 and anti-CD28 scFvs.
Figure 54, comprising Figures 54A-54B, is a panel of graphs showing cytokine production in electroporated T cells. Figure 54A is a graph showing IL-2 production by T cells electroporated with the different RNAs and activated by incubation with tumor cells. Figure 54B is a graph showing IFN-gamma production by T cells electroporated with the different RNAs and activated by incubation with tumor cells.
Figure 55 is an illustration showing construction of bi-specific antibodies using anti-TGFb receptor II and anti-CD28 scFvs.

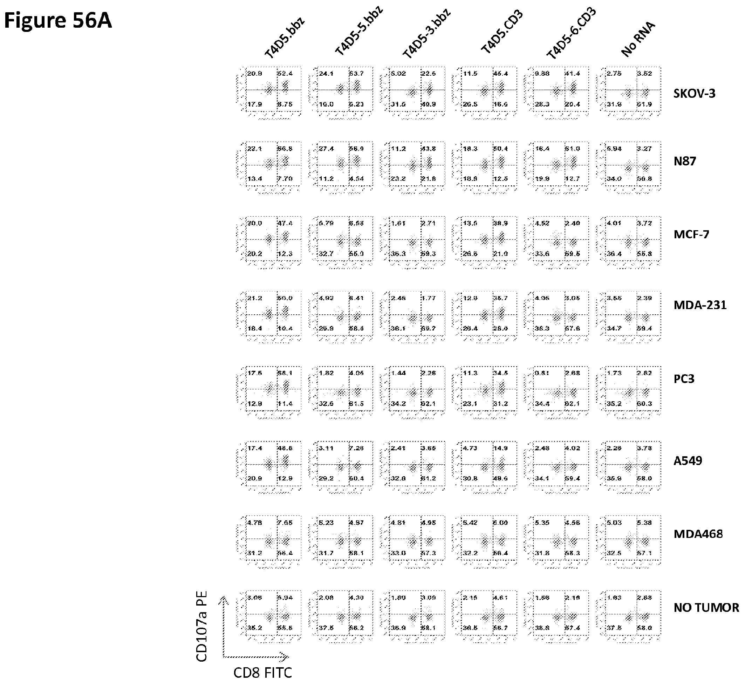

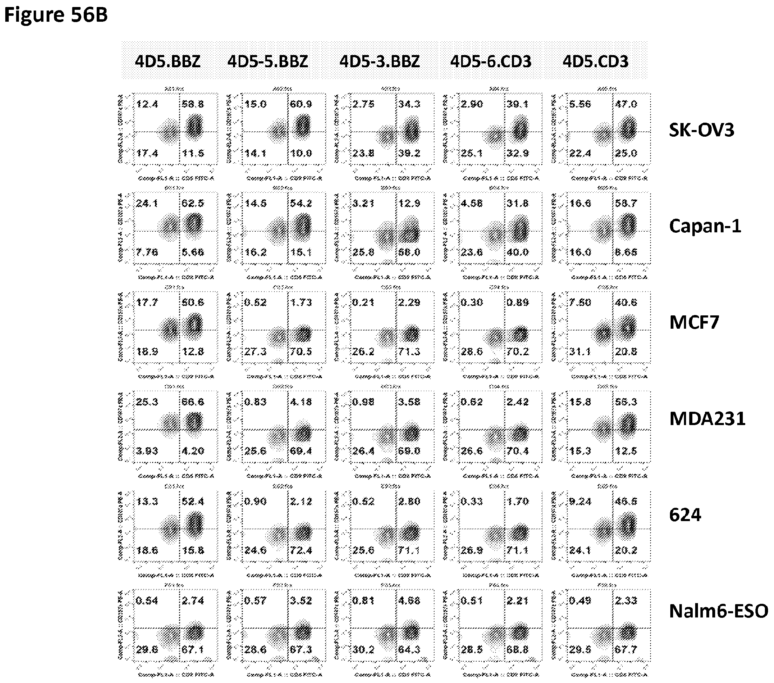

Figure 56, comprising Figures 56A-56B, is a panel of graphs showing reactivity of electroporated T cells. Figure 56A is a graph showing T cells electroporated with 4D5-CD3 Bis-RNA were reactive to ErbB2 over expressing tumor cells. T cells electroporated with ErbB2 CARs or Bis-RNA were stimulated with tumor lines expressing high levels of ErbB2, SK-OV3 and N87, or low levels, MFC-7, MDA-231, PC3 and A549. A CD107a assay showed that T cells expressing 4D5-6.CD3 were only reactive to ErbB2 over expressing tumor cells. Figure 56B is a panel of graphs showing results of a repeat of the experiment of Figure 56A.
Figure 57 is a panel of graphs showing lytic activity of T cells electroporated with 4D5-CD3 Bis-RNA to ErbB2 over expressing tumor cells. T cells electroporated with ErbB2 CARs or Bis-RNA were tested for their lytic activity against ErbB2 over-expressing tumor cells, SK-OV3-CBG, or ErbB2 low expressing tumor cells, mel624 (624-CBG). The luciferase based CTL assay showed that, like affinity tuned BebB2 CAR, 4D5-5.BBZ and 4D5-3.BBZ, T cells expressing 4D5-6.CD3 were only reactive to ErbB2 over expressing tumor cells, SK-OV3.
Figure 58 is a panel of graphs showing T cells lenti-virally transduced with 4D5-CD3 Bis-RNA were reactive to ErbB2 over expressing tumor cells. T cells transduced with ErbB2 CARs or Bis-RNA were stimulated with tumor lines expressing high levels of ErbB2, SK-OV3 and N87, or low levels, MDA-231, PC3 and A549. A CD107a assay showed that T cells expressing 4D5-CD3 were only reactive to ErbB2 over expressing tumor cells.
Figure 59 is a panel of graphs showing T cells lenti-virally transduced with 4D5-CD3 Bis-RNA were reactive to ErbB2 over expressing tumor cells. T cells transduced with ErbB2 CARs or Bis-RNA as indicated were stimulated with tumor lines expressing high levels of ErbB2, SK-OV3 and N87, or low levels, MDA-231, PC3 and A549. Cytokine production as assayed by ELISA indicates T cells expressing T4D5-CD3 were only reactive to ErbB2 over expressing tumor cells.

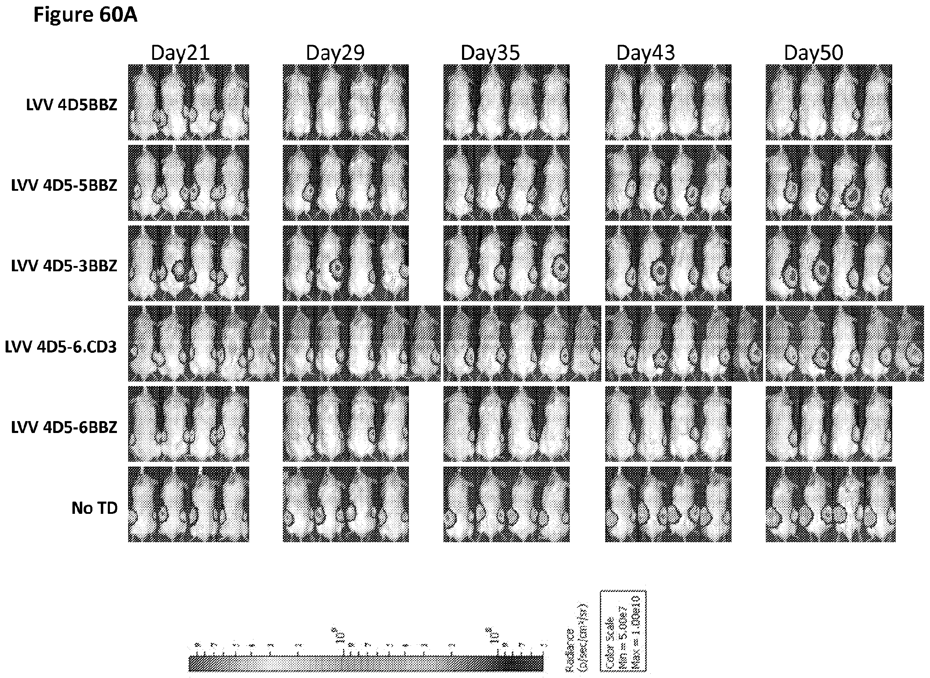

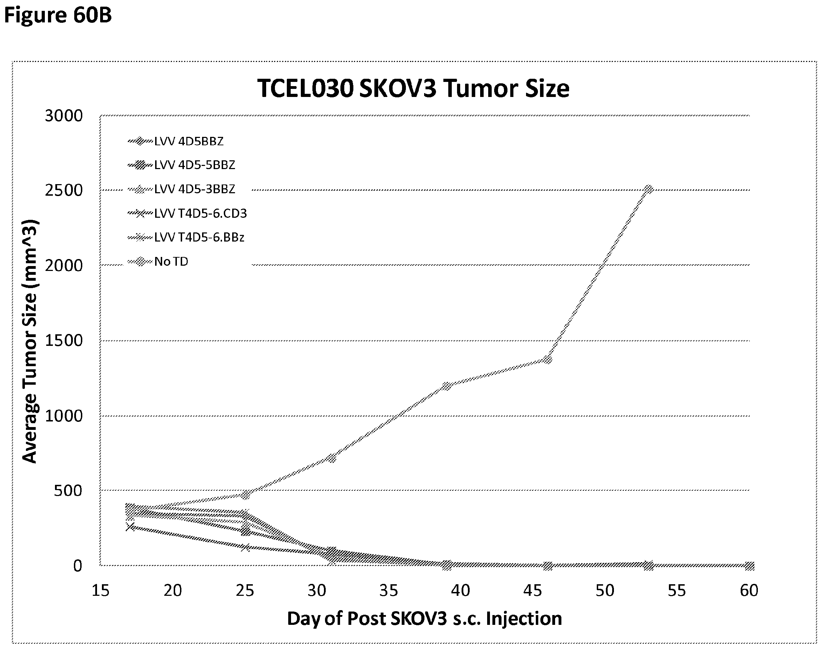

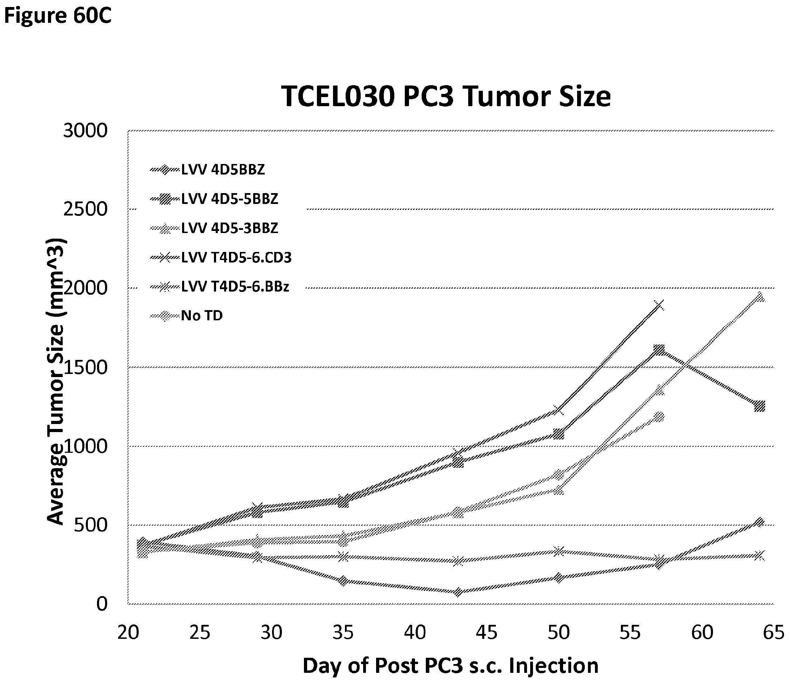

Figure 60, comprising Figures 60A and 60C, is a panel of images showing regression of advanced vascularized tumors in T cell treated mice. Figure 60A is a panel of images showing affinity-tuned ErbB2 BiTE cells increase the therapeutic index and induce regression of advanced vascularized tumors in mice. T cells modified with different affinity ErbB2 CARs or BiTEs by lentiviral transduction were tested in dual-tumor engrafted NSG mice. Mice (n=4-5) were implanted with PC3-CBG tumor cells (1x10⁶ cells/mouse, s.c.) on the right flank on day 0. On day 5, the same mice were given SK-OV3-CBG tumor cells (5x10⁶ cells/mouse, s.c.) on the left flank. The mice were treated with T cells (i.v.) at day 23 after PC3 tumor inoculation. T cells were administered as a single injection of 1x10⁷/mouse. Mice treated with non-transduced T cells (No TD) served as controls. Animals were imaged at the indicated time post PC3 tumor inoculation. Figure 60B is a graph showing SK-OV3 tumor size in dual-tumor grafted NSG mice model. Tumor sizes were measured, and the tumor volume was calculated and plotted. Figure 60C is a graph showing PC3 tumor sizes in dual-tumor grafted NSG mice model. Tumor sizes were measured, and the tumor volume was calculated and plotted.
Figure 61, comprising Figures 61A-61B, is a panel of images showing the PD1-CD28 switch receptors. Figure 61A is an illustration of constructs for co-expressing PD1-CD28 switch receptor and affinity tuned T4D5-6.CD3 BiTEs. Figure 61B is a panel of graphs showing detection of PD1-CD28 switch receptor of T cells lentivirally transduced with PD1-CD28 and T4D5-CD3 co-expression vectors.

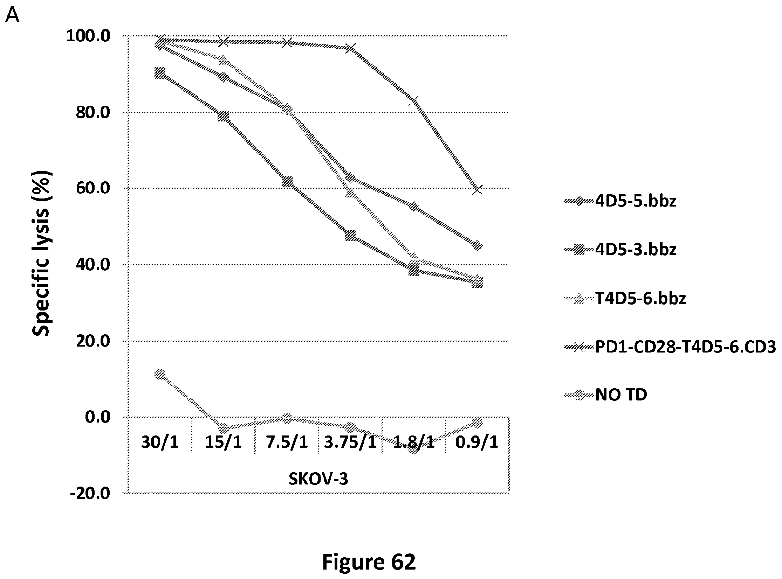

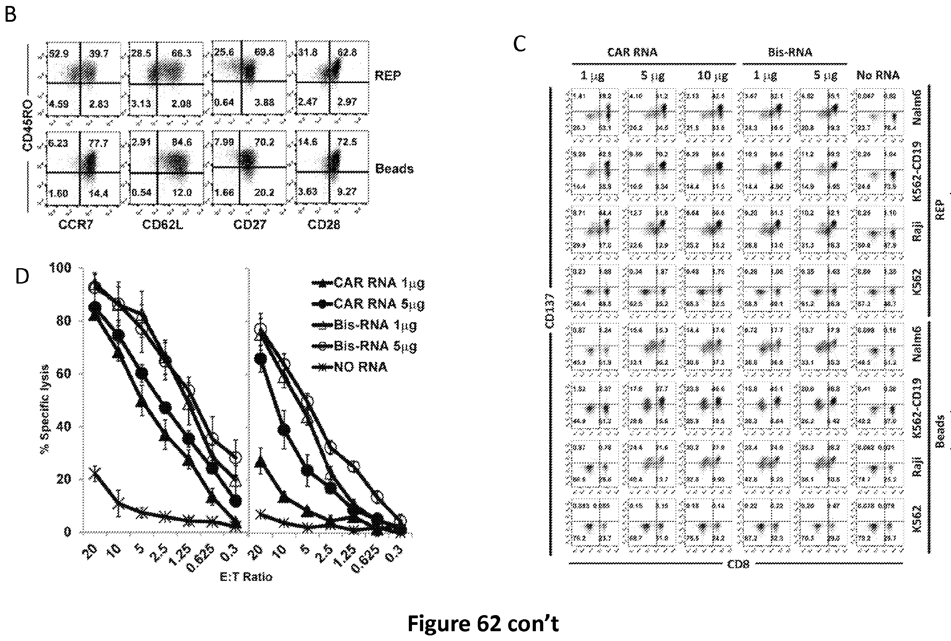

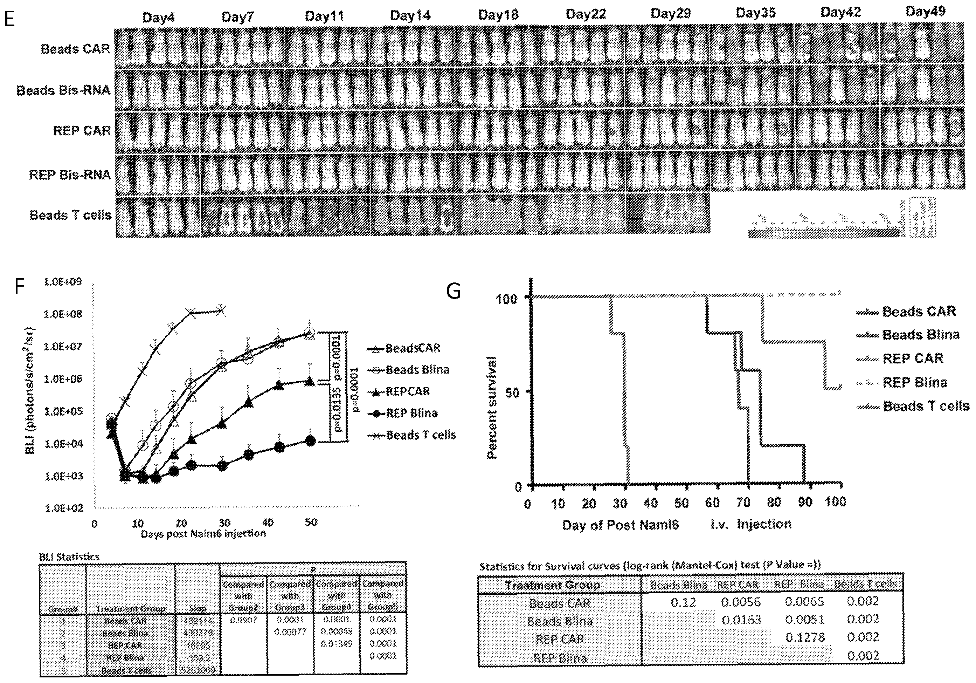

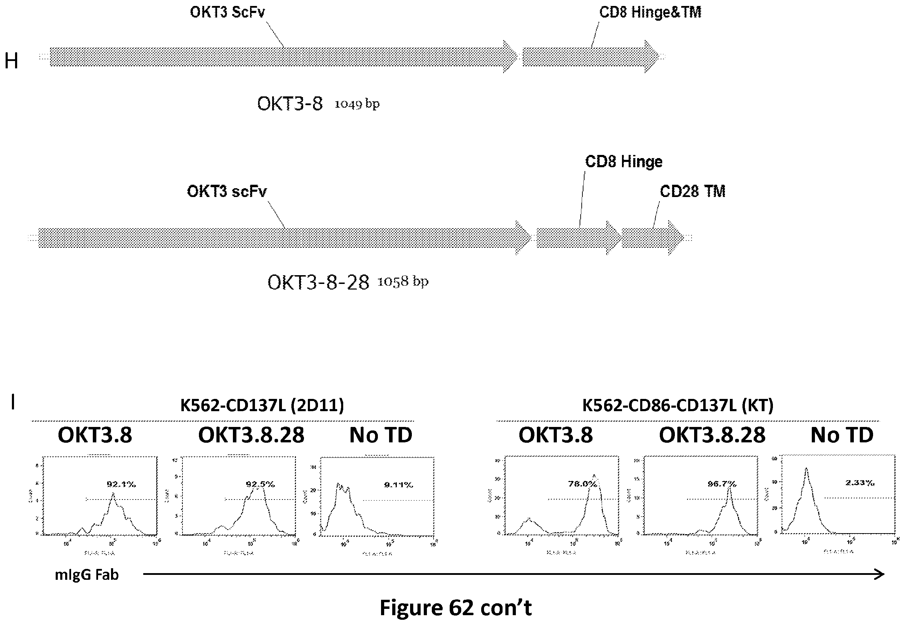

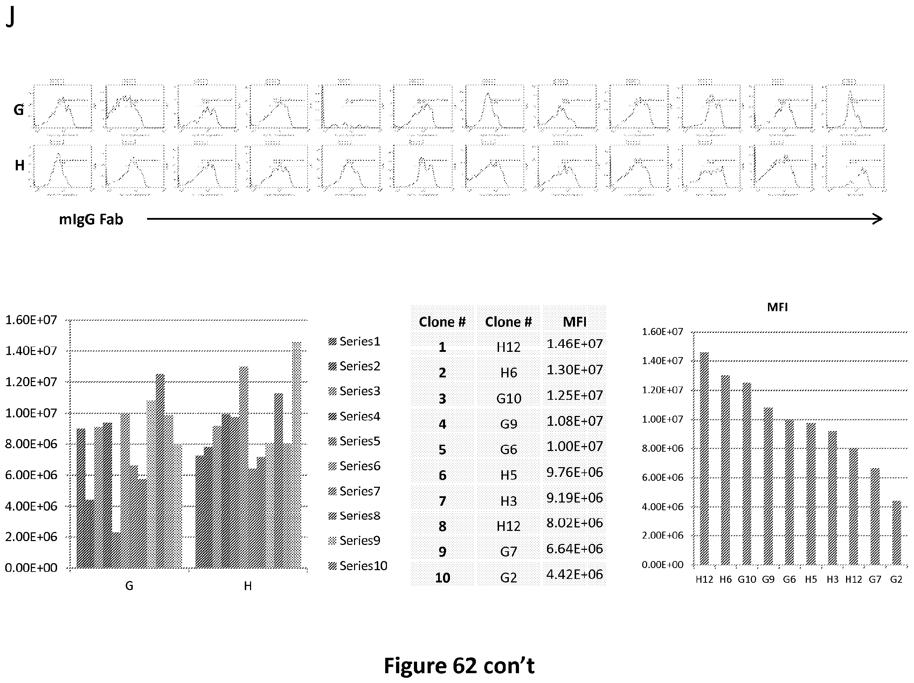

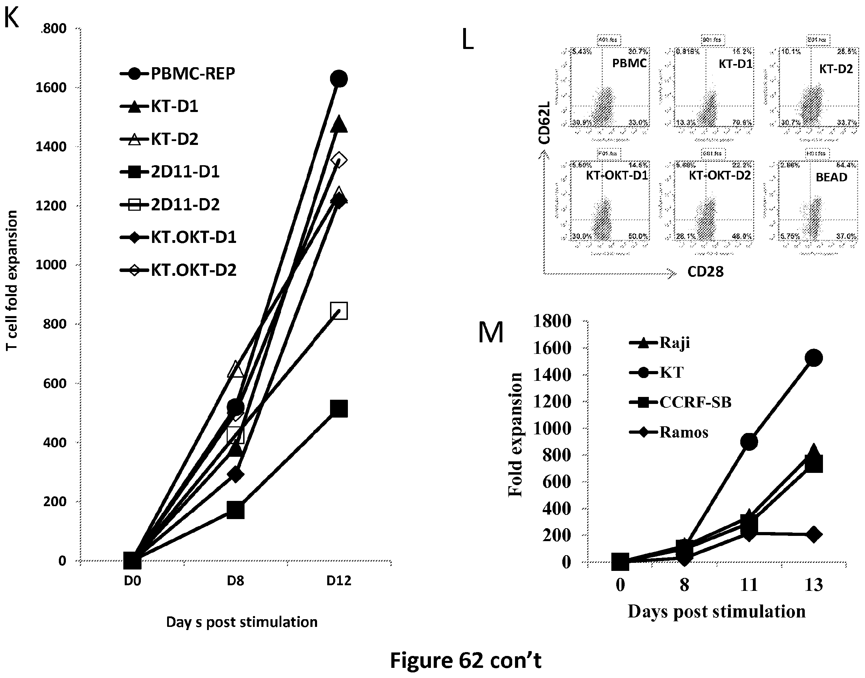

Figure 62, comprising Figures 62A-62M, is a panel of images. Figure 62A is a graph showing increased lytic activity of T cells co-expressing both PD1-CD28 switch receptor and T4D5-6.CD3 affinity tuned BiTEs. Figures 62B-62G are a panel of images showing Bis-RNA electroporated T cells generated by rapid expansion protocol (REP) further improved in vivo anti-leukemia activity. The phenotype of T cells expanded by REP or anti-CD3/anti-CD28 beads (Beads) was assessed (Figure 62B). REP T cells or anti-CD3/anti-CD28 bead T cells were electroporated with different amounts of CAR RNA or Bis-RNA and stimulated with different cell lines for 18 hr. CD137 up-regulation was analyzed by flow cytometry (gated on CD3+ T cells) (Figure 62C). Lytic activity was measured in REP T cells (Figure 62D, left panel) or anti-CD3/anti-CD28 beads T cells (Figure 62D, right panel) electroporated with different amounts of CAR RNA or Blinatumomab Bis-RNA. NSG mice were injected with 1x10⁶ Nalm6-CBG intravenously and 5 days later treated with 30x10⁶ CAR RNA or Blinatumomab Bis-RNA (Blina) T cells for the first treatment, followed by 5x10⁶ each, twice a week for three weeks starting day at 8 after Nalm6-CBG injection. Bioluminescence imaging (BLI) was conducted at the indicated times (Figure 62E) and the results of BLI and survival were plotted in Figures 62F and Figure 62G, respectively. Figures 62H-62I are a panel of images showing the generation of K562 based artificial antigen presenting cells (aAPC) expressing membrane bound OKT3. Lentiviral vectors (pLENS) expressing chimeric protein for membrane forms of OKT3 with either CD8 hinge and transmembrane (OKT3.8) or with CD8 hinge and CD28 transmembrane (OKT3.8.28) are illustrated in Figure 62H. K562 based aAPC, K562-CD86-CD137L (KT) or K562-CD137L (2D11) cell lines were transduced with lentiviral OKT3.8 or OKT3.8.28 and the expression of the membrane bound OKT3 was detected using an antibody against murine IgG Fab (Figure 62I). Figure 62J is a panel of images showing characterization of membrane bound OKT3 transduced K562 aAPC clones. Selection of the clones by limiting dilution was based on expression of membrane band OKT3 from OKT3.8.28 transduced KT aAPC. Figure 62K-62M are a panel of graphs showing REP with K562 based artificial aAPC. Figure 62K is a graph showing OKT3 loaded K562-CD86-CD137L (KT) or K562-CD137L (2D11), or KT expressing membrane bound OKT3 (KT.OKT) were irradiated and cultured for one day (D1) or two days (D2) before being used to stimulated T cells at T cell: aAPC ratio of 1:250. Figure 62L is a panel of graphs showing the expanded T cells, independently expanded in the REP, stained for CD62L and CD28. Figure 62M is a graph showing different B cell lines in a REP experiment, as compared with KT cells.
Figure 63 is a graph showing NSG mice with established leukemia by intravenous injection of 1x10⁶ Naml6-CBG cells. Seven days later, the mice were treated with 20x10⁶ RNA electroporated T cells. On day 13 and 27, the mice were treated with 60 mg/kg cytoxan intraperitoneal 24 hours prior to injection with 5x10⁶ T cells electroporated with RNA. Animals were images at the indicated time points post injection, with total photon flux indicated on the Y-axis (n=5).
Figure 64 is a graph showing NSG mice with established leukemia by intravenous injection of 1x10⁶ Naml6-CBG cells. Seven days later, the mice were treated with 20x10⁶ RNA electroporated T cells. On day 13 and 27, the mice were treated with 60 mg/kg cytoxan intraperitoneal 24 hours prior to injection with 5x10⁶ T cells electroporated with RNA. Animals were images at the indicated time points post injection, with total photon flux indicated on the Y-axis (n=5).
Figure 65 is a graph showing TCR RNA electroporated T cells controlled tumor growth better than lenti-transduced T cells. 2.5x10⁶ A549-ESO/A2 tumor cells were injected intravenously at day 0. Treatment started at day 5. Lenti-T cells were administered in a single dose of 10x10⁶ at day 5 and RNA electroporated T cells were injected four doses of 30x10⁶, 10x10⁶, 10x10⁶, and 10x10⁶ starting at day 5, twice a week.
Figure 66 is a panel of images showing TCR RNA electroporated T cells controlled tumor cell growth.
Figure 67 is a panel of images showing that lenti-transduced T cells did not control tumor growth as efficiently as TCR RNA electroporated T cells.
Figure 68 is a graph showing that controlled tumor growth in a tumor model more efficiently than lenti-transduced T cells.
Figure 69 is a panel of images showing an illustration of a CD3 construct and graphs showing TCR and CD3 expression in TCR and CD3 RNA electroporated T cells.
Figure 70 is a graph showing expression levels of TCR (vb13.1), CD3 and TCR (vb13.1)/CD3 were detected in electroporated T cells.
Figure 71 is a panel of graphs expression of electroporated TCR RNA in T cells.
Figure 72 is a table showing titer of the lenti-viral vector for 1G4 wildtype (1G4wt) and high affinity (1G4.LY95a) NY-ESO-1 TCR.
Figure 73 is a table showing titer of the different lenti-viral vectors for 1G4 wildtype (1G4wt) and high affinity (1G4.LY95a) NY-ESO-1 TCR.
Figure 74 is a table showing viral infection efficiency of T cells.
Figure 75 is panel of graphs showing transgene expression in T cells co-electroporated with TCR and CD3.
Figure 76 is panel of graphs showing transgene expression in T cells electroporated with TCR and CD3 RNA.
Figure 77 is panel of graphs showing transgene expression in T cells co-electroporated with TCR and CD3 or T cells electroporated with TCR and stimulated with CD3 beads.
Figure 78 is a panel of flow diagrams showing T cells electroporated with TCR RNA (y-axis) with or without CD3 and incubated with Naml6 tumor cells (x-axis).
Figure 79 is a panel of flow diagrams showing T cells electroporated with TCR RNA (y-axis) with or without CD3 and incubated with OKT expressing Naml6 tumor cells (x-axis).
Figure 80 is a panel of flow diagrams showing T cells electroporated with TCR RNA (y-axis) with or without CD3 and incubated with ND340 expressing Naml6 tumor cells (x-axis).
Figure 81 is a panel of flow graphs showing TCR and CD3 expression in T cells electroporated with CD3 delta, gamma, epsilon and zeta, either with all the units (4 subunits), or less (3 subunits, 2 subunits, or 1 subunit), together with NY-ESO-1 TCR (1G4) alpha and beta chain RNA. CD3 and TCR (vb13.1) were detected by flow cytometry 18 hours post electroporation.
Figure 82 is a bar graph showing the mean fluorescence intensity (MFI) of CD3 and TCR expression after CD3 RNA electroporated into 293 cell lines.
Figure 83 is a panel of flow graphs showing CD107a up-regulation in TCR RNA and CD3 RNA co-electroporated T cells stimulated with tumor cells.
Figure 84 is a panel of graphs showing IFN-gamma expression in RNA electroporated T cells incubated with different tumor cell lines. T cells were electroporated with NY-ESO-1 TCR alpha and beta RNA together with different combinations of CD3 RNA. The cells were co-cultured with Naml6-ESO or 624mel tumor cells that are NY-ESO-1/HLA-A2 positive. IFN-gamma production levels were measured after 18 hours.
Figure 85 is a graph showing IFN-gamma expression of different RNAs electroporated T cells incubated with tumor cell lines.
Figure 86 is a panel of graphs showing IL-2 expression in RNA electroporated T cells incubated with different tumor cell lines. T cells were electroporated with NY-ESO-1 TCR alpha and beta RNA together with different combinations of CD3 RNA. The cells were co-cultured with Naml6-ESO or 624mel tumor cells that are NY-ESO-1/HLA-A2 positive. IL-2 production levels were measured after 18 hours.
Figure 87 is a panel of graphs showing that the TCR was expressed by T cells electroporated with 4-1BB containing TCR or CD3 RNA constructs. T cells were co-electroporated with 1G4 TCR alpha (or alpha.BB) and beta (alpha/beta or alpha.BB/beta), or alpha/beta with CD3 zeta or epsilon with or without 4-1BB (zeta, zeta.BB, epsilon, or epsilon.BB). After 18 hours, CD3 and vb13.1 were determined by flow cytometry.

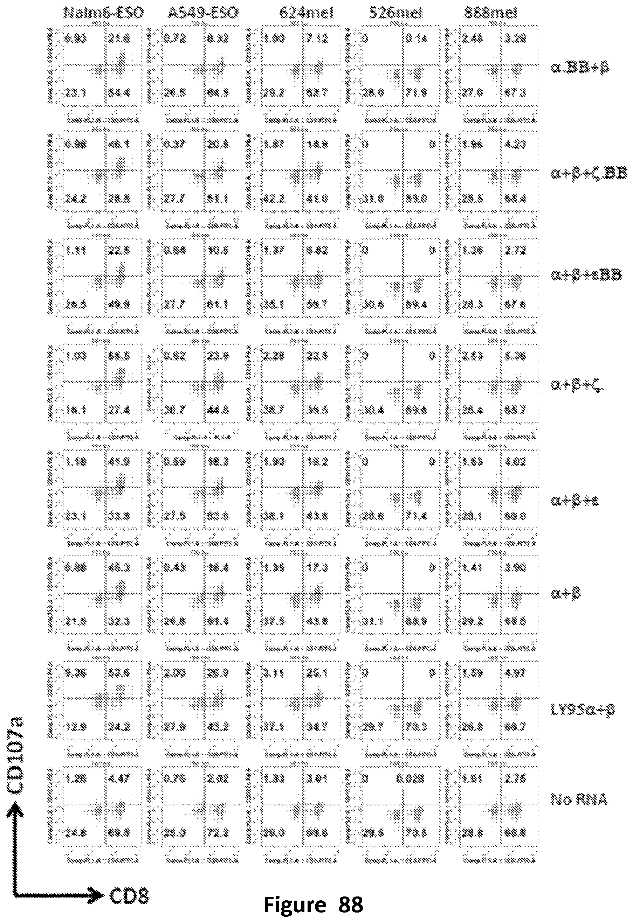

Figure 88 is a panel of graphs showing RNA electroporated T cells incubated with different tumor cell lines were affective against multiple tumor cell lines. Providing co-stimulatory signals to the C terminal of either the TCR or CD3 maintained T cell function and potentially provided T cells with a direct co-stimulatory signal. T cells co-electroporated with 1G4 TCR alpha (or alpha.BB) and beta (alpha/beta or alpha.BB/beta), or alpha/beta with CD3 zeta and epsilon with or without 4-1BB (zeta, zeta.BB, epsilon or epsilon.BB). Eighteen hours later, T cells were stimulated by tumor cell lines, Naml6-ESO(HLA-A2+/NY-ESO-1+), A549ENA(HLA-A2+/NY-ESO-1+), 624mel(HLA-A2+/NY-ESO-1+), 526mel(HLA-A2+/NY-ESO-1+), or 888mel(HLA-A2+/NY-ESO-1+) and CD107a production was measured.
Figure 89 is a panel of flow diagrams showing Naml6 tumor cells (x-axis) and T cells electroporated with RNA encoding different TCR chains (y-axis) with or without CD3 chains.
Figure 90 is a panel of flow diagrams showing 624 tumor cells (x-axis) and T cells electroporated with RNA encoding different TCR chains (y-axis) with or without CD3 chains.
Figure 91 is a panel of flow diagrams showing 526 tumor cells (x-axis) and T cells electroporated with RNA encoding different TCR chains (y-axis) with or without CD3 chains.
Figure 92 is a panel of flow diagrams showing 888 tumor cells (x-axis) and T cells electroporated with RNA encoding different TCR chains (y-axis) with or without CD3 chains.
Figure 93, comprising Figures 93A-93D, is a panel of graphs that show the maximum transgene expression (Figure 93A) achieved and function of electroporated T cells using TCR with a disulfide bond and beta chain having both a disulfide bond and N-deglycosylation. N-deglycosylation of the TCR alpha chain impaired the function of electroporated T cells (Figures 93A-93D).
Figure 94 is a graph showing expression of 10x10⁶ Naml6-CBG-ESO-GFP (click beetle green) cells that express both NY-ESO-1 and GFP after intravenous injection into NOD/SCID mice. Five days after tumor inoculation, first CBR (click beetle red) transduced and RNA electroporated T cells were intravenously injected as indicated (n=5). Wt1G4: wildtype 1G4 TCR, m1G4: second disulfide bond alpha/second disulfide bond alpha and N-deglycosylation beta, CD19: CD19BBZ CAR, meso: ss1BBZ CAR, and saline.
Figure 95 is a panel of images showing N-deglycosylation in the TCR alpha chain impaired function of RNA electroporated T cells.
Figure 96 is a panel of images showing transgene expression and functionality of T cells electroporated with RNA encoding hybrid TCR containing murine constant regions.
Figure 97 is a panel of images showing fluorescence of injected tumor and hybrid TCR T cells in mouse models over time.
Figure 98, comprising Figures 98A-98D, is a panel of images showing the addition of disulfide bonds to the alpha and beta chains, or N-deglycosylation of the beta chain of the TCR enhanced transgene expression and function of electroporated T cells as compared to adding disulfide bonds to the TCR or hybrid TCRs.
Figure 99 is a panel of images showing TCR expressed by T cells electroporated with 4-1BB containing TCR or CD3. T cells were co-electroporated with 1G4 TCR alpha (or alpha.BB) and beta (a/b or a.BB/b), or a/b with CD3 zeta or epsilon with or without 4-1BB (z , z.BB, e or e.BB). Eighteen hours later, CD3 and vb13.1 were determined by flow cytometry.
Figure 100 is a panel of images showing T cells co-electroporated with 1G4 TCR alpha (or alpha.BB) and beta (a/b or a.BB/b), or a/b with CD3 zeta or epsilon with or without 4-1BB (z, z.BB, e or e.BB). Eighteen hours later, the T cells were stimulated with tumor lines, Nalm6-ESO (HLA-A2+/NY-ESO-1+), A549-ESO(HLA-A2+/NY-ESO-1+), 624mel (HLA-A2+/NY-ESO-1+), 526mel (HLA-A2+/NY-ESO-1-), or 888mel (HLA-A2-/NY-ESO-1-) and assessed in a CD107a assay.
Figure 101 is a panel of images showing T cells co-electroporated with 1G4 TCR alpha and beta, or a/b with CD3 epsilon (E) and/or zeta (Z) with or without CD27 (or CD28). Eighteen hours later, the T cells were stimulated with tumor lines, Nalm6-ESO (HLA-A2+/NY-ESO-1+), 624mel (HLA-A2+/NY-ESO-1+), U266 (HLA-A2+/NY-ESO-1+), or 888mel (HLA-A2-/NY-ESO-1-) and assessed in a CD107a assay.
Figure 102 is a panel of images showing the PD1 constructs and detection of PD1 and vb13.1 in T cells lentivirally transduced with PD1-CD28 switch receptor and 1G4 TCR with or without CD27.
Figure 103 is a graph showing 5E6 A549-ESO (HLA-A2 and NY-ESO-1 transduced A549) were injected subcutaneously at day 0. 1x10⁶ transduced T cells were injected at day 14 and the tumor size was measured. The preliminary results indicated no effect for TCR alone (1G4), while tumor growth was delayed for CD27 co-stimulatory signal (1G4.CD27), or PD1-CD28 switch receptor (PD1-CD28.1G4), or both CD27 co-stimulatory signal and PD1-CD28 switch receptor (PD1-CD28.1G4.CD27).
Figure 104 is a schematic drawing of a modified TCR capable of non-MHC restricted tumor antigen recognition with functional cognate antigen recognition.
Figure 105 is a panel of images showing transgene expression of co-electroporated T cells. Seven amino acid flu (HA1) peptide sequence was added to the N'-terminus of either the TCR alpha chain or CD3 zeta or epsilon chains to generate HA1.alpha (HA1.a), HA1.zeta (HA1.z) and HA1.epsilon (HA1.e). T cells were co-electroporated with the modified TCR along with RNA encoding a bi-specific antibody against Flu, HA1 (17-9 or 26-9), and tumor antigen (CD19 or Her2 (4D5)). Eighteen hours post electroporation, transgeneic TCR (vb13.1) expression was examined by flow cytometry.
Figure 106 is a panel of images showing the T cells with a modified NY-ESO-1 TCR recognized cognate antigen (HLA-A2/NY-ESO-1) along with CD19 or Her2.
Figure 107 is a graph showing IFN-gamma secretion of T cells transferred with modified TCR co-introduced with bis-RNA.
Figure 108 is a graph showing IL-2 secretion of T cells transferred with modified TCR co-introduced with bis-RNA.
Figure 109 is a panel of images showing anti-tumor activity of T cells with modified TCR and bis-RNA administered to tumor bearing mice. Mice were injected with Nalm6-ESO (i.v.) and treated with T cells electroporated with RNA as indicated.
Figure 110, comprising Figures 110A-110B, is a panel of images showing T cells with a modified TCR. Figure 110A is diagrammatic sketch of a small molecule (AFFIBODY^{®}) modified TCR (Affi-TCR) by adding the small molecule and His-tag **sequence to the N'** terminus of either alpha or beta chain of the TCR. Figure 110B is a panel of images showing T cells were co-electroporated with NY-ESO-1 (1G4) TCR alpha (a) and beta (b), or their ErbB2 small molecule (342, 342.15, 342 or 342.4) modification. After overnight, vb13.1 and His-Tag were detected by flow cytometry.
Figure 111 is a panel of images showing CD107 up-regulation of Affi-TCR RNA electroporated T cells.

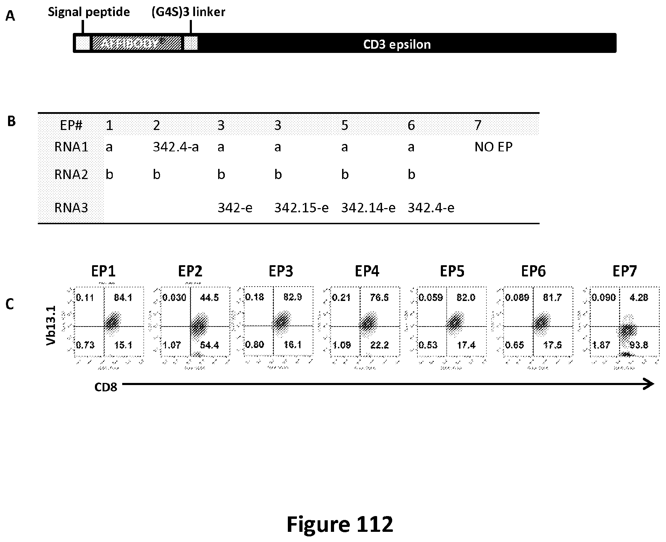

Figure 112, comprising Figures 112A-112C, is a panel of images showing TCR expression and dual targeting by co-delivering small molecule modified CD3 epsilon. Figure 112A is a diagrammatic sketch of small molecule modified CD3 epsilon by adding the small molecule **and G4S linker to the N'** terminus of either CD3 epsilon chain. Figure 112B is a table showing T cells co-electroporated with NY-ESO-1 (1G4) TCR alpha (a) and beta (b), or together with ErbB2 small molecule (342, 342.15, 342 or 342.4) modified CD3 epsilon (e). Figure 112C is a panel of images showing expression of vb13.1 detected by flow cytometry after overnight incubation.
Figure 113 is a panel of images showing CD107a up-regulation of Affi-TCR RNA electroporated T cells. T cells were co-electroporated with NY-ESO-1 (1G4) TCR alpha(a) and beta(b), or together with ErbB2 small molecule (342, 342.15, 342 or 342.4) modified CD3 epsilon(e) as indicated in Figure 101 and stimulated with tumor cells, Nalm-6-ESO (NY-eso-1+, ErbB2-), Nalm6 (NY-eso-1-, ErbB2-), SK-OV3 (NY-eso-1-, ErbB2+) or MDA231 (NY-eso-1-, ErbB2+), and assessed for CD107a expression.

### DETAILED DESCRIPTION

### Definitions

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

It is also to be understood that the terminology used herein is for the purpose of describing particular embodiments only, and is not intended to be limiting.

The articles "a" and "an" are used herein to refer to one or to more than one (*i.e.,* to at least one) of the grammatical object of the article. By way of example, "an element" means one element or more than one element.

**"About" as used herein when referring to a measurable value** such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

**"Activation," as used herein, refers to the state of a T cell that has been** sufficiently stimulated to induce detectable cellular proliferation. Activation can also be associated with induced cytokine production, and detectable effector functions. The term "activated T cells" refers to, among other things, T cells that are undergoing cell division.

**The** phrase "affinity **molecule chimeric** receptor" **refers** to **recombinant receptor** comprising an affinity molecule, such as a small molecule, antibody mimetic, Affibody^{™}, or any **fragment thereof, that binds to target protein or peptide with high** affinity. In some embodiments, the affinity molecule chimeric receptor comprises a transmembrane domain and intracellular domain. In some other embodiments, the affinity molecule chimeric receptor comprises T cell receptor domains, such as constant and variable domains.

**The term "antibody," as used herein, refers to an immunoglobulin molecule which** specifically binds with an antigen. Antibodies can be intact immunoglobulins obtained from natural sources or from recombinant sources and can be immunoreactive portions of intact immunoglobulins. Antibodies are typically tetramers of immunoglobulin molecules. Tetramers may be naturally occurring or reconstructed from single chain antibodies or antibody fragments. Antibodies also include dimers that may be naturally occurring or constructed from single chain antibodies or antibody fragments. The antibodies in the present invention may exist in a variety of forms including, for example, polyclonal antibodies, monoclonal antibodies, Fv, Fab and F(ab')₂, as well as single chain antibodies (scFv), humanized antibodies, and human antibodies (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426).

**The term "antibody fragment" refers to a region of an intact antibody and refers to** the antigenic determining variable regions of an intact antibody. Examples of antibody fragments include, but are not limited to, Fab, Fab', F(ab')₂, and Fv fragments, linear antibodies, scFv antibodies, single-domain antibodies, such as camelid antibodies (Riechmann, 1999, Journal of Immunological Methods 231:25-38), composed of either a VL or a VH domain which exhibit sufficient affinity for the target, and multispecific antibodies formed from antibody fragments. The antibody fragment also includes a human antibody or a humanized antibody or a fragment of a human antibody or a humanized antibody thereof.

An **"antibody heavy chain,"** as used herein, refers to the larger of the two types of polypeptide chains present in all antibody molecules in their naturally occurring conformations.

An **"antibody light chain," as used herein, refers** to the smaller of the two types of polypeptide chains present in all antibody molecules in their naturally occurring conformations. **α and β light chains refer to the two major antibody light chain isotypes.**

**A "bispecific antibody," as used herein, refers to an antibody having binding** specificities for at least two different antigenic epitopes. In one embodiment, the epitopes are from the same antigen. In another embodiment, the epitopes are from two different antigens. Methods for making bispecific antibodies are known in the art. For example, bispecific antibodies can be produced recombinantly using the co-expression of two immunoglobulin heavy chain/light chain pairs. See, e.g., Milstein et al. (1983) Nature 305: 537-39. Alternatively, bispecific antibodies can be prepared using chemical linkage. See, e.g., Brennan et al. (1985) Science 229:81. Bispecific antibodies include bispecific antibody fragments. See, e.g., Holliger et al. (1993) Proc. Natl. Acad. Sci. U.S.A. 90:6444-48, Gruber et al. (1994) J. Immunol. 152:5368.

**By the term "synthetic antibody" as used herein, is meant an antibody which is** generated using recombinant DNA technology, such as, for example, an antibody expressed by a bacteriophage as described herein. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using synthetic DNA or amino acid sequence technology which is available and well known in the art.

**The term "antigen" or "Ag" as used herein is defined as a molecule that provokes** an immune response. This immune response may involve either antibody production, or the activation of specific immunologically-competent cells, or both. The skilled artisan will understand that any macromolecule, including virtually all proteins or peptides, can serve as an antigen. Furthermore, antigens can be generated from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to elicit the desired immune response. Moreover, a skilled artisan will **understand that an antigen need not be encoded by a "gene" at all. It is readily apparent** that an antigen can be generated, synthesized or originate from a biological sample. Such a biological sample can include, but is not limited to a tissue sample, a tumor sample, a cell or a biological fluid.

**The term "anti-tumor effect" as used herein, refers to a biological effect which can** be manifested by a decrease in tumor volume, a decrease in the number of tumor cells, a decrease in the number of metastases, an increase in life expectancy, or amelioration of **various physiological symptoms associated with the cancerous condition. An "anti-tumor effect" can also be manifested** by the ability of the peptides, polynucleotides, cells and antibodies of the invention in prevention of the occurrence of tumor in the first place.

**The term "auto-antigen" means, in accordance with the present invention, any** self-antigen which is recognized by the immune system as being foreign. Auto-antigens comprise, but are not limited to, cellular proteins, phosphoproteins, cellular surface proteins, cellular lipids, nucleic acids, glycoproteins, including cell surface receptors.

**The term "autoimmune disease" as used herein is defined as a disorder that results** from an autoimmune response. An autoimmune disease is the result of an inappropriate and excessive response to a self-antigen. Examples of autoimmune diseases include but are not limited to, Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, among others.

**As used herein, the term "autologous" is meant to refer to any material originating** from the same individual to which it is later to be re-introduced into the individual.

**"Allogeneic" refers to a graft derived from a different animal of the same species.**

**"Xenogeneic" refers to a graft derived from an animal of a different species.**

**The phrase "bispecific affinity molecule" refers to a molecule comprising two** different binding specificities and thus is capable of binding two targets, molecules, or antigens at the same time. The bispecific affinity molecule includes an affinity domain capable of binding an antigen on a target cell and an affinity domain capable of binding an antigen on an activating T cell. In some embodiments, one or more affinity domains is a small molecule antigen binding domain, which may comprise a small molecule, **antibody mimetic, Affibody^{™}, or any fragment thereof.**

**"Bispecificity," as used herein, refers** to a molecule having binding specificities for at least two different binding epitopes. In one embodiment, the epitopes are from the same binding partner. In another embodiment, the epitopes are from two different binding partners. The molecule with bispecificity to different epitopes may include a bispecific antibody.

**The term "cancer" as used herein is defined as disease characterized by the rapid** and uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer, thyroid cancer, and the like.

**The term "chimeric antigen receptor" or "CAR," as used herein, refers to an** artificial T cell receptor that is engineered to be expressed on an immune effector cell and specifically bind an antigen. CARs may be used as a therapy with adoptive cell transfer. T cells are removed from a patient and modified so that they express the receptors specific to a particular form of antigen. In some embodiments, the CARs have been expressed with specificity to a tumor associated antigen, for example. CARs may also comprise an intracellular activation domain, a transmembrane domain and an extracellular domain comprising a tumor associated antigen binding region. In some aspects, CARs comprise fusions of single-chain variable fragments (scFv) derived monoclonal antibodies, fused to CD3 -zeta transmembrane and intracellular domain. The specificity of CAR designs may be derived from ligands of receptors (e.g., peptides). In some embodiments, a CAR can target cancers by redirecting the specificity of a T cell expressing the CAR specific for tumor associated antigens.

As used herein, **the phrase "chimeric ligand engineered activation receptor," or** "CLEAR" refers **to an engineered receptor comprising of at least an extracellular domain** for ligand recognition and an intracellular domain for activation signal transduction within a T cell. The CLEAR may be engineered to include an extracellular domain that binds to either a tumor or viral antigen or other molecule, while the intracellular domain provides an activation signal within the T cell after binding of ligand or antigen to the extracellular domain of the receptor.

**The term "chimeric membrane protein" refers to an engineered membrane** protein having an extracellular domain and intracellular domain derived from or capable of activating one or more signaling and/or receptor molecules. For example, the chimeric membrane protein described herein comprises a single chain variable fragment (scFv) directed against CD3 and an intracellular domain comprising a fragment of an intracellular domain of CD28 and 4-1BB.

**As used herein, the term "conservative sequence modifications" is intended to** refer to amino acid modifications that do not significantly affect or alter the binding characteristics of the antibody containing the amino acid sequence. Such conservative modifications include amino acid substitutions, additions and deletions. Modifications can be introduced into an antibody of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions of an antibody can be replaced with other amino acid residues from the same side chain family and the altered antibody can be tested for the ability to bind antigens using the functional assays described herein.

**"Co-stimulatory ligand," as the term** is used herein, includes a molecule on an antigen presenting cell (e.g., an aAPC, dendritic cell, B cell, and the like) that specifically binds a cognate co-stimulatory molecule on a T cell, thereby providing a signal which, in addition to the primary signal provided by, for instance, binding of a TCR/CD3 complex with an MHC molecule loaded with peptide, mediates a T cell response, including, but not limited to, proliferation, activation, differentiation, and the like. A co-stimulatory ligand can include, but is not limited to, CD7, B7-1 (CD80), B7-2 (CD86), PD-L1, PD-L2, 4-1BBL, OX40L, inducible costimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as, but not limited to, CD27, CD28, 4-1BB, OX40, CD30, CD40, PD-1, ICOS, lymphocyte function-associated antigen-1 (LFA-1), CD2, CD7, LIGHT, NKG2C, B7-H3, and a ligand that specifically binds with CD83.

**A "co-stimulatory molecule" refers to the cognate** binding partner on a T cell that specifically binds with a co-stimulatory ligand, thereby mediating a co-stimulatory response by the T cell, such as, but not limited to, proliferation. Co-stimulatory molecules include, but are not limited to an MHC class I molecule, BTLA and a Toll ligand receptor.

**A "co-stimulatory signal", as used herein, refers to a signal, which in combination** with a primary signal, such as TCR/CD3 ligation, leads to T cell proliferation and/or upregulation or downregulation of key molecules.

**The term "derived from" refers to being generated, synthesized, or originating** from a particular source, such that the derived matter is related to the source. The derived matter does not need to be identical to the particular source. In one embodiment, an antigen is derived from a protein. In another embodiment, a single-chain variable fragment is derived from a monoclonal antibody.

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

"Effective amount" or "therapeutically effective amount" are used interchangeably herein, and refer to an amount of a compound, formulation, material, or composition, as described herein effective to achieve a particular biological result or provides a therapeutic or prophylactic benefit. Such results may include, but are not limited to, anti-tumor activity as determined by any means suitable in the art.

**"Encoding" refers to the inherent property of specific sequences of nucleotides in** a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

**As used herein "endogenous" refers to any material from or produced inside an** organism, cell, tissue or system.

**As used herein, the term "exogenous" refers to any material introduced from or** produced outside an organism, cell, tissue or system.

**The term "expand" as used herein refers to increasing in number, as in an increase** in the number of T cells. In one embodiment, the T cells that are expanded ex vivo increase in number relative to the number originally present in the culture. In another embodiment, the T cells that are expanded ex vivo increase in number relative to other cell types in the culture. The term "ex vivo," as used herein, refers to cells that have been removed from a living organism, (e.g., a human) and propagated outside the organism (e.g., in a culture dish, test tube, or bioreactor).

**The term "expression" as used herein is defined as the** transcription and/or translation of a particular nucleotide sequence driven by its promoter.

**"Expression vector" refers to a vector comprising a recombinant polynucleotide** comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (*e.g.,* naked or contained in liposomes) and viruses (*e.g.,* lentiviruses, retroviruses, adenoviruses, and adeno-associated viruses) that incorporate the recombinant polynucleotide.

**"Humanized" forms of non-human** (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies are human immunoglobulins (recipient antibody) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies can comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

**"Fully human" refers to an immunoglobulin, such as an antibody, where the whole** molecule is of human origin or consists of an amino acid sequence identical to a human form of the antibody.

**"Identity" as used herein refers to** the subunit sequence identity between two polymeric molecules particularly between two amino acid molecules, such as, between two polypeptide molecules. When two amino acid sequences have the same residues at the same positions; *e.g.*, if a position in each of two polypeptide molecules is occupied by an Arginine, then they are identical at that position. The identity or extent to which two amino acid sequences have the same residues at the same positions in an alignment is often expressed as a percentage. The identity between two amino acid sequences is a direct function of the number of matching or identical positions; *e.g.,* if half (*e.g.,* five positions in a polymer ten amino acids in length) of the positions in two sequences are identical, the two sequences are 50% identical; if 90% of the positions (*e.g.*, 9 of 10), are matched or identical, the two amino acids sequences are 90% identical.

**The term "immunoglobulin" or "Ig," as used herein is defined as a class of** proteins, which function as antibodies. Antibodies expressed by B cells are sometimes referred to as the BCR (B cell receptor) or antigen receptor. The five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitourinary tracts. IgG is the most common circulating antibody. IgM is the main immunoglobulin produced in the primary immune response in most subjects. It is the most efficient immunoglobulin in agglutination, complement fixation, and other antibody responses, and is important in defense against bacteria and viruses. IgD is the immunoglobulin that has no known antibody function, but may serve as an antigen receptor. IgE is the immunoglobulin that mediates immediate hypersensitivity by causing release of mediators from mast cells and basophils upon exposure to allergen.

**The term "immune response" as used herein is defined as a cellular response to an** antigen that occurs when lymphocytes identify antigenic molecules as foreign and induce the formation of antibodies and/or activate lymphocytes to remove the antigen.

**The phrases "an immunologically effective amount", "an** anti-immune response effective **amount", "an immune response-inhibiting effective amount", or "therapeutic amount" refer to the amount of the composition of the present invention to be** administered to a subject which amount is determined by a physician, optionally in consultation with a scientist, in consideration of individual differences in age, weight, immune response, type of disease/condition, and the health of the subject (patient) so that the desired result is obtained in the subject.

**As used herein, an "instructional material"** includes a publication, a recording, a diagram, or any other medium of expression which can be used to communicate the usefulness of the compositions and methods of the invention. The instructional material of the kit of the invention may, for example, be affixed to a container which contains the nucleic acid, peptide, and/or composition of the invention or be shipped together with a container which contains the nucleic acid, peptide, and/or composition. Alternatively, the instructional material may be shipped separately from the container with the intention that the instructional material and the compound be used cooperatively by the recipient.

**"Isolated" means altered or removed from the natural state. For example, a** nucleic acid or a peptide naturally present **in a living animal is not "isolated," but the** same nucleic acid or peptide partially or completely separated from the coexisting **materials of its natural state is "isolated." An isolated nucleic acid or protein can exist in** substantially purified form, or can exist in a non-native environment such as, for example, a host cell.

**A "lentivirus" as used herein refers to a genus of the Retroviridae family.** Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses. Vectors derived from lentiviruses offer the means to achieve significant levels of gene transfer in vivo.

**By the term "modified" as used herein, is meant a changed state or structure of a** molecule or cell of the invention. Molecules may be modified in many ways, including chemically, structurally, and functionally. Cells may be modified through the introduction of nucleic acids.

**By the term "modulating," as used herein, is meant mediating a detectable** increase or decrease in the level of a response in a subject compared with the level of a response in the subject in the absence of a treatment or compound, and/or compared with the level of a response in an otherwise identical but untreated subject. The term encompasses perturbing and/or affecting a native signal or response thereby mediating a beneficial therapeutic response in a subject, preferably, a human.

In the context of the present invention, the following abbreviations for the **commonly occurring nucleic acid bases are used. "A" refers to adenosine, "C" refers to cytosine, "G" refers to guanosine, "T" refers to thymidine, and "U" refers to uridine.**

**Unless otherwise specified, a "nucleotide sequence encoding an amino acid sequence" includes all nucleotide sequences that are degenerate versions of each other** and that encode the same amino acid sequence. The phrase nucleotide sequence that encodes a protein or an RNA may also include introns to the extent that the nucleotide sequence encoding the protein may in some version contain an intron(s).

**Unless otherwise specified, a "nucleotide sequence encoding an amino acid sequence" includes all nucleotide** sequences that are degenerate versions of each other and that encode the same amino acid sequence. Nucleotide sequences that encode proteins and RNA may include introns.

**The term "operably linked" refers to functional linkage between a regulatory** sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

**The term "overexpressed" tumor antigen or "overexpression" of a tumor antigen** is intended to indicate an abnormal level of expression of a tumor antigen in a cell from a disease area like a solid tumor within a specific tissue or organ of the patient relative to the level of expression in a normal cell from that tissue or organ. Patients having solid tumors or a hematological malignancy characterized by overexpression of the tumor antigen can be determined by standard assays known in the art.

**"Parenteral" administration of an immunogenic composition includes, *e.g.*,** subcutaneous (s.c.), intravenous (i.v.), intramuscular (i.m.), or intrasternal injection, or infusion techniques.

**The term "polynucleotide" as used herein is defined as a chain of nucleotides.** Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the **monomeric "nucleotides." The monomeric nucleotides can be hydrolyzed into** nucleosides. As used herein polynucleotides include, but are not limited to, all nucleic acid sequences which are obtained by any means available in the art, including, without limitation, recombinant means, i.e., the cloning of nucleic acid sequences from a recombinant library or **a cell genome, using ordinary cloning technology and PCR^{™}, and** the like, and by synthetic means.

**As used herein, the terms "peptide," "polypeptide," and "protein" are used** interchangeably, and refer to a compound comprised of amino acid residues covalently linked by peptide bonds. A protein or peptide must contain at least two amino acids, and no limitation is placed on the maximum number of amino acids that can comprise a **protein's or peptide's sequence. Polypeptides include any peptide or protein comprising** two or more amino acids joined to each other by peptide bonds. As used herein, the term refers to both short chains, which also commonly are referred to in the art as peptides, oligopeptides and oligomers, for example, and to longer chains, which generally are **referred to in the art as proteins, of which there are many types. "Polypeptides" include,** for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

**The term "promoter" as used herein is defined as a DNA sequence recognized by** the synthetic machinery of the cell, or introduced synthetic machinery, required to initiate the specific transcription of a polynucleotide sequence.

**As used herein, the term "promoter/regulatory sequence" means a nucleic acid** sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

**A "constitutive" promoter is a nucleotide sequence which, when operably** linked with a polynucleotide which encodes or specifies a gene product, causes the gene product to be produced in a cell under most or all physiological conditions of the cell.

**An "inducible" promoter is a nucleotide sequence which, when operably linked** with a polynucleotide which encodes or specifies a gene product, causes the gene product to be produced in a cell substantially only when an inducer which corresponds to the promoter is present in the cell.

**A "tissue-specific" promoter is a nucleotide** sequence which, when operably linked with a polynucleotide encodes or specified by a gene, causes the gene product to be produced in a cell substantially only if the cell is a cell of the tissue type corresponding to the promoter.

**A "signal transduction pathway" refers to the biochemical relationship between a** variety of signal transduction molecules that play a role in the transmission of a signal from one portion of a cell to another portion of a cell. The phrase "cell surface receptor" includes molecules and complexes of molecules capable of receiving a signal and **transmitting signal across the plasma membrane of a cell. An example of a "cell surface receptor" is human FSHR.**

**"Similarity" as used herein, refers to the subunit sequence identity between two** polymeric molecules, e.g., between two nucleic acid molecules, such as, two DNA molecules or two RNA molecules, or between two polypeptide molecules. When a subunit position in both of the two molecules is occupied by the same monomeric subunit; *e.g.*, if a position in each of two DNA molecules is occupied by adenine, then they are similar at that position. The similarity between two sequences is a direct function of the number of matching or similar positions; *e.g.,* if half (*e.g.,* five positions in a polymer ten subunits in length) of the positions in two sequences are similar, the two sequences are 50% similar; if 90% of the positions (*e.g*., 9 of 10), are matched or similar, the two sequences are 90% similar.

**"Single chain antibodies" refer to antibodies** formed by recombinant DNA techniques in which immunoglobulin heavy and light chain fragments are linked to the Fv region via an engineered span of amino acids. Various methods of generating single chain antibodies are known, including those described in U.S. Pat. No. 4,694,778; Bird (1988) Science 242:423-442; Huston et al. (1988) Proc. Natl. Acad. Sci. USA 85:5879-5883; Ward et al. (1989) Nature 334:54454; Skerra et al. (1988) Science 242:1038-1041.

**The term "small molecule" refers to a peptide having** about 10, 11, 12, 13, 14, 15, 16, 17, 18, 19, or 20 amino acids with the capacity to bind a target, such as a molecule, or antigen. The small molecule comprises a low molar mass, such as less than about 12 kD, 11 kD, 10 kD, 9 kD, 8 kD, 7 kD, 6 kD, 5 kD, or any molar mass therebetween or less. In some embodiments, the small molecule is a small molecule extracellular domain of an affinity molecule chimeric receptor. In some embodiments, the small molecule is a small molecule binding domain of a bispecific affinity molecule. Small molecule may be characterized by their ability to bind a target and their structure. In some embodiments, the small molecule comprises at least one helix, such an alpha-helix, or two helices, three helices or more. The small molecule may also be chemically inert and withstand high temperatures, such as 85°C or higher.

**By the term "specifically binds," as used herein with respect to an antibody, is** meant an antibody which recognizes a specific antigen, but does not substantially recognize or bind other molecules in a sample. For example, an antibody that specifically binds to an antigen from one species may also bind to that antigen from one or more species. But, such cross-species reactivity does not itself alter the classification of an antibody as specific. In another example, an antibody that specifically binds to an antigen may also bind to different allelic forms of the antigen. However, such cross reactivity does not itself alter the classification of an antibody as specific. In some instances, the **terms "specific binding" or "specifically binding," can be used in reference to the** interaction of an antibody, a protein, or a peptide with a second chemical species, to mean that the interaction is dependent upon the presence of a particular structure (e.g., an antigenic determinant or epitope) on the chemical species; for example, an antibody recognizes and binds to a specific protein structure rather than to proteins generally. If an **antibody is specific for epitope "A", the presence** of a molecule containing epitope A (or **free, unlabeled A), in a reaction containing labeled "A" and the antibody, will reduce the** amount of labeled A bound to the antibody.

**By the term "stimulation," is meant a primary response induced by binding of a** stimulatory molecule (*e.g.,* a TCR/CD3 complex) with its cognate ligand thereby mediating a signal transduction event, such as, but not limited to, signal transduction via the TCR/CD3 complex. Stimulation can mediate altered expression of certain molecules, such as downregulation of TGF-beta, and/or reorganization of cytoskeletal structures, and the like.

**A "stimulatory molecule," as the term is used herein, means a molecule on a T** cell that specifically binds with a cognate stimulatory ligand present on an antigen presenting cell.

**A "stimulatory ligand," as used herein, means a ligand that when present on an** antigen presenting cell (*e.g.,* an aAPC, a dendritic cell, a B-cell, and the like) can specifically bind with a cognate binding partner (referred to herein as a "stimulatory molecule") on a T cell, thereby mediating a primary response by the T cell, including, but not limited to, activation, initiation of an immune response, proliferation, and the like. Stimulatory ligands are well-known in the art and encompass, *inter alia,* an MHC Class I molecule loaded with a peptide, an anti-CD3 antibody, a superagonist anti-CD28 antibody, and a superagonist anti-CD2 antibody.

**The term "subject" is intended to include living organisms in which an immune** response can be elicited (e.g., mammals). A "subject" or "patient," as used therein, may be a human or non-human mammal. Non-human mammals include, for example, livestock and pets, such as ovine, bovine, porcine, canine, feline and murine mammals. Preferably, the subject is human.

**As used herein, a "substantially purified" cell is a cell that is essentially free of** other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some embodiments, the cells are cultured *in vitro.* In other embodiments, the cells are not cultured *in vitro.*

**A "target site" or "target sequence" refers to a** genomic nucleic acid sequence that defines a region of a nucleic acid to which a binding molecule may specifically bind under conditions sufficient for binding to occur.

**As used herein, the term "T cell receptor" or "TCR" refers** to a complex of membrane proteins that participate in the activation of T cells in response to the presentation of antigen. The TCR is responsible for recognizing antigens bound to major histocompatibility complex molecules. TCR is composed of a heterodimer of an alpha **(a) and beta (β) chain, although in some cells the TCR consists of gamma and delta (γ/δ)** chains. TCRs may exist in alpha/beta and gamma/delta forms, which are structurally similar but have distinct anatomical locations and functions. Each chain is composed of two extracellular domains, a variable and constant domain. In some embodiments, the TCR may be modified on any cell comprising a TCR, including, for example, a helper T cell, a cytotoxic T cell, a memory T cell, regulatory T cell, natural killer T cell, and gamma delta T cell.

**The term "therapeutic" as used herein means a treatment and/or prophylaxis.** A therapeutic effect is obtained by suppression, remission, or eradication of a disease state.

**The term "transfected" or "transformed" or "transduced" as used herein refers to a** process by which exogenous nucleic acid is transferred or introduced into the host cell. A **"transfected" or "transformed" or "transduced" cell is one which has been transfected,** transformed or transduced with exogenous nucleic acid. The cell includes the primary subject cell and its progeny.

**To "treat" a disease as the term** is used herein, means to reduce the frequency or severity of at least one sign or symptom of a disease or disorder experienced by a subject.

**The phrase "under transcriptional control" or "operatively linked" as used herein** means that the promoter is in the correct location and orientation in relation to a polynucleotide to control the initiation of transcription by RNA polymerase and expression of the polynucleotide.

**A "vector" is a composition of matter which comprises an isolated nucleic acid** and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, lentiviral vectors, and the like.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The invention is defined in the appended claims. The references to methods of treatment by therapy in this description and examples are to be interpreted as references to modified T cells, pharmaceutical compositions and medicaments of the invention for use in those methods. The present invention includes methods and compositions for generating a modified T cell capable of expressing a bispecific antibody, as defined in the claims. In some embodiments, the invention includes a method for generating the modified T cell, as defined in the claims. Other embodiments include a modified T cell or a population of modified T cells, as defined in the claims. The bispecific antibody comprises bispecificity for an antigen on a target cell and an antigen on an activating T cell, wherein the activating T cell antigen is CD3,as defined in the claims.

### T Cell Receptor

The present invention includes a modified T cell with an exogenous T cell receptor (TCR), as defined in the claims. In one aspect, the invention includes a method for generating a modified T cell comprising expanding a population of T cells, and introducing a nucleic acid encoding a modified T cell receptor (TCR) comprising affinity for an antigen on a target cell into the expanded T cells, as defined in the claims. In this embodiment, the T cells are capable of expressing the modified TCR.

In another aspect, the invention includes a method for generating a modified T cell comprising expanding a population of T cells, and introducing a nucleic acid encoding a modified T cell receptor (TCR) comprising affinity for a surface antigen on a target cell into the expanded T cells, as defined in the claims. In this embodiment, the T cells are capable of expressing the modified TCR.

A T cell receptor is a complex of membrane proteins that participate in the activation of T cells in response to the presentation of antigen. Stimulation of the TCR is triggered by major histocompatibility complex molecules (MHC) on antigen presenting cells that present antigen peptides to the T cells and bind to the TCR complexes to induce a series of intracellular signaling cascades.

The TCR is generally composed of six different membrane bound chains that form the TCR heterodimer responsible for ligand recognition. TCRs exist in alpha/beta and gamma/delta forms, which are structurally similar but have distinct anatomical locations and functions. In one embodiment, the TCR comprises a TCR alpha and beta chain, such as the nucleic acid encoding the TCR comprises a nucleic acid encoding a TCR alpha and a TCR beta chain. In another embodiment, an alpha or beta chain or both comprises at least one N-deglycosylation.

Each chain is composed of two extracellular domains, a variable and constant domain. In one embodiment, the TCR comprises at least one murine constant region. The constant domain is proximal to the cell membrane, followed by a transmembrane domain and a short cytoplasmic tail. In one embodiment, the co-stimulatory signaling domain is a 4-1BB co-stimulatory signaling domain. The variable domain contributes to the determination of the particular antigen and MHC molecule to which the TCR has binding specificity. In turn, the specificity of a T cell for a unique antigen-MHC complex resides in the particular TCR expressed by the T cell.

Each of the constant and variable domains may include an intra-chain disulfide bond. In one embodiment, TCR comprises at least one disulfide bond. The variable domains include the highly polymorphic loops analogous to the complementarity determining regions (CDRs) of antibodies. The diversity of TCR sequences is generated via somatic rearrangement of linked variable (V), diversity (D), joining (J), and constant genes.

Functional alpha and gamma chain polypeptides are formed by rearranged V-J-C regions, whereas beta and delta chains consist of V-D-J-C regions. The extracellular constant domain includes a membrane proximal region and an immunoglobulin region.

In one embodiment, the TCR includes a wildtype TCR, a high affinity TCR, and a chimeric TCR. When the TCR is modified, it may have higher affinity for the target cell antigen than a wildtype TCR. In embodiments where the TCR is a chimeric TCR, the TCR may include chimeric domains, such as the TCR comprises a co-stimulatory **signaling domain at a C' terminal of at least one of the chains. In other embodiment, the** TCR may include a modified chain, such as a modified alpha or beta chain. Such modifications may include, but are not limited to, N-deglycosylation, altered domain (such as an engineered variable region to target a specific antigen or increase affinity), addition of one or more disulfide bonds, entire or fragment of a chain derived from a different species, and any combination thereof.

Examples of target cell associated antigens are described elsewhere herein, all of which may be targeted by the TCR of the present invention, as defined in the claims.

In one aspect, the invention includes a population of modified T cells comprising an electroporated RNA encoding a modified T cell receptor (TCR) comprising affinity for an antigen on a target cell, wherein the population of T cells was expanded prior to electroporation with the TCR RNA, as defined in the claims.

In another aspect, the invention includes a modified T cell comprising an exogenous nucleic acid encoding a T cell receptor (TCR) comprising affinity for an antigen on a target cell; and an electroporated nucleic acid encoding a costimulatory molecule, wherein the T cell expresses the TCR and co-stimulatory molecule, as defined in the claims. The co-stimulatory molecule may be selected from the group consisting of CD3, CD27, CD28, CD83, CD86, CD127, 4-1BB, 4-1BBL, PD1 and PD1L.

In another aspect, the invention includes a modified T cell comprising an exogenous nucleic acid encoding a T cell receptor (TCR) comprising affinity for a surface antigen on a target cell; and an electroporated nucleic acid encoding a costimulatory molecule, wherein the T cell expresses the TCR and co-stimulatory molecule, as defined in the claims.

In yet another aspect, the invention includes a modified T cell comprising an electroporated RNA encoding a modified T cell receptor (TCR), wherein the modified T cell receptor (TCR) comprises affinity for an antigen on a target cell and the T cell was expanded prior to electroporation with the TCR RNA, as defined in the claims.

In yet another aspect, the invention includes a modified T cell comprising an electroporated RNA encoding a modified T cell receptor (TCR), wherein the modified T cell receptor (TCR) comprises affinity for a surface antigen on a target cell and the T cell was expanded prior to electroporation with the TCR RNA, as defined in the claims.

In one embodiment, the invention includes introducing a nucleic acid encoding a modified T cell receptor (TCR) comprising affinity for an antigen on a target cell into the expanded T cells, as defined in the claims. In this embodiment, the T cells are capable of expressing the modified TCR.

Techniques for engineering and expressing T cell receptors include, but are not limited to, the production of TCR heterodimers which include the native disulphide bridge which connects the respective subunits (Garboczi, et al., (1996), Nature 384(6605): 134-41; Garboczi, et al., (1996), J Immunol 157(12): 5403-10; Chang et al., (1994), PNAS USA 91: 11408-11412; Davodeau et al., (1993), J. Biol. Chem. 268(21): 15455-15460; Golden et al., (1997), J. Imm. Meth. 206: 163-169; U.S. Pat. No. 6,080,840).

In one embodiment, the TCR comprises specificity to a target cell antigens as defined in the claims. The target cell antigen may include any type of protein associated with a target cell. For example, the target cell antigen may be chosen to recognize a particular disease state of the target cell. Thus examples of cell surface markers that may act as ligands for the antigen binding domain of the TCR including those associated with viral, bacterial and parasitic infections, autoimmune disease and cancer cells. In one embodiment, the target cell antigen includes any tumor associated antigen (TAA) and viral antigen, or any fragment thereof.

The target cell antigen may include any protein that may be processed and presented by major histocompability complexes. For example, the target antigen may be associated with a particular disease state. Thus examples of cell markers that may act as targets of the TCR include those associated with viral, bacterial and parasitic infections, autoimmune disease and cancer cells. In one embodiment, the target antigen includes any of tumor associated antigens (TAA) and viral antigens, or any fragment thereof

### Bispecific Antibodies

The invention is defined in the appended claims. The present invention includes a bispecific antibody, as defined in the claims. A bispecific antibody comprises two different binding specificities and thus binds to two different antigens.

The bispecific antibody comprises a first antigen binding domain that binds to a first antigen and a second antigen binding domain that binds to a second antigen.

The bispecific antibody comprises an antigen binding domain comprising a first and a second single chain variable fragment (scFv) molecules. In such an embodiment, the first and second scFv bind an antigen on a target cell and an antigen on an activating T cell. The first scFv molecule is specific for at least one antigen on a target cell and the second scFv molecule is specific for an antigen on an activating T cell, wherein the activating T cell antigen is CD3. In these examples, the bispecific antibody recognizes a T cell antigen and is referred to as a Bispecific T Cell Engager (BiTE).

However, the present invention is not limited by the use of any particular bispecific antibody. Rather, any bispecific antibody or BiTE can be used. The bispecific antibody or BiTE molecule may also be expressed as a membrane protein with specificity for at least one target cell associated antigen. Examples of target cell associated antigens are described elsewhere herein, all of which may be targeted by the bispecific antibody of the present invention. Techniques for making human and humanized antibodies are also described elsewhere herein. In one embodiment, the bispecific antibody or BiTE molecule comprises a bispecific antigen binding domain. In this embodiment, the bispecific antigen binding domain includes a synthetic antibody, human antibody, a humanized antibody, single chain variable fragment, single domain antibody, an antigen binding fragment thereof, and any combination thereof.

In one aspect, the invention includes a population of modified T cells comprising a nucleic acid, such as RNA, encoding a bispecific antibody comprising bispecificity for an antigen on a target cell and an antigen on the T cell, as defined in the claims. The population of T cells is expanded prior to introduction of the nucleic acid.

In one aspect, the invention includes a population of modified T cells comprising an electroporated mRNA encoding a bispecific antibody comprising bispecificity for an antigen on a target cell and an antigen on an activating T cell, as defined in the claims. The population of T cells was expanded prior to the BiTE electroporation.

In another aspect, the invention includes a modified T cell comprising an electroporated mRNA encoding a bispecific T-cell engager (BiTE) molecule. In this embodiment, the BiTE molecule comprises bispecificity for an antigen on a target cell and an antigen on an activating T cell, wherein the activating T cell antigen is CD3.

In yet another aspect, the invention includes a population of modified T cells comprising a nucleic acid, such as RNA, encoding a bispecific antibody comprising bispecificity for an antigen on a target cell and an antigen on the T cell, as defined in the claims. The population of T cells can be expanded prior to introduction of the nucleic acid.

In yet another aspect, the invention includes a modified T cell comprising an electroporated RNA encoding a bispecific antibody, as defined in the claims. The bispecific antibody comprises bispecificity for an antigen on a target cell and an antigen on an activating T cell, wherein the activating T cell antigen is CD3. This embodiment also includes the T cell being expanded prior to electroporation with the bispecific antibody mRNA.

In one embodiment, the invention includes electroporating the expanded T cells with mRNA encoding a bispecific antibody, such as a BiTE molecule, as defined in the claims. In another embodiment, the invention includes introducing the expanded T cells with a nucleic acid encoding a bispecific antibody, such as a BiTE molecule. In such embodiments, theT cells are capable of expressing the bispecific antibody. Techniques for engineering and expressing bispecific antibodies include, but are not limited to, recombinant co-expression of two immunoglobulin heavy chain-light chain pairs having different specificities (see Milstein and Cuello, Nature 305: 537 (1983), WO 93/08829, and Traunecker et al., EMBO J. 10: 3655 (1991)), and "knob-in-hole" engineering (see, e.g., U.S. Pat. No. 5,731,168). Multi-specific antibodies may also be made by engineering electrostatic steering effects for making antibody Fc-heterodimeric molecules (WO 2009/089004A1); cross-linking two or more antibodies or fragments (see, e.g., U.S. Pat. No. 4,676,980, and Brennan et al., Science 229:81 (1985)); using leucine zippers to produce bispecific antibodies (see, e.g., Kostelny et al., J. Immunol. 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see, e.g. Gruber et al., J. Immunol., 152:5368 (1994)); and preparing trispecific antibodies as described, e.g., in Tutt et al. J. Immunol. 147: 60 (1991). Engineered antibodies with three or more functional antigen binding sites, including "Octopus antibodies," are also included herein (see, e.g. US 2006/0025576A1). Bispecific antibodies can be constructed by linking two different antibodies, or portions thereof. For example, a bispecific antibody can comprise Fab, F(ab')₂, Fab', scFv, and sdAb from two different antibodies.

In one embodiment, the bispecific antibody and/or BiTE molecule comprises a bispecific antigen binding domain that comprises a first and a second single chain variable fragment (scFv) molecules, as defined in the claims. In such an embodiment, the bispecific antigen binding domain can comprise bispecificity for an antigen on a target cell and an antigen on the T cell, such as the first scFv molecule is specific for at least one antigen on a target cell and the second scFv molecule is specific for at least one antigen on the T cell, as defined in the claims. The bispecific antigen binding domain can comprise bispecificity for an antigen on a target cell and an antigen on an activating T cell, such as the first scFv molecule is specific for at least one antigen on a target cell and the second scFv molecule is specific for at least one antigen on an activating T cell, wherein the activating T cell antigen is CD3 . In another embodiment, the bispecific antibody is expressed as a membrane protein.

In one embodiment, the bispecific antibody comprises specificity to a target cell antigen. The target cell antigen may include the same target cell antigen that the T cell receptor binds or may include a different target cell antigen. The target cell antigen may include any type of ligand that defines the target cell. For example, the target cell antigen may be chosen to recognize a ligand that acts as a cell marker on target cells associated with a particular disease state. Thus examples of cell markers that may act as ligands for the antigen moiety domain in a BiTE molecule, including those associated with viral, bacterial and parasitic infections, autoimmune disease and cancer cells.

In one embodiment, the target cell antigen includes any tumor associated antigen (TAA) and viral antigen, or any fragment thereof. In this embodiment, the bispecific antibody and/or BiTE molecule comprises an antibody, such as a synthetic antibody, human antibody, a humanized antibody, single chain variable fragment, single domain antibody, an antigen binding fragment thereof, and any combination thereof, that specifically binds to the target cell antigen. Examples of the activating T cell antigen may include tumor antigens, viral antigens, and fragments thereof. Examples of the target cell antigen may include tumor antigens, viral antigens, and fragments thereof.

In one embodiment, the bispecific antibody and/or BiTE molecule comprises specificity to at least one antigen on an activating T cell, wherein the activating T cell antigen is CD3, as defined in the appended claims.

In one embodiment, the activating T cell antigen is CD3, or any fragment thereof. In this embodiment, the bispecific antibody and/or BiTE molecule comprises an antibody, such as a synthetic antibody, human antibody, a humanized antibody, single chain variable fragment, single domain antibody, an antigen binding fragment thereof, and any combination thereof, that specifically binds to the activating T cell antigen.

### Co-Stimulatory Molecule

In one embodiment, the modified T cell of the invention further includes a nucleic acid encoding a co-stimulatory molecule, such that the modified T cell expresses the co-stimulatory molecule, as defined in the claims. The nucleic acid may be introduced into the T cell by transducing the T cell, transfecting the T cell, or electroporating the T cell. In another embodiment, the co-stimulatory molecule is selected from CD3, CD27, CD28, CD83, CD86, CD127, 4-1BB, 4-1BBL, PD1 and PD1L. In yet another embodiment, the co-stimulatory molecule is CD3, and CD3 comprises at least two different CD3 chains, such as CD3 zeta and CD3 epsilon chains. In an exemplary embodiment, RNA encoding the co-stimulatory molecule, such as CD3, is electroporated into the T cell or cell. In another embodiment, the nucleic acid encoding the costimulatory molecule, such as CD3 RNA, is co-electroporated with another nucleic acid, such as the nucleic acid or RNA encoding the affinity molecule chimeric receptor.

In another embodiment, the TCR is modified to include a co-stimulatory domain selected from at least one domain from CD3, CD27, CD28, CD83, CD86, CD127, 4-1BB, 4-1BBL, PD1 and PD1L.

### Introduction of Nucleic Acids

Methods of introducing nucleic acids into a cell include physical, biological and chemical methods. Physical methods for introducing a polynucleotide, such as RNA, into a host cell include calcium phosphate precipitation, lipofection, particle bombardment, microinjection, electroporation, and the like. RNA can be introduced into target cells using commercially available methods which include electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany). RNA can also be introduced into cells using cationic liposome mediated transfection using lipofection, using polymer encapsulation, using peptide mediated transfection, or using biolistic particle delivery systems such as "gene guns" (see, for example, Nishikawa, et al. Hum Gene Ther., 12(8):861-70 (2001).

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present invention, in order to confirm the presence of the nucleic acids in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the invention.

In one embodiment, a nucleic acid encoding a T cell receptor (TCR) comprising affinity for an antigen on a target cell is introduced into the T cells. The nucleic acid may be introduced by any means, such as transducing the expanded T cells, transfecting the expanded T cells, and electroporating the expanded T cells.

In another embodiment, a nucleic acid nucleic acid encoding an affinity molecule chimeric receptor or a bispecific affinity molecule is introduced by a method selected from the group consisting of transducing the population of cells, transfecting the population of cells, and electroporating the population of cells.

In another embodiment, a nucleic acid encoding a bispecific affinity molecule comprising an affinity domain capable of binding an antigen on a target cell and an affinity domain capable of binding an antigen on an activating T cell into a population of cells by a method selected from the group consisting of transducing the population of cells, transfecting the population of cells, and electroporating the population of cells.

In yet another embodiment, a nucleic acid encoding a bispecific antibody, such as a BiTE molecule, is introduced into the T cells. The nucleic acid may be introduced by any means, such as transducing the expanded T cells, transfecting the expanded T cells, and electroporating the expanded T cells.

### RNA

In one embodiment, the nucleic acids introduced into the T cell are RNA. In another embodiment, the RNA is mRNA that comprises in vitro transcribed RNA or synthetic RNA. The RNA is produced by in vitro transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. The desired template for in vitro transcription is a chimeric membrane protein. By way of example, the template encodes an antibody, a fragment of an antibody or a portion of an antibody. By way of another example, the template comprises an extracellular domain comprising a single chain variable domain of an antibody, such as anti-CD3, and an intracellular domain of a co-stimulatory molecule. In one embodiment, the template for the RNA chimeric membrane protein encodes a chimeric membrane protein comprising an extracellular domain comprising an antigen binding domain derived from an antibody to a co-stimulatory molecule, and an intracellular domain derived from a portion of an intracellular domain of CD28 and 4-1BB.

PCR can be used to generate a template for in vitro transcription of mRNA which is then introduced into cells. Methods for performing PCR are well known in the art. Primers for use in PCR are designed to have regions that are substantially complementary to regions of the DNA to be used as a template for the PCR. "Substantially complementary", as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a gene that is normally transcribed in cells (the open reading frame), including 5' and 3' UTRs. The primers can also be designed to amplify a portion of a gene that encodes a particular domain of interest. In one embodiment, the primers are designed to amplify the coding region of a human cDNA, including all or portions of the 5' and 3' UTRs. Primers useful for PCR are generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Chemical structures that have the ability to promote stability and/or translation efficiency of the RNA may also be used. The RNA preferably has 5' and 3' UTRs. In one embodiment, the 5' UTR is between zero and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the gene of interest. Alternatively, UTR sequences that are not endogenous to the gene of interest can be added by incorporating the UTR sequences into the forward and reverse primers or by any other modifications of the template. The use of UTR sequences that are not endogenous to the gene of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous gene. Alternatively, when a 5' UTR that is not endogenous to the gene of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be derived from an RNA virus whose RNA genome is stable in cells. In other embodiments various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In one embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (size can be 50-5000 T), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines.

Poly(A) tails of RNAs can be further extended following in vitro transcription with the use of a poly(A) polymerase, such as E. coli polyA polymerase (E-PAP). In one embodiment, increasing the length of a poly(A) tail from 100 nucleotides to between 300 and 400 nucleotides results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

The disclosed methods can be applied to the modulation of T cell activity in basic research and therapy, in the fields of cancer, stem cells, acute and chronic infections, and autoimmune diseases, including the assessment of the ability of the genetically modified T cell to kill a target cancer cell.

The methods also provide the ability to control the level of expression over a wide range by changing, for example, the promoter or the amount of input RNA, making it possible to individually regulate the expression level. Furthermore, the PCR-based technique of mRNA production greatly facilitates the design of the chimeric receptor mRNAs with different structures and combination of their domains. For example, varying of different intracellular effector/costimulator domains on multiple chimeric receptors in the same cell allows determination of the structure of the receptor combinations which assess the highest level of cytotoxicity against multi-antigenic targets, and at the same time lowest cytotoxicity toward normal cells.

One advantage of RNA transfection methods of the invention is that RNA transfection is essentially transient and a vector-free. A RNA transgene can be delivered to a lymphocyte and expressed therein following a brief in vitro cell activation, as a minimal expressing cassette without the need for any additional viral sequences. Under these conditions, integration of the transgene into the host cell genome is unlikely. Cloning of cells is not necessary because of the efficiency of transfection of the RNA and its ability to uniformly modify the entire lymphocyte population.

Genetic modification of T cells with in vitro-transcribed RNA (IVT-RNA) makes use of two different strategies both of which have been successively tested in various animal models. Cells are transfected with in vitro-transcribed RNA by means of lipofection or electroporation. It is desirable to stabilize IVT-RNA using various modifications in order to achieve prolonged expression of transferred IVT-RNA.

Some IVT vectors are known in the literature which are utilized in a standardized manner as template for in vitro transcription and which have been genetically modified in such a way that stabilized RNA transcripts are produced. Currently protocols used in the art are based on a plasmid vector with the following structure: a 5' RNA polymerase promoter enabling RNA transcription, followed by a gene of interest which is flanked either 3' and/or 5' by untranslated regions (UTR), and a 3' polyadenyl cassette containing 50-70 A nucleotides. Prior to in vitro transcription, the circular plasmid is linearized downstream of the polyadenyl cassette by type II restriction enzymes (recognition sequence corresponds to cleavage site). The polyadenyl cassette thus corresponds to the later poly(A) sequence in the transcript. As a result of this procedure, some nucleotides remain as part of the enzyme cleavage site after linearization and extend or mask the poly(A) sequence at the 3' end. It is not clear, whether this nonphysiological overhang affects the amount of protein produced intracellularly from such a construct.

RNA has several advantages over more traditional plasmid or viral approaches. Gene expression from an RNA source does not require transcription and the protein product is produced rapidly after the transfection. Further, since the RNA has to only gain access to the cytoplasm, rather than the nucleus, and therefore typical transfection methods result in an extremely high rate of transfection. In addition, plasmid based approaches require that the promoter driving the expression of the gene of interest be active in the cells under study.

In another aspect, the RNA construct is delivered into the cells by electroporation. See, e.g., the formulations and methodology of electroporation of nucleic acid constructs into mammalian cells as taught in US 2004/0014645, US 2005/0052630A1, US 2005/0070841A1, US 2004/0059285A1, US 2004/0092907A1. The various parameters including electric field strength required for electroporation of any known cell type are generally known in the relevant research literature as well as numerous patents and applications in the field. See e.g., U.S. Pat. No. 6,678,556, U.S. Pat. No. 7,171,264, and U.S. Pat. No. 7,173,116. Apparatus for therapeutic application of electroporation are available commercially, e.g., the MedPulser^{™} DNA Electroporation Therapy System (Inovio/Genetronics, San Diego, Calif.), and are described in patents such as U.S. Pat. No. 6,567,694; U.S. Pat. No. 6,516,223, U.S. Pat. No. 5,993,434, U.S. Pat. No. 6,181,964, U.S. Pat. No. 6,241,701, and U.S. Pat. No. 6,233,482; electroporation may also be used for transfection of cells in vitro as described e.g. in US20070128708A1. Electroporation may also be utilized to deliver nucleic acids into cells in vitro. Accordingly, electroporation-mediated administration into cells of nucleic acids including expression constructs utilizing any of the many available devices and electroporation systems known to those of skill in the art presents an exciting new means for delivering an RNA of interest to a target cell.

In some embodiments, the RNA encoding a TCR is electroporated into the cells. In one embodiment, the RNA encoding the TCR is in vitro transcribed RNA. In some embodiments, the mRNA encoding bispecific antibodies are electroporated into the cells. In another embodiment, the mRNA encoding bispecific antibodies is in vitro transcribed mRNA.

In some embodiments, the RNA encoding bispecific antibodies is electroporated into the cells. In one embodiment, the RNA encoding bispecific antibodies is in vitro transcribed RNA.

In one embodiment, the method includes electroporating a RNA encoding a TCR alpha and beta chain. The TCR alpha and beta chain can be encoded on the same or separate RNAs, such as co-electroporating a RNA encoding the TCR alpha chain and a separate RNA encoding the TCR beta chain. When the alpha and beta are encoded by separate RNAs, the RNA may be co-electroporated.

In some embodiments, the method further includes electroporating a nucleic acid encoding a bispecific antibody or BiTE molecule. The bispecific antibody nucleic acid may be co-electroporated with the TCR RNA.

In another embodiment, the method may further include electroporating a nucleic acid encoding a costimulatory molecule. The costimulatory molecule nucleic acid may be co-electroporated with the TCR RNA.

### Chimeric Membrane Protein

In one embodiment, the modified T cells are expanded prior to introduction of the nucleic acid, as defined in the claims. In another embodiment, the modified T cells are expanded prior to electroporation with RNA encoding the TCR or the bispecific antibody or BiTE molecule. The expansion of the T cells may include electroporating the T cells with RNA encoding a chimeric membrane protein, and culturing the electroporated T cells. The chimeric membrane protein of the invention comprises an extracellular and intracellular domain. The extracellular domain comprises a target-specific binding element, such as an antibody. In one embodiment, the extracellular domain of the chimeric membrane protein targets a molecule on a T cell that includes but is not limited to TCR, CD3, CD28, and the like.

### Extracellular Domain

The present invention includes an extracellular domain comprising an antigen binding domain comprising an antibody or fragment thereof directed against a molecule on T cells, as defined in the claims. The molecule can include any molecule that co-stimulates T cells, such as, but not limited to, TCR, CD3, CD28, or a combination thereof. In one embodiment, the extracellular domain can include an antigen binding domain comprising the CD3 binding domain of an anti-CD3 antibody, an anti-TCR antibody, anti-CD28 antibody, or a combination thereof.

In another embodiment, the extracellular domain can include any fragment of an antibody that binds to antigen including, but not limited to, the antigen binding domain of a synthetic antibody, human antibody, humanized antibody, single domain antibody, single chain variable fragments, and fragments thereof. In some instances, it is beneficial for the extracellular domain to be derived from the same species in which the chimeric membrane protein will ultimately be used in. For example, for use in humans, it may be beneficial for the extracellular domain of the chimeric membrane protein to comprise a human antibody or fragment thereof. Thus, in one embodiment, the extracellular domain comprises a human antibody or a fragment thereof.

In one embodiment, the antibody is a synthetic antibody, human antibody, humanized antibody, single domain antibody, single chain variable fragment, and antigen-binding fragments thereof.

### Intracellular Domain

The intracellular domain or cytoplasmic domain comprises a costimulatory signaling region. The costimulatory signaling region refers to an intracellular domain of a costimulatory molecule. Costimulatory molecules are cell surface molecules other than antigen receptors or their ligands that are required for an efficient response of lymphocytes to antigen.

The cytoplasmic domain or the intracellular signaling domain of the chimeric membrane protein is responsible for activation of at least one of effector functions of the T cell. The term "effector function" refers to a specialized function of a cell. Effector function of a T cell, for example, may be cytolytic activity or helper activity including the secretion of cytokines. Thus the term "intracellular signaling domain" refers to the portion of a protein which transduces the effector function signal and directs the cell to perform a specialized function. While usually the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

Nonlimiting examples of intracellular signaling domains for use in the chimeric membrane protein include any fragment of the intracellular domain of CD28, 4-1BB, T cell receptor (TCR), co-stimulatory molecules, any derivative or variant of these sequences, any synthetic sequence that has the same functional capability, and any combination thereof.

### Other Domains of the Chimeric Membrane Protein

Between the extracellular domain and the transmembrane domain of the chimeric membrane protein, or between the cytoplasmic domain and the transmembrane domain of the chimeric membrane protein, there may be incorporated a spacer domain. As used herein, the term "spacer domain" generally means any oligo- or polypeptide that functions to link the transmembrane domain to, either the extracellular domain or, the cytoplasmic domain in the polypeptide chain. A spacer domain may comprise up to 300 amino acids, preferably 10 to 100 amino acids and most preferably 25 to 50 amino acids.

In some embodiments, the chimeric membrane protein further comprises a transmembrane domain. In some embodiment, the chimeric membrane protein further comprises a hinge domain. In one embodiment, the mRNA encoding the chimeric membrane protein further comprises a transmembrane and hinge domain, such as a CD28 transmembrane domain and a CD8-alpha hinge domain.

### Human Antibodies

For in vivo use of antibodies in humans, it may be preferable to use human antibodies. Completely human antibodies are particularly desirable for therapeutic treatment of human subjects. Human antibodies can be made by a variety of methods known in the art including phage display methods using antibody libraries derived from human immunoglobulin sequences, including improvements to these techniques. See, also, U.S. Pat. Nos. 4,444,887 and 4,716,111; and PCT publications WO 98/46645, WO 98/50433, WO 98/24893, WO98/16654, WO 96/34096, WO 96/33735, and WO 91/10741. A human antibody can also be an antibody wherein the heavy and light chains are encoded by a nucleotide sequence derived from one or more sources of human DNA.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. For example, it has been described that the homozygous deletion of the antibody heavy chain joining region (JH) gene in chimeric and germ-line mutant mice results in complete inhibition of endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, e.g., all or a portion of a polypeptide of the invention. Antibodies directed against the target of choice can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies, including, but not limited to, IgG1 (gamma 1) and IgG3. For an overview of this technology for producing human antibodies, see, Lonberg and Huszar (Int. Rev. Immunol., 13:65-93 (1995)). For a detailed discussion of this technology for producing human antibodies and human monoclonal antibodies and protocols for producing such antibodies, see, e.g., PCT Publication Nos. WO 98/24893, WO 96/34096, and WO 96/33735; and U.S. Pat. Nos. 5,413,923; 5,625,126; 5,633,425; 5,569,825; 5,661,016; 5,545,806; 5,814,318; and 5,939,598. In addition, companies such as Abgenix, Inc. (Freemont, Calif.) and Genpharm (San Jose, Calif.) can be engaged to provide human antibodies directed against a selected antigen using technology similar to that described above. For a specific discussion of transfer of a human germ-line immunoglobulin gene array in germ-line mutant mice that will result in the production of human antibodies upon antigen challenge see, e.g., Jakobovits et al., Proc. Natl. Acad. Sci. USA, 90:2551 (1993); Jakobovits et al., Nature, 362:255-258 (1993); Bruggermann et al., Year in Immunol., 7:33 (1993); and Duchosal et al., Nature, 355:258 (1992).

Human antibodies can also be derived from phage-display libraries (Hoogenboom et al., J. Mol. Biol., 227:381 (1991); Marks et al., J. Mol. Biol., 222:581-597 (1991); Vaughan et al., Nature Biotech., 14:309 (1996)). Phage display technology (McCafferty et al., Nature, 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, e.g., Johnson, Kevin S, and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of unimmunized mice. A repertoire of V genes from unimmunized human donors can be constructed and antibodies to a diverse array of antigens (including self-antigens) can be isolated essentially following the techniques described by Marks et al., J. Mol. Biol., 222:581-597 (1991), or Griffith et al., EMBO J., 12:725-734 (1993). See, also, U.S. Pat. Nos. 5,565,332 and 5,573,905.

Human antibodies may also be generated by in vitro activated B cells (see, U.S. Pat. Nos. 5,567,610 and 5,229,275). Human antibodies may also be generated in vitro using hybridoma techniques such as, but not limited to, that described by Roder et al. (Methods Enzymol., 121:140-167 (1986)).

### Humanized Antibodies

Alternatively, in some embodiments, a non-human antibody is humanized, where specific sequences or regions of the antibody are modified to increase similarity to an antibody naturally produced in a human. In one embodiment, the antigen binding domain is humanized.

A "humanized" antibody retains a similar antigenic specificity as the original antibody. However, using certain methods of humanization, the affinity and/or specificity of binding of the antibody for human CD3 antigen may be increased using methods of "directed evolution," as described by Wu et al., J. Mol. Biol., 294:151 (1999).

A humanized antibody has one or more amino acid residues introduced into it from a source which is nonhuman. These nonhuman amino acid residues are often referred to as "import" residues, which are typically taken from an "import" variable domain. Thus, humanized antibodies comprise one or more CDRs from nonhuman immunoglobulin molecules and framework regions from human. Humanization of antibodies is well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640). In such humanized chimeric antibodies, substantially less than an intact human variable domain has been substituted by the corresponding sequence from a nonhuman species. In practice, humanized antibodies are typically human antibodies in which some CDR residues and possibly some FR residues are substituted by residues from analogous sites in rodent antibodies. Humanization of antibodies can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987). Another method uses a particular framework derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993).

Antibodies can be humanized with retention of high affinity for the target antigen and other favorable biological properties. According to one aspect of the invention, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind the target antigen. In this way, FR residues can be selected and combined from the recipient and import sequences so that the desired antibody characteristic, such as increased affinity for the target antigen, is achieved. In general, the CDR residues are directly and most substantially involved in influencing antigen binding.

### Sources of T Cells

Prior to expansion, a source of T cells is obtained from a subject. Non-limiting examples of subjects include humans, dogs, cats, mice, rats, and transgenic species thereof. Preferably, the subject is a human. T cells can be obtained from a number of sources, including peripheral blood mononuclear cells, bone marrow, lymph node tissue, spleen tissue, umbilical cord, and tumors. In certain embodiments, any number of T cell lines available in the art, may be used. In certain embodiments, T cells can be obtained from a unit of blood collected from a subject using any number of techniques known to the skilled artisan, such as Ficoll separation. In one embodiment, cells from the circulating blood of an individual are obtained by apheresis or leukapheresis. The apheresis product typically contains lymphocytes, including T cells, monocytes, granulocytes, B cells, other nucleated white blood cells, red blood cells, and platelets. The cells collected by apheresis may be washed to remove the plasma fraction and to place the cells in an appropriate buffer or media, such as phosphate buffered saline (PBS) or wash solution lacks calcium and may lack magnesium or may lack many if not all divalent cations, for subsequent processing steps. After washing, the cells may be resuspended in a variety of biocompatible buffers, such as, for example, Ca-free, Mg-free PBS. Alternatively, the undesirable components of the apheresis sample may be removed and the cells directly resuspended in culture media.

In another embodiment, T cells are isolated from peripheral blood by lysing the red blood cells and depleting the monocytes, for example, by centrifugation through a PERCOLL^{™} gradient. Alternatively, T cells can be isolated from umbilical cord. In any event, a specific subpopulation of T cells can be further isolated by positive or negative selection techniques.

The cord blood mononuclear cells so isolated can be depleted of cells expressing certain antigens, including, but not limited to, CD34, CD8, CD14, CD19 and CD56. Depletion of these cells can be accomplished using an isolated antibody, a biological sample comprising an antibody, such as ascites, an antibody bound to a physical support, and a cell bound antibody.

Enrichment of a T cell population by negative selection can be accomplished using a combination of antibodies directed to surface markers unique to the negatively selected cells. A preferred method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail typically includes antibodies to CD14, CD20, CD11b, CD16, HLA-DR, and CD8.

For isolation of a desired population of cells by positive or negative selection, the concentration of cells and surface (e.g., particles such as beads) can be varied. In certain embodiments, it may be desirable to significantly decrease the volume in which beads and cells are mixed together (i.e., increase the concentration of cells), to ensure maximum contact of cells and beads. For example, in one embodiment, a concentration of 2 billion cells/ml is used. In one embodiment, a concentration of 1 billion cells/ml is used. In a further embodiment, greater than 100 million cells/ml is used. In a further embodiment, a concentration of cells of 10, 15, 20, 25, 30, 35, 40, 45, or 50 million cells/ml is used. In yet another embodiment, a concentration of cells from 75, 80, 85, 90, 95, or 100 million cells/ml is used. In further embodiments, concentrations of 125 or 150 million cells/ml can be used. Using high concentrations can result in increased cell yield, cell activation, and cell expansion.

T cells can also be frozen after the washing step, which does not require the monocyte-removal step. While not wishing to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, in a non-limiting example, one method involves using PBS containing 20% DMSO and 8% human serum albumin, or other suitable cell freezing media. The cells are then frozen to -80°C at a rate of 1° per minute and stored in the vapor phase of a liquid nitrogen storage tank. Other methods of controlled freezing may be used as well as uncontrolled freezing immediately at -20°C or in liquid nitrogen.

In one embodiment, the population of T cells is comprised within cells such as peripheral blood mononuclear cells, cord blood cells, a purified population of T cells, and a T cell line. In another embodiment, peripheral blood mononuclear cells comprise the population of T cells. In yet another embodiment, purified T cells comprise the population of T cells.

In another embodiment, the T cells are isolated from cells such as peripheral blood mononuclear cells, cord blood cells, a purified population of T cells, and a T cell line. In another embodiment, the method described herein further comprises isolating a population of T cells from peripheral blood mononuclear cells, cord blood cells, a purified population of T cells, or a T cell line.

### Expansion of T Cells

The invention is defined in the appended claims. In one embodiment, expanding the T cells further includes culturing the electroporated T cells, as defined in the claims. In another embodiment, the source of the T cells to be electroporated and expanded is peripheral blood mononuclear cells.

Generally, T cells are expanded by contact with a surface having attached thereto an agent that stimulates a CD3/TCR complex associated signal and a ligand that stimulates a co-stimulatory molecule on the surface of the T cells. The present invention comprises a novel method of expanding a population of electroporated T cells comprising culturing the electroporated population, wherein the electroporated T cells within the population expand at least 10 fold. Expression of the chimeric membrane protein allows interaction with other cells in the population to stimulate and activate expansion of the electroporated T cells. In one embodiment, at least one cell in the population of cells expresses CD3. Not being held to any particular theory, the cells that express CD3 may come into contact and bind with the chimeric membrane protein that is expressed on the surface of the electroporated cells. At least one cell expressing the chimeric membrane protein may interact with another cell expressing CD3. This interaction may stimulate expansion of the electroporated T cells.

Alternatively, the cells can be ex vivo expanded using a method described in U.S. Pat. No. 5,199,942. Expansion, such as described in U.S. Pat. No. 5,199,942 can be an alternative or in addition to other methods of expansion described herein. Briefly, ex vivo culture and expansion of T cells comprises the addition to the cellular growth factors, such as those described in U.S. Pat. No. 5,199,942, or other factors, such as flt3-L, IL-1, IL-2, IL-3 and c-kit ligand, for example as those described in Dudley et al., J. Immunol., 26(4):332-342, 2003, for a Rapid Expansion Protocol (REP). In one embodiment, expanding the T cells comprises culturing the T cells with a factor selected from the group consisting of flt3-L, IL-1, IL-2, IL-3 and c-kit ligand.

As demonstrated by the data disclosed herein, expanding the electroporated T cells by the methods disclosed herein can be multiplied by about 10 fold, 20 fold, 30 fold, 40 fold, 50 fold, 60 fold, 70 fold, 80 fold, 90 fold, 100 fold, 200 fold, 300 fold, 400 fold, 500 fold, 600 fold, 700 fold, 800 fold, 900 fold, 1000 fold, 2000 fold, 3000 fold, 4000 fold, 5000 fold, 6000 fold, 7000 fold, 8000 fold, 9000 fold, 10,000 fold, 100,000 fold, 1,000,000 fold, 10,000,000 fold, or greater, and any and all whole or partial intergers therebetween. In one embodiment, the T cells expand in the range of about 20 fold to about 50 fold.

Following culturing, the T cells can be incubated in cell medium in a culture apparatus for a period of time or until the cells reach confluency or high cell density for optimal passage before passing the cells to another culture apparatus. The culturing apparatus can be of any culture apparatus commonly used for culturing cells in vitro. A period of time can be any time suitable for the culture of cells in vitro. The T cell medium may be replaced during the culture of the T cells at any time. Preferably, the T cell medium is replaced about every 2 to 3 days. The T cells are then harvested from the culture apparatus whereupon the T cells can be used immediately or cryopreserved to be stored for use at a later time. In one embodiment, the invention includes cryopreserving the expanded T cells. The cryopreserved, expanded T cells are then thawed prior to electroporation with RNA. In another embodiment, the cryopreserved T cells are thawed prior to introducing the nucleic acid into the T cell.

In one aspect, the method of expanding the T cells can further comprise isolating the T cells and a subsequent electroporation followed by culturing. In another embodiment, the invention further comprises cryopreserving the expanded T cells. In yet another embodiment, the cryopreserved T cells are thawed for electroporation with the RNA encoding the bispecific antibody or BiTE molecule. In yet another embodiment, the cryopreserved T cells are thawed for introduction with the affinity molecule chimeric receptor or bispecific affinity molecule nucleic acid. In yet another embodiment, the cryopreserved T cells are thawed for electroporation with the RNA encoding the TCR.

The culturing step as described herein (contact with agents as described herein) can be very short, for example less than 24 hours such as 1, 2, 3, 4, 5, 6, 7, 8, 9, 10, 11, 12, 13, 14, 15, 16, 17, 18, 19, 20, 21, 22, or 23 hours. The culturing step as described further herein (contact with agents as described herein) can be longer, for example 1, 2, 3, 4, 5, 6, 7, 8, 9, 10, 11, 12, 13, 14, or more days.

Various terms are used to describe cells in culture. Cell culture refers generally to cells taken from a living organism and grown under controlled condition. A primary cell culture is a culture of cells, tissues or organs taken directly from an organism and before the first subculture. Cells are expanded in culture when they are placed in a growth medium under conditions that facilitate cell growth and/or division, resulting in a larger population of the cells. When cells are expanded in culture, the rate of cell proliferation is typically measured by the amount of time required for the cells to double in number, otherwise known as the doubling time.

Each round of subculturing is referred to as a passage. When cells are subcultured, they are referred to as having been passaged. A specific population of cells, or a cell line, is sometimes referred to or characterized by the number of times it has been passaged. For example, a cultured cell population that has been passaged ten times may be referred to as a P10 culture. The primary culture, i.e., the first culture following the isolation of cells from tissue, is designated P0. Following the first subculture, the cells are described as a secondary culture (P1 or passage 1). After the second subculture, the cells become a tertiary culture (P2 or passage 2), and so on. It will be understood by those of skill in the art that there may be many population doublings during the period of passaging; therefore the number of population doublings of a culture is greater than the passage number. The expansion of cells (i.e., the number of population doublings) during the period between passaging depends on many factors, including but is not limited to the seeding density, substrate, medium, and time between passaging.

In one embodiment, the cells may be cultured for several hours (about 3 hours) to about 14 days or any hourly integer value in between. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-gamma, IL-4, IL-7, GM-CSF, IL-10, IL-12, IL-15, TGF-beta, and TNF-α. or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature *(e.g.,* 37° C) and atmosphere *(e.g.,* air plus 5% CO₂).

The medium used to culture the T cells may include an agent that can co-stimulate the T cells. For example, an agent that can stimulate CD3 is an antibody to CD3, and an agent that can stimulate CD28 is an antibody to CD28. This is because, as demonstrated by the data disclosed herein, a cell isolated by the methods disclosed herein can be expanded approximately 10 fold, 20 fold, 30 fold, 40 fold, 50 fold, 60 fold, 70 fold, 80 fold, 90 fold, 100 fold, 200 fold, 300 fold, 400 fold, 500 fold, 600 fold, 700 fold, 800 fold, 900 fold, 1000 fold, 2000 fold, 3000 fold, 4000 fold, 5000 fold, 6000 fold, 7000 fold, 8000 fold, 9000 fold, 10,000 fold, 100,000 fold, 1,000,000 fold, 10,000,000 fold, or greater. In one embodiment, the T cells expand in the range of about 20 fold to about 50 fold, or more by culturing the electroporated population.

In one embodiment, the method includes introducing a nucleic acid encoding a T cell receptor (TCR) comprising affinity for a surface antigen on a target cell into the expanded T cells, and electroporating a RNA encoding a co-stimulatory molecule into the T cells, wherein the electroporated T cells are capable of expressing the TCR and the co-stimulatory molecule.

In one embodiment, the method further comprises stimulating the expanded T cells with at least one co-stimulatory molecule selected from the group consisting of CD3, CD27, CD28, CD83, CD86, CD127, 4-1BB, 4-1BBL, PD1 and PD1L. The stimulation may include co-electroporation with RNA encoding the co-stimulatory molecule. In such an embodiment, the expanded T cells are further electroporated or co-electroporated with a RNA encoding CD3. The CD3 includes comprises at least two different CD3 chains, such as CD3 zeta and epsilon chains.

In another embodiment, the method of expanding the T cells can further comprise isolating the expanded T cells for further applications. In yet another embodiment, the method of expanding can further comprise a subsequent electroporation of the expanded T cells followed by culturing. The subsequent electroporation may include electroporating a RNA encoding an agent, such as a bispecific antibody or BiTE molecule, into the expanded population of T cells, wherein the agent further stimulates the T cell. In another embodiment, the subsequent electroporation may include introducing a nucleic acid encoding an agent, such as a transducing the expanded T cells, transfecting the expanded T cells, or electroporating the expanded T cells with a nucleic acid encoding a TCR, into the expanded population of T cells, wherein the agent further stimulates the T cell.

The agent may stimulate the T cells, such as by stimulating further expansion, effector function, or another T cell function. In one embodiment, the agent nucleic acid is co-electroporated with the chimeric membrane protein RNA. In another embodiment, the agent nucleic acid, such as a bispecific antibody or BiTE molecule RNA or TCR RNA, is electroporated after culturing the electroporated population.

In a further embodiment, the agent RNA, such as a bispecific antibody or BiTE molecule RNA, is electroporated into expanded T cells that were cryopreserved. In another embodiment, the agent nucleic acid, such as a TCR RNA, is electroporated after culturing the electroporated population. **In** a further embodiment, the agent nucleic acid, such as a TCR RNA, is electroporated into expanded T cells that were cryopreserved.

**In** yet another embodiment, the modified T cells are cryopreserved after electroporation with the RNA encoding the bispecific antibody or BiTE molecule.

**In** yet another embodiment, the modified T cells are cryopreserved after introduction with the nucleic acid encoding the TCR.

### Therapy

The invention is defined in the appended claims. The references to methods of treatment by therapy in this description and examples are to be interpreted as references to modified T cells, pharmaceutical compositions and medicaments of the invention for use in those methods. The modified T cells described herein may be included in a composition for therapy, as defined in the claims. The composition may include a pharmaceutical composition and further include a pharmaceutically acceptable carrier. A therapeutically effective amount of the pharmaceutical composition comprising the modified T cells may be administered.

In one aspect, the invention includes the modified T cell described herein for use in stimulating a T cell-mediated immune response to a target cell or tissue in a subject; optionally further comprising inducing lysis of the target cell or tissue, preferably wherein the induced lysis is antibody-dependent cell-mediated cytotoxicity (ADCC).

The invention further provides the modified T cell of the invention for use in adoptive cell transfer therapy for preventing or treating an immune reaction that is adverse to the subject.

The invention further provides the modified T cell of the invention for use in treating an autoimmune disease or cancer.

In one embodiment of the above aspects, the modified T cells have been expanded and electroporated with RNA encoding a modified T cell receptor (TCR) comprising affinity for a surface antigen on a target cell. In another embodiment, the modified T cells have been expanded and electroporated with RNA encoding a modified T cell receptor (TCR) and RNA encoding a bispecific antibody, such as a bispecific T-cell engager (BiTE) molecule, comprising bispecificity for an antigen on a target cell and an antigen on an activating T cell. wherein the activating T cell antigen is CD3.

In another embodiment, the modified T cells have been expanded and electroporated with RNA encoding a bispecific T-cell engager (BiTE) molecule comprising bispecificity for an antigen on a target cell, and an antigen on an activating T cell wherein the activating T cell antigen is **CD3** as defined in the appended claims.

The modified T cells may be administered to induce lysis of the target cell or tissue, such as where the induced lysis is antibody-dependent cell-mediated cytotoxicity (ADCC).

The modified T cells generated as described herein possess T cell function. Further, the modified T cells can be administered to a mammal, preferably a human, to suppress an immune reaction, such as those common to autoimmune diseases such as diabetes, psoriasis, rheumatoid arthritis, multiple sclerosis, GVHD, enhancing allograft tolerance induction, transplant rejection, and the like. In addition, the cells of the present invention can be used for the treatment of any condition in which a diminished or otherwise inhibited immune response, especially a cell-mediated immune response, is desirable to treat or alleviate the disease. In one aspect, the invention includes treating a condition, such as an autoimmune disease, in a subject, comprising administering to the subject a therapeutically effective amount of a pharmaceutical composition comprising a population of modified T cells.

Examples of autoimmune disease include but are not limited to, Acquired Immunodeficiency Syndrome (AIDS, which is a viral disease with an autoimmune component), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barre syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Meniere's disease, mixed connective tissue disease, multiple sclerosis, myasthenia gravis, pernacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjogren's syndrome, stiff-man syndrome, systemic lupus erythematosus, Takayasu arteritis, temporal arteritis/giant cell arteritis, ulcerative colitis, uveitis, vitiligo and Wegener's granulomatosis.

The modified T cells generated as described herein can also be expanded and used to treat inflammatory disorders. Examples of inflammatory disorders include but are not limited to, chronic and acute inflammatory disorders. Examples of inflammatory disorders include Alzheimer's disease, asthma, atopic allergy, allergy, atherosclerosis, bronchial asthma, eczema, glomerulonephritis, graft vs. host disease, hemolytic anemias, osteoarthritis, sepsis, stroke, transplantation of tissue and organs, vasculitis, diabetic retinopathy and ventilator induced lung injury.

In another embodiment, the T cells described herein may be used for the manufacture of a medicament for the treatment of an immune response in a subject in need thereof. In another embodiment, the invention includes the modified cell described herein for use in a method of treating an immune response in a subject in need thereof.

The cells of the present invention can be administered to an animal, preferably a mammal, even more preferably a human, to treat a cancer. In addition, the cells of the present invention can be used for the treatment of any condition related to a cancer, especially a cell-mediated immune response against a tumor cell(s), where it is desirable to treat or alleviate the disease. Examples of cancers include but are not limited breast cancer, prostate cancer, ovarian cancer, cervical cancer, skin cancer, pancreatic cancer, colorectal cancer, renal cancer, liver cancer, brain cancer, lymphoma, leukemia, lung cancer, thyroid cancer, and the like.

Cells of the invention can be administered in dosages and routes and at times to be determined in appropriate pre-clinical and clinical experimentation and trials. Cell compositions may be administered multiple times at dosages within these ranges. Administration of the cells of the invention may be combined with other methods useful to treat the desired disease or condition as determined by those of skill in the art.

The cells of the invention to be administered may be autologous, allogeniec or xenogenic with respect to the subject undergoing therapy.

The administration of the cells of the invention may be carried out in any convenient manner known to those of skill in the art. The cells of the present invention may be administered to a subject by aerosol inhalation, injection, ingestion, transfusion, implantation or transplantation. The compositions described herein may be administered to a patient transarterially, subcutaneously, intradermally, intratumorally, intranodally, intramedullary, intramuscularly, by intravenous (i.v.) injection, or intraperitoneally. In other instances, the cells of the invention are injected directly into a site of inflammation in the subject, a local disease site in the subject, alymph node, an organ, a tumor, and the like.

The cells described herein can also be administered using any number of matrices. The present invention utilizes such matrices within the novel context of acting as an artificial lymphoid organ to support, maintain, or modulate the immune system, typically through modulation of T cells. Accordingly, the present invention can utilize those matrix compositions and formulations which have demonstrated utility in tissue engineering. Accordingly, the type of matrix that may be used in the compositions, devices and methods of the invention is virtually limitless and may include both biological and synthetic matrices. In one particular example, the compositions and devices set forth by U.S. Pat. Nos. 5,980,889; 5,913,998; 5,902,745; 5,843,069; 5,787,900; or 5,626,561 are utilized. Matrices comprise features commonly associated with being biocompatible when administered to a mammalian host. Matrices may be formed from natural and/or synthetic materials. The matrices may be non-biodegradable in instances where it is desirable to leave permanent structures or removable structures in the body of an animal, such as an implant; or biodegradable. The matrices may take the form of sponges, implants, tubes, telfa pads, fibers, hollow fibers, lyophilized components, gels, powders, porous compositions, or nanoparticles. In addition, matrices can be designed to allow for sustained release of seeded cells or produced cytokine or other active agent. In certain embodiments, the matrix of the present invention is flexible and elastic, and may be described as a semisolid scaffold that is permeable to substances such as inorganic salts, aqueous fluids and dissolved gaseous agents including oxygen.

A matrix is used herein as an example of a biocompatible substance. However, the current invention is not limited to matrices and thus, wherever the term matrix or matrices appears these terms should be read to include devices and other substances which allow for cellular retention or cellular traversal, are biocompatible, and are capable of allowing traversal of macromolecules either directly through the substance such that the substance itself is a semi-permeable membrane or used in conjunction with a particular semi-permeable substance.

### Pharmaceutical compositions

The invention is defined in the appended claims. The references to methods of treatment by therapy in this description and examples are to be interpreted as references to modified T cells, pharmaceutical compositions and medicaments of the invention for use in those methods. Pharmaceutical compositions of the present invention may comprise a modified T cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

It can generally be stated that a pharmaceutical composition comprising the modified T cells described herein may be administered at a dosage of 10⁴ to 10⁹ cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, *e.g.*, Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

In certain embodiments, it may be desired to administer activated T cells to a subject and then subsequently redraw blood (or have an apheresis performed), activate T cells therefrom according to the present invention, and reinfuse the patient with these activated and expanded T cells. This process can be carried out multiple times every few weeks. In certain embodiments, T cells can be activated from blood draws of from 10 ml to 400 ml. In certain embodiments, T cells are activated from blood draws of 20 ml, 30 ml, 40 ml, 50 ml, 60 ml, 70 ml, 80 ml, 90 ml, or 100 ml. Not to be bound by theory, using this multiple blood draw/multiple reinfusion protocol, may select out certain populations of T cells.

In certain embodiments of the present invention, cells expanded and modified using the methods described herein, or other methods known in the art where T cells are expanded to therapeutic levels, are administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. In further embodiments, the T cells of the invention may be used in combination with chemotherapy, radiation, immunosuppressive agents, such as cyclosporin, azathioprine, methotrexate, mycophenolate, and FK506, antibodies, or other immunoablative agents such as CAM PATH, anti-CD3 antibodies or other antibody therapies, cytoxin, fludaribine, cyclosporin, FK506, rapamycin, mycophenolic acid, steroids, FR901228, cytokines, and irradiation. These drugs inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993). In a further embodiment, the cell compositions of the present invention are administered to a patient in conjunction with *(e.g.,* before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In another embodiment, the cell compositions of the present invention are administered following B-cell ablative therapy such as agents that react with CD20, *e.g.,* Rituxan. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

It should be understood that the method and compositions that would be useful in the present invention are not limited to the particular formulations set forth in the examples. The following examples are put forth so as to provide those of ordinary skill in the art with a complete disclosure and description of how to make and use the cells, expansion and culture methods, and therapeutic methods of the invention, and are not intended to limit the scope of what the inventors regard as their invention.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Oligonucleotide Synthesis" (Gait, 1984); "Culture of Animal Cells" (Freshney, 2010); "Methods in Enzymology" "Handbook of Experimental Immunology" (Weir, 1997); "Gene Transfer Vectors for Mammalian Cells" (Miller and Calos, 1987); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques are applicable to the production of the polynucleotides and polypeptides of the invention, and, as such, may be considered in making and practicing the invention. Particularly useful techniques for particular embodiments will be discussed in the sections that follow.

### EXPERIMENTAL EXAMPLES

The invention is defined in the appended claims. The references to methods of treatment by therapy in this description and examples are to be interpreted as references to modified T cells, pharmaceutical compositions and medicaments of the invention for use in those methods. The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

The materials and methods employed in experiments of Example 1 are now described.

*Primary human lymphocytes.* Primary lymphocytes were stimulated with microbeads coated with CD3 and CD28 stimulatory antibodies (Life Technologies, Grand Island, NY, Catalog) as described *(*Human gene therapy 2011, 22(12):1575-1586). T cells were cryopreserved at day 10 in a solution of 90% fetal calf serum and 10% dimethylsulfoxide (DMSO) at 1 x 10⁸ cells/vial.

*Generation of TCR constructs for mRNA electroporation and lentiviral transduction.* 1G4 NY-ESO-1 TCR with different mutations were synthesized and/or amplified by PCR, based on sequencing information provided by the relevant publications *(*The Journal of experimental medicine 2005, 201(8):1243-1255; J Immunol 2008, 180(9):6116-6131), and subcloned into pGEM.64A RNA based vector or pTRPE lentiviral vectors.

*mRNA in vitro transcription and T cell electroporation.* T7 mscript systems kit (CellScript) was used to generate in vitro transcribed (IVT) RNA. CD3/CD28 bead stimulated T cells were electroporated with IVT RNA using BTX EM830 (Harvard Apparatus BTX) as previously described *(*Cancer research 2010, 70(22):9053-9061). Briefly, T cells were washed three times and resuspended in OPTI-MEM (Invitrogen) at a final concentration of 1-3 x 10⁸ cells/ml. Subsequently, 0.1 ml of cells were mixed with 10 ug IVT RNA (or as indicated) and electroporated in a 2 mm cuvette.

*ELISA assays.* Target cells, different tumor cell lines expressing CD19, were washed and suspended at 1x10⁶ cells/ml in R10 medium (RPMI 1640 supplemented with 10% fetal calf serum; Invitrogen). 100 ul of each target cell type was added in duplicate to a 96 well round bottom plate (Corning). Effector T cells were washed, and re-suspended at 1x10⁶ cells/ml in R10 medium and then 100 ul of T cells were combined with the target cells in the indicated wells. In addition, wells containing T cells alone were prepared as a control. The plates were incubated at 37° C for 18 to 20 hours. After the incubation, supernatant was harvested and subjected to an ELISA assay (eBioscience, 88-7316-77; 88-7025-77).

*CD107a staining* Cells were plated at an Effector cell:T cell ratio of 1:1 (1x10⁵ effectors to 1x10⁵ targets) in 160 µl of complete RPMI medium in a 96 well plate. 20 µl of phycoerythrin-labeled anti-CD107a antibody (BD Biosciences, 555801) was added and the plate was incubated at 37° C for 1 hour before adding Golgi Stop (2 ul Golgi Stop in 3 ml RPMI medium, 20 ul/well; BD Biosciences, 51-2092KZ ) and incubating the plate for another 2.5 hours. Then 5 µl FITC-anti-CD8 and 5 ul APC-anti-CD3 were added and incubated at 37° C for 30 min. After incubation, the samples were washed with FACS buffer and analyzed by flow cytometry.

*Luciferase based CTL assay.* Naml6-CBG tumor cells were generated and employed in a modified version of a luciferase based cytotoxic T lymphocyte assay. Briefly, click beetle green luciferase (CBG) was cloned into the pELNS vector, packaged into lentivirus, transduced into Naml6 tumor cells and sorted for CBG expression. The resulting Naml6-CBG cells were washed and resuspended at 1x10⁵ cells/ml in R10 medium, and 100 ul of CBG-labeled cells were incubated with different ratios of T cells (e.g. 30:1, 15:1, etc) overnight at 37° C. 100 ul of the mixture was transferred to a 96 well white luminometerplate. 100 ul of substrate was added to the cells and luminescence was immediately determined.

*Mouse xenograft studies.* Studies were performed as previously described with certain modifications *(*Human gene therapy 2011, 22(12):1575-1586; Proceedings of the National Academy of Sciences of the United States of America 2009, 106(9):3360-3365). Briefly, 6-10 week old NOD/SCID gamma (NSG) mice were injected subcutaneously with 1x10⁶ PC3-CBG tumors cells on the right flank at day 0 and the same mice were given SK-OV3-CBG tumor cells (5x10⁶ cells/mouse, subcutaneously.) on the left flank at day 5. The mice were treated with T cells via the tail vein at day 23 post PC3-CBG tumor inoculation, such that both tumors were approximately 200 mm³ in volume. Lentivirally transduced T cells were given at 1x10⁷ cells/mouse (10M), or 3x10⁶ cells/mouse (3M).

The materials and methods employed in experiments of Example 2 are now described.

*Construction of in vitro transcription (IVT) mRNA and lentiviral vectors for CARs.* All genes were synthesised and/or amplified and assembled by PCR using publically available sequence information. The PCR products were subcloned into pGEM.64A based vector by replacing GFP of pGEM-GFP.64A to produce pGEM.64A based IVT vectors based on the publically available sequence information.

*RNA in vitro transcription (IVT).* In vitro transcription to synthesize mRNA was performed with a kit, such as mMESSAGE mMACHINE^{®} T7 Ultra (Ambion, Inc), to generate IVT RNA with Anti-Reverse Cap Analog (ARCA, 7-methyl(3'-Omethyl) GpppG)m7G(5')ppp(5')G). The IVT RNA products were purified using an RNeasy Mini Kit (Qiagen, Inc., Valencia, CA) and purified RNA was eluted in RNase-free water at 1-2 mg/ml.

*RNA electroporation of T cells.* Purified resting T cells or CD3/CD28 bead-stimulated T cells were electroporated using BTX EM830 (Harvard Apparatus BTX, Holliston, MA, USA). The T cells subjected to electroporation were washed three times with OPTI-MEM (Invitrogen) and were re-suspended in OPTI-MEM at the final concentration of 1-3x10⁸/ml. Subsequently, 0.1 ml of the cells was mixed with 10 ug IVT RNA (or as indicated) and electroporated in a 2-mm cuvette as described (Zhao et al., 2010, Cancer Res 70:9053-9061*).*

*CLEAR detection on electroporated T Cells.* Cells were washed and suspended in flow activated cell sorting (FACs) buffer (PBS plus 0.1% sodium azide and 0.4% BSA). Anti-PD1 (APC) and anti-CD27 (PE) were used for PD1 or CD27 based CLEAR detection respectively. Biotin-labeled polyclonal goat anti-mouse F(ab)2 antibodies (for murine scFv) or anti-human anti-F(ab)2 (for human scFv) (Jackson Immunoresearch, West Grove, PA) was added to the cells and the cells were incubated at 4°C for 25 minutes and washed twice. The cells were then stained with phycoerythrin-labeled streptavidin (BD Pharmingen, San Diego, CA).

*ELISA and Luminex assays.* Target cells were washed and suspended at 10⁶ cells/mL in R10. One hundred thousand cells of each target cell type were added to each of 2 wells of a 96 well round bottom plate (Corning). Effector T cell cultures were washed and suspended at 10⁶ cells/mL in R10. One hundred thousand effector T cells were combined with target cells in the indicated wells of the 96 well plate. In addition, wells containing T cells alone were prepared. The plates were incubated at 37°C for 18 to 20 hours. After the incubation, supernatant was harvested and subjected to an ELISA assay using standard methods (Pierce, Rockford, IL). *CD107a staining.* Cells were plated at an E:T of 1:1 (10⁵ effectors: 10⁵ targets) in 160 µl of complete RPMI medium in a 96 well plate. 20 µl of phycoerythrin-labeled anti-CD107a antibody (BD Pharmingen, San Diego, CA) was added and the plate was incubated at 37°C for 1 hour before adding Golgi Stop and incubating for another 2.5 hours. After 2.5 hours 10 µl FITC-anti-CD8 and APC-anti-CD3 was added and incubated at 37°C for 30 min. After incubation, the samples were washed once with FACS buffer. Flow cytometry acquisition was performed with a BD FacsCalibur (BD Biosciences), and analysis was performed with FlowJo (Treestar Inc, Ashland, OR).

The materials and methods employed in experiments of Example 3 are now described.

*Primary human lymphocytes.* Primary lymphocytes were stimulated with microbeads coated with CD3 and CD28 stimulatory antibodies (Life Technologies, Grand Island, NY, Catalog) as previously described (Barrett, et al., Human gene therapy 2011, 22(12):1575-1586). T cells were cryopreserved at day 10 in a solution of 90% fetal calf serum and 10% dimethylsulfoxide (DMSO) at 1 x 10⁸ cells /vial.

*Generation of ART constructs for mRNA electroporation.* The following Affinity antibody mimetic Redirected T cell (ART) sequences for either EGFR (Friedman et al., Journal of molecular biology 2008, 376(5):1388-1402; and Friedman et al., Protein engineering, design & selection : PEDS 2007, 20(4):189-199) or ErbB2 (Fedwisch et al., Journal of molecular biology 2010, 398(2):232-247) were used to generated ARTs.

### For EGFR:

SEQ ID NO:1,
ZEGFR955:VDNKFNKELEKAYNEIRNLPNLNGWQMTAFIASLVDDPSQSANLLAE
AKKLNDAQAPK

SEQ ID NO:2, ZEGFR942: 
VDNKFNKEMLIAMEEIGSLPNLNWGQEQAFILSLWDDPSQSANLLAEAKKLNDA
QAPK
SEQ ID NO:3, ZEGFR1853: 
VDNKFNKEFWWASDEIRNLPNLNGWQMTAFIASLADDPSQSANLLAEAKKLND
AQAPK
SEQ ID NO:4, ZEGFR1907:

VDNKFNKEMWAAWEEIRNLPNLNGWQMTAFIASLVDDPSQSANLLAEAKKLND
AQAPK

### For ErbB2:

SEQ ID NO:5, ZHER2- 
342VDNKFNKEMRNAYWEIALLPNLNNQQKRAFIRSLYDDPSQSANLLAEAKKL
NDAQAPK
SEQ ID NO:6, ZHER2-342-15 
VDNKFNKEMRNAYWEIALLPNLTNQQKRAFIRSLYKDPSQSANLLAEAKKLNDA
QAPK
SEQ ID NO:7, ZHER2-342-14 
VDNKFNKEMRNAYWEIALLPNLNNQQKRAFIRSLYADPSQSANLLAEAKKLND
AQAPK
SEQ ID NO:8, ZHER2-342-4

VDNKFEKEMRNAYWEIALLPNLTNQQKRAFIRSLYDDPSQSANLLAEAKKLNDA
QAPK

**Table 1: Affinity of the Affinity Antibody Mimetics.**

| **ARTs** | **Kd** |
|---|---|
| ZEGFR955 | 185 nM |
| ZEGFR942 | 130 nM |
| ZEGFR1853 | 9.2 nM |
| ZEGFR1907 | 5.4nM |
| | |
| ZHER2.342 | 22 pM |
| ZHER2.342-15 | 180 pM |
| ZHER2.342-14 | 76 pM |
| ZHER2.342-4 | 44 pM |

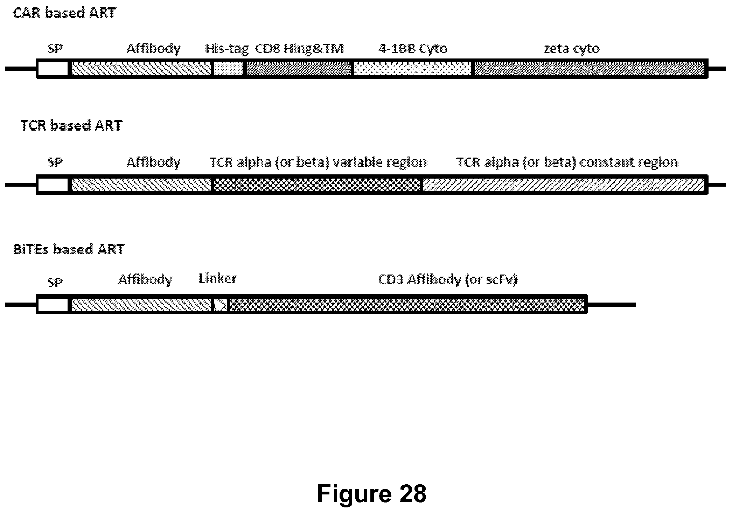

Three different kinds of ARTs were constructed, as showed in **Figure 28****.**

The first is CAR based ART **(****Figure 28****,** upper panel): that is composed of a signal peptide (SP) from human CD8 alpha, affinity antibody mimetic, 6X His tag (His-Tag), human CD8 alpha hinge and transmembrane (CD8 Hinge&TM), 4-1BB cytoplasmic domain (4-1BB Cyto) and CD3 zeta cytoplasmic domain (zeta Cyto). The second is TCR based ART (Fig. 28 lower panel): that potentially could target multiple tumor antigens simultaneously, which is composed of a signal peptide (SP) from human CD8 alpha, ErbB2 affinity antibody mimetic (ZHER2.342, ZHER2.342-15, ZHER2.342-14 or ZHER2.342-4) and full length 1G4 NY-ESO-1 TCR alpha or beta chain. The third is Bi-specific T-cell engager (BiTEs) based ART: which is composed of a signal peptide (SP) from human CD8 alpha, ErbB2 affinity antibody mimetic (ZHER2.342,

ZHER2.342-15, ZHER2.342-14 or ZHER2.342-4), GS linker(or EGFR affinity antibody mimetic and GS linker) and CD3 affinity antibody mimetic (or scFv).

All the (bellow) affinity antibody mimetic DNA sequences were generated by UpGene, an application of a DNA codon optimization algorithm, which was used to generate PCR primer sequences based on above protein sequence info, to synthesis all the ART constructs (via over lapping PCR) in pGEM.64A based RNA in vitro transcription (IVT) vector (Zhao, et al., Cancer research 2010, 70(22):9053-9061).
SEQ ID NO:9, ZEGFR955: 
atg gcg ctg ccc gtg acg gcc ctg ctg ctc ccc ctg gca ctg ctt ctg cac gcc gcc cga ccc tct cag
gtc gac aac aag ttt aac aag gaa ttg gag aag gcg tac aac gaa atc cgg aac ctg ccc aac ctc aac
gga tgg cag atg act gct ttc atc gcg tcc ctg gtg gac gac ccc tcc cag tcg gcg aac ctg ctg gcc
gag gcc aag aaa ctg aat gac gcc caa gcc cct aag
SEQ ID NO:10, ZEGFR942: 
atg gcg ctg ccc gtg acg gcc cta ctc ctg cca ctg gca ctc ctg ctc cac gcc gcg cgg cca tcc cag
gtg gac aac aaa ttc aac aag gag atg ctc atc gca atg gaa gaa ata ggc tcc ctc ccc aac ctg aac
tgg ggg cag gag cag gcg ttc ata ctc tcg ctc tgg gat gac cca agc cag tct gcg aac ctc ctt gcc
gag gcg aag aag ctc aat gac gct cag gca ccc aag
SEQ ID NO:11, ZEGFR1853: 
atg gcg ctg ccc gtg acg gcc CTC TTG ctg CCC CTG GCC CTC CTG ctc cac GCA GCC
AGG CCG AGT CAA GTC gac aac AAG ttt aac aag gaa TTC tgg tgg GCC TCC GAC
GAG atc CGC aat CTG ccc AAC CTG AAC ggc tgg cag atg ACC gcc ttc atc GCA AGC
ctg GCG gac gac CCT tcc cag agt GCC aac CTG ctg gca gag gcc aag aag ctg AAC GAC
GCC CAA GCG CCA aag
SEQ ID NO:12, ZEGFR1907: 
atg gcg ctg ccc gtg acg gcc ctg ctg ctg ccg ctc gcc tta ctg ctc cac gcg gct agg ccc tcc cag
gtg gat aac aag ttc aac aag gag atg tgg gcc gcc tgg gag gaa atc cgc aac ctc cca aat ctg aac
ggc tgg cag atg aca gca ttc att gcc tcc ctg gtg gac gat cca tcg cag tcg gcc aac ctc cta gcc
gag gcc aag aag ctg aac gac gcc cag gcg ccc aag CatcatcaccatcatcatAccacgacg
SEQ ID NO:13, ZHER2-342 
atg gcg ctg ccc gtg acg gcc ctt ctg ctc ccg ctg gcc ctg ctg ctg cat gcc gcc cgc ccc tca caa
gtc gac aac aaa ttc aat aag gag atg aga aac gct tac tgg gag atc gcc ctg ctt ccc aac ctg aac
aac cag cag aaa cga gca ttc att cgc agc ctc tac gac gat ccg tcg cag tca gct aat ctc ctc gcc
gag gcg aag aag ctg aac gac gcc cag gcc ccc aag
SEQ ID NO:14, ZHER2-342-15 
atg gcg ctg ccc gtg acg gcc ctc ctg ctg ccc ctg gcc ctg ctg ctc cac gct gca aga ccc agc cag
gtg gac aac aag ttt aac aag gag atg cgc aac gcc tac tgg gag atc gcc ctc ctg ccc aac tta acc
aac cag caa aaa aga gct ttc att cgt tcc ctg tac aaa gac cct tcg cag tcc gcc aat ctg ctt gcc gag
gct aag aag ctg aac gac gcg cag gcc ccc aag
SEQ ID NO:15, ZHER2-342-14 
atg gcg ctg ccc gtg acg gcc ctc ctg ctg ccc ctc gcg ctc ctg ctc cac gcc gcc cgg ccc tcc caa
gtc gac aac aag ttc aat aag gag atg cgg aac gca tac tgg gag atc gcc ctg ctg ccg aac ctg aac
aac cag cag aag cgt gcc ttt atc agg tct ctc tac gca gat cca agc cag tct gcg aac ctg ctg gcc
gag gcg aag aaa ctc aac gac gcg cag gca ccg aag
SEQ ID NO:16, ZHER2-342-4 
atg gcg ctg ccc gtg acg gcc ctg ctc ctg ccg ctc gcc ctc tta ctt cac gcc gcg cga ccg tcc cag
gtt gac aac aag ttc gag aaa gag atg agg aac gca tac tgg gag atc gcc tta ctg cct aat ctg act
aac cag cag aag cgc gcc ttc atc cgc tcc ctg tac gac gat ccg tcg cag tcc gct aac ttg ctg gcc
gag gcc aag aag ctc aac gat gcc caa gcc ccc aag
SEQ ID NO:17, 6X His tag:
   Catcatcaccatcatcat
SEQ ID NO:18, CD8Hinge&TM-BBZ: 
Accacgacgccagcgccgcgaccaccaacaccggcgcccaccatcgcgtcgcagcccctgtccctgcgcccagaggcgtg
ccggccagcggcggggggcgcagtgcacacgagggggctggacttcgcctgtgatatctacatctgggcgcccttggccgg
gacttgtggggtccttctcctgtcactggttatcaccctttactgcaaacggggcagaaagaaactcctgtatatattcaaacaacca
tttatgagaccagtacaaactactcaagaggaagatggctgtagctgccgatttccagaagaagaagaaggaggatgtgaactg
agagtgaagttcagcaggagcgcagacgcccccgcgtacaagcagggccagaaccagctctataacgagctcaatctaggac
gaagagaggagtacgacgttttggacaagagacgtggccgggaccctgagatggggggaaagccgagaaggaagaaccct
caggaaggcctgtacaatgaactgcagaaagataagatggcggaggcctacagtgagattgggatgaaaggcgagcgccgg
aggggcaaggggcacgatggcctttaccagggtctcagtacagccaccaaggacacctacgacgcccttcacatgcaggccc
tgccccctcgcTAA
SEQ ID NO:19, Linker-CD3 scFv

ggaggtggtggatccgatatcaaactgcagcagtcaggggctgaactggcaagacctggggcctcagtgaagatgtcctgcaa
gacttctggctacacctttactaggtacacgatgcactgggtaaaacagaggcctggacagggtctggaatggattggatacatta
atcctagccgtggttatactaattacaatcagaagttcaaggacaaggccacattgactacagacaaatcctccagcacagcctac
atgcaactgagcagcctgacatctgaggactctgcggtctatttctgtgcaagatattatgatgatcattactgccttgactactggg
gccaaggcaccactctcacagtctcctcagtcgaaggtggaagtggaggttctggtggaagtggaggttcaggtggagtcgac
gacgccgccattcagctgacccagtctccagcaatcatgtctgcatctccaggggagaaggtcaccatgacctgcagagccagt
tcaagtgtaagttacatgaactggtaccagcagaagtcaggcacctcccccaaaagatggatttatgacacatccaaagtggcttc
tggagtcccttatcgcttcagtggcagtgggtctgggacctcatactctctcacaatcagcagcatggaggctgaagatgctgcca
cttattactgccaacagtggagtagtaacccgctcacgttcggtgctgggaccaagctggagctgaaacatcatcaccatcatcat
taataa

*mRNA in vitro transcription and T cell electroporation.* mMESSAGE mMACHINE^{®} T7 ULTRA Transcription Kit (Invitrogen) was used to generate IVT RNA. CD3/CD28 bead stimulated T cells were electroporated with IVT RNA using BTX EM830 (Harvard Apparatus BTX) as previously described (Zhao, et al., Cancer research 2010, 70(22):9053-9061). Briefly, T cells were washed three times and resuspended in OPTI-MEM (Invitrogen) at a final concentration of 1-3 x 10⁸ cells /ml. Subsequently, 0.1 ml of cells were mixed with 10ug IVT RNA (or as indicated) and electroporated in a 2mm cuvette.

*ELISA assays.* Target cells were washed and suspended at 1x 10⁶ cells/ml in R10 medium (RPMI 1640 supplemented with 10% fetal calf serum; Invitrogen). 100ul each target cell type were added in duplicate to a 96 well round bottom plate (Corning). Effector T cells were washed, and re-suspended at 1x 10⁶ cells/ml in R10 medium and then 100ul of T cells were combined with target cells in the indicated wells. In addition, wells containing T cells alone were prepared. The plates were incubated at 37°C for 18 to 20 hours. After the incubation, supernatant was harvested and subjected to an ELISA assay (eBioscience, 88-7316-77; 88-7025-77).

*CD107a staining.* Cells were plated at an E:T of 1:1 (1 x 10⁵ effectors: 1 x 10⁵ targets) in 160 µl of complete RPMI medium in a 96 well plate. 20µl of phycoerythrin-labeled anti-CD107a Ab (BD Biosciences, 555801) was added and the plate was incubated at 37°C for 1 hour before adding Golgi Stop (2ul Golgi Stop in 3ml RPMI medium, 20ul/well; BD Biosciences, 51-2092KZ ) and incubating for another 2.5 hours. Then 5 µl FITC-anti-CD8 and 5ul APC-anti-CD3 were added and incubated at 37°C for 30 min. After incubation, the samples were washed with FACS buffer and analyzed by flow cytometry.

*Luciferase based CTL assay.* SK-OV3-CBG tumor cells were generated and employed in a modified version of a luciferase based CTL assay as follows: Click beetle green luciferase (CBG) was cloned into the pELNS vector, packaged into lentivirus, transduced into SK-OV3 tumor cells and sorted for CBG expression. Resulting SK-OV3-CBG cells were washed and resuspended at 1 x 10⁵ cells /ml in R10 medium, and 100 ul of CBG-labeled cells were incubated with different ratios of T cells (e.g. 30:1, 15:1, etc) 8h at 37°C. 100ul of the mixture was transferred to a 96 well white luminometerplate, 100ul of substrate was added and the luminescence was immediately determined.

The materials and methods employed in experiments of Example 4 are now described.

*Construction of in vitro transcription (IVT) mRNA and lentiviral vectors for CARs.* All CARs (CD19, mesothelin, cMet, GD2, PSCA, EGFRviii and ErBB2) and the RNA encoding bispecific antibodies (Bis-RNAs) for the same molecules were synthesized and/or amplified and assembled by PCR. The PCR products were subcloned into pGEM.64A based vector by replacing GFP of pGEM-GFP.64A (Zhao et al., 2003, Blood 102:4137-4142) to produce pGEM.64A based CAR or Bis-RNA vectors. DNA encoding Blinatumomab and the fully human CD19 CAR (21D4-BBZ) and Bis-RNA (21D4-F11) were synthesised and assembled by PCR based on sequencing information provided from the published patent No.: US2013/050275.

*RNA in vitro transcription (IVT).* In vitro transcription to synthesize mRNA was performed with a kit, such as mMESSAGE mMACHINE^{®} T7 Ultra (Ambion, Inc), to generate IVT RNA with Anti-Reverse Cap Analog (ARCA, 7-methyl(3'-Omethyl) GpppG)m7G(5')ppp(5')G). The IVT RNA products were purified using an RNeasy Mini Kit (Qiagen, Inc., Valencia, CA) and purified RNA was eluted in RNase-free water at 1-2 mg/ml.

*RNA electroporation of T cells.* Purified resting T cells or CD3/CD28 bead-stimulated T cells were electroporated using BTX EM830 (Harvard Apparatus BTX, Holliston, MA, USA). The T cells subjected to electroporation were washed three times with OPTI-MEM (Invitrogen) and were re-suspended in OPTI-MEM at the final concentration of 1-3x10⁸/ml. Subsequently, 0.1 ml of the cells was mixed with 10 ug IVT RNA (or as indicated) and electroporated in a 2-mm cuvette as described (Zhao et al., 2010, Cancer Res 70:9053-9061*).*

*CAR detection on electroporated T Cells.* Cells were washed and suspended in flow activated cell sorting (FACs) buffer (PBS plus 0.1% sodium azide and 0.4% BSA). Biotin-labeled polyclonal goat anti-mouse F(ab)2 antibodies ( for murine scFv) or anti-human anti-F(ab)2 (for human scFv) (Jackson Immunoresearch, West Grove, PA) was added to the cells and the cells were incubated at 4°C for 25 minutes and washed twice. The cells were then stained with phycoerythrin-labeled streptavidin (BD Pharmingen, San Diego, CA).

*ELISA and Luminex assays.* Target cells were washed and suspended at 10⁶ cells/mL in R10. One hundred thousand cells of each target cell type were added to each of 2 wells of a 96 well round bottom plate (Corning). Effector T cell cultures were washed and suspended at 10⁶ cells/mL in R10. One hundred thousand effector T cells were combined with target cells in the indicated wells of the 96 well plate. In addition, wells containing T cells alone were prepared. The plates were incubated at 37°C for 18 to 20 hours. After the incubation, supernatant was harvested and subjected to an ELISA assay using standard methods (Pierce, Rockford, IL).

*CD107a staining.* Cells were plated at an E:T of 1:1 *(10⁵* effectors: 10⁵ targets) in 160 µl of complete RPMI medium in a 96 well plate. 20 µl of phycoerythrin-labeled anti-CD107a antibody (BD Pharmingen, San Diego, CA) was added and the plate was incubated at 37°C for 1 hour before adding Golgi Stop and incubating for another 2.5 hours. After 2.5 hours 10 µl FITC-anti-CD8 and APC-anti-CD3 was added and incubated at 37°C for 30 min. After incubation, the samples were washed once with FACS buffer. Flow cytometry acquisition was performed with a BD FacsCalibur (BD Biosciences), and analysis was performed with FlowJo (Treestar Inc, Ashland, OR).

*CFSE based T cells proliferation assay.* T cells at a concentration of 10x10⁶/ mL in PBS were labeled with CFSE at 3 µM for 3 min and 30 sec at room temperature. The labeling was stopped 5% FBS (in PBS) and washed twice with R10 and cultured in R10 with 10 IU/ml IL-2. After overnight culture, the CFSE labeled T cells were electroporated. Two to four hours after electroporation, the T cells were stimulated with irradiated tumor or K562 cell lines at T:stimulator at 1:1. CFSE dilution was examined by flow cytometry and cell number was counted at the time as indicated.

*Flow CTL.* A slightly modified version of a flow cytometry cytotoxicity assay was used as described previously (Zhao et al., 2010, Cancer Res 70:9053-9061, Hermans et al., 2004, J Immunol Methods 285:25-40). Click beetle green luciferase (CBG) tumor cells were generated and employed in a luciferase based CTL assay as follows: CBG was cloned into the pELNS vector, packaged into lentivirus, and transduced into tumor cells. CBG tumor cells were sorted for CBG expression. The resulting CBG tumor cells were washed and resuspended at 1x10⁵ cells/ml in R10 medium, and 100 ul of CBG-labeled cells were incubated with different ratios of T cells (e.g. 30:1, 15:1, etc) overnight at 37⁰C. 100 ul of the mixture was transferred to a 96 well white luminometer plate. 100 ul of substrate was added to each well and luminescence was immediately determined.

*Mouse xenograft studies.* Studies were performed as previously described with certain modifications (Barrett et al., Hum Gene Ther 22:1575-1586). Briefly, 6-10 week old NOD-SCID-?c-/- (NSG) mice were obtained from the Jackson Laboratory (Bar Harbor, ME) or bred in-house under an approved institutional animal care and use committee (IACUC) protocol and maintained under pathogen-free conditions. Nalm-6, a CD19+ human ALL line, was transduced with CBG (Nalm6-CBG) and injected via the tail vein in 0.2 ml of sterile PBS in the dosage of one million cells. T cells were injected via the tail vein 7 days after injection of Nalm-6-CBG.

*Bioluminescence imaging (BLI).* Tumor growth was monitored by BLI. Anesthetized mice were imaged using a Xenogen Spectrum system and Living Image v3.2 software. D-luciferin (Caliper Life Sciences, Hopkinton, MA) was resuspended in sterile PBS at a concentration of 15 mg/mL (100 µL luciferin solution/10 g mouse body weight) and administered to the mice via intraperitoneal (IP) injection at ratio of 150 mg/kg body weight. Previous titration of M108-Luc indicated the time to peak of photon emission at approx. five minutes, with peak emission lasting for about 6-10 minutes. Each animal was imaged alone (for photon quantitation) or in groups of up to 5 mice (for display purposes) in the anterior-posterior prone position at the same relative time point after luciferin injection (6 minutes). Data were collected until the midrange of the linear scale was reached (600 to 60000 counts) or maximal exposure settings reached (f/stop 1, large binning and 1-2 seconds), and then converted to photons/second/cm2/steradian to normalize each image for exposure time, f/stop, binning and animal size. For anatomic localization, a pseudocolor map representing light intensity was superimposed over the grayscale body-surface reference image. For data display purposes, mice without luciferase containing cells were imaged at maximal settings and a mean value of 3.6 x10⁵ p/s/cm2/sr was obtained.

The materials and methods employed in experiments of Example 5 are now described.

*Primary human lymphocytes.* Primary lymphocytes were stimulated with microbeads coated with CD3 and CD28 stimulatory antibodies (Life Technologies, Grand Island, NY, Catalog) as described *(*Human gene therapy 2011, 22(12):1575-1586). T cells were cryopreserved at day 10 in a solution of 90% fetal calf serum and 10% dimethylsulfoxide (DMSO) at 1 x 10⁸ cells/vial.

*Generation of constructs for mRNA electroporation and lentiviral transduction.* 1G4 NY-ESO-1 TCR with different mutations were synthesized and/or amplified by PCR, based on sequencing information provided by the relevant publications *(*The Journal of experimental medicine 2005, 201(8):1243-1255; J Immunol 2008, 180(9):6116-6131), and subcloned into pGEM.64A RNA based vector or pTRPE lentiviral vectors.

### ErbB2 affibody sequences:

SEQ ID NO:5, ZHER2-342 (342) 
VDNKFNKEMRNAYWEIALLPNLNNQQKRAFIRSLYDDPSQSANLLAEAKKLND
AQAPK
SEQ ID NO:6, ZHER2-342-15 (342-15) 
VDNKFNKEMRNAYWEIALLPNLTNQQKRAFIRSLYKDPSQSANLLAEAKKLNDA
QAPK
SEQ ID NO:7, ZHER2-342-14 (342-14) 
VDNKFNKEMRNAYWEIALLPNLNNQQKRAFIRSLYADPSQSANLLAEAKKLND
AQAPK
SEQ ID NO:8, ZHER2-342-4 (342-4)

VDNKFEKEMRNAYWEIALLPNLTNQQKRAFIRSLYDDPSQSANLLAEAKKLNDA
QAPK

*mRNA in vitro transcription and T cell electroporation.* T7 mscript systems kit (CellScript) was used to generate in vitro transcribed (IVT) RNA. CD3/CD28 bead stimulated T cells were electroporated with IVT RNA using BTX EM830 (Harvard Apparatus BTX) as previously described *(*Cancer research 2010, 70(22):9053-9061). Briefly, T cells were washed three times and resuspended in OPTI-MEM (Invitrogen) at a final concentration of 1-3 x 10⁸ cells/ml. Subsequently, 0.1 ml of cells were mixed with 10 ug IVT RNA (or as indicated) and electroporated in a 2 mm cuvette.

*ELISA assays.* Target cells, different tumor cell lines expressing CD19, were washed and suspended at 1x10⁶ cells/ml in R10 medium (RPMI 1640 supplemented with 10% fetal calf serum; Invitrogen). 100 ul of each target cell type was added in duplicate to a 96 well round bottom plate (Corning). Effector T cells were washed, and re-suspended at 1x10⁶ cells/ml in R10 medium and then 100 ul of T cells were combined with the target cells in the indicated wells. In addition, wells containing T cells alone were prepared as a control. The plates were incubated at 37° C for 18 to 20 hours. After the incubation, supernatant was harvested and subjected to an ELISA assay (eBioscience, 88-7316-77; 88-7025-77).

*CD107a staining* Cells were plated at an Effector cell:T cell ratio of 1:1 (1x10⁵ effectors to 1x10⁵ targets) in 160 µl of complete RPMI medium in a 96 well plate. 20 µl of phycoerythrin-labeled anti-CD107a antibody (BD Biosciences, 555801) was added and the plate was incubated at 37° C for 1 hour before adding Golgi Stop (2 ul Golgi Stop in 3 ml RPMI medium, 20 ul/well; BD Biosciences, 51-2092KZ ) and incubating the plate for another 2.5 hours. Then 5 µl FITC-anti-CD8 and 5 ul APC-anti-CD3 were added and incubated at 37° C for 30 min. After incubation, the samples were washed with FACS buffer and analyzed by flow cytometry.

*Luciferase based CTL assay.* Naml6-CBG tumor cells were generated and employed in a modified version of a luciferase based cytotoxic T lymphocyte assay. Briefly, click beetle green luciferase (CBG) was cloned into the pELNS vector, packaged into lentivirus, transduced into Naml6 tumor cells and sorted for CBG expression. The resulting Naml6-CBG cells were washed and resuspended at 1x10⁵ cells/ml in R10 medium, and 100 ul of CBG-labeled cells were incubated with different ratios of T cells (e.g. 30:1, 15:1, etc) overnight at 37° C. 100 ul of the mixture was transferred to a 96 well white luminometerplate. 100 ul of substrate was added to the cells and luminescence was immediately determined.

*Mouse xenograft studies.* Studies were performed as previously described with certain modifications *(*Human gene therapy 2011, 22(12):1575-1586; Proceedings of the National Academy of Sciences of the United States of America 2009, 106(9):3360-3365). Briefly, 6-10 week old NOD/SCID gamma (NSG) mice were injected subcutaneously with 1x10⁶ PC3-CBG tumors cells on the right flank at day 0 and the same mice were given SK-OV3-CBG tumor cells (5x10⁶ cells/mouse, subcutaneously.) on the left flank at day 5. The mice were treated with T cells via the tail vein at day 23 post PC3-CBG tumor inoculation, such that both tumors were approximately 200 mm³ in volume. Lentivirally transduced T cells were given at 1x10⁷ cells/mouse (10M), or 3x10⁶ cells/mouse (3M).

The results of the experiments are now described.

### Example 1: T cells expressing TCRs and bispecific antibodies.

Cancer patients treated with anti-tumor antigen TCR re-directed T lymphocytes by lentiviral or retroviral vectors show promising results. In this study, RNA was electroporated into T cells to determine if an efficient therapy for cancer adoptive immunotherapy could be developed. The T cells were compared to assess the in vivo potency of T lymphocytes that expressed exogenous TCR and bispecific antibodies in Naml6 leukemia and A549 lung cancer mouse models.

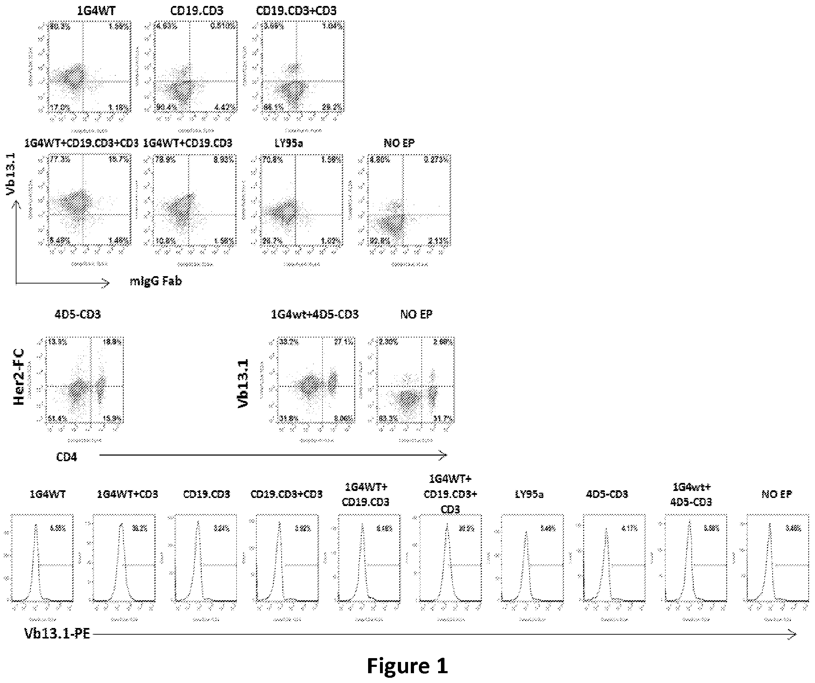

To improve TCR redirected T cell adoptive immunotherapy, T cells were electroporated with TCR RNA and bispecific T cell engagers (BiTEs). **Figure** 1 shows transgene expression in the T cells co-electroporated with TCR and BiTEs. T cells were co-electroporated with CD19.CD3 (upper panel) or 4D5.CD3 (ErbB2) (middle panel) BiTEs with or without CD3zeta and epsilon. Eighteen hours post electroporation, T cells were stained for TCR vb13.1 and mIgG Fab (or Her2-Fc). Lower panel shows TCR (vb13.1) expression 3 days after electroporation

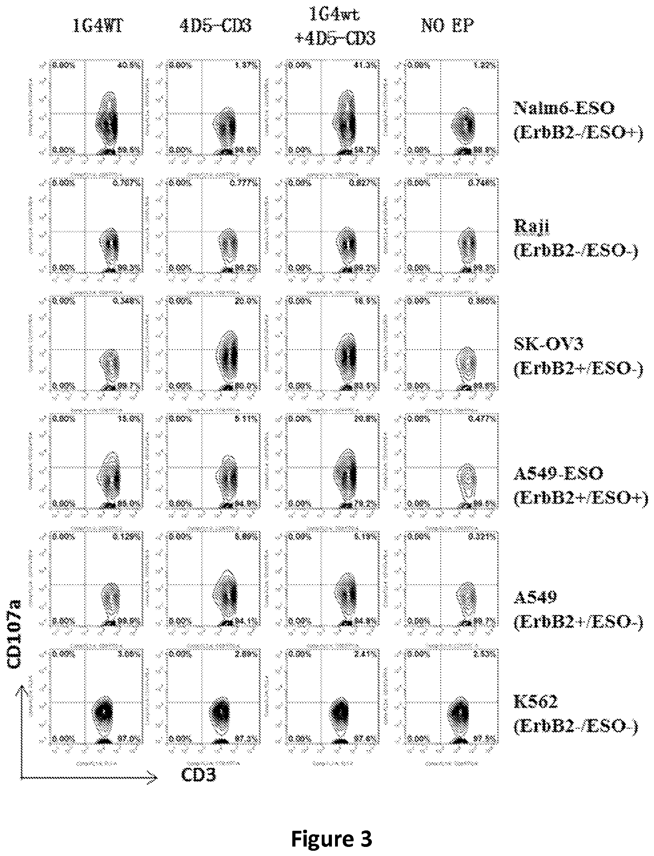

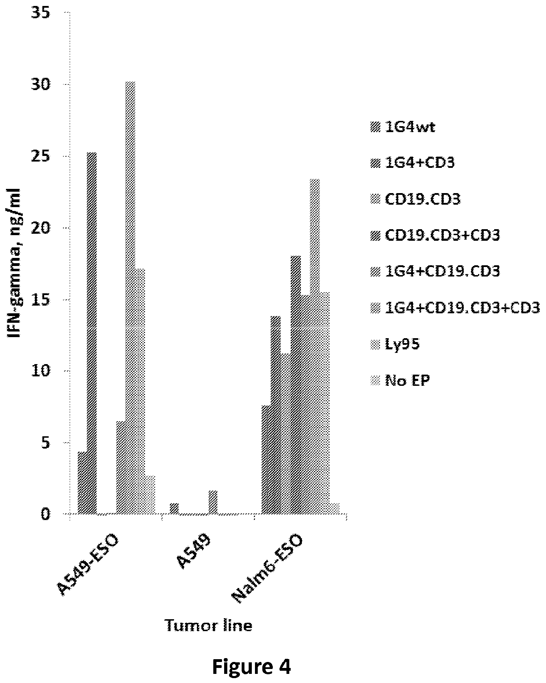

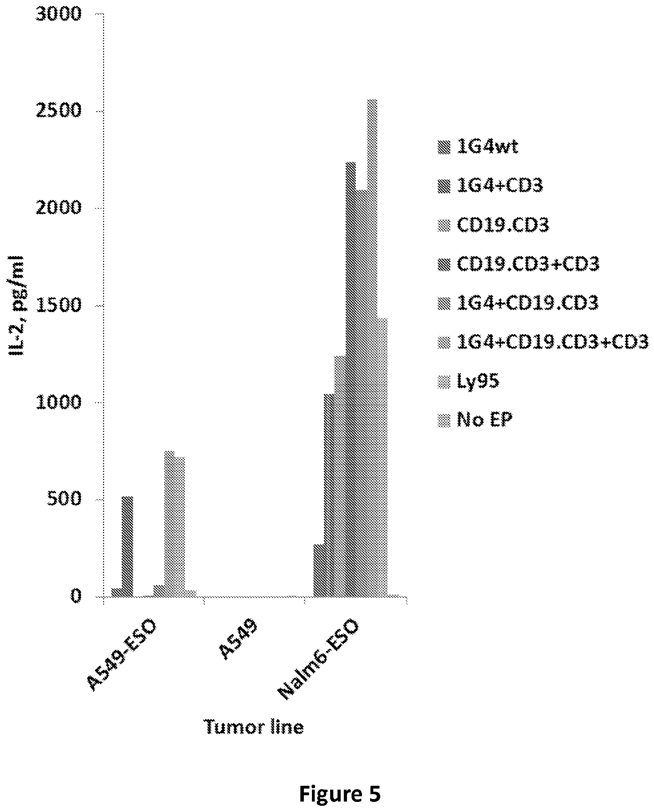

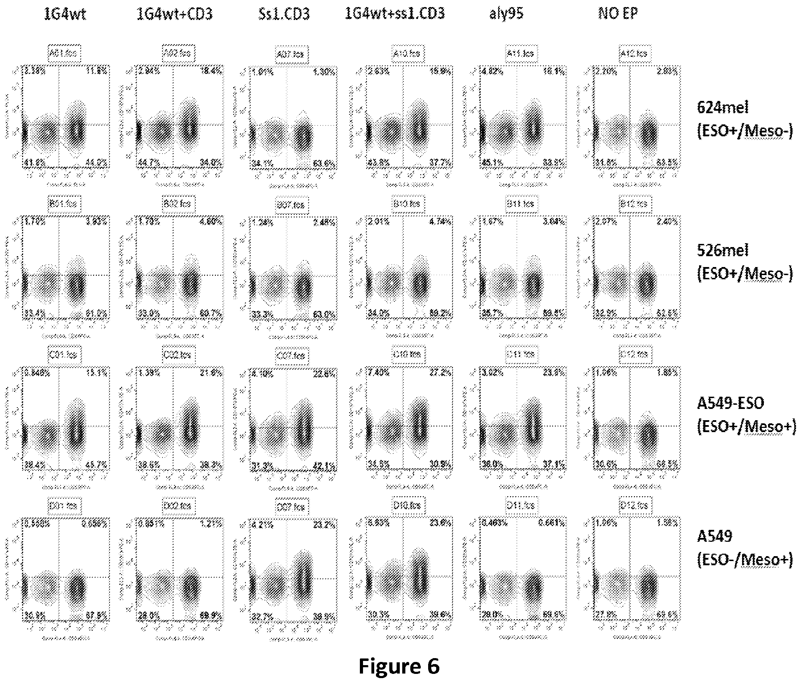

Functionality was improved in the co-electroporated T cells. **Figures 2** and **3** illustrate CD107a was up-regulated in T cells after stimulation with tumor cells. The T cells were co-electroporated with TCR RNA and BiTEs RNA, then stimulated with tumor cell lines having single or double positivity for CD19 and NY-ESO-1 (ESO). Both IFN-gamma and IL-2 production was increased in co-electroporated T cells **(****Figures 4** **and** **5****).** Moreover, CD107a was up-regulated in tumor stimulated T cells expressing NY-ESO-1 TCR (1G4) and mesothelin BiTEs (ss1.CD3) **(****Figure 6****).**

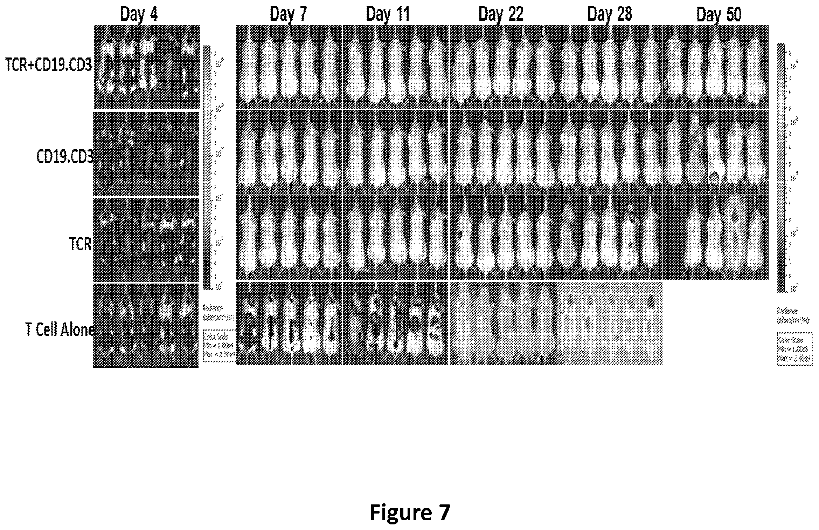

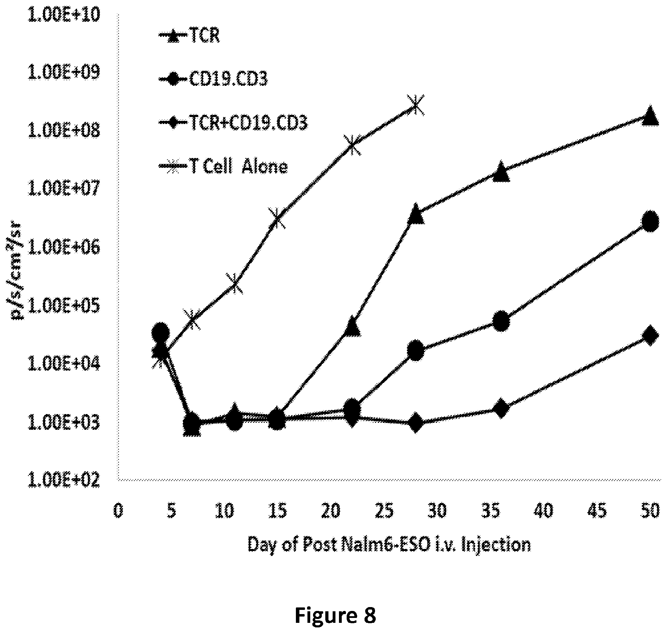

The cells co-expressing the TCR and BiTEs were injected into a leukemia mouse model. In NOD/SCID (NSG) mouse models, RNA electroporated T cells expressing NY-EOS-1 wildtype TCR and CD19.CD3 BiTEs were injected five days after tumor cell injection **(****Figure 7****).** It was found that T cells electroporated with NY-ESO-1 wildtype TCR RNA and CD19.CD3 BiTEs showed potent anti-tumor activity, while T cells with NY-ESO-1 TCR were much less potent **(****Figure 8****).**

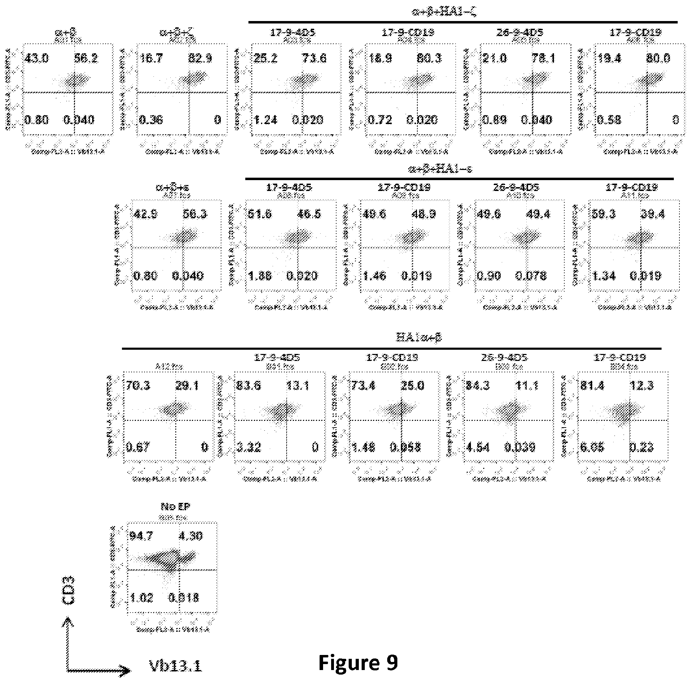

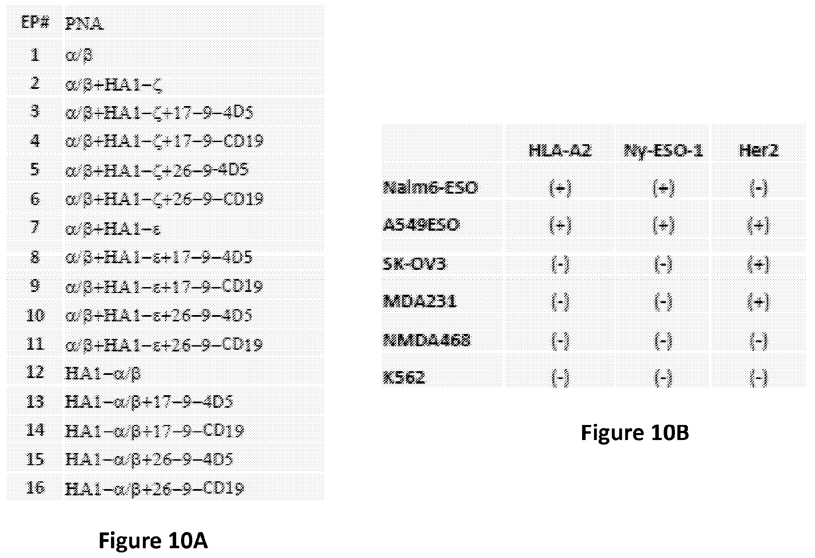

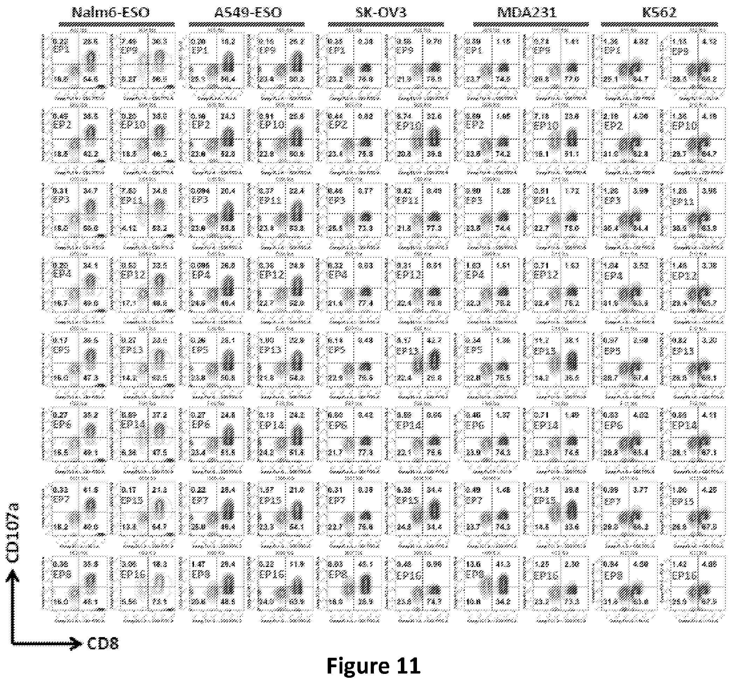

T cells expressing modified TCRs also recognized both cognate and MHC/peptide and surface tumor antigens without HLA restriction when combined with bi-specific antibodies in the T cells **(****Figure 9****).** A list of the constructs of bi-specific antibody RNAs and TCR RNAs electroporated into T cells is shown in **Figures 10A** and **10B****.** T cells expressing the modified NY-ESO-1 TCR and bi-specific antibodies recognized both cognate antigen (HLA-A2/NY-ESO-1) and CD19 or Her2 **(****Figure 11****).**

### Example 2: T cells modified with bispecific antibodies and CLEARs.

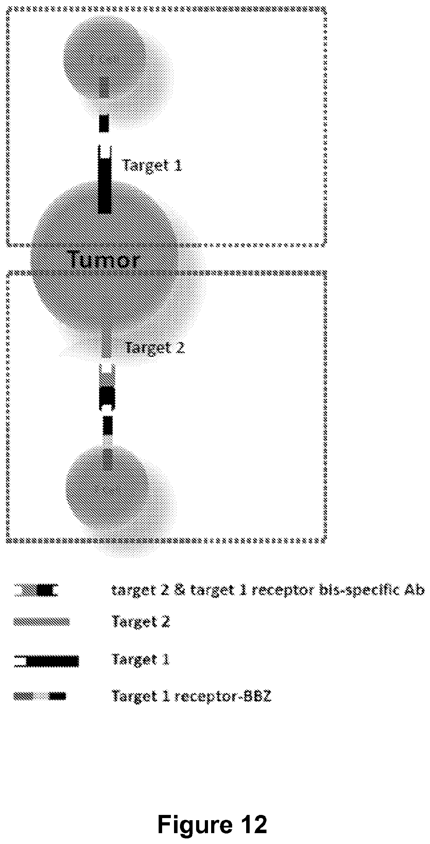

Adoptive immunotherapy of cancers using chimeric antigen receptor (CAR) or T cell receptor (TCR) modified T cells has been shown to be a promising strategy for the treatment of cancers. Due to the heterogeneous properties of cancers, especially solid tumors, targeting single tumor antigens to treat cancers likely leads to immune escape of tumor cells that are either negative for the targeted antigen or down-regulate the targeted antigen. Therefore, targeting multiple tumor antigens simultaneously has the potential to enhance treatment. Instead of pooling multiple single chain variable fragment (scFv) CARs that potentially interfere with each other due to structural similarity, a novel method of targeting multiple tumor antigens by co-introduction of two molecules was developed. The novel molecule was named "chimeric ligand engineered activation receptors (CLEARs)" (target-1) and was composed of an intracellular T cell activation signaling domain, such as CD3 zeta with or without co-stimulatory signal, and an extracellular domain. The extracellular domain was chosen to: 1) recognize an antibody or a specific receptor/ligand and, 2) specifically bind to either a tumor antigen or other molecule that is not expressed on healthy tissues. The cells were also engineered to express bi-specific antibodies or fusion proteins bind tumor antigen (target 2) on one side and the extracellular domain of the CLEARs on the other side. This was to enable T cell recognition of a second tumor expression target **(****Figure 12****).**

After these two molecules were introduced into T lymphocytes, the CLEAR targeted target-1 and at the same time bound to the secreted bi-specific antibody (or fusion protein). The T cells were triggered by either direct recognition of target-1 and/or by simultaneous engagement with CLEARs at target-2 on the tumor cell and secreted bi-specific antibody (or fusion protein).

As a proof of concept, two receptor/ligand targets PD1/PD-L1 and CD27/CD70, important targets in immunotherapy of cancers, were chosen to generate PD1 or CD27 CLEARs. Mesothelin, ErbB2 and CD19 were used as target 2 antigens. Three scFv with different affinities against PD1 (2D3, 4A11 and 4H1)(5) or CD27 (C2177, M709 and M708)(6) were chosen and bi-specific constructs were made with scFv against all the target-2 ligands mentioned above.

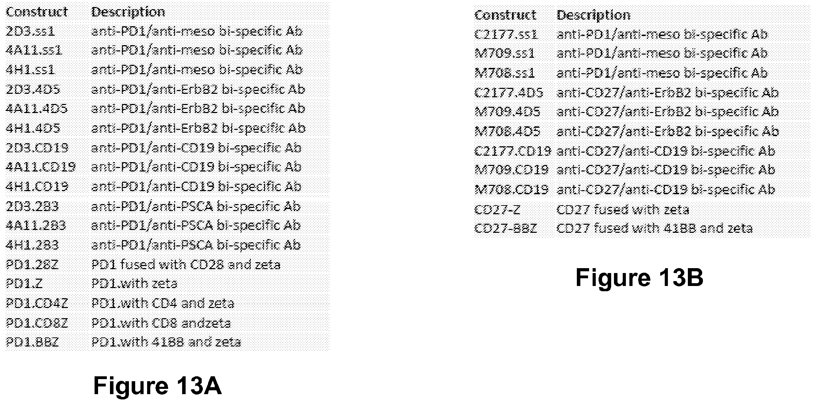

PD1 CLEARs with different co-stimulatory or co-receptor signaling domains were also constructed. CD27 CLEARs were constructed using either CD27 (CD27-Z) (7) or CD27-4-1BB (CD27-BBZ) signaling domains **(****Figures 13A** and **13B****).** All the constructs were made into RNA via in vitro transcription (IVT) vectors and IVT RNA made and used to electroporate CD3/CD28 bead stimulated T cells.

### PD1 or CD27 CLEARs redirected T cells targeting PDL1 or CD70 positive tumors.

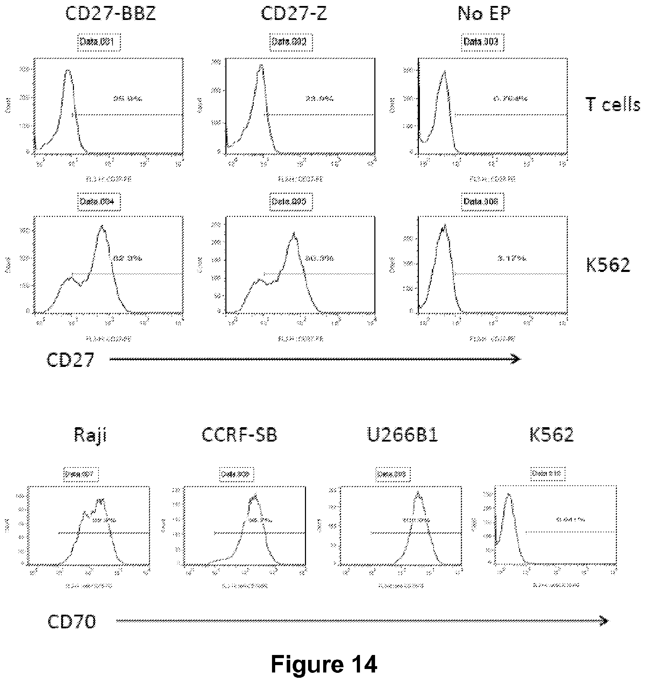

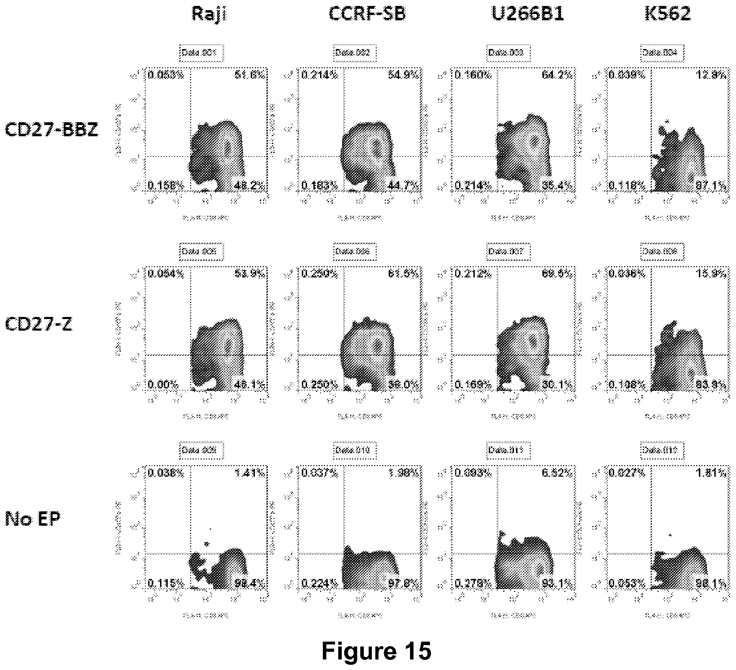

The function of T cells expressing either CD27 or PD1 CLEARs alone was tested. It was found that CD27 CLEARs could be expressed on the cell surface **(****Figure 14****).** When the T cells were stimulated with tumor lines that express CD70, the T cells were specifically activated, which was evidenced by significant up-regulation of CD107a expression **(****Figure 15****).**

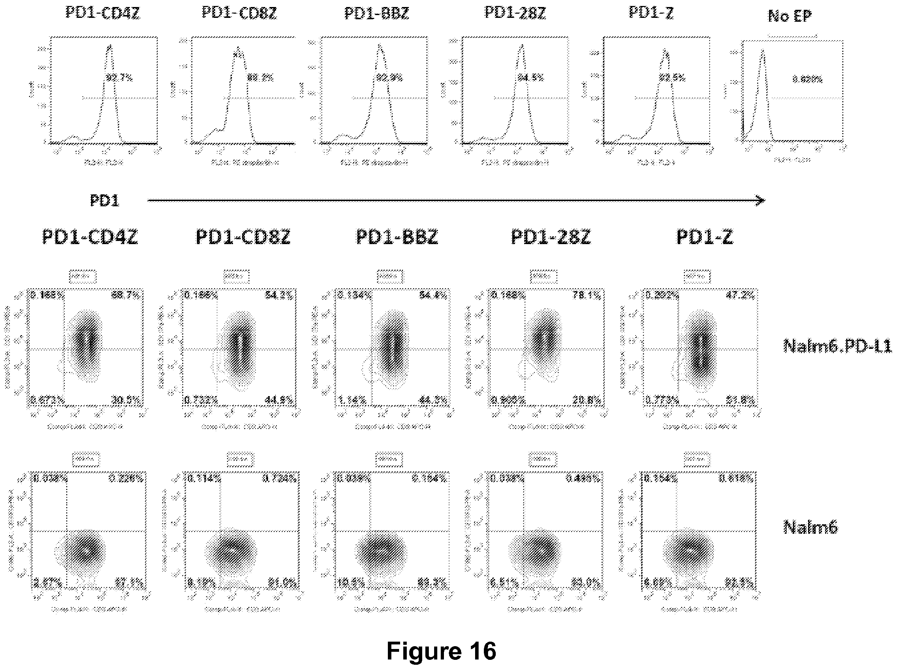

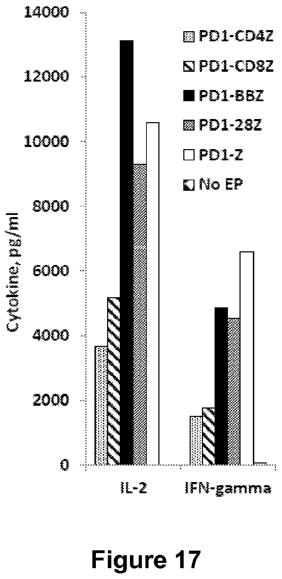

To test if T cell function by using different co-stimulatory (CD28 or 4-1BB) or co-receptor (CD4 or CD8) molecules, five PD1 CLEARs were tested by their expression on T cells **(****Figure 16****)** and their reactivities against PDL1 positive cell line Nalm6-PD-L1 was determined. T cells with all PD1 CLEARs responded to PD-L1 positive tumor strongly as evidenced by about 50% to about 70% CD107a up-regulation and cytokine production. Interestingly, T cells PD1 CLEARs with CD4 or CD8 co-receptor signaling showed comparable CD107a expression with other PD1 CLEARs: CD28, or 4-1BB or without any co-signaling (zeta alone, PD1-Z). Both IFN-gamma and IL-2 secretion was significantly lower **(****Figure 17****),** indicating PD1 CLEAR T cells with CD4 or CD8 co-signaling behaved differently from the others, which may beneficial for the treatment with less toxicity due to cytokine storm.

### Targeting multiple tumor antigens by eCLEARs combining PD1 CLEAR with bispecific antibody.

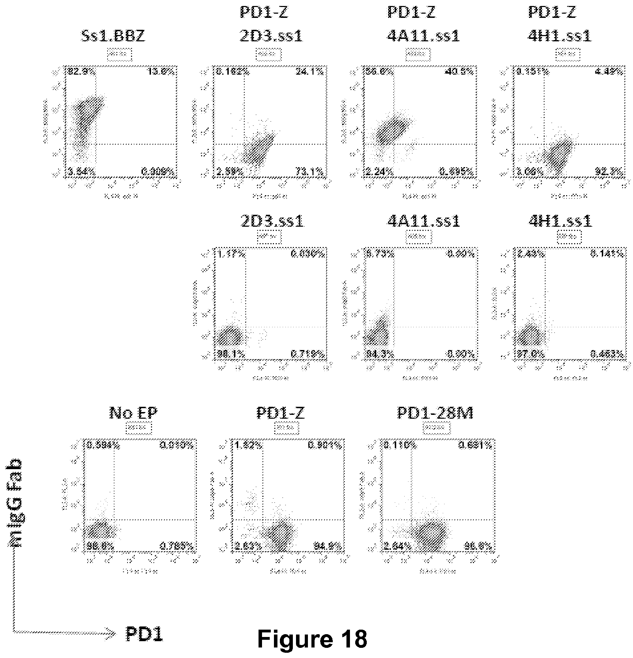

T cells with PD1 eCLEARs were tested by co-electroporating RNAs for both PD1 CLEAR (PD1-Z) and RNAs for bi-specific antibody against PD1 and mesothelin (2D3-ss1, 4A11-ss1 and 4H1-ss1) into T cells. Flow cytometry staining showed that both CLEARs and the bi-specific antibodies could be detected **(****Figure 18****).** More importantly, when these T cells were stimulated with different cell lines that expressed either single or double target antigens, they recognized, in an antigen specific manner, either single antigen positive tumor lines, or double positive tumor lines.

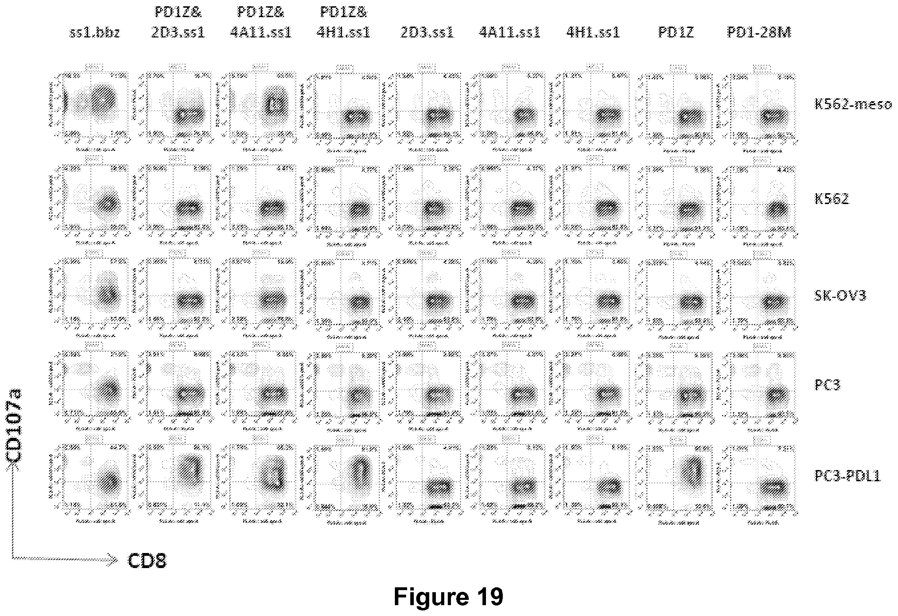

Comparing with the CARc ss1.BBZ, T cells with CLEARs (PD1Z&4A11.ss1) showed comparable lytic ability against mesothelin single positive cell line, K562-meso, in a CD107a up-regulation assay. However, for tumor cell line, PC3-PDL1, that was lenti-virally transduced with PD-L1 and weakly positive for medothelin, T cells with eCLEARs had much higher CD107a up-regulation than T cells with ss1.bbz CAR **(****Figure 19****).**

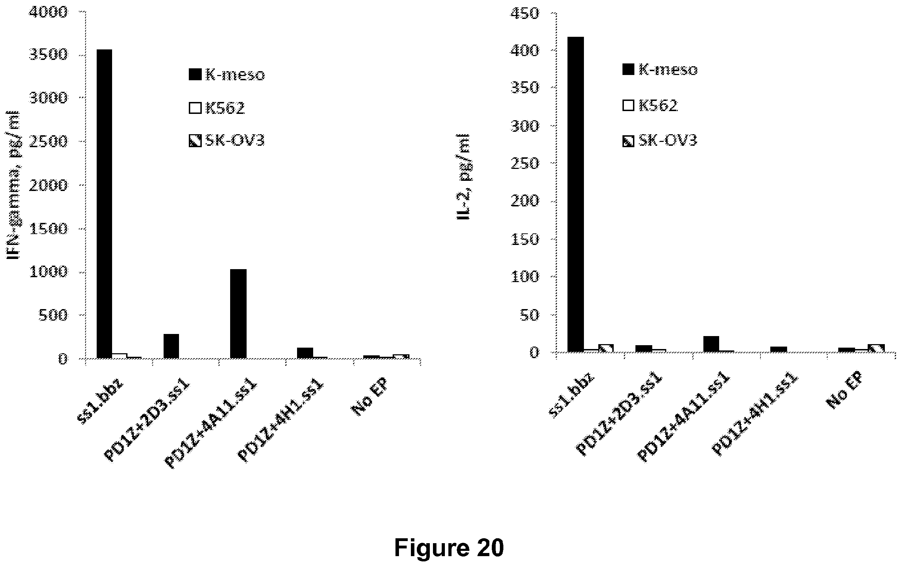

Cytokine production (IFN-gamma and IL-2) of the stimulated T cells was tested by ELISA. Although T cells transferred with CLEARs RNA showed as high as , or even higher than ss1.BBZ CAR transferred T cells cytokine production, there was nearly no detectable cytokines for both IL-2 and IFN-gamma for CLEARs transferred T cells stimulated with either single positive or double positive target cell lines. In contrast, T cells transferred with ss1.bbz CAR secreted high levels of both IL-2 and IFN-gamma **(****Figure 20****).** The property of high lytic ability with low cytokine production may have benefit in treating cancer patients, since the chance of developing an adverse cytokine storm could be reduced.

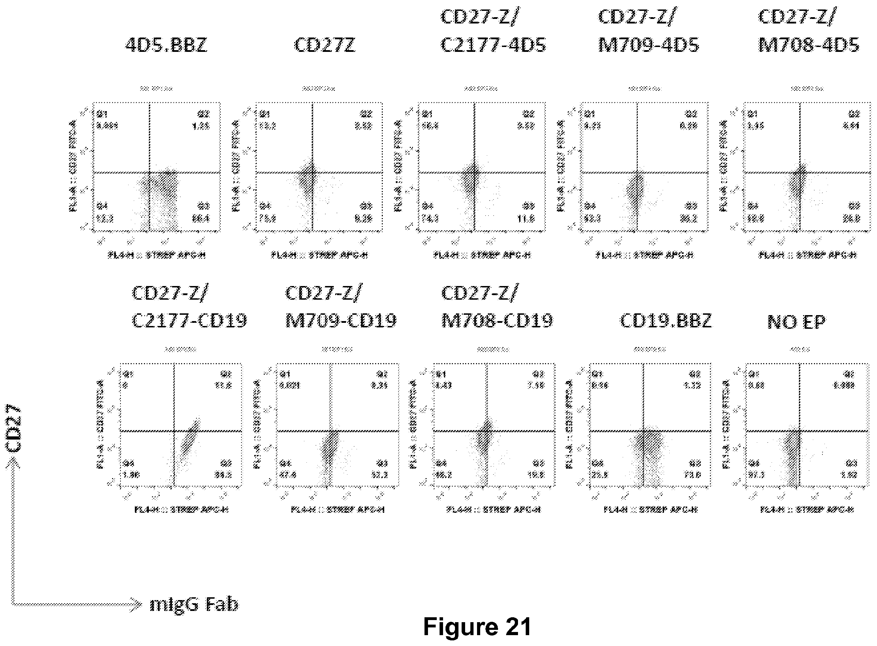

To test if CLEARs could efficiently target tumor antigens other than mesothelin, bi-specific antibody constructs against PD1 with either ErbB2 (4D5), or CD19, or PSCA (2B3) were generated. RNA for those new bi-specifc antibodies were co-electroporated into T cells and compared with their relevant CARs (4D5.BBZ, 19.BBZ or 2B3.BBZ). CD107a expression was examined after these T cells were stimulated with tumor lines and the results showed that PD1/CD19 CLEARs (PD1-Z/2D3-CD19, PD1-Z/4A11-CD19) could recognize CD19 single positive tumor cells, Nalm6, as efficiently as CD19 CAR (19.BBZ). CD19 CAR T cells decreased their reactively against CD19/PDL1 double positive Nalm6-PDL1 cells (52.2% for CD107a+/CD8+ versus 56.6%). CD107a was also slightly increased for PD1/CD19 CLEARs T cells. The results also showed that, for both PD1/4D5 and PD1/2B3 CLEARs, CD107a expressed higher in T cells with CLEARs than with the relevant CARs when the T cells were stimulated with double positive tumors **(****Figure 21****).**

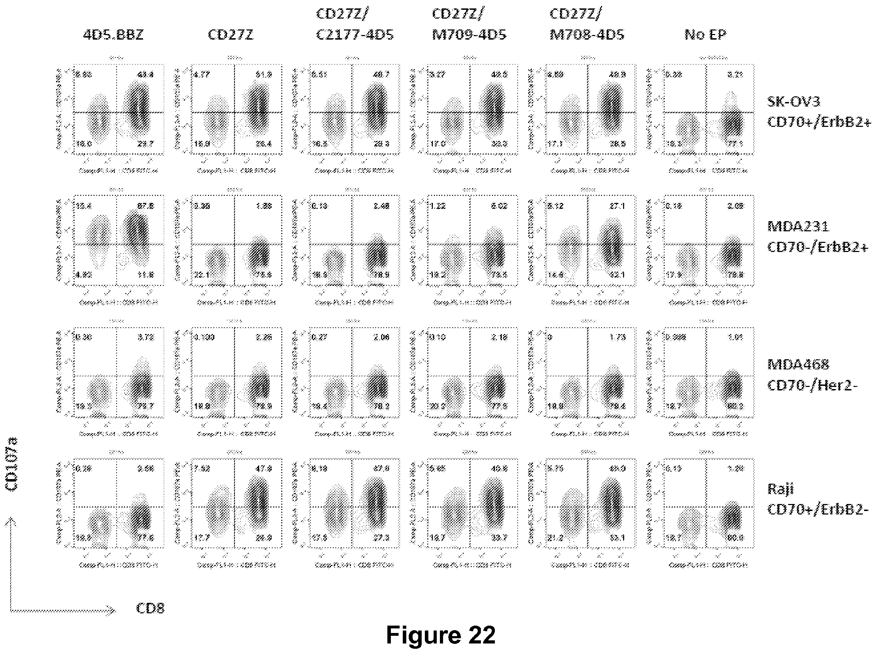

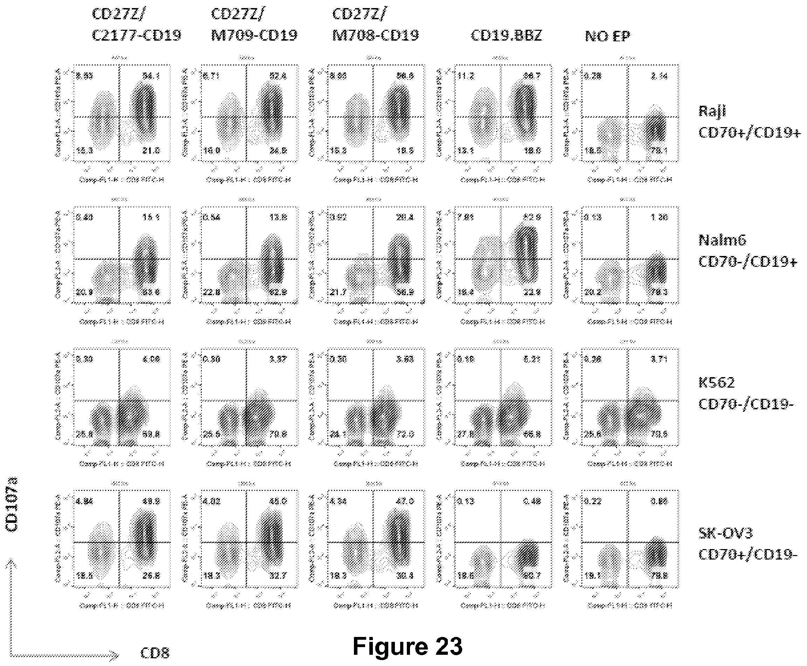

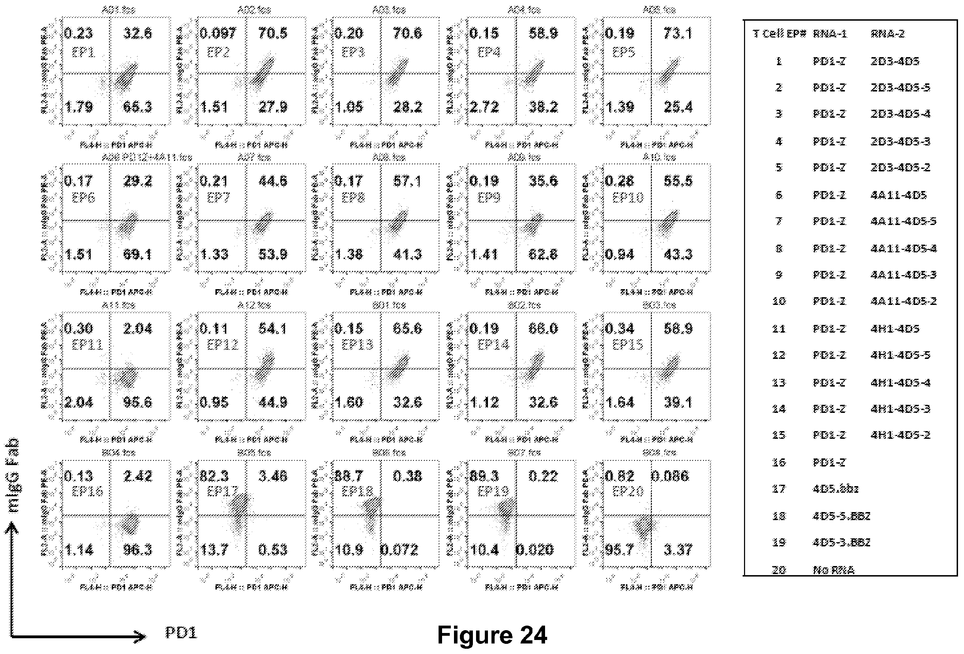

CD27 CLEARs against two tumor antigens, ErbB2 and CD19 were also tested. As shown in **Figure 22****,** surface staining of both CD27 and mIgG Fab (upper panel for CD27-z with ErbB2 target and lower panel for CD27-z with CD19 target) showed transgene expression of both CD27 and secreted bi-specific antibody that bound to the T cells at different levels. When the T cells were stimulated with tumor lines that are single or double positive for CD70 and ErbB2, antigen specific T cell activation was observed. In particular, CD70-/ErbB2+ tumor MDA231, CD27-Z/M708-4D5 showed significantly increased CD107a expression, at about 33.2%, over the about 3% background levels **(****Figure 23****).** When the T cells with CD27/CD19 CLEARs were stimulated with cell lines that are single or double positive for CD70 and CD19 **(****Figure 24****),** same as seen for CD27/ErbB2, antigen specific T cell activation was observed and CD27/CD17 CLEARs showed clear anti-tumor activity against both CD27/CD19 double positive cells, or either CD70 or CD19 single positive cells. Taken together with the results from PD1/Mesothelin eCLEARs, the data shown herein indicates that tumor antigens could be targeted indirectly using CLEARs and bi-specific antibodies. Thus, two tumor antigens could be targeted simultaneously without interfering each other.

### Targeting ErbB2 using affinity decreased anti-ErbB2 scFv (4D5-5, 4D5-4, 4D4-3 and 4D5-2) with eCLEARs combined with PD1 CLEAR and bispecific Ab.

Anti-ErbB2 4D5 is a high affinity scFv that is not for use in T cell based cancer treatments. T cells with PD1 eCLEARs were tested by co-electroporating RNAs for both PD1 CLEAR (PD1-Z) and bispecific antibodies against PD1 (2D3, 4A11 or 4H1) and ErbB2 (4D5, 4D5-2, 4D5-3, 4D5-4 or 4D5-5). Flow cytometry staining showed that both PD1 CLEAR and the bi-specific antibody was detectable in most of the co-electroporated T cells **(****Figure 24****).**

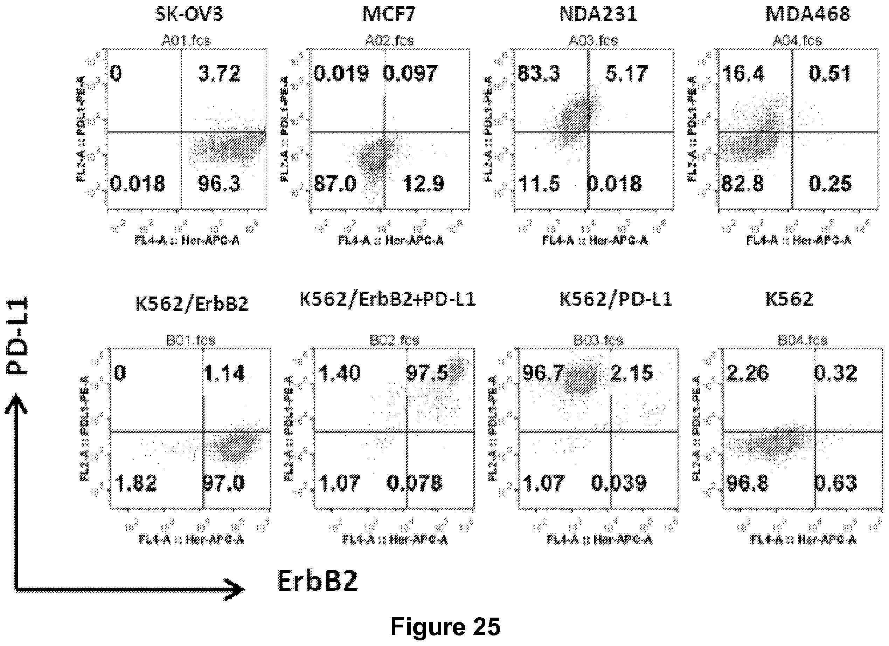

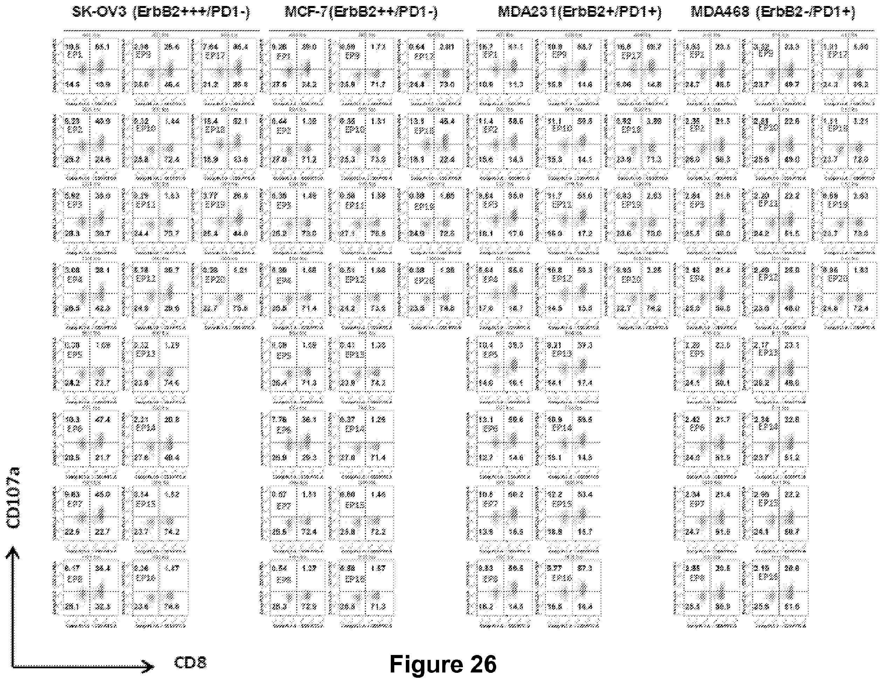

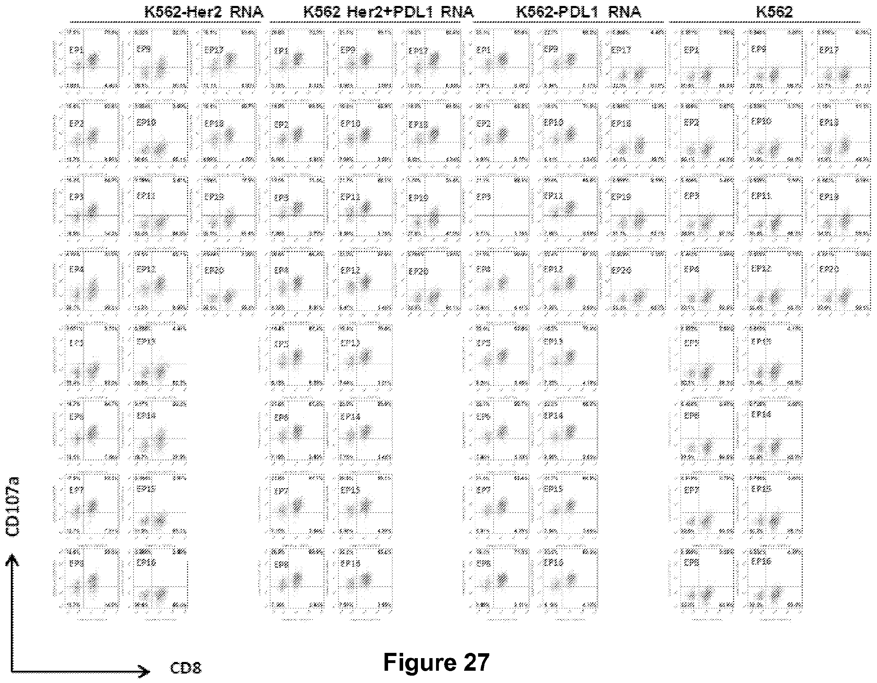

T cells were then stimulated with different tumor cell lines that expressed either single or double target antigens or K562 cells electroporated with ErbB2 and/or PD-L1 RNA **(****Figure 25****).** As showed in **Figures 26** and **27****,** it was found that T cells co-electroporated with both PD1 CLEAR and anti-PD1/anti-ErbB2 bispecific antibody showed affinity associated T cell activity against ErbB2 high expressing cell lines, SK-OV3 and ErbB2 RNA electroporated K562. 4H1-4D5, 4H1-4D5-4 and 4D5-2 were found to have insufficient affinity to be conclusive.

T cells co-electroporated with PD1 CLEAR and bispecific antibody with high affinity against ErbB2 scFv (4D5), 2D3-4D5 and 4A11-4D5 showed reactivity against PD1 negative and low level ErbB2 expressing tumor cells, MCF7. T cells co-electroporated with PD1 CLEAR and bispecific antibody with lower affinity against ErbB2 scFv (4D5), 4D5-5, 4D5-4 and 4D5-3 showed completely no reactivity against PD1 negative and low level ErbB2 expressing tumor cells, MCF7. This suggested that the CLEARs and bi-specific antibodies could be safely used for treating cancer patient with ErbB2 overexpressing tumors.
SEQ ID NO:20, 2D3.ss1: 
atgggctggtcttgcatcatcctgtttctggtcgccaccgccaccggggtccacagtgagatcgttctcacccaatcccccgccac
actgtcgctctccccaggcgagcgggccacgctgtcgtgtcgcgcgagccagagcgtttcctcctacctcgcctggtaccagca
gaagccgggccaggcccctcgcctgctgatctacgatacttcgaatagagccaccggtatccctgcaaggttctccggatccgg
gtcagggacggatttcaccctgaccatttcctccctggagccagaggatttcgccgtgtactactgccagcagcgcagtaactgg
cctctgactttcggtggcggcacaaaggtggagatcaaggggggcggcggcagtgggggcgggggcagcgggggcggag
gctcgcaggtccagctcgtggagtcggggggggacgtggtccagccaggcaggagcctgaggctgtcatgcgctgcgtccg
gcctgacgttcaccaactacggcttccactgggtgagacaggcacccgggaagggcctggaatgggtggccgtgatctggtac
gacgggagcaagaagtactacgcagactcagtgaagggccgattcaccatcagccgggacaactctaagaacacactgtacct
gcagatgaacaatttgcgcgccgaggataccgccgtatattattgcgccacaggcgacgactattggggacagggcaccctggt
cacggtaagtagtggaggtggtggatcccaggtacaactgcagcagtctgggcctgagctggagaagcctggcgcttcagtga
agatatcctgcaaggcttctggttactcattcactggctacaccatgaactgggtgaagcagagccatggaaagagccttgagtg
gattggacttattactccttacaatggtgcttctagctacaaccagaagttcaggggcaaggccacattaactgtagacaagtcatc
cagcacagcctacatggacctcctcagtctgacatctgaagactctgcagtctatttctgtgcaagggggggttacgacgggagg
ggttttgactactggggccaagggaccacggtcaccgtctcctcaggtggaggcggttcaggcggcggtggctctagcggtgg
cggatcggacatcgagctcactcagtctccagcaatcatgtctgcatctccaggggagaaggtcaccatgacctgcagtgccag
 
ctcaagtgtaagttacatgcactggtaccagcagaagtcaggcacctcccccaaaagatggatttacgacacatccaaactggct
tctggagtcccaggtcgcttcagtggcagtgggtctggaaactcttactctctcacaatcagcagcgtggaggctgaagacgacg
caacttattactgccagcagtggagtaagcaccctctcacgtacggtgctgggacaaagttggaaatcaaataa
SEQ ID NO:21, 4A11.ssl: 
atgggctggtcctgtatcatattattcctggtcgcgaccgccaccggggtgcactccgacatccagatgactcagtccccatcctc
cctgtcggcgtccgttggcgaccgggtctcgattacatgccgcgcctcccagggcatctcctcctggctcgcctggtaccagca
gaagcctgagaaggcccccaagtcattaatctacgcagcctccaatctgaggtccggcgtgcccagtagattctcgggctccgg
gtcgggcactgattttaccctcaccatcagttcgctccagccagaggacttcgctacgtactattgccagcagtactattcctaccc
acgcaccttcgggcagggcaccaaggtcgagatcaagggcggcggcgggtccggcggaggcgggtctggcggtggtggtt
cgcagctgcagctgcaagagtccgggcctggcctcgtgaagcccagcgagaccctgtcattgacatgcaccgtgagtggggg
gagtctgtcccgctcctccttcttctggggctggattcgccagccgcccggcaaggggctggaatggatcgggtcgatctactac
agtgggtcgacttactacaatccaagtctgaagagccgtgtgaccatctcagtggatacctcgaagaatcagttcagcctgaagct
ctcctccgtcaccgcggccgatacggcagtgtactactgtgtgcgcgattacgacatcctcactggcgatgaggactactggggt
caaggcaccctagtcacggtgtcgtcgggaggtggtggatcccaggtacaactgcagcagtctgggcctgagctggagaagc
ctggcgcttcagtgaagatatcctgcaaggcttctggttactcattcactggctacaccatgaactgggtgaagcagagccatgga
aagagccttgagtggattggacttattactccttacaatggtgcttctagctacaaccagaagttcaggggcaaggccacattaact
gtagacaagtcatccagcacagcctacatggacctcctcagtctgacatctgaagactctgcagtctatttctgtgcaaggggggg
ttacgacgggaggggttttgactactggggccaagggaccacggtcaccgtctcctcaggtggaggcggttcaggcggcggtg
gctctagcggtggcggatcggacatcgagctcactcagtctccagcaatcatgtctgcatctccaggggagaaggtcaccatga
cctgcagtgccagctcaagtgtaagttacatgcactggtaccagcagaagtcaggcacctcccccaaaagatggatttacgaca
catccaaactggcttctggagtcccaggtcgcttcagtggcagtgggtctggaaactcttactctctcacaatcagcagcgtggag
gctgaagacgacgcaacttattactgccagcagtggagtaagcaccctctcacgtacggtgctgggacaaagttggaaatcaaa
taa
SEQ ID NO:22, 4H1.ss1: 
atggggtggagttgcatcattctgttcctcgtggcgaccgcaacaggcgtgcacagcgagatcgtgctcacccagtcaccagcc
accttatccttaagtcccggcgaacgcgccaccctgtcctgcagagcgtcccagtcggtcagcagttatctggcgtggtaccagc
agaagcccggccaagccccacgcctgctcatctacgacgcgtcgaatcgcgccacgggtattcccgcacggtttagcggctcc
ggttcagggactgactttaccctgacgatctcgagtctagagccggaggacttcgcggtgtactactgccagcagtccagcaact
ggccgcgcaccttcggccagggcacaaaggtggagatcaaggggggcgggggctcgggtggcgggggctccggaggcgg
gggctcccaagtgtacctggttgaatcgggcgggggcgtggtgcagcctgggcgctcgctgcgcctcagttgcgccgcgtcc
ggattcacattttccaactacgggatgcactgggtgcgccaagctccgggaaaggggctggagtgggtggccctgatctggtac
gacggttccaataagtactatgccgatagcgtgaagggccggttcacgatatccagggataactccaagaacactctctatctgc
 
agatgacctcactgcgcgtggaggacactgccgtctattactgcgcctccaatgtggatcactggggacagggcaccctggtga
ccgtaagctcgggaggtggtggatcccaggtacaactgcagcagtctgggcctgagctggagaagcctggcgcttcagtgaag
atatcctgcaaggcttctggttactcattcactggctacaccatgaactgggtgaagcagagccatggaaagagccttgagtggat
tggacttattactccttacaatggtgcttctagctacaaccagaagttcaggggcaaggccacattaactgtagacaagtcatccag
cacagcctacatggacctcctcagtctgacatctgaagactctgcagtctatttctgtgcaagggggggttacgacgggaggggt
tttgactactggggccaagggaccacggtcaccgtctcctcaggtggaggcggttcaggcggcggtggctctagcggtggcgg
atcggacatcgagctcactcagtctccagcaatcatgtctgcatctccaggggagaaggtcaccatgacctgcagtgccagctca
agtgtaagttacatgcactggtaccagcagaagtcaggcacctcccccaaaagatggatttacgacacatccaaactggcttctg
gagtcccaggtcgcttcagtggcagtgggtctggaaactcttactctctcacaatcagcagcgtggaggctgaagacgacgcaa
cttattactgccagcagtggagtaagcaccctctcacgtacggtgctgggacaaagttggaaatcaaataa
SEQ ID NO:23, 2D3.BBZ: 
atgggctggtcttgcatcatcctgtttctggtcgccaccgccaccggggtccacagtgagatcgttctcacccaatcccccgccac
actgtcgctctccccaggcgagcgggccacgctgtcgtgtcgcgcgagccagagcgtttcctcctacctcgcctggtaccagca
gaagccgggccaggcccctcgcctgctgatctacgatacttcgaatagagccaccggtatccctgcaaggttctccggatccgg
gtcagggacggatttcaccctgaccatttcctccctggagccagaggatttcgccgtgtactactgccagcagcgcagtaactgg
cctctgactttcggtggcggcacaaaggtggagatcaaggggggcggcggcagtgggggcgggggcagcgggggcggag
gctcgcaggtccagctcgtggagtcggggggggacgtggtccagccaggcaggagcctgaggctgtcatgcgctgcgtccg
gcctgacgttcaccaactacggcttccactgggtgagacaggcacccgggaagggcctggaatgggtggccgtgatctggtac
gacgggagcaagaagtactacgcagactcagtgaagggccgattcaccatcagccgggacaactctaagaacacactgtacct
gcagatgaacaatttgcgcgccgaggataccgccgtatattattgcgccacaggcgacgactattggggacagggcaccctggt
cacggtaagtagtaccacgacgccagcgccgcgaccaccaacaccggcgcccaccatcgcgtcgcagcccctgtccctgcg
cccagaggcgtgccggccagcggcggggggcgcagtgcacacgagggggctggacttcgcctgtgatatctacatctgggc
gcccttggccgggacttgtggggtccttctcctgtcactggttatcaccctttactgcaaacggggcagaaagaaactcctgtatat
attcaaacaaccatttatgagaccagtacaaactactcaagaggaagatggctgtagctgccgatttccagaagaagaagaagg
aggatgtgaactgagagtgaagttcagcaggagcgcagacgcccccgcgtacaagcagggccagaaccagctctataacga
gctcaatctaggacgaagagaggagtacgacgttttggacaagagacgtggccgggaccctgagatggggggaaagccgag
aaggaagaaccctcaggaaggcctgtacaatgaactgcagaaagataagatggcggaggcctacagtgagattgggatgaaa
ggcgagcgccggaggggcaaggggcacgatggcctttaccagggtctcagtacagccaccaaggacacctacgacgccctt
cacatgcaggccctgccccctcgctaa
SEQ ID NO:24, 4A11.BBZ: 
atgggctggtcctgtatcatattattcctggtcgcgaccgccaccggggtgcactccgacatccagatgactcagtccccatcctc
cctgtcggcgtccgttggcgaccgggtctcgattacatgccgcgcctcccagggcatctcctcctggctcgcctggtaccagca
 
gaagcctgagaaggcccccaagtcattaatctacgcagcctccaatctgaggtccggcgtgcccagtagattctcgggctccgg
gtcgggcactgattttaccctcaccatcagttcgctccagccagaggacttcgctacgtactattgccagcagtactattcctaccc
acgcaccttcgggcagggcaccaaggtcgagatcaagggcggcggcgggtccggcggaggcgggtctggcggtggtggtt
cgcagctgcagctgcaagagtccgggcctggcctcgtgaagcccagcgagaccctgtcattgacatgcaccgtgagtggggg
gagtctgtcccgctcctccttcttctggggctggattcgccagccgcccggcaaggggctggaatggatcgggtcgatctactac
agtgggtcgacttactacaatccaagtctgaagagccgtgtgaccatctcagtggatacctcgaagaatcagttcagcctgaagct
ctcctccgtcaccgcggccgatacggcagtgtactactgtgtgcgcgattacgacatcctcactggcgatgaggactactggggt
caaggcaccctagtcacggtgtcgtcgaccacgacgccagcgccgcgaccaccaacaccggcgcccaccatcgcgtcgcag
cccctgtccctgcgcccagaggcgtgccggccagcggcggggggcgcagtgcacacgagggggctggacttcgcctgtgat
atctacatctgggcgcccttggccgggacttgtggggtccttctcctgtcactggttatcaccctttactgcaaacggggcagaaa
gaaactcctgtatatattcaaacaaccatttatgagaccagtacaaactactcaagaggaagatggctgtagctgccgatttccaga
agaagaagaaggaggatgtgaactgagagtgaagttcagcaggagcgcagacgcccccgcgtacaagcagggccagaacc
agctctataacgagctcaatctaggacgaagagaggagtacgacgttttggacaagagacgtggccgggaccctgagatggg
gggaaagccgagaaggaagaaccctcaggaaggcctgtacaatgaactgcagaaagataagatggcggaggcctacagtga
gattgggatgaaaggcgagcgccggaggggcaaggggcacgatggcctttaccagggtctcagtacagccaccaaggacac
ctacgacgcccttcacatgcaggccctgccccctcgctaa
SEQ ID NO:25, 4H1.BBZ: 
atggggtggagttgcatcattctgttcctcgtggcgaccgcaacaggcgtgcacagcgagatcgtgctcacccagtcaccagcc
accttatccttaagtcccggcgaacgcgccaccctgtcctgcagagcgtcccagtcggtcagcagttatctggcgtggtaccagc
agaagcccggccaagccccacgcctgctcatctacgacgcgtcgaatcgcgccacgggtattcccgcacggtttagcggctcc
ggttcagggactgactttaccctgacgatctcgagtctagagccggaggacttcgcggtgtactactgccagcagtccagcaact
ggccgcgcaccttcggccagggcacaaaggtggagatcaaggggggcgggggctcgggtggcgggggctccggaggcgg
gggctcccaagtgtacctggttgaatcgggcgggggcgtggtgcagcctgggcgctcgctgcgcctcagttgcgccgcgtcc
ggattcacattttccaactacgggatgcactgggtgcgccaagctccgggaaaggggctggagtgggtggccctgatctggtac
gacggttccaataagtactatgccgatagcgtgaagggccggttcacgatatccagggataactccaagaacactctctatctgc
agatgacctcactgcgcgtggaggacactgccgtctattactgcgcctccaatgtggatcactggggacagggcaccctggtga
ccgtaagctcgaccacgacgccagcgccgcgaccaccaacaccggcgcccaccatcgcgtcgcagcccctgtccctgcgcc
cagaggcgtgccggccagcggcggggggcgcagtgcacacgagggggctggacttcgcctgtgatatctacatctgggcgc
ccttggccgggacttgtggggtccttctcctgtcactggttatcaccctttactgcaaacggggcagaaagaaactcctgtatatatt
caaacaaccatttatgagaccagtacaaactactcaagaggaagatggctgtagctgccgatttccagaagaagaagaaggag
gatgtgaactgagagtgaagttcagcaggagcgcagacgcccccgcgtacaagcagggccagaaccagctctataacgagct
caatctaggacgaagagaggagtacgacgttttggacaagagacgtggccgggaccctgagatggggggaaagccgagaag
gaagaaccctcaggaaggcctgtacaatgaactgcagaaagataagatggcggaggcctacagtgagattgggatgaaaggc
 
gagcgccggaggggcaaggggcacgatggcctttaccagggtctcagtacagccaccaaggacacctacgacgcccttcac
atgcaggccctgccccctcgctaa
SEQ ID NO:26, PD1.28M: 
atgcagatcccacaggcgccctggccagtcgtctgggcggtgctacaactgggctggcggccaggatggttcttagactcccc
agacaggccctggaacccccccaccttctccccagccctgctcgtggtgaccgaaggggacaacgccaccttcacctgcagct
tctccaacacatcggagagcttcgtgctaaactggtaccgcatgagccccagcaaccagacggacaagctggccgccttcccc
gaggaccgcagccagcccggccaggactgccgcttccgtgtcacacaactgcccaacgggcgtgacttccacatgagcgtgg
tcagggcccggcgcaatgacagcggcacctacctctgtggggccatctccctggcccccaaggcgcagatcaaagagagcct
gcgggcagagctcagggtgacagagagaagggcagaagtgcccacagcccaccccagcccctcacccaggccagccggc
cagttccaaaccctggtgttttgggtgctggtggtggttggtggagtcctggcttgctatagcttgctagtaacagtggcctttattatt
ttctgggtgaggagtaagaggagcaggctcctgcacagtgacttcttctttactgcccgccgcgccgggcccacccgcaag
cattaccaggcctatgccgcaccacgcgacttcgcagcctatcgctcc
SEQ ID NO:27, PD1.Z: 
atgcagatcccacaggcgccctggccagtcgtctgggcggtgctacaactgggctggcggccaggatggttcttagactcccc
agacaggccctggaacccccccaccttctccccagccctgctcgtggtgaccgaaggggacaacgccaccttcacctgcagct
tctccaacacatcggagagcttcgtgctaaactggtaccgcatgagccccagcaaccagacggacaagctggccgccttcccc
gaggaccgcagccagcccggccaggactgccgcttccgtgtcacacaactgcccaacgggcgtgacttccacatgagcgtgg
tcagggcccggcgcaatgacagcggcacctacctctgtggggccatctccctggcccccaaggcgcagatcaaagagagcct
gcgggcagagctcagggtgacagagagaagggcagaagtgcccacagcccaccccagcccctcacccaggccagccggc
cagttccaaaccctggtgttttgggtgctggtggtggttggtggagtcctggcttgctatagcttgctagtaacagtggcctttattatt
ttctgggtggatagagtgaagttcagcaggagcgcagacgcccccgcgtaccagcagggccagaaccagctctataacgagc
tcaatctaggacgaagagaggagtacgatgttttggacaagagacgtggccgggaccctgagatggggggaaagccgagaa
ggaagaaccctcaggaaggcctgtacaatgaactgcagaaagataagatggcggaggcctacagtgagattgggatgaaagg
cgagcgccggaggggcaaggggcacgatggcctttaccagggtctcagtacagccaccaaggacacctacgacgcccttca
catgcaggccctgccccctcgctaa
SEQ ID NO:28, 2D3.4D5: 
atgggctggtcttgcatcatcctgtttctggtcgccaccgccaccggggtccacagtgagatcgttctcacccaatcccccgccac
actgtcgctctccccaggcgagcgggccacgctgtcgtgtcgcgcgagccagagcgtttcctcctacctcgcctggtaccagca
gaagccgggccaggcccctcgcctgctgatctacgatacttcgaatagagccaccggtatccctgcaaggttctccggatccgg
gtcagggacggatttcaccctgaccatttcctccctggagccagaggatttcgccgtgtactactgccagcagcgcagtaactgg
cctctgactttcggtggcggcacaaaggtggagatcaaggggggcggcggcagtgggggcgggggcagcgggggcggag
 
gctcgcaggtccagctcgtggagtcggggggggacgtggtccagccaggcaggagcctgaggctgtcatgcgctgcgtccg
gcctgacgttcaccaactacggcttccactgggtgagacaggcacccgggaagggcctggaatgggtggccgtgatctggtac
gacgggagcaagaagtactacgcagactcagtgaagggccgattcaccatcagccgggacaactctaagaacacactgtacct
gcagatgaacaatttgcgcgccgaggataccgccgtatattattgcgccacaggcgacgactattggggacagggcaccctggt
cacggtaagtagtggaggtggtggatccgacatccagatgacccagtccccttcctccctctctgcctctgtgggagaccgcgtt
accatcacatgccgagcttcccaggacgtgaacacagccgtggcctggtaccagcagaagcccgggaaggcacccaaactcc
tcatctactccgcctccttcctatacagtggcgtgccttcccgattctccggctccaggagtggcacggactttacgctcaccattag
tagcctgcagcccgaagacttcgcgacctactattgtcagcaacactacacgacgccaccaactttcggccagggtaccaaggt
cgagattaagcgaaccggcagtaccagtgggtctggcaagcccggcagcggcgagggatccgaggtccagctggtcgagtc
cggcgggggcctggtgcagccgggcggctcgctgaggttatcttgcgccgccagtggcttcaacatcaaggatacttacatcca
ctgggtgaggcaggctccgggcaagggcctggaatgggtggctaggatctaccctactaacgggtacacacgctacgcagatt
cggtgaaaggccgcttcactatctccgccgacacctcgaagaacactgcttacctgcagatgaactccctcagggccgaagata
ctgcagtctactactgctcccgctggggtggggacggcttctacgccatggacgtgtggggtcagggcactctagttacagtgtc
atcctaa
SEQ ID NO:29, 4A11.4D5: 
atgggctggtcctgtatcatattattcctggtcgcgaccgccaccggggtgcactccgacatccagatgactcagtccccatcctc
cctgtcggcgtccgttggcgaccgggtctcgattacatgccgcgcctcccagggcatctcctcctggctcgcctggtaccagca
gaagcctgagaaggcccccaagtcattaatctacgcagcctccaatctgaggtccggcgtgcccagtagattctcgggctccgg
gtcgggcactgattttaccctcaccatcagttcgctccagccagaggacttcgctacgtactattgccagcagtactattcctaccc
acgcaccttcgggcagggcaccaaggtcgagatcaagggcggcggcgggtccggcggaggcgggtctggcggtggtggtt
cgcagctgcagctgcaagagtccgggcctggcctcgtgaagcccagcgagaccctgtcattgacatgcaccgtgaggggg
gagtctgtcccgctcctccttcttctggggctggattcgccagccgcccggcaaggggctggaatggatcgggtcgatctactac
agtgggtcgacttactacaatccaagtctgaagagccgtgtgaccatctcagtggatacctcgaagaatcagttcagcctgaagct
ctcctccgtcaccgcggccgatacggcagtgtactactgtgtgcgcgattacgacatcctcactggcgatgaggactactggggt
caaggcaccctagtcacggtgtcgtcgggaggtggtggatccgacatccagatgacccagtccccttcctccctctctgcctctgt
gggagaccgcgttaccatcacatgccgagcttcccaggacgtgaacacagccgtggcctggtaccagcagaagcccgggaa
ggcacccaaactcctcatctactccgcctccttcctatacagtggcgtgccttcccgattctccggctccaggagtggcacggact
ttacgctcaccattagtagcctgcagcccgaagacttcgcgacctactattgtcagcaacactacacgacgccaccaactttcggc
cagggtaccaaggtcgagattaagcgaaccggcagtaccagtgggtctggcaagcccggcagcggcgagggatccgaggtc
cagctggtcgagtccggcgggggcctggtgcagccgggcggctcgctgaggttatcttgcgccgccagtggcttcaacatcaa
ggatacttacatccactgggtgaggcaggctccgggcaagggcctggaatgggtggctaggatctaccctactaacgggtaca
cacgctacgcagattcggtgaaaggccgcttcactatctccgccgacacctcgaagaacactgcttacctgcagatgaactccct
cagggccgaagatactgcagtctactactgctcccgctggggtggggacggcttctacgccatggacgtgtggggtcagggca
 ctctagttacagtgtcatcctaa
SEQ ID NO:30, 4H1.4D5: 
atggggtggagttgcatcattctgttcctcgtggcgaccgcaacaggcgtgcacagcgagatcgtgctcacccagtcaccagcc
accttatccttaagtcccggcgaacgcgccaccctgtcctgcagagcgtcccagtcggtcagcagttatctggcgtggtaccagc
agaagcccggccaagccccacgcctgctcatctacgacgcgtcgaatcgcgccacgggtattcccgcacggtttagcggctcc
ggttcagggactgactttaccctgacgatctcgagtctagagccggaggacttcgcggtgtactactgccagcagtccagcaact
ggccgcgcaccttcggccagggcacaaaggtggagatcaaggggggcgggggctcgggtggcgggggctccggaggcgg
gggctcccaagtgtacctggttgaatcgggcgggggcgtggtgcagcctgggcgctcgctgcgcctcagttgcgccgcgtcc
ggattcacattttccaactacgggatgcactgggtgcgccaagctccgggaaaggggctggagtgggtggccctgatctggtac
gacggttccaataagtactatgccgatagcgtgaagggccggttcacgatatccagggataactccaagaacactctctatctgc
agatgacctcactgcgcgtggaggacactgccgtctattactgcgcctccaatgtggatcactggggacagggcaccctggtga
ccgtaagctcgggaggtggtggatccgacatccagatgacccagtccccttcctccctctctgcctctgtgggagaccgcgttac
catcacatgccgagcttcccaggacgtgaacacagccgtggcctggtaccagcagaagcccgggaaggcacccaaactcctc
atctactccgcctccttcctatacagtggcgtgccttcccgattctccggctccaggagtggcacggactttacgctcaccattagta
gcctgcagcccgaagacttcgcgacctactattgtcagcaacactacacgacgccaccaactttcggccagggtaccaaggtcg
agattaagcgaaccggcagtaccagtgggtctggcaagcccggcagcggcgagggatccgaggtccagctggtcgagtccg
gcgggggcctggtgcagccgggcggctcgctgaggttatcttgcgccgccagtggcttcaacatcaaggatacttacatccact
gggtgaggcaggctccgggcaagggcctggaatgggtggctaggatctaccctactaacgggtacacacgctacgcagattc
ggtgaaaggccgcttcactatctccgccgacacctcgaagaacactgcttacctgcagatgaactccctcagggccgaagatac
tgcagtctactactgctcccgctggggtggggacggcttctacgccatggacgtgtggggtcagggcactctagttacagtgtca
tcctaa
SEQ ID NO:31, 2D3.CD19: 
atgggctggtcttgcatcatcctgtttctggtcgccaccgccaccggggtccacagtgagatcgttctcacccaatcccccgccac
actgtcgctctccccaggcgagcgggccacgctgtcgtgtcgcgcgagccagagcgtttcctcctacctcgcctggtaccagca
gaagccgggccaggcccctcgcctgctgatctacgatacttcgaatagagccaccggtatccctgcaaggttctccggatccgg
gtcagggacggatttcaccctgaccatttcctccctggagccagaggatttcgccgtgtactactgccagcagcgcagtaactgg
cctctgactttcggtggcggcacaaaggtggagatcaaggggggcggcggcagtgggggcgggggcagcgggggcggag
gctcgcaggtccagctcgtggagtcggggggggacgtggtccagccaggcaggagcctgaggctgtcatgcgctgcgtccg
gcctgacgttcaccaactacggcttccactgggtgagacaggcacccgggaagggcctggaatgggtggccgtgatctggtac
gacgggagcaagaagtactacgcagactcagtgaagggccgattcaccatcagccgggacaactctaagaacacactgtacct
gcagatgaacaatttgcgcgccgaggataccgccgtatattattgcgccacaggcgacgactattggggacagggcaccctggt
cacggtaagtagtggaggtggtggatccgacatccagatgacacagactacatcctccctgtctgcctctctgggagacagagtc
 
accatcagttgcagggcaagtcaggacattagtaaatatttaaattggtatcagcagaaaccagatggaactgttaaactcctgatc
taccatacatcaagattacactcaggagtcccatcaaggttcagtggcagtgggtctggaacagattattctctcaccattagcaac
ctggagcaagaagatattgccacttacttttgccaacagggtaatacgcttccgtacacgttcggaggggggaccaagctggag
atcacaggtggcggtggctcgggcggtggtgggtcgggtggcggcggatctgaggtgaaactgcaggagtcaggacctggc
ctggtggcgccctcacagagcctgtccgtcacatgcactgtctcaggggtctcattacccgactatggtgtaagctggattcgcca
gcctccacgaaagggtctggagtggctgggagtaatatggggtagtgaaaccacatactataattcagctctcaaatccagactg
accatcatcaaggacaactccaagagccaagttttcttaaaaatgaacagtctgcaaactgacgacacagccatttactactgtgc
caaacattattactacggtggtagctacgctatggactactggggccaaggaacctcagtcaccgtctcctctaa
SEQ ID NO:32, 4A11.CD19: 
atgggctggtcctgtatcatattattcctggtcgcgaccgccaccggggtgcactccgacatccagatgactcagtccccatcctc
cctgtcggcgtccgttggcgaccgggtctcgattacatgccgcgcctcccagggcatctcctcctggctcgcctggtaccagca
gaagcctgagaaggcccccaagtcattaatctacgcagcctccaatctgaggtccggcgtgcccagtagattctcgggctccgg
gtcgggcactgattttaccctcaccatcagttcgctccagccagaggacttcgctacgtactattgccagcagtactattcctaccc
acgcaccttcgggcagggcaccaaggtcgagatcaagggcggcggcgggtccggcggaggcgggtctggcggtggtggtt
cgcagctgcagctgcaagagtccgggcctggcctcgtgaagcccagcgagaccctgtcattgacatgcaccgtgagtggggg
gagtctgtcccgctcctccttcttctggggctggattcgccagccgcccggcaaggggctggaatggatcgggtcgatctactac
agtgggtcgacttactacaatccaagtctgaagagccgtgtgaccatctcagtggatacctcgaagaatcagttcagcctgaagct
ctcctccgtcaccgcggccgatacggcagtgtactactgtgtgcgcgattacgacatcctcactggcgatgaggactactggggt
caaggcaccctagtcacggtgtcgtcgggaggtggtggatccgacatccagatgacacagactacatcctccctgtctgcctctc
tgggagacagagtcaccatcagttgcagggcaagtcaggacattagtaaatatttaaattggtatcagcagaaaccagatggaac
tgttaaactcctgatctaccatacatcaagattacactcaggagtcccatcaaggttcagtggcagtgggtctggaacagattattct
ctcaccattagcaacctggagcaagaagatattgccacttacttttgccaacagggtaatacgcttccgtacacgttcggagggggg
accaagctggagatcacaggtggcggtggctcgggcggtggtgggtcgggtggcggcggatctgaggtgaaactgcagga
gtcaggacctggcctggtggcgccctcacagagcctgtccgtcacatgcactgtctcaggggtctcattacccgactatggtgta
agctggattcgccagcctccacgaaagggtctggagtggctgggagtaatatggggtagtgaaaccacatactataattcagctc
tcaaatccagactgaccatcatcaaggacaactccaagagccaagttttcttaaaaatgaacagtctgcaaactgacgacacagc
catttactactgtgccaaacattattactacggtggtagctacgctatggactactggggccaaggaacctcagtcaccgtctcctct
aa
SEQ ID NO:33, 4H1.CD19: 
atggggtggagttgcatcattctgttcctcgtggcgaccgcaacaggcgtgcacagcgagatcgtgctcacccagtcaccagcc
accttatccttaagtcccggcgaacgcgccaccctgtcctgcagagcgtcccagtcggtcagcagttatctggcgtggtaccagc
agaagcccggccaagccccacgcctgctcatctacgacgcgtcgaatcgcgccacgggtattcccgcacggtttagcggctcc
 
ggttcagggactgactttaccctgacgatctcgagtctagagccggaggacttcgcggtgtactactgccagcagtccagcaact
ggccgcgcaccttcggccagggcacaaaggtggagatcaaggggggcgggggctcgggtggcgggggctccggaggcgg
gggctcccaagtgtacctggttgaatcgggcgggggcgtggtgcagcctgggcgctcgctgcgcctcagttgcgccgcgtcc
ggattcacattttccaactacgggatgcactgggtgcgccaagctccgggaaaggggctggagtgggtggccctgatctggtac
gacggttccaataagtactatgccgatagcgtgaagggccggttcacgatatccagggataactccaagaacactctctatctgc
agatgacctcactgcgcgtggaggacactgccgtctattactgcgcctccaatgtggatcactggggacagggcaccctggtga
ccgtaagctcgggaggtggtggatccgacatccagatgacacagactacatcctccctgtctgcctctctgggagacagagtca
ccatcagttgcagggcaagtcaggacattagtaaatatttaaattggtatcagcagaaaccagatggaactgttaaactcctgatct
accatacatcaagattacactcaggagtcccatcaaggttcagtggcagtgggtctggaacagattattctctcaccattagcaacc
tggagcaagaagatattgccacttacttttgccaacagggtaatacgcttccgtacacgttcggaggggggaccaagctggagat
cacaggtggcggtggctcgggcggtggtgggtcgggtggcggcggatctgaggtgaaactgcaggagtcaggacctggcct
ggtggcgccctcacagagcctgtccgtcacatgcactgtctcaggggtctcattacccgactatggtgtaagctggattcgccag
cctccacgaaagggtctggagtggctgggagtaatatggggtagtgaaaccacatactataattcagctctcaaatccagactga
ccatcatcaaggacaactccaagagccaagttttcttaaaaatgaacagtctgcaaactgacgacacagccatttactactgtgcc
aaacattattactacggtggtagctacgctatggactactggggccaaggaacctcagtcaccgtctcctctaa
SEQ ID NO:34, 2D3.2B3: 
atgggctggtcttgcatcatcctgtttctggtcgccaccgccaccggggtccacagtgagatcgttctcacccaatcccccgccac
actgtcgctctccccaggcgagcgggccacgctgtcgtgtcgcgcgagccagagcgtttcctcctacctcgcctggtaccagca
gaagccgggccaggcccctcgcctgctgatctacgatacttcgaatagagccaccggtatccctgcaaggttctccggatccgg
gtcagggacggatttcaccctgaccatttcctccctggagccagaggatttcgccgtgtactactgccagcagcgcagtaactgg
cctctgactttcggtggcggcacaaaggtggagatcaaggggggcggcggcagtgggggcgggggcagcgggggcggag
gctcgcaggtccagctcgtggagtcggggggggacgtggtccagccaggcaggagcctgaggctgtcatgcgctgcgtccg
gcctgacgttcaccaactacggcttccactgggtgagacaggcacccgggaagggcctggaatgggtggccgtgatctggtac
gacgggagcaagaagtactacgcagactcagtgaagggccgattcaccatcagccgggacaactctaagaacacactgtacct
gcagatgaacaatttgcgcgccgaggataccgccgtatattattgcgccacaggcgacgactattggggacagggcaccctggt
cacggtaagtagtggaggtggtggatccgatatccagctgacccaatcaccgtcgtccctgtctgcctccgtgggcgaccgggt
gacgatcacctgtagtgcctcgagcagtgtacggttcatccactggtaccaacagaagcccggcaaggcaccaaagcggctga
tctacgacaccagcaagctggcgtctggggtgcccagcaggttctcgggaagtggtagtggcacagacttcactctcaccatca
gttcactccagccggaggactttgccacctactattgccagcagtggtcctcgtccccctttaccttcggccagggaacaaaggtg
gaaattaagggttcgacctccggggggggctccggtgggggctccggcggggggggctcatcggaggttcagctggtggag
agcggcggcggcctggtgcagcccggcgggagtctgcggctgtcctgtgccgccagcggcttcaacatcaaggactactaca
ttcactgggtgcggcaagccccaggcaagggtctggagtgggtggcttggattgaccctgaaaacggcgacactgagttcgtg
ccaaaattccaggggcgggcgaccatctccgccgacacctccaagaatacggcctacctgcagatgaactccctgcgcgccg
 aagacacagcggtctactactgcaagacagggggtttctggggccagggcaccctcgtgaccgtttcgagtgccgccggctaa
SEQ ID NO:35, 4A11.2B3: 
atgggctggtcctgtatcatattattcctggtcgcgaccgccaccggggtgcactccgacatccagatgactcagtccccatcctc
cctgtcggcgtccgttggcgaccgggtctcgattacatgccgcgcctcccagggcatctcctcctggctcgcctggtaccagca
gaagcctgagaaggcccccaagtcattaatctacgcagcctccaatctgaggtccggcgtgcccagtagattctcgggctccgg
gtcgggcactgattttaccctcaccatcagttcgctccagccagaggacttcgctacgtactattgccagcagtactattcctaccc
acgcaccttcgggcagggcaccaaggtcgagatcaagggcggcggcgggtccggcggaggcgggtctggcggtggtggtt
cgcagctgcagctgcaagagtccgggcctggcctcgtgaagcccagcgagaccctgtcattgacatgcaccgtgagtggggg
gagtctgtcccgctcctccttcttctggggctggattcgccagccgcccggcaaggggctggaatggatcgggtcgatctactac
agtgggtcgacttactacaatccaagtctgaagagccgtgtgaccatctcagtggatacctcgaagaatcagttcagcctgaagct
ctcctccgtcaccgcggccgatacggcagtgtactactgtgtgcgcgattacgacatcctcactggcgatgaggactactggggt
caaggcaccctagtcacggtgtcgtcgggaggtggtggatccgatatccagctgacccaatcaccgtcgtccctgtctgcctccg
tgggcgaccgggtgacgatcacctgtagtgcctcgagcagtgtacggttcatccactggtaccaacagaagcccggcaaggca
ccaaagcggctgatctacgacaccagcaagctggcgtctggggtgcccagcaggttctcgggaagtggtagtggcacagactt
cactctcaccatcagttcactccagccggaggactttgccacctactattgccagcagtggtcctcgtccccctttaccttcggcca
gggaacaaaggtggaaattaagggttcgacctccggggggggctccggtgggggctccggcggggggggctcatcggagg
ttcagctggtggagagcggcggcggcctggtgcagcccggcgggagtctgcggctgtcctgtgccgccagcggcttcaacat
caaggactactacattcactgggtgcggcaagccccaggcaagggtctggagtgggtggcttggattgaccctgaaaacggcg
acactgagttcgtgccaaaattccaggggcgggcgaccatctccgccgacacctccaagaatacggcctacctgcagatgaact
ccctgcgcgccgaagacacagcggtctactactgcaagacagggggtttctggggccagggcaccctcgtgaccgtttcgagt
gccgccggctaa
SEQ ID NO:36, 4H1.2B3: 
atggggtggagttgcatcattctgttcctcgtggcgaccgcaacaggcgtgcacagcgagatcgtgctcacccagtcaccagcc
accttatccttaagtcccggcgaacgcgccaccctgtcctgcagagcgtcccagtcggtcagcagttatctggcgtggtaccagc
agaagcccggccaagccccacgcctgctcatctacgacgcgtcgaatcgcgccacgggtattcccgcacggtttagcggctcc
ggttcagggactgactttaccctgacgatctcgagtctagagccggaggacttcgcggtgtactactgccagcagtccagcaact
ggccgcgcaccttcggccagggcacaaaggtggagatcaaggggggcgggggctcgggtggcgggggctccggaggcgg
gggctcccaagtgtacctggttgaatcgggcgggggcgtggtgcagcctgggcgctcgctgcgcctcagttgcgccgcgtcc
ggattcacattttccaactacgggatgcactgggtgcgccaagctccgggaaaggggctggagtgggtggccctgatctggtac
gacggttccaataagtactatgccgatagcgtgaagggccggttcacgatatccagggataactccaagaacactctctatctgc
agatgacctcactgcgcgtggaggacactgccgtctattactgcgcctccaatgtggatcactggggacagggcaccctggtga
ccgtaagctcgggaggtggtggatccgatatccagctgacccaatcaccgtcgtccctgtctgcctccgtgggcgaccgggtga
 
cgatcacctgtagtgcctcgagcagtgtacggttcatccactggtaccaacagaagcccggcaaggcaccaaagcggctgatct
acgacaccagcaagctggcgtctggggtgcccagcaggttctcgggaagtggtagtggcacagacttcactctcaccatcagtt
cactccagccggaggactttgccacctactattgccagcagtggtcctcgtccccctttaccttcggccagggaacaaaggtgga
aattaagggttcgacctccggggggggctccggtgggggctccggcggggggggctcatcggaggttcagctggtggagag
cggcggcggcctggtgcagcccggcgggagtctgcggctgtcctgtgccgccagcggcttcaacatcaaggactactacattc
actgggtgcggcaagccccaggcaagggtctggagtgggtggcttggattgaccctgaaaacggcgacactgagttcgtgcca
aaattccaggggcgggcgaccatctccgccgacacctccaagaatacggcctacctgcagatgaactccctgcgcgccgaag
acacagcggtctactactgcaagacagggggtttctggggccagggcaccctcgtgaccgtttcgagtgccgccggctaa
SEQ ID NO:37, C2177.ss1: 
atgggttggagctgcatcattctgttcctcgtggcgacggctaccggggtgcactcggatatcgtgctgacccagtcgccggcaa
gcctcgccgtgtctctggggcagagagctacaatctcatgcaaagccagtcaaagtgtggattatgcgggggattcgtacatgaa
ctggtatcagcagaagcccgggcagcctcccaagctgttgatctacgcggctagtaacctggagagcgggatcccagctcggtt
ctccgggtccgggtccggaaccgacttcaccctcaacatccatccggtcgaggaggaggatgcggcaacctactactgccagc
agtcgaatgaagatccgtacactttcggcgggggcaccaagctcgagataaagggaggaggcggctctggcgggggaggat
ctgggggcggcggctctcaggtccagctgcaacagtcaggccctgaactggtaaagcccggcgcttcggttaaaatctcgtgta
aggcttccgggtacgcgttctcctcctcctggatgaactgggtcaagcagcgcccaggaaagggcctggagtggattggccgg
atctatccgggcgatggcgacacaaactacaatggcaagttcaaggggaaggctactctcaccgcagataagtcctcgtctactg
cttacatgcaacttagtagcctcacctcagaggattccgccgtgtatttctgcgcccgatccgattactatggcgattacggatttgc
atattggggacaaggcaccctggttacggtcagcgccggaggtggtggatcccaggtacaactgcagcagtctgggcctgagc
tggagaagcctggcgcttcagtgaagatatcctgcaaggcttctggttactcattcactggctacaccatgaactgggtgaagcag
agccatggaaagagccttgagtggattggacttattactccttacaatggtgcttctagctacaaccagaagttcaggggcaaggc
cacattaactgtagacaagtcatccagcacagcctacatggacctcctcagtctgacatctgaagactctgcagtctatttctgtgca
agggggggttacgacgggaggggttttgactactggggccaagggaccacggtcaccgtctcctcaggtggaggcggttcag
gcggcggtggctctagcggtggcggatcggacatcgagctcactcagtctccagcaatcatgtctgcatctccaggggagaag
gtcaccatgacctgcagtgccagctcaagtgtaagttacatgcactggtaccagcagaagtcaggcacctcccccaaaagatgg
atttacgacacatccaaactggcttctggagtcccaggtcgcttcagtggcagtgggtctggaaactcttactctctcacaatcagc
agcgtggaggctgaagacgacgcaacttattactgccagcagtggagtaagcaccctctcacgtacggtgctgggacaaagttg
gaaatcaaataa
SEQ ID NO:38, M709.ss1: 
atgggctggtcctgcatcatcctgttcctggtggctaccgccaccggtgttcactccgatatcgtcatgacccagtcgccagactc
ccttgctgtaagcctgggggagcgcgccaccatcaactgtaaagccagtcagtccgttgattacgccggcgagtcattcatgaac
tggtaccagcagaagcccggccagcctcctaagctactgatctacgccgcgtccaatctggagtcgggcgtcccagaccgcttt
 
tccggcagcgggtcgggtaccgatttcaccctgacgatctcctcgctccaggccgaggatgtggccgtgtattactgccagcag
agtaacgaggatccatacaccttcggccagggaactaagctcgagatcaaagggggcggcggctccggcggcgggggctcc
ggcggagggggcagtgaggtgcagctggtgcagtctggcgcagaggtgaagaaacccggagagtcgctcaagatctcgtgc
aagggctcgggctacgccttttcgagttcgtggatgaactgggtgcgtcagatgcccggcaagggcctggagtggatgggccg
gatctatgcgggggatgcagataccgcgtactcgccgtcgtttcagggccaggttaccatcagcgcggacaaaagtatttccac
ggcgtacctgcagtggagttccctcaaggcgtccgacaccgccatgtactactgcgcccgcagtgactactatggggactacgg
gtttgcatactggggtcaggggaccctggtgaccgtgtcatcgggaggtggtggatcccaggtacaactgcagcagtctgggcc
tgagctggagaagcctggcgcttcagtgaagatatcctgcaaggcttctggttactcattcactggctacaccatgaactgggtga
agcagagccatggaaagagccttgagtggattggacttattactccttacaatggtgcttctagctacaaccagaagttcaggggc
aaggccacattaactgtagacaagtcatccagcacagcctacatggacctcctcagtctgacatctgaagactctgcagtctatttc
tgtgcaagggggggttacgacgggaggggttttgactactggggccaagggaccacggtcaccgtctcctcaggtggaggcg
gttcaggcggcggtggctctagcggtggcggatcggacatcgagctcactcagtctccagcaatcatgtctgcatctccagggg
agaaggtcaccatgacctgcagtgccagctcaagtgtaagttacatgcactggtaccagcagaagtcaggcacctcccccaaaa
gatggatttacgacacatccaaactggcttctggagtcccaggtcgcttcagtggcagtgggtctggaaactcttactctctcacaa
tcagcagcgtggaggctgaagacgacgcaacttattactgccagcagtggagtaagcaccctctcacgtacggtgctgggaca
aagttggaaatcaaataa
SEQ ID NO:39, M708.ss1: 
atgggctggtcctgcatcattctgttcctggtggccaccgccacgggggtgcactccgacattcagatgacccagtccccctcaa
gtctgtcagccagtgtgggcgaccgtgtgacaatcacctgcagggcctctaagtccgtgtccacttcggggtactcattcatgcac
tggtaccagcagaagcccggcaaagcacccaagctgctgatctacgtggggagccgcctggactatggcgtgccttcgcgcttt
tcgggcagcggatcgggcactgatttcactctgaccatctcctcactccaacccgaagacttcgcgacgtactactgccaacact
cccgcgagctgccctggactttcggacagggcaccaaggttgagattaaaggcgggggcgggtcaggcgggggcggctccg
gcggcggaggaagtgaggtgcagctgctggagtcgggcggaggcctggtgcagcctggggggtcactccgactgagctgc
gctgcatccgggttcaccttcagctcgtataccatgagctgggtgcgccaggcccccgggaagggcctggagtgggtgtctgtg
atcgaccacgggggcgggtccacgcattatcccgatagcgtcaagggccggttcacgatctcgcgcgacaactctaagaatact
ctgtacctgcagatgaactccctgcgcgccgaagacactgcggtgtactattgtgccaggcaccgcggcaacccattcgactact
ggggccagggcaccctggtcacagtctcctccggaggtggtggatcccaggtacaactgcagcagtctgggcctgagctgga
gaagcctggcgcttcagtgaagatatcctgcaaggcttctggttactcattcactggctacaccatgaactgggtgaagcagagc
catggaaagagccttgagtggattggacttattactccttacaatggtgcttctagctacaaccagaagttcaggggcaaggccac
attaactgtagacaagtcatccagcacagcctacatggacctcctcagtctgacatctgaagactctgcagtctatttctgtgcaag
ggggggttacgacgggaggggttttgactactggggccaagggaccacggtcaccgtctcctcaggtggaggcggttcaggc
ggcggtggctctagcggtggcggatcggacatcgagctcactcagtctccagcaatcatgtctgcatctccaggggagaaggtc
accatgacctgcagtgccagctcaagtgtaagttacatgcactggtaccagcagaagtcaggcacctcccccaaaagatggatt
 
acgacacatccaaactggcttctggagtcccaggtcgcttcagtggcagtgggtctggaaactcttactctctcacaatcagcagc
gtggaggctgaagacgacgcaacttattactgccagcagtggagtaagcaccctctcacgtacggtgctgggacaaagttggaa
atcaaataa
SEQ ID NO:40, C2177.4D5: 
atgggttggagctgcatcattctgttcctcgtggcgacggctaccggggtgcactcggatatcgtgctgacccagtcgccggcaa
gcctcgccgtgtctctggggcagagagctacaatctcatgcaaagccagtcaaagtgtggattatgcgggggattcgtacatgaa
ctggtatcagcagaagcccgggcagcctcccaagctgttgatctacgcggctagtaacctggagagcgggatcccagctcggtt
ctccgggtccgggtccggaaccgacttcaccctcaacatccatccggtcgaggaggaggatgcggcaacctactactgccagc
agtcgaatgaagatccgtacactttcggcgggggcaccaagctcgagataaagggaggaggcggctctggcgggggaggat
ctgggggcggcggctctcaggtccagctgcaacagtcaggccctgaactggtaaagcccggcgcttcggttaaaatctcgtgta
aggcttccgggtacgcgttctcctcctcctggatgaactgggtcaagcagcgcccaggaaagggcctggagtggattggccgg
atctatccgggcgatggcgacacaaactacaatggcaagttcaaggggaaggctactctcaccgcagataagtcctcgtctactg
cttacatgcaacttagtagcctcacctcagaggattccgccgtgtatttctgcgcccgatccgattactatggcgattacggatttgc
atattggggacaaggcaccctggttacggtcagcgccggaggtggtggatccgacatccagatgacccagtccccttcctccct
ctctgcctctgtgggagaccgcgttaccatcacatgccgagcttcccaggacgtgaacacagccgtggcctggtaccagcagaa
gcccgggaaggcacccaaactcctcatctactccgcctccttcctatacagtggcgtgccttcccgattctccggctccaggagtg
gcacggactttacgctcaccattagtagcctgcagcccgaagacttcgcgacctactattgtcagcaacactacacgacgccacc
aactttcggccagggtaccaaggtcgagattaagcgaaccggcagtaccagtgggtctggcaagcccggcagcggcgaggg
atccgaggtccagctggtcgagtccggcgggggcctggtgcagccgggcggctcgctgaggttatcttgcgccgccagtggct
tcaacatcaaggatacttacatccactgggtgaggcaggctccgggcaagggcctggaatgggtggctaggatctaccctacta
acgggtacacacgctacgcagattcggtgaaaggccgcttcactatctccgccgacacctcgaagaacactgcttacctgcaga
tgaactccctcagggccgaagatactgcagtctactactgctcccgctggggtggggacggcttctacgccatggacgtgtggg
gtcagggcactctagttacagtgtcatcctaa
SEQ ID NO:41, M709.4D5: 
atgggctggtcctgcatcatcctgttcctggtggctaccgccaccggtgttcactccgatatcgtcatgacccagtcgccagactc
ccttgctgtaagcctgggggagcgcgccaccatcaactgtaaagccagtcagtccgttgattacgccggcgagtcattcatgaac
tggtaccagcagaagcccggccagcctcctaagctactgatctacgccgcgtccaatctggagtcgggcgtcccagaccgcttt
tccggcagcgggtcgggtaccgatttcaccctgacgatctcctcgctccaggccgaggatgtggccgtgtattactgccagcag
agtaacgaggatccatacaccttcggccagggaactaagctcgagatcaaagggggcggcggctccggcggcgggggctcc
ggcggagggggcagtgaggtgcagctggtgcagtctggcgcagaggtgaagaaacccggagagtcgctcaagatctcgtgc
aagggctcgggctacgccttttcgagttcgtggatgaactgggtgcgtcagatgcccggcaagggcctggagtggatgggccg
gatctatgcgggggatgcagataccgcgtactcgccgtcgtttcagggccaggttaccatcagcgcggacaaaagtatttccac
 
ggcgtacctgcagtggagttccctcaaggcgtccgacaccgccatgtactactgcgcccgcagtgactactatggggactacgg
gtttgcatactggggtcaggggaccctggtgaccgtgtcatcgggaggtggtggatccgacatccagatgacccagtccccttc
ctccctctctgcctctgtgggagaccgcgttaccatcacatgccgagcttcccaggacgtgaacacagccgtggcctggtacca
gcagaagcccgggaaggcacccaaactcctcatctactccgcctccttcctatacagtggcgtgccttcccgattctccggctcc
aggagtggcacggactttacgctcaccattagtagcctgcagcccgaagacttcgcgacctactattgtcagcaacactacacga
cgccaccaactttcggccagggtaccaaggtcgagattaagcgaaccggcagtaccagtgggtctggcaagcccggcagcgg
cgagggatccgaggtccagctggtcgagtccggcgggggcctggtgcagccgggcggctcgctgaggttatcttgcgccgcc
agtggcttcaacatcaaggatacttacatccactgggtgaggcaggctccgggcaagggcctggaatgggtggctaggatctac
cctactaacgggtacacacgctacgcagattcggtgaaaggccgcttcactatctccgccgacacctcgaagaacactgcttacc
tgcagatgaactccctcagggccgaagatactgcagtctactactgctcccgctggggtggggacggcttctacgccatggacg
tgtggggtcagggcactctagttacagtgtcatcctaa
SEQ ID NO:42, M708.4D5: 
atgggctggtcctgcatcattctgttcctggtggccaccgccacgggggtgcactccgacattcagatgacccagtccccctcaa
gtctgtcagccagtgtgggcgaccgtgtgacaatcacctgcagggcctctaagtccgtgtccacttcggggtactcattcatgcac
tggtaccagcagaagcccggcaaagcacccaagctgctgatctacgtggggagccgcctggactatggcgtgccttcgcgcttt
tcgggcagcggatcgggcactgatttcactctgaccatctcctcactccaacccgaagacttcgcgacgtactactgccaacact
cccgcgagctgccctggactttcggacagggcaccaaggttgagattaaaggcgggggcgggtcaggcgggggcggctccg
gcggcggaggaagtgaggtgcagctgctggagtcgggcggaggcctggtgcagcctggggggtcactccgactgagctgc
gctgcatccgggttcaccttcagctcgtataccatgagctgggtgcgccaggcccccgggaagggcctggagtgggtgtctgtg
atcgaccacgggggcgggtccacgcattatcccgatagcgtcaagggccggttcacgatctcgcgcgacaactctaagaatact
ctgtacctgcagatgaactccctgcgcgccgaagacactgcggtgtactattgtgccaggcaccgcggcaacccattcgactact
ggggccagggcaccctggtcacagtctcctccggaggtggtggatccgacatccagatgacccagtccccttcctccctctctg
cctctgtgggagaccgcgttaccatcacatgccgagcttcccaggacgtgaacacagccgtggcctggtaccagcagaagccc
gggaaggcacccaaactcctcatctactccgcctccttcctatacagtggcgtgccttcccgattctccggctccaggagtggcac
ggactttacgctcaccattagtagcctgcagcccgaagacttcgcgacctactattgtcagcaacactacacgacgccaccaactt
tcggccagggtaccaaggtcgagattaagcgaaccggcagtaccagtgggtctggcaagcccggcagcggcgagggatccg
aggtccagctggtcgagtccggcgggggcctggtgcagccgggcggctcgctgaggttatcttgcgccgccagtggcttcaac
atcaaggatacttacatccactgggtgaggcaggctccgggcaagggcctggaatgggtggctaggatctaccctactaacgg
gtacacacgctacgcagattcggtgaaaggccgcttcactatctccgccgacacctcgaagaacactgcttacctgcagatgaac
tccctcagggccgaagatactgcagtctactactgctcccgctggggtggggacggcttctacgccatggacgtgtggggtcag
ggcactctagttacagtgtcatcctaa
SEQ ID NO:43, C2177.BBZ 
atgggttggagctgcatcattctgttcctcgtggcgacggctaccggggtgcactcggatatcgtgctgacccagtcgccggcaa
gcctcgccgtgtctctggggcagagagctacaatctcatgcaaagccagtcaaagtgtggattatgcgggggattcgtacatgaa
ctggtatcagcagaagcccgggcagcctcccaagctgttgatctacgcggctagtaacctggagagcgggatcccagctcggtt
ctccgggtccgggtccggaaccgacttcaccctcaacatccatccggtcgaggaggaggatgcggcaacctactactgccagc
agtcgaatgaagatccgtacactttcggcgggggcaccaagctcgagataaagggaggaggcggctctggcgggggaggat
ctgggggcggcggctctcaggtccagctgcaacagtcaggccctgaactggtaaagcccggcgcttcggttaaaatctcgtgta
aggcttccgggtacgcgttctcctcctcctggatgaactgggtcaagcagcgcccaggaaagggcctggagtggattggccgg
atctatccgggcgatggcgacacaaactacaatggcaagttcaaggggaaggctactctcaccgcagataagtcctcgtctactg
cttacatgcaacttagtagcctcacctcagaggattccgccgtgtatttctgcgcccgatccgattactatggcgattacggatttgc
atattggggacaaggcaccctggttacggtcagcgccaccacgacgccagcgccgcgaccaccaacaccggcgcccaccat
cgcgtcgcagcccctgtccctgcgcccagaggcgtgccggccagcggcggggggcgcagtgcacacgagggggctggact
tcgcctgtgatatctacatctgggcgcccttggccgggacttgtggggtccttctcctgtcactggttatcaccctttactgcaaacg
gggcagaaagaaactcctgtatatattcaaacaaccatttatgagaccagtacaaactactcaagaggaagatggctgtagctgc
cgatttccagaagaagaagaaggaggatgtgaactgagagtgaagttcagcaggagcgcagacgcccccgcgtacaagcag
ggccagaaccagctctataacgagctcaatctaggacgaagagaggagtacgacgttttggacaagagacgtggccgggacc
ctgagatggggggaaagccgagaaggaagaaccctcaggaaggcctgtacaatgaactgcagaaagataagatggcggag
gcctacagtgagattgggatgaaaggcgagcgccggaggggcaaggggcacgatggcctttaccagggtctcagtacagcc
accaaggacacctacgacgcccttcacatgcaggccctgccccctcgctaa
SEQ ID NO:44, M709.BBZ: 
atgggctggtcctgcatcatcctgttcctggtggctaccgccaccggtgttcactccgatatcgtcatgacccagtcgccagactc
ccttgctgtaagcctgggggagcgcgccaccatcaactgtaaagccagtcagtccgttgattacgccggcgagtcattcatgaac
tggtaccagcagaagcccggccagcctcctaagctactgatctacgccgcgtccaatctggagtcgggcgtcccagaccgcttt
tccggcagcgggtcgggtaccgatttcaccctgacgatctcctcgctccaggccgaggatgtggccgtgtattactgccagcag
agtaacgaggatccatacaccttcggccagggaactaagctcgagatcaaagggggcggcggctccggcggcgggggctcc
ggcggagggggcagtgaggtgcagctggtgcagtctggcgcagaggtgaagaaacccggagagtcgctcaagatctcgtgc
aagggctcgggctacgccttttcgagttcgtggatgaactgggtgcgtcagatgcccggcaagggcctggagtggatgggccg
gatctatgcgggggatgcagataccgcgtactcgccgtcgtttcagggccaggttaccatcagcgcggacaaaagtatttccac
ggcgtacctgcagtggagttccctcaaggcgtccgacaccgccatgtactactgcgcccgcagtgactactatggggactacgg
gtttgcatactggggtcaggggaccctggtgaccgtgtcatcgaccacgacgccagcgccgcgaccaccaacaccggcgccc
accatcgcgtcgcagcccctgtccctgcgcccagaggcgtgccggccagcggcggggggcgcagtgcacacgagggggct
ggacttcgcctgtgatatctacatctgggcgcccttggccgggacttgtggggtccttctcctgtcactggttatcaccctttactgc
aaacggggcagaaagaaactcctgtatatattcaaacaaccatttatgagaccagtacaaactactcaagaggaagatggctgta
 
gctgccgatttccagaagaagaagaaggaggatgtgaactgagagtgaagttcagcaggagcgcagacgcccccgcgtacaa
gcagggccagaaccagctctataacgagctcaatctaggacgaagagaggagtacgacgttttggacaagagacgtggccgg
gaccctgagatggggggaaagccgagaaggaagaaccctcaggaaggcctgtacaatgaactgcagaaagataagatggcg
gaggcctacagtgagattgggatgaaaggcgagcgccggaggggcaaggggcacgatggcctttaccagggtctcagtaca
gccaccaaggacacctacgacgcccttcacatgcaggccctgccccctcgctaa
SEQ ID NO:45, M708.BBZ: 
atgggctggtcctgcatcattctgttcctggtggccaccgccacgggggtgcactccgacattcagatgacccagtccccctcaa
gtctgtcagccagtgtgggcgaccgtgtgacaatcacctgcagggcctctaagtccgtgtccacttcggggtactcattcatgcac
tggtaccagcagaagcccggcaaagcacccaagctgctgatctacgtggggagccgcctggactatggcgtgccttcgcgcttt
tcgggcagcggatcgggcactgatttcactctgaccatctcctcactccaacccgaagacttcgcgacgtactactgccaacact
cccgcgagctgccctggactttcggacagggcaccaaggttgagattaaaggcgggggcgggtcaggcgggggcggctccg
gcggcggaggaagtgaggtgcagctgctggagtcgggcggaggcctggtgcagcctggggggtcactccgactgagctgc
gctgcatccgggttcaccttcagctcgtataccatgagctgggtgcgccaggcccccgggaagggcctggagtgggtgtctgtg
atcgaccacgggggcgggtccacgcattatcccgatagcgtcaagggccggttcacgatctcgcgcgacaactctaagaatact
ctgtacctgcagatgaactccctgcgcgccgaagacactgcggtgtactattgtgccaggcaccgcggcaacccattcgactact
ggggccagggcaccctggtcacagtctcctccaccacgacgccagcgccgcgaccaccaacaccggcgcccaccatcgcgt
cgcagcccctgtccctgcgcccagaggcgtgccggccagcggcggggggcgcagtgcacacgagggggctggacttcgcc
tgtgatatctacatctgggcgcccttggccgggacttgtggggtccttctcctgtcactggttatcaccctttactgcaaacggggca
gaaagaaactcctgtatatattcaaacaaccatttatgagaccagtacaaactactcaagaggaagatggctgtagctgccgatttc
cagaagaagaagaaggaggatgtgaactgagagtgaagttcagcaggagcgcagacgcccccgcgtacaagcagggccag
aaccagctctataacgagctcaatctaggacgaagagaggagtacgacgttttggacaagagacgtggccgggaccctgagat
ggggggaaagccgagaaggaagaaccctcaggaaggcctgtacaatgaactgcagaaagataagatggcggaggcctaca
gtgagattgggatgaaaggcgagcgccggaggggcaaggggcacgatggcctttaccagggtctcagtacagccaccaagg
acacctacgacgcccttcacatgcaggccctgccccctcgctaa
SEQ ID NO:46, C2177.CD19: 
atgggttggagctgcatcattctgttcctcgtggcgacggctaccggggtgcactcggatatcgtgctgacccagtcgccggcaa
gcctcgccgtgtctctggggcagagagctacaatctcatgcaaagccagtcaaagtgtggattatgcgggggattcgtacatgaa
ctggtatcagcagaagcccgggcagcctcccaagctgttgatctacgcggctagtaacctggagagcgggatcccagctcggtt
ctccgggtccgggtccggaaccgacttcaccctcaacatccatccggtcgaggaggaggatgcggcaacctactactgccagc
agtcgaatgaagatccgtacactttcggcgggggcaccaagctcgagataaagggaggaggcggctctggcgggggaggat
ctgggggcggcggctctcaggtccagctgcaacagtcaggccctgaactggtaaagcccggcgcttcggttaaaatctcgtgta
aggcttccgggtacgcgttctcctcctcctggatgaactgggtcaagcagcgcccaggaaagggcctggagtggattggccgg
 
atctatccgggcgatggcgacacaaactacaatggcaagttcaaggggaaggctactctcaccgcagataagtcctcgtctactg
cttacatgcaacttagtagcctcacctcagaggattccgccgtgtatttctgcgcccgatccgattactatggcgattacggatttgc
atattggggacaaggcaccctggttacggtcagcgccggaggtggtggatccgacatccagatgacacagactacatcctccct
gtctgcctctctgggagacagagtcaccatcagttgcagggcaagtcaggacattagtaaatatttaaattggtatcagcagaaac
cagatggaactgttaaactcctgatctaccatacatcaagattacactcaggagtcccatcaaggttcagtggcagtgggtctgga
acagattattctctcaccattagcaacctggagcaagaagatattgccacttacttttgccaacagggtaatacgcttccgtacacgt
tcggaggggggaccaagctggagatcacaggtggcggtggctcgggcggtggtgggtcgggtggcggcggatctgaggtg
aaactgcaggagtcaggacctggcctggtggcgccctcacagagcctgtccgtcacatgcactgtctcaggggtctcattaccc
gactatggtgtaagctggattcgccagcctccacgaaagggtctggagtggctgggagtaatatggggtagtgaaaccacatac
tataattcagctctcaaatccagactgaccatcatcaaggacaactccaagagccaagttttcttaaaaatgaacagtctgcaaact
gacgacacagccatttactactgtgccaaacattattactacggtggtagctacgctatggactactggggccaaggaacctcagt
caccgtctcctctaa
SEQ ID NO:47, M709.CD19: 
atgggctggtcctgcatcatcctgttcctggtggctaccgccaccggtgttcactccgatatcgtcatgacccagtcgccagactc
ccttgctgtaagcctgggggagcgcgccaccatcaactgtaaagccagtcagtccgttgattacgccggcgagtcattcatgaac
tggtaccagcagaagcccggccagcctcctaagctactgatctacgccgcgtccaatctggagtcgggcgtcccagaccgcttt
tccggcagcgggtcgggtaccgatttcaccctgacgatctcctcgctccaggccgaggatgtggccgtgtattactgccagcag
agtaacgaggatccatacaccttcggccagggaactaagctcgagatcaaagggggcggcggctccggcggcgggggctcc
ggcggagggggcagtgaggtgcagctggtgcagtctggcgcagaggtgaagaaacccggagagtcgctcaagatctcgtgc
aagggctcgggctacgccttttcgagttcgtggatgaactgggtgcgtcagatgcccggcaagggcctggagtggatgggccg
gatctatgcgggggatgcagataccgcgtactcgccgtcgtttcagggccaggttaccatcagcgcggacaaaagtatttccac
ggcgtacctgcagtggagttccctcaaggcgtccgacaccgccatgtactactgcgcccgcagtgactactatggggactacgg
gtttgcatactggggtcaggggaccctggtgaccgtgtcatcgggaggtggtggatccgacatccagatgacacagactacatc
ctccctgtctgcctctctgggagacagagtcaccatcagttgcagggcaagtcaggacattagtaaatatttaaattggtatcagca
gaaaccagatggaactgttaaactcctgatctaccatacatcaagattacactcaggagtcccatcaaggttcagtggcagtgggt
ctggaacagattattctctcaccattagcaacctggagcaagaagatattgccacttacttttgccaacagggtaatacgcttccgta
cacgttcggaggggggaccaagctggagatcacaggtggcggtggctcgggcggtggtgggtcgggtggcggcggatctga
ggtgaaactgcaggagtcaggacctggcctggtggcgccctcacagagcctgtccgtcacatgcactgtctcaggggtctcatt
acccgactatggtgtaagctggattcgccagcctccacgaaagggtctggagtggctgggagtaatatggggtagtgaaaccac
atactataattcagctctcaaatccagactgaccatcatcaaggacaactccaagagccaagttttcttaaaaatgaacagtctgca
aactgacgacacagccatttactactgtgccaaacattattactacggtggtagctacgctatggactactggggccaaggaacct
cagtcaccgtctcctctaa
SEQ ID NO:48, M708.CD19: 
atgggctggtcctgcatcattctgttcctggtggccaccgccacgggggtgcactccgacattcagatgacccagtccccctcaa
gtctgtcagccagtgtgggcgaccgtgtgacaatcacctgcagggcctctaagtccgtgtccacttcggggtactcattcatgcac
tggtaccagcagaagcccggcaaagcacccaagctgctgatctacgtggggagccgcctggactatggcgtgccttcgcgcttt
tcgggcagcggatcgggcactgatttcactctgaccatctcctcactccaacccgaagacttcgcgacgtactactgccaacact
cccgcgagctgccctggactttcggacagggcaccaaggttgagattaaaggcgggggcgggtcaggcgggggcggctccg
gcggcggaggaagtgaggtgcagctgctggagtcgggcggaggcctggtgcagcctggggggtcactccgactgagctgc
gctgcatccgggttcaccttcagctcgtataccatgagctgggtgcgccaggcccccgggaagggcctggagtgggtgtctgtg
atcgaccacgggggcgggtccacgcattatcccgatagcgtcaagggccggttcacgatctcgcgcgacaactctaagaatact
ctgtacctgcagatgaactccctgcgcgccgaagacactgcggtgtactattgtgccaggcaccgcggcaacccattcgactact
ggggccagggcaccctggtcacagtctcctccggaggtggtggatccgacatccagatgacacagactacatcctccctgtctg
cctctctgggagacagagtcaccatcagttgcagggcaagtcaggacattagtaaatatttaaattggtatcagcagaaaccagat
ggaactgttaaactcctgatctaccatacatcaagattacactcaggagtcccatcaaggttcagtggcagtgggtctggaacaga
ttattctctcaccattagcaacctggagcaagaagatattgccacttacttttgccaacagggtaatacgcttccgtacacgttcgga
ggggggaccaagctggagatcacaggtggcggtggctcgggcggtggtgggtcgggtggcggcggatctgaggtgaaact
gcaggagtcaggacctggcctggtggcgccctcacagagcctgtccgtcacatgcactgtctcaggggtctcattacccgactat
ggtgtaagctggattcgccagcctccacgaaagggtctggagtggctgggagtaatatggggtagtgaaaccacatactataatt
cagctctcaaatccagactgaccatcatcaaggacaactccaagagccaagttttcttaaaaatgaacagtctgcaaactgacgac
acagccatttactactgtgccaaacattattactacggtggtagctacgctatggactactggggccaaggaacctcagtcaccgt
ctcctctaa
SEQ ID NO:49, PD1.CD8Z: 
atgcagatcccacaggcgccctggccagtcgtctgggcggtgctacaactgggctggcggccaggatggttcttagactcccc
agacaggccctggaacccccccaccttctccccagccctgctcgtggtgaccgaaggggacaacgccaccttcacctgcagct
tctccaacacatcggagagcttcgtgctaaactggtaccgcatgagccccagcaaccagacggacaagctggccgccttcccc
gaggaccgcagccagcccggccaggactgccgcttccgtgtcacacaactgcccaacgggcgtgacttccacatgagcgtgg
tcagggcccggcgcaatgacagcggcacctacctctgtggggccatctccctggcccccaaggcgcagatcaaagagagcct
gcgggcagagctcagggtgacagagagaagggcagaagtgcccacagcccaccccagcccctcacccaggccagccggc
cagttccaaaccctggtgttttgggtgctggtggtggttggtggagtcctggcttgctatagcttgctagtaacagtggcctttattatt
ttctgggtgctttactgcaaccacaggaaccgaagacgtgtttgcaaatgtccccggcctgtggtcaaatcgggagacaagccca
gcctttcggcgagatacgtcctgagagtgaagttcagcaggagcgcagacgcccccgcgtacaagcagggccagaaccagct
ctataacgagctcaatctaggacgaagagaggagtacgacgttttggacaagagacgtggccgggaccctgagatgggggga
aagccgagaaggaagaaccctcaggaaggcctgtacaatgaactgcagaaagataagatggcggaggcctacagtgagattg
ggatgaaaggcgagcgccggaggggcaaggggcacgatggcctttaccagggtctcagtacagccaccaaggacacctacg
 acgcccttcacatgcaggccctgccccctcgctaa
SEQ ID NO:50, PD1.CD4Z: 
atgcagatcccacaggcgccctggccagtcgtctgggcggtgctacaactgggctggcggccaggatggttcttagactcccc
agacaggccctggaacccccccaccttctccccagccctgctcgtggtgaccgaaggggacaacgccaccttcacctgcagct
tctccaacacatcggagagcttcgtgctaaactggtaccgcatgagccccagcaaccagacggacaagctggccgccttcccc
gaggaccgcagccagcccggccaggactgccgcttccgtgtcacacaactgcccaacgggcgtgacttccacatgagcgtgg
tcagggcccggcgcaatgacagcggcacctacctctgtggggccatctccctggcccccaaggcgcagatcaaagagagcct
gcgggcagagctcagggtgacagagagaagggcagaagtgcccacagcccaccccagcccctcacccaggccagccggc
cagttccaaaccctggtgttttgggtgctggtggtggttggtggagtcctggcttgctatagcttgctagtaacagtggcctttattatt
ttctgggtgtgtgtcaggtgccggcaccgaaggcgccaagcagagcggatgtctcagatcaagagactcctcagtgagaagaa
gacctgccagtgccctcaccggtttcagaagacatgtagccccattctgagagtgaagttcagcaggagcgcagacgcccccg
cgtacaagcagggccagaaccagctctataacgagctcaatctaggacgaagagaggagtacgacgttttggacaagagacgt
ggccgggaccctgagatggggggaaagccgagaaggaagaaccctcaggaaggcctgtacaatgaactgcagaaagataa
gatggcggaggcctacagtgagattgggatgaaaggcgagcgccggaggggcaaggggcacgatggcctttaccagggtct
cagtacagccaccaaggacacctacgacgcccttcacatgcaggccctgccccctcgctaa
SEQ ID NO:51, PD1.28Z: 
atgcagatcccacaggcgccctggccagtcgtctgggcggtgctacaactgggctggcggccaggatggttcttagactcccc
agacaggccctggaacccccccaccttctccccagccctgctcgtggtgaccgaaggggacaacgccaccttcacctgcagct
tctccaacacatcggagagcttcgtgctaaactggtaccgcatgagccccagcaaccagacggacaagctggccgccttcccc
gaggaccgcagccagcccggccaggactgccgcttccgtgtcacacaactgcccaacgggcgtgacttccacatgagcgtgg
tcagggcccggcgcaatgacagcggcacctacctctgtggggccatctccctggcccccaaggcgcagatcaaagagagcct
gcgggcagagctcagggtgacagagagaagggcagaagtgcccacagcccaccccagcccctcacccaggccagccggc
cagttccaaaccctggtgttttgggtgctggtggtggttggtggagtcctggcttgctatagcttgctagtaacagtggcctttattatt
ttctgggtgaggagtaagaggagcaggctcctgcacagtgactacatgaacatgactccccgccgccccgggcccacccgca
agcattaccagccctatgccccaccacgcgacttcgcagcctatcgctccgatagagtgaagttcagcaggagcgcagacgcc
cccgcgtaccagcagggccagaaccagctctataacgagctcaatctaggacgaagagaggagtacgatgttttggacaagag
acgtggccgggaccctgagatggggggaaagccgagaaggaagaaccctcaggaaggcctgtacaatgaactgcagaaag
ataagatggcggaggcctacagtgagattgggatgaaaggcgagcgccggaggggcaaggggcacgatggcctttaccagg
gtctcagtacagccaccaaggacacctacgacgcccttcacatgcaggccctgccccctcgctaa
SEQ ID NO:52, PD1.BBZ:

atgcagatcccacaggcgccctggccagtcgtctgggcggtgctacaactgggctggcggccaggatggttcttagactcccc

agacaggccctggaacccccccaccttctccccagccctgctcgtggtgaccgaaggggacaacgccaccttcacctgcagct
tctccaacacatcggagagcttcgtgctaaactggtaccgcatgagccccagcaaccagacggacaagctggccgccttcccc
gaggaccgcagccagcccggccaggactgccgcttccgtgtcacacaactgcccaacgggcgtgacttccacatgagcgtgg
tcagggcccggcgcaatgacagcggcacctacctctgtggggccatctccctggcccccaaggcgcagatcaaagagagcct
gcgggcagagctcagggtgacagagagaagggcagaagtgcccacagcccaccccagcccctcacccaggccagccggc
cagttccaaaccctggtgttttgggtgctggtggtggttggtggagtcctggcttgctatagcttgctagtaacagtggcctttattatt
ttctgggtgcggggcagaaagaaactcctgtatatattcaaacaaccatttatgagaccagtacaaactactcaagaggaagatg
gctgtagctgccgatttccagaagaagaagaaggaggatgtgaagatagagtgaagttcagcaggagcgcagacgcccccgc
gtaccagcagggccagaaccagctctataacgagctcaatctaggacgaagagaggagtacgatgttttggacaagagacgtg
gccgggaccctgagatggggggaaagccgagaaggaagaaccctcaggaaggcctgtacaatgaactgcagaaagataag
atggcggaggcctacagtgagattgggatgaaaggcgagcgccggaggggcaaggggcacgatggcctttaccagggtctc
agtacagccaccaaggacacctacgacgcccttcacatgcaggccctgccccctcgctaa

### Example 3: T cells expressing affinity molecule chimeric receptors or bispecific antibodies.

**Figure 29** is a panel of graphs showing expression of Affinity antibody mimetic redirected T cells (ARTs) by staining with anti-His antibody. Ten micrograms of RNA encoding ARTs against either EGFR (955.BBZ, 1853.BBZ or 1970.BBZ) or ErbB2 (342.BBZ, 432-15.BBZ, 342-14.BBZ or 342-4.BBZ) was electroporated T cells and cultured in R10 overnight. One hundred microliters of electroporated T cells were stained by an anti-His tag antibody for flow cytometry detection of ART expression (lower panel) and showed that T cells electroporated with all the ART RNA were stained positive, comparing with the T cells that were no electroporated (No EP).

**Figure 30** is a panel of graphs showing specific CD107a up-regulation of EGFR and ErbB2 ARTs. Electroporated T cells as shown in **Figure 29** were stimulated with 4 different tumor cell lines that express EGFR and/or ErbB2 as indicated below the name of each tumor. After 4h incubation, CD107a upregulation was measured by staining the cell with CD107a-PE, CD3-APC and CD8-FITC. As shown in the **Figure 29****,** all the EGFR ARTs only strongly reactive to the tumor lines that are EGFR positive (SK-OV3, MDA231 and MDA468), but not EGFR negative tumor MCF7. While all the ErbB2 ARTs strongly reactive to the tumor lines that are ErbB2 positive (SK-OV3, MDA231 and MCF7), but not ErbB2negative tumor MDA468. 4D5.BBZ and 2224.BBZ were CARs against ErbB2 and EGFR respectively.

**Figure 31** is a graph showing specific IFN-gamma production of EGFR and ErbB2 ARTs. Electroporated T cells as shown in **Figure 29** were stimulated with 4 different tumor cell lines that express EGFR and/or ErbB2 as indicated below the name of each tumor. After overnight incubation, supernatant was used to measure INF-gamma production by ELISA. IFN-gamma could be detected at different levels for all the EGFR ARTs stimulated by the tumor lines that are EGFR positive (SK-OV3, MDA231 and MDA468), but not EGFR negative tumor MCF7. While all the ErbB2 ARTs strongly reactive to the tumor lines that are ErbB2 positive (SK-OV3, MDA231 and MCF7), but not ErbB2negative tumor MDA468. 4D5.BBZ and 2224.BBZ were CARs against ErbB2 and EGFR respectively.

**Figure 32** is a graph showing specific IL-2 production of EGFR and ErbB2 ARTs. Electroporated T cells as shown in **Figure 29** were stimulated with four different tumor cell lines that express EGFR and/or ErbB2 as indicated below the name of each tumor. After overnight incubation, supernatant was used to measure IL-2 production by ELISA. IL-2 could be detected at different levels for all the EGFR ARTs stimulated by the tumor lines that are EGFR positive (SK-OV3, MDA231 and MDA468), but not EGFR negative tumor MCF7. While all the ErbB2 ARTs strongly reactive to the tumor lines that are ErbB2 positive (SK-OV3, MDA231 and MCF7), but not ErbB2negative tumor MDA468. 4D5.BBZ and 2224.BBZ were CARs against ErbB2 and EGFR respectively.

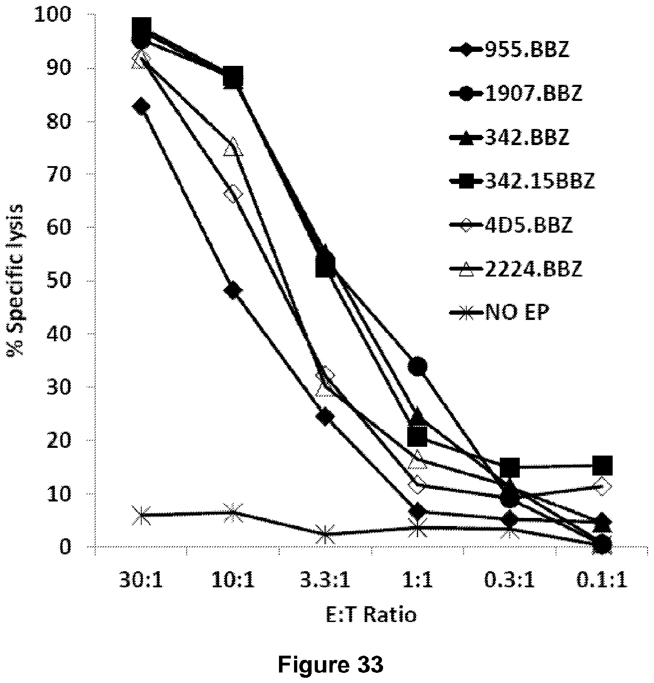

**Figure 33** is a graph showing specific lytic activities of EGFR and ErbB2 ARTs. In a separate experiment to test the killing ability of two EGFR and two ErbB2 ARTs, comparing to their relevant CARs, against tumor cell line SK-OV3 that is positive for both EGFR and ErbB2. ART T cells killed the tumor cells as efficiently as relevant CAR T cells.

**Figure 34A** is a diagrammatic sketch of Affinity antibody mimetic modified TCR (Affi-TCR) by adding affinity antibody mimetic and His -tag sequence to the N' of either alpha or beta chain of a TCR..

**Figure 34B** is a panel of graphs showing Vb13.1 TCR and His-tag detection of affinity antibody mimetic redirected TCR ( Affi-TCR) RNA electroporated T cells. T cells were co-electroporated with NY-ESO-1 (1G4) TCR alpha (a) and beta (b), or their ErbB2 affinity antibody mimetic (342, 342.15, 342 or 342.4) modification . After overnight, vb13.1 and His-Tag were detected by flow cytometry.

**Figure 34C** shows the ErbB2 affinity antibody mimetic sequences.

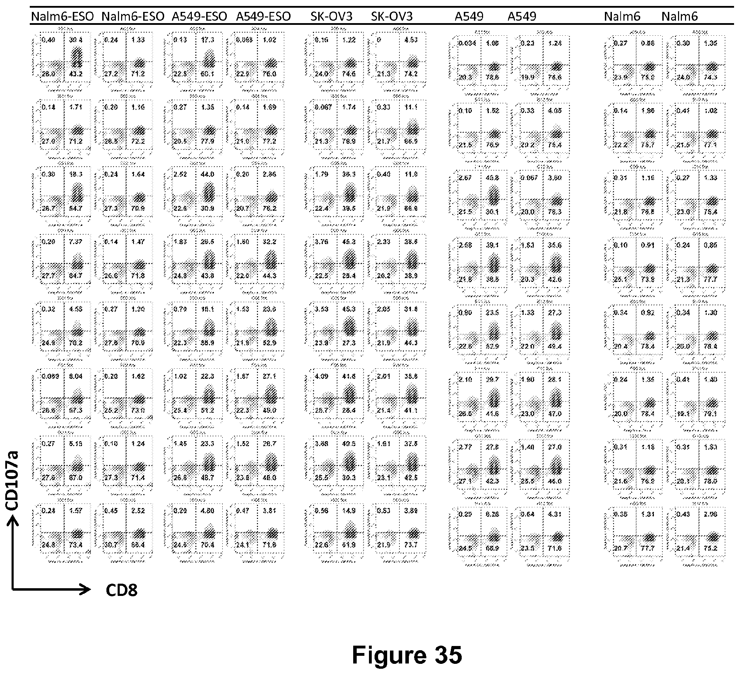

**Figure 35** is a panel of graphs showing CD107a up-regulation of Affi-TCR RNA electroporated T cells. T cells were co-electroporated with TCR alpha (a) and beta (b), or their ErbB2 affinity antibody mimetic (342, 342.15, 342 or 342.4) modification as indicated in **Figure 34B** and stimulated with tumor line Nalm-6-ESO (NY-eso-1+, ErbB2-), A549-ESO (NY-eso-1+, ErbB2+), SK-OV3 (NY-eso-1-, ErbB2+), A549 (NY-eso-1-, ErbB2+), or Nalm6 (NY-eso-1-, ErbB2-), for CD107a assay. The results show that T cells with ErbB2 Affi-TCR could specifically recognize both Ny-ESO-1 and ErbB2 positive tumors.

**Figure 36A** is a diagrammatic sketch of affinity antibody mimetic modified CD3 epsilon by adding affinity antibody mimetic and G4S linker to the N' of either CD3 epsilon.

**Figure 36B** is a table showing electroporation of T cells. T cells were co-electroporated with NY-ESO-1 (1G4) TCR alpha (a) and beta (b), or together with ErbB2 affinity antibody mimetic (342, 342.15, 342 or 342.4) modified CD3 epsilon (e).

**Figure 36C** is a panel of graphs showing t maintainenance of TCR expression and dual tageting by co-delivering affinity antibody mimeti modified CD3 epsilon. After overnight of the T cells of **Figure 36B****,** vb13.1 expression was detected by flow cytometry.

The results show that adding affinity antibody mimetic to CD3 epsilon minimally influenced NY-ESO-1 TCR expression, as shown 76.5% to 82.9% CD8/vb13.1 double positive for all Affi-epsilon co-electroporated T cells (EP3 to EP6), compared with 44.5% for affinity antibody mimetic modified TCR (EP2).

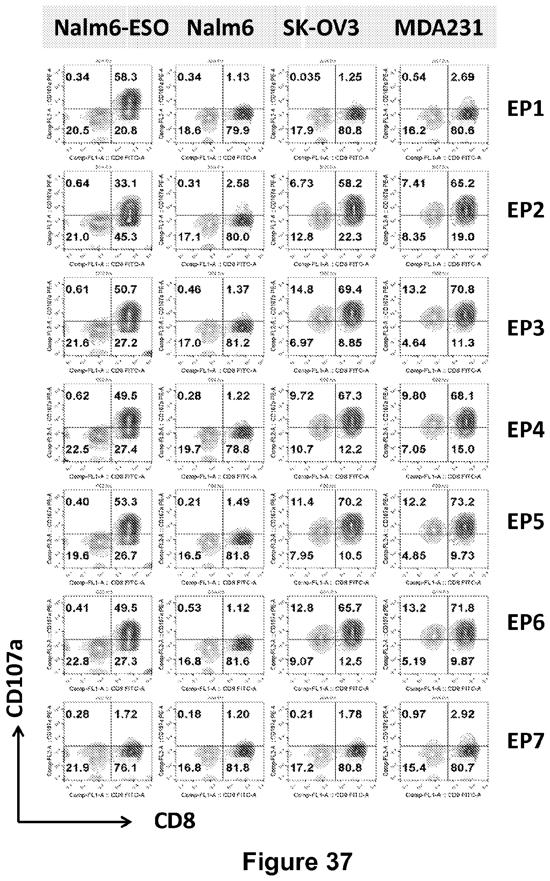

**Figure 37** is a panel of graphs showing CD107a up-regulation of Affi-TCR RNA electroporated T cells. T cells were co-electroporated with NY-ESO-1 (1G4) TCR alpha (a) and beta (b), or together with ErbB2 affinity antibody mimetic (342, 342.15, 342 or 342.4) modified CD3 epsilon (e) as indicated in **Figure 36B** and stimulated with tumor line Nalm-6-ESO (NY-eso-1+, ErbB2-), Nalm6 (NY-eso-1-, ErbB2-), SK-OV3 (NY-eso-1-, ErbB2+) or MDA231 (NY-eso-1-, ErbB2+), for CD107a assay.

The results suggest that adding affinity antibody mimetic to CD3 epsilon could minimally influence NY-ESO-1 TCR function of recognizing NY-ESO-1 single positive tumor Nalm6-ESO, as shown about 49.5% to 53.3% CD8/CD107a double positive for all Affi-epsilon co-electroporated T cells (EP3 to EP6), compared with 33.1% for affinity antibody mimetic modified TCR (EP2). Moreover, those Affi-epsilon co-electroporated T cells (EP3 to EP6) demonstrated higher antitumor activity than affinity antibody mimetic modified TCR (EP2) against ErbB2 positive tumor, SK-OV3 and MDA231, cells.

### Example 4: Bispecific T cell engagers (BiTEs), modified T cells with bispecific antibodies

### Functional BiTEs could be secreted from Bis-RNA electroporated T cells and engage to T cells with specific tumor reactivity.

T cells transferred with Bis-RNA (Bis-RNA T) were tested for their ability to secrete functional bispecific T cell engagers (BiTEs), and the roles of the BiTEs engaged in both Bis-RNA electroporated and non-Bis-RNA electroporated T cells were identified. Also, the potential increase of T cell anti-tumor activity was assessed when co-introducing CAR RNA and Bis-RNA.

First, surface staining of the T cells was conducted with an antibody that can detect murine origin Fab (mIgGFab). Positive staining was found for the T cells electroporated with either CAR (CAR RNA) or Bis-RNA **(****Figure 38A****,** upper panel). To test if Blinatumomab secreted by the Bis-RNA T could also be loaded to other T cells, GFP RNA electroporated T cells (GFP T cells) were mixed with either Bis-RNA T or CAR-RNA T, either immediately after electroporation **(****Figure 38A****,** middle panel) or before staining **(****Figure 38A****,** lower panel). It was found that GFP-RNA T cells could be stained positive for mIgGFab, as long as they were co-incubated with Bis-RNA T cells, but not with CAR19-RNA T, suggesting that Blinatumomab secreted from Bis-RNA T could not only be engaged to Bis-RNA T cells, but also to other T cells.

The function of the Bis-RNA T cells, or the GFP T cells co-incubated with Bis-RNA T cells were tested in a four hour CD107a assay by stimulating the T cells with CD19 positive tumor lines (Nalm6, K562-CD19 and Raji). As shown in **Figure 38B****,** antigen specific CD107a up-regulation for the Bis-RNA T cells was as efficient as for CAR-RNA T cells. Moreover, significant antigen specific CD107a up-regulation, at the similar levels of CAR-RNA T cells and Bis-RNA T cells, was also evidenced for GFP-RNA T cells for cells co-incubated with Bis-RNA-T, but not with CAR-RNA T cells.

In a four hour cytotoxic T lymphocyte killing assay, it was found that Bis-RNA T cells killed tumor as efficiently as CAR19-RNA T cells (CD19) **(****Figure 38C****).** However, when these T cells were mixed with equal amount of non-tumor reactive GFP-RNA T cells, Bis-RNA T cells mixed with GFP-RNA T cells (Bis- RNA/GFP-T cells) showed significant higher lytic ability over CAR19-RNA T cells mixed with GFP RNA T cells (CD19/GFP-T cells). This could be due to the decreased E:T ratio when mixing CAR19-RNA T cells with GFP-RNA T cells. While mixing Bis-RNA T cells with GFP-RNA T cells, the E:T ratio was maintained via enabling the GFP-RNA T cells specific tumor reactivity by engaging them with Blinatumomab secreted from Bis-RNA T cells. It is remarkable that the Blinatumomab Bis-RNA T cells were only activated when they were stimulated by CD19 positive cell lines, such as K562-CD19, Nalm6 and Raji, but not CD19 negative cell line K562. This indicated the BiTEs introduced in the form of Bis-RNA would only activate T cells in an antigen specific manner.

To further confirm that BiTEs could be produced by the Bis-RNA electroporated T cells, ten or 100 times diluted supernatant harvested from the electroporated T cells was added to non-electroporated T cells and mixed with tumors for a CD107a assay. It was found that the non-electroporated non-tumor reactive T cells became highly tumor reactive by adding the supernatant collected from the Bis- RNA T cells **(****Figures 45A and 45B****).**

### Increased specific T cell activation sensitive and tumor killing ability and prolonged tumor reactivity of Bis-RNA electroporated T cells.

To test the sensitivity of Bis-RNA T cells for tumor recognition, T cells were electroporated with different doses of Bis-RNA and compared with CD19 CAR RNA. Similar results were obtained from CD107a up-regulation **(****Figure 39A****).**

In the experiments of IFN-gamma/Granzyme B intracellular staining **(****Figure 39B****)**and IFN-gamma production assayed by ELISA **(****Figure 39C****),** significant T cell activation was observed when as low as 0.5 µg Bis-RNA was used. Yet, 1-2 µg of Bis-RNA remained comparable to 5-10 µg CD19 CAR RNA. Despite the Bis-RNA being at 0.5 µg in the low dose, Bis-RNA T cells and co-incubated GFP-RNA T cells still showed efficient anti-tumor activities.

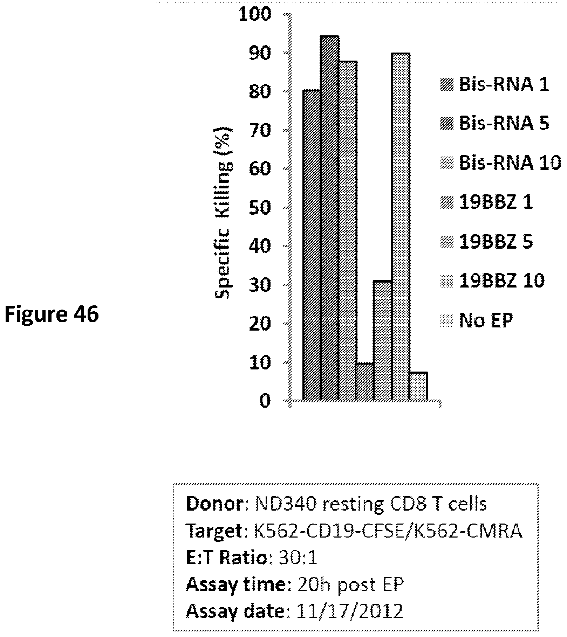

In a four hour cytotoxic T lymphocyte assay, the lytic ability of T cells with different amounts of RNA at the doses of 1, 5 and 10 µg of either Blinatumomab Bis-RNA or CD19BBZ CAR RNA was compared. As shown in **Figure 39D****,** the killing ability of T cells showed correlation with the RNA doses for both Bis- RNA and CAR RNA. Yet, T cells with 1 µg fo Bis-RNA killed tumors at similar levels as T cells with 10 µg CD19BBZ CAR RNA at effector to target ratios from 1:1 to 30:1 **(****Figure 46****).**

To test the functional persistence of the T cells electroporated with Blinatumomab RNA, increasing doses of RNA were electroporated into the T cells, in comparison with CD19 CAR RNA. The antigen specific T cell reactivation by stimulating T cells with CD19+ tumor lines at different times after electroporation was followed by looking at CD107a up-regulation levels. As already shown in **Figure 39A** for day 1 after electroporation, as long as the RNA dose was over 1 µg, Bis-RNA T cells could be activated as efficient as 5-10 µg CD19-CAR-T cells.

A new experiment was set to follow the T cells functional persistence starting at day 3 post electroporation, up to 14 days post electroporation **(****Figures 40A** **and** **40B****).** It was found that day 3 post electroporation, Bis-RNA T cells with 5 and 10 µg RNA still showed very strong anti-tumor reactivity, evidenced by high CD107a upregulation, while the anti-tumor reactivity of 10 µg CAR19-RNA T cells declined to 1 µg Bis-RNA T cell levels. At day 8, the tumor reactivity of 10 µg CAR19-RNA T cells was much lower than 1 µg Bis-RNA T cells and decreased to nearly negative levels, while the tumor reactivity still remain at high levels for Bis-RNA T cells with 5 and 10 µg RNA. Most significantly, at day 12 post electroporation, significant amount of CD107a remained detectable for Bis-RNA T cells at 5 and 10 µg RNA dose. The results indicate that BiTEs could be stably loaded on T cells with a low turnover rate and maintain T cell tumor reactivity for a relatively longer amount of time, compared with functional CAR expressed on T cells introduced by RNA electroporation.

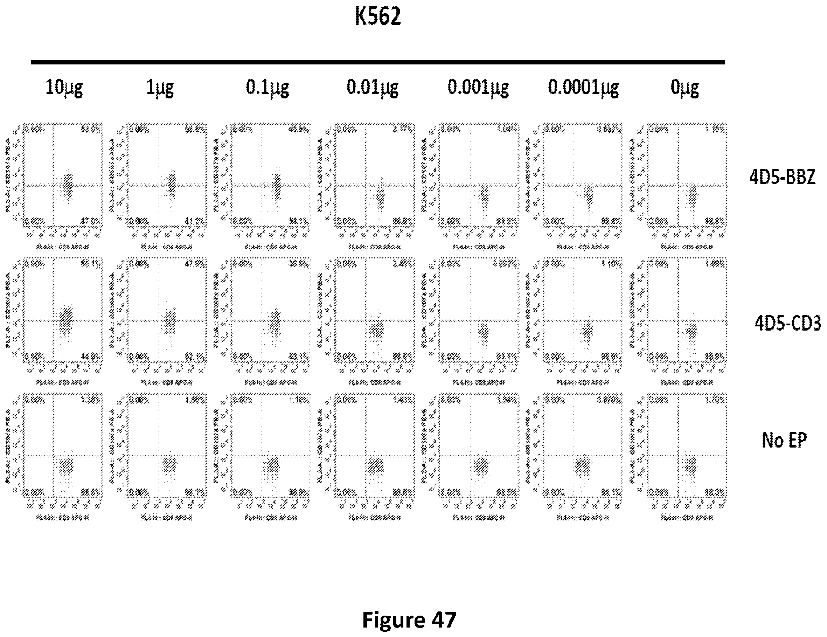

To test the recognition sensitivity of BiTEs provided to the T cells through Bis-RNA, K562 cell line expressing CD19 at different levels was used to stimulate T cells electroporated with either CAR RNA or Bis-RNA at 5 µg or 10 µg respectively. As shown in **Figure 40C****,** T cells for both CAR RNA and Bis-RNA recognized CD19 at the same level of 0.001µg. K562 cell line expressing ErbB2 at different levels was also used to stimulate T cells electroporated with either 4D5BBZ CAR RNA or 4D5-CD3 Bis-RNA. Similar to what was found for CD19 antigen testing, both ErbB2 (4D5) CAR RNA and Bis-RNA could recognize the antigen at the 0.1 ug level as evidenced by both IFN-gamma secretion **(****Figure 40D****)** and CD107a up-regulation **(****Figure 47****).**

### Less co-stimulatory dependent division and proliferation of Bis-RNA electroporated T cells.

The T cell division and proliferation were tested by stimulating the Blinatumomab Bis- RNA T cells, or CD19 CAR-RNA T cells with K562 expressing CD19 and K562 (K562-CD19/K562) or with K562-CD19 and K562 expressing CD86 (K562-CD19/K562-CD86). When the T cells were stimulated with K562-CD19 without providing co-stimulation, Bis-RNA T cells could divide efficiently, even at RNA doses as low as 1 µg. On the contrary, the division of CAR-RNA T cells remained much less efficient, which was evidenced by the fact that there was little detection of T cell division at the RNA dose of 1 µg. Even at the RNA dose of 10 µg, the CAR-RNA T cells did not divide as efficiently as Bis-RNA T cells at the RNA dose of 1 µg **(****Figures 41A** **and** **48A****).**

The extent of T cell division of Bis-RNA T cells was correlated with cell proliferation and expansion, which relys on both T cell division and survival. As shown in **Figure 41B****,** left panel, only Bis-RNA T cells showed T cell expansion, even at the 1 µg RNA dose. CAR-RNA T cells showed no T cell expansion at all RNA doses. Although CAR-RNA T cells at the 5 and 10 µg RNA doses showed significant CFSE dilution, there was no T cell expansion detected. These results suggest that without providing additional co-stimulatory signals during the stimulation process, Bis-RNA T cells were still capable of receiving sufficient stimulation signals to maintain T cell division, proliferation and survival, while stimulating signals received by CAR-RNA T cells were insufficient for most of the dividing cells to survive.

By adding CD28 signal to the co-stimulation, it was found that CD28 co-stimulation greatly enhanced both division (for all RNA doses) and proliferation (only at RNA dose of 10 µg) of CAR-RNA T cells. While, CD28 co-stimulation slightly increased the Bis-RNA T cells division with enhanced cell proliferation at all the Bis-RNA doses **(****Figures 41B****,** **48A****, and** **48B****).**

To further test if T cells could be efficiently activated with decreasing amounts of Bis-RNA and the influence of T cell status on stimulation, CFSE dilution and the cell proliferation of CD45RO+ memory or CD45RO- naive T cells were assessed with different doses of RNA. It was found that T cells electroporated with as low as 0.2 µg of RNA could be efficiently activated, as evidenced by CFSE dilution of T cells and cell expansion for CD45RO- naive cells or CD45RO+ memory cells co-stimulated with K562-CD86 (equivalent as T cells with 5 µg CD19BBZ RNA in most cases) **(****Figures 41C****,** **41D****, and** **48C****).** This further confirms that T cells with Bis-RNA were more sensitive to T cell activation and less dependent on co-stimulation than T cells with CAR RNA.

Interestingly, a direct correlation was found between CFSE dilution and IFN-gamma levels of Bis- RNA T cells and CAR-RNA T cells, but a reversed correlation was found between CFSE dilution and IL-2 levels. Eight days after stimulation of CFSE labeled T cells, the cytokines in the culture supernatant were examined **(****Figures 41A** **and** **48D****).** As a result, Bis-RNA T cells showed higher IFN-gamma production, indicating more completed T cell activation. Bis-RNA T cells also showed higher consumption of medium-supplemented IL-2(10IU/mL), suggesting higher levels of T cell proliferation **(****Figure 48E****).** Increased T cell activation of Bis-RNA T cells was also evidenced by examining a panel of cytokines and chemokines, which showed a global increase in most cytokines and chemokines assayed, without a polarization preference **(****Figure 49B****).**

To exclude that the CD28 co-stimulation dependence of CAR RNA T cells was due to the use of BBZ configuration, in which there is no CD28 signaling moiety, CD19-28Z CAR RNA was added to assess the division and proliferation capacity of CAR RNA and Bis- RNA electroporated T cells. It was found that both CFSE dilution **(****Figure 48F****)** and T cell expansion **(****Figure 48G****)** for CD19-28Z CAR RNA T cells were slightly increased, compared to CD19BBZ CAR RNA T cells upon stimulation with a CD19+ tumor line with or without additional CD28 co-stimulation.

Both the CFSE dilution and cell expansion assessments of 5 ug CD19- 28BBZ CAR RNA T cells were significantly lower than 1 ug Bis-RNA T cells, even in the presence of additional CD28 co-stimulation. In a four hour cytotoxic T lymphocyte assay, it was shown that both CD19BBZ and CD19-28Z RNA electroporated T cells had similar lytic ability, yet T cells with Blinatumomab RNA (Blina) showed stronger killing ability over CAR RNA T cells **(****Figure 48H****).**

### Enhanced anti-tumor activity of Bis-RNA T in a leukemia mouse model.

The findings suggest that Bis-RNA T cells may have enhanced functionality in vivo demonstrated by increased CD107a up-regulation, cytokine production, tumor lytic ability, and cell dividing and proliferation capacity as compared to CAR RNA T cells. Seven days after NOD-SCID-?c-/- (NSG) mice received green luciferase-transduced Nalm6 cells (Nalm6-CBG), the mice were treated with T cells that were electroporated with CD19BBZ RNA CAR (RNA CAR) and/or Blinatumomab Bis-RNA as indicated **(****Figures 42A** **and** **42B****).**

For mice treated with a single dose of T cells, CAR RNA T cells showed significant tumor burden reduction compared with mice treated with control CAR RNA T cells (ss1BBZ). Mice treated by Bis-RNA T cells, or T cells co-electroporated with both CAR RNA and Bis-RNA, demonstrated enhanced tumor regression, evidenced by approximately 1-2 log tumor density reduction. When the tumor bearing mice were treated with multiple T cell dose injections, the tumor burden for both groups of mice, treated with CAR RNA T cells and Bis-RNA T cells, were reduced to background levels.

To evaluate if enhanced anti-tumor activity of Bis-RNA T cells in vivo is due to enhanced persistence of the cells, mice given a single injection of human T cells were euthanized and cells from the bone marrow and spleens were purified for functional analysis. T cells were analyzed for CD137 expression **(****Figure 42C****),** IFN-gamma production **(****Figure 42D****),** and CD107a **(Figure 42E)** expression after stimulation with a CD19 positive cell line, K562-CD19. CD19BBZ CAR T cells (CAR RNA T) and CD19 Bis-RNA T cells (Bis-RNA T) demonstrated high reactivity to specific antigen stimulation at day 1 post T cell injection in all three functional assays. While at day 3 post injection, CAR RNA T cells still demonstrated some anti-tumor activity, at day 7 post injection, nearly all anti-tumor activity was absent for both CAR RNA and Bis-RNA T cells.

### Generating Bis-RNA using different scFvs targeting different tumor antigens.

The immunogenicity of the CARs derived from mouse scFv is a potential limitation threatening CAR therapy. In cases where an adverse immune response is provoked, often times the immune response is directed against the extracellular antigen recognition domain that is usually derived from mouse monoclonal antibodies (Lamer et al., *Blood* 117:72-82, 2011). A human anti-CD19 scFv (21D4) and a human anti-CD3 scFv (28F11) were chosen to make a fully human CD19-CD3 Bis-RNA. Initial attempts at making VH and VL chains of each single chain variable fragment (scFv) showed that the fully human CD19-CD3 Bis-RNA lacked any detectable function. New constructs were made having different VL/VH sequences, D4-F11HLHL.

As shown in **Figure 43A****,** expression of D4-F11HLHL Bis-RNA demonstrated over 90% CD107a up-regulation, compared with T cells electroporated with either CD19 CAR RNA (CD19BBZ) and Blinatumomab Bis-RNA (Blina-Bis-RNA), which upregulated CD107a at 78.89% and 80.96% respectively.

To further confirm functionality of the fully human D4-F11HLHL Bis-RNA, electroporated T cells were stimulated with different CD19 positive cell lines. IFN-gamma and IL-2 levels **(****Figure 49A****)** were detected after overnight co-culturing with the CD19 cells. T cells expressing D4-F11 HLHL produced significant higher levels of IFN-gamma, compared with T cells expressing CD19 CAR RNA or Blinatumomab Bis-RNA **(****Figure 43B****).** Cytokine production profiles showed that about 2-5 fold increased in cytokine production was observed from D4-F11 Bis-RNA T as compared to CD19 CAR RNA T cells for TNF-alpha, IL-2, IL-10, IFN-gamma and GM-CSF **(****Figure 49B****).** These results indicated an overall enhancement of T cell function for T cells electroporated with CD19-CD3 Bis-RNA.

FMC63 anti-CD19 scFv based CAR was used for most of the comparisons between CD19 CAR RNA and Blinatumomab based Bis-RNA. Despite difficulties in generating Bis-RNA from FMC63 scFv, the CAR RNA from FMC63 scFv demonstrated more functionality than the Bis-RNA derived from Blinatumomab anti-CD19 svFc (HD37).

To determine if functional differences between CAR RNA T cells and Bis-RNA T cells was due to different scFvs used in the CAR and bispecific antibody, a fully human CAR, D4BBZ, was generated using anti-CD19 scFv (21D4) and compared to two CD19 CARs (FMC63 CAR and 21D4 CAR) and two CD19-CD3 Bis-RNAs (HD37 Bis-RNA and 21D4 Bis-RNA) at RNA doses of 1 µg and 10 µg. Cytokine production of IFN-gamma and IL-2 **(****Figure 49C****)** and CD107a expression showed that 21D4 CAR was comparable to and slightly more functional than the FMC63 CAR. T cells with 21D4 Bis-RNA (D4-F11) showed much stronger anti-tumor activity, evidenced by significantly increased cytokine production and CD107a expression, especially at lower RNA doses **(****Figure 49D****).**

To test the anti-tumor activity of the fully human CD19 Bis-RNA (D4-F11), NSG mice (N=6) bearing Nalm6-CBG cells were treated with CD19BBZ or D4F11 lentiviral transduced T cells, or 19BBZ or D4F11 RNA electroporated T cells **(****Figure 43E****).** Comparing CD19BBZ or D4F11 lentiviral transduced T cells, lentiviral transduction with D4F11 produced T cells more anti-tumor activity than CD19BBZ lentiviral transduction. T cells electroporated with either CD19BBZ CAR RNA and D4F11 Bis-RNA initially controlled tumor growth in animals **(****Figure 49E****).** However, residual disease remained in both groups of animals, whereas animals receiving lentiviral transduced T cells lacked any residual disease.

To test the feasibility of converting other tumor antigen specific scFvs into Bis-RNAs and whether Bis-RNAs could be generated, scFv from mesothelin, cMet, PSCA or GD2 CARs were used to generate Bis-RNAs. Surface staining for scFv on T cells electroporated with these Bis-RNAs showed positive staining for mesothelin, cMet and PSCA, but not for GD2 **(****Figure 43C****).** Similarly, antigen specific CD107a up-regulation was observed in the T cells with relevant CAR RNAs, as well as T cells electroporated with mesothelin, cMet and PSCA Bis-RNAs, but not for GD2 Bis-RNA **(****Figure 43D****).**

Furthermore, three functional Bis-RNAs for EGFRviii (MR-1 and 139) and ErbB2 (4D5) were generated **(****Figures 49F****,** **49G****, and** **49H****).** Thus, 7 out of 8 CARs could be converted into Bis-RNA.

Moreover, it was found that there was little nonspecific T cell activation when T cells electroporated with mesothelin Bis-RNA were incubated with mesothelin negative cell lines, K562-PSCA, LY5Y and K562. However, T cells with mesothelin CAR RNA showed frequent non-specific T cell activation and CD107a expression remained at 46% to 55% over the negative control **(****Figure 43D****).** These data suggest that converting CARs to Bis-RNA may have potential therapeutic benefits, such as preventing on- or off-target toxicities associated with some CARs.

### Resistance to tumor associated suppression in Bis-RNA T cells.

Bis-RNA T cells showed higher cytokine production, increased proliferation, increased lytic ability and less co-stimulation dependency, when compared to CAR-*RNA T* cells. Such properties indicate that the Bis-*RNA T* cells were fully functional and less prone to tumor associated suppressions, such as suppression by regulatory T cells and influences from programmed cell death 1 (PD1) receptor-ligand interaction.

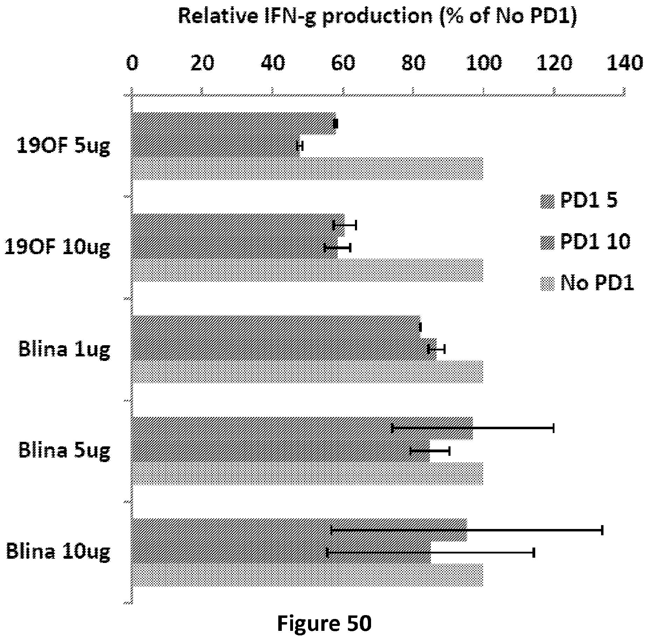

To test the influence of PD1 on T cell function, T cells were co-electroporated with CD19 Bis-RNA or CAR RNA and PD1 RNA at different RNA doses and stimulated with CD19 positive tumor cells. The results showed that T cells co-electroporated with either 5ug or 10ug PD1 RNA significantly reduced CAR-RNA T cell response to specific antigen stimulation, reduced CD107a expression **(****Figure 44A****)** and cytokine production (IFN-gamma and IL-2) **(****Figures 44B** **and** **50****).** However, T cell functions were minimally affected by PD1 at 1 ug and 5 ug of Bis-RNA with 10 ug PD1 RNA and no obvious negative influence on T cell function at 10 ug of Bis-RNA.

To test if Bis-RNA T cells could show more resistance to regulatory T cell (Treg) induced suppression, purified Tregs were added to CFSE labeled T effector cells. T effector cells were electroporated with Bis-RNA or CAR RNA and stimulated with a CD19 positive cell lines at different T effector:Treg ratios. Suppression of proliferation was evidenced as a maintanence of CFSE labled cells, as compared to non-Treg suppressed cells that displayed a dilution in CFSE signal over time **(****Figure 44D****).** Decreases in CFSE signal indicated Treg suppressed proliferation of T cells electroporated with CD19 CAR (19BBZ) at both 4:1 and 8:1 T effector:Treg ratios. However, less Treg suppression was evident with T cells electroporated with Blinatumomab Bis-RNA (Bis-RNA) (4:1 T effector:Treg ratio). No significant Treg suppression was observed above an 8:1 T effector:Treg ratio for Bis-RNA T cells **(****Figure 44C****).**

The combining stimulation (anti-CD3 scFv expressed on the cell surface) and co-stimulation (CD28 and 4-1BB) in a single molecule provides essential components to support efficient T cell expansion and activation. By co-introducing co-stimulatory molecules, novel T cell populations having a central memory phenotype with strong lytic capacity are two critical characteristics for T cell therapeutic efficacy, which is lacking in current T cell methodologies.

To test the sensitivity of Blinatumomab RNA (Bis-RNA) T cells for tumor recognition, T cells were electroporated with different doses of Bis-RNA and compared with CD19 CAR RNA. Similar results were obtained from CD107a up-regulation **(****Figure 51A****).**

In the experiments using IFN-gamma/Granzyme B intracellular staining **(****Figure 51B****)** and IFN-gamma production assayed by ELISA **(****Figure 51C****),** significant T cell activation was observed, even when only 0.5 µg Bis-RNA was electroporated. Yet, 1-2 µg of Bis-RNA remained comparable to 5-10 µg CD19 CAR RNA in the assay. Even at low amounts of Bis-RNA at 0.5 µg, Bis-RNA T cells and co-incubated GFP-RNA T cells still showed efficient anti-tumor activities.

In a four hour cytotoxic T lymphocyte assay, the lytic ability of T cells with different amounts of RNA at doses of 1, 5 and 10 µg of either Bis-RNA or CD19BBZ CAR RNA was compared. As shown in **Figure 51D****,** the killing ability of T cells correlated with the RNA doses for both Bis-RNA and CAR RNA.

To test the functional persistence of the T cells electroporated with bis-RNA, increasing doses of RNA were electroporated into the T cells, in comparison with CD19 CAR RNA. CD107a up-regulation levels were assessed after antigen specific reactivation of the T cells with CD19+ tumor cell lines at different times after electroporation. At day 1 after electroporation, as long as the RNA dose was over 1 µg, Bis-RNA T cells were activated as efficiently as T cells electroporated with 5-10 µg CD19-CAR RNA **(****Figure 51A****).**

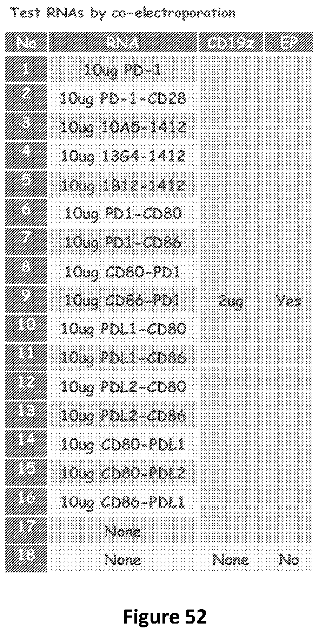

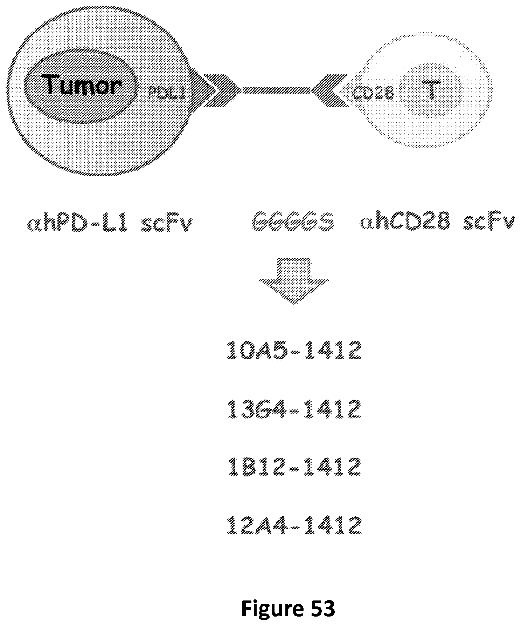

Constructs of four bi-specific antibodies with single chain variable fragments (scFvs) that block PD-L1(from patent No. AU2006265108A1) and an anti-CD28 scFv (1412, US7,585,960 B2) were designed and synthesized by PCR **(****Figure 52****).** Sequence verified DNA was properly cloned into pGEM.64A based RNA in vitro transcription vectors to generate pGEM.10A5-1-1412, pGEM.13G4-1412, pGEM.1b12-1412 and pGEM.12A4-1412, see **Figure 53****.**

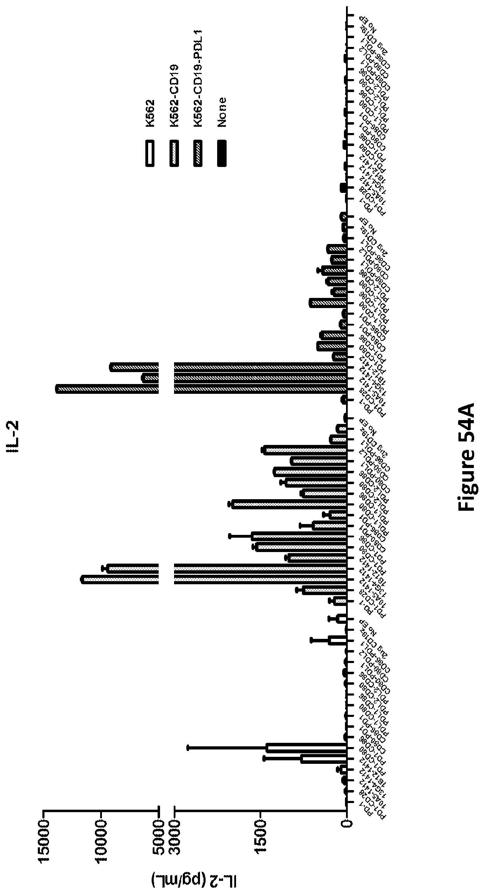

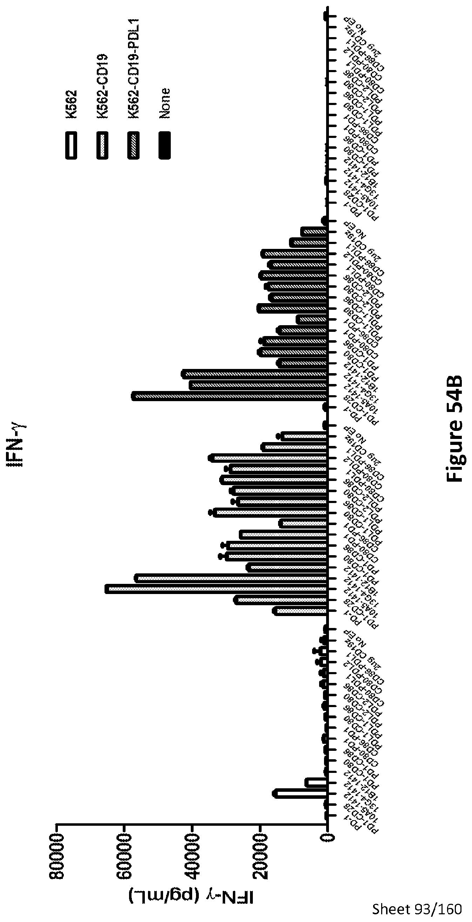

Cytokine (IL2, **Figure 54A****,** and IFN-gamma ,**Figure 54B****)** production detected by ELISA showed that PD1-CD28 switch receptors, 10A5-1412 (aPDL1-aCD28 bi-RNA) and 13G4-1412 (aPDL1-aCD28 bi-RNA) significantly improved T cell function by increasing the secretion of both IL-2 and IFN-gamma. The switch in function suggests a PD1 negative signal was switched to a CD28 positive signal. T cells with Bis-RNA switch receptors, 10A5-1412 (aPDL1-aCD28 bi-RNA) and 13G4-1412 (aPDL1-aCD28 bi-RNA), showed increased cytokine production, suggesting that these engineered T cells delivered activation molecules that positively improved T cell function.

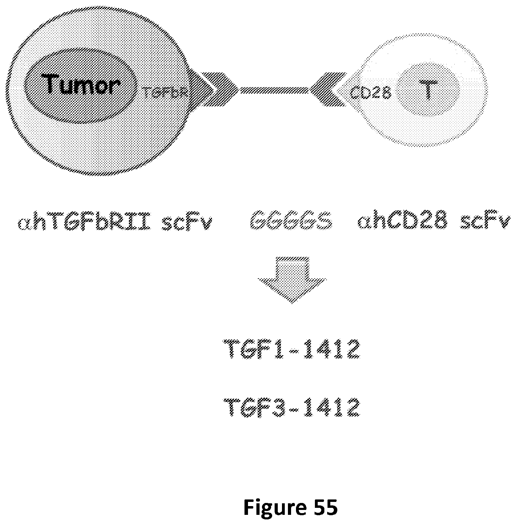

Constructs including anti-aTGFbRII-1, anti-aTGFbRII-3 (from US Patent No. 8,147,834) and an anti-CD28 scFv (1412, US Patent No. 7,585,960) were designed and synthesized by PCR. Sequence verified DNA was properly cloned into pGEM.64A based RNA in vitro transcription vectors to generate pGEM.aTGFbR-1-1412 and pGEM.aTGFbR-3-1412, see **Figure 55****.**

**Figure 56A** is a graph showing T cells electroporated with 4D5-CD3 Bis-RNA were reactive to ErbB2 over expressing tumor cells. T cells electroporated with ErbB2 CARs or Bis-RNA were stimulated with tumor lines expressing high levels of ErbB2, SK-OV3 and N87, or low levels, MFC-7, MDA-231, PC3 and A549. A CD107a assay showed that T cells expressing 4D5-6.CD3 were only reactive to ErbB2 over expressing tumor cells. **Figure 56B** is a panel of graphs showing results of a repeat of the experiment of **Figure 56A****.**

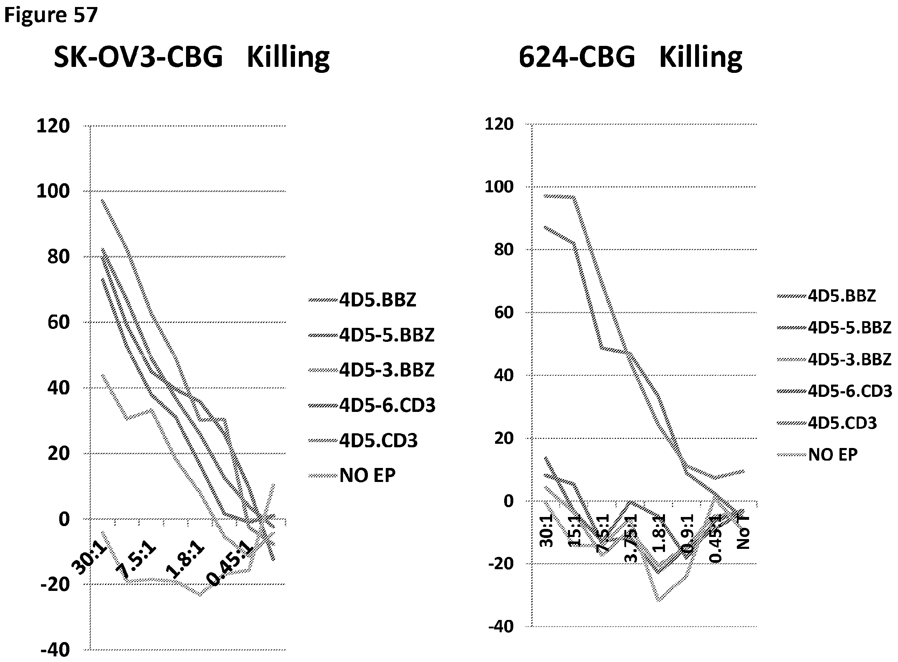

**Figure 57** is a panel of graphs showing lytic activity of T cells electroporated with 4D5-CD3 Bis-RNA to ErbB2 over expressing tumor cells. T cells electroporated with ErbB2 CARs or Bis-RNA were tested for their lytic activity against ErbB2 overexpressing tumor cells, SK-OV3-CBG, or ErbB2 low expressing tumor cells, mel624 (624-CBG). The luciferase based CTL assay showed that, like affinity tuned BebB2 CAR, 4D5-5.BBZ and 4D5-3.BBZ, T cells expressing 4D5-6.CD3 were only reactive to ErbB2 over expressing tumor cells, SK-OV3.

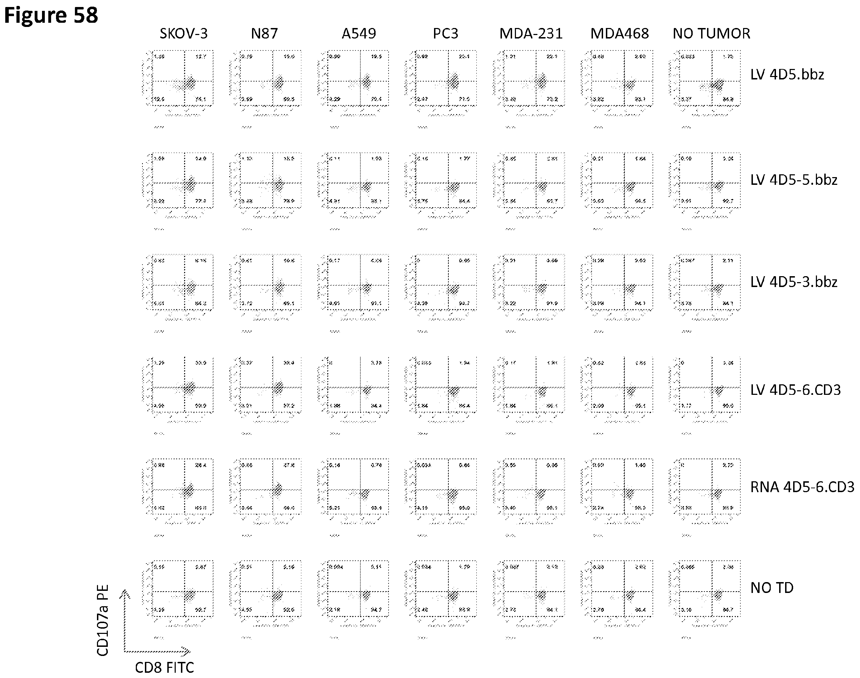

**Figure 58** is a panel of graphs showing T cells lenti-virally transduced with 4D5-CD3 Bis-RNA were reactive to ErbB2 over expressing tumor cells. T cells transduced with ErbB2 CARs or Bis-RNA were stimulated with tumor lines expressing high levels of ErbB2, SK-OV3 and N87, or low levels, MDA-231, PC3 and A549. A CD107a assay showed that T cells expressing 4D5-CD3 were only reactive to ErbB2 over expressing tumor cells.

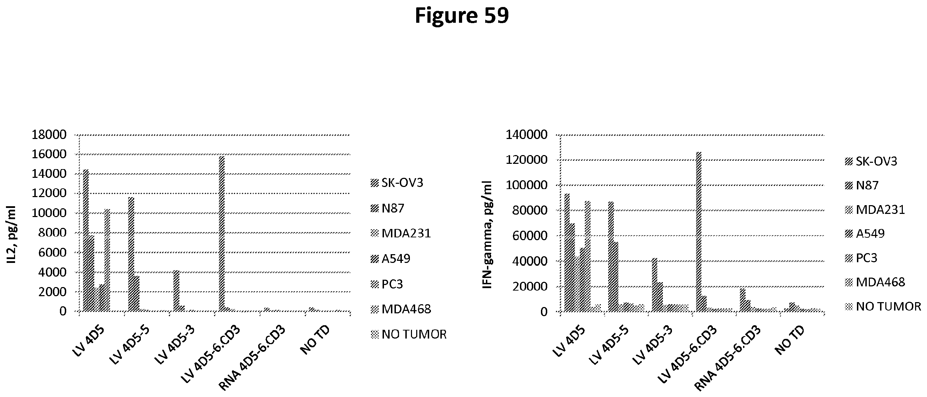

**Figure 59** is a panel of graphs showing T cells lenti-virally transduced with 4D5-CD3 Bis-RNA were reactive to ErbB2 over expressing tumor cells. T cells transduced with ErbB2 CARs or Bis-RNA as indicated were stimulated with tumor lines expressing high levels of ErbB2, SK-OV3 and N87, or low levels, MDA-231, PC3 and A549. Cytokine production as assayed by ELISA indicates T cells expressing T4D5-CD3 were only reactive to ErbB2 over expressing tumor cells.

**Figure 60A** is a panel of images showing affinity-tuned ErbB2 BiTE cells increase the therapeutic index and induce regression of advanced vascularized tumors in mice. T cells modified with different affinity ErbB2 CARs or BiTEs by lentiviral transduction were tested in dual-tumor engrafted NSG mice. Mice (n=4-5) were implanted with PC3-CBG tumor cells (1x10⁶ cells/mouse, s.c.) on the right flank on day 0. On day 5, the same mice were given SK-OV3-CBG tumor cells (5x10⁶ cells/mouse, s.c.) on the left flank. The mice were treated with T cells (i.v.) at day 23 after PC3 tumor inoculation. T cells were administered as a single injection of 1x10⁷/mouse. Mice treated with non-transduced T cells (No TD) served as controls. Animals were imaged at the indicated time post PC3 tumor inoculation. **Figure 60B** is a graph showing SK-OV3 tumor size in dual-tumor grafted NSG mice model. Tumor sizes were measured, and the tumor volume was calculated and plotted. **Figure 60C** is a graph showing PC3 tumor sizes in dual-tumor grafted NSG mice model. Tumor sizes were measured, and the tumor volume was calculated and plotted.

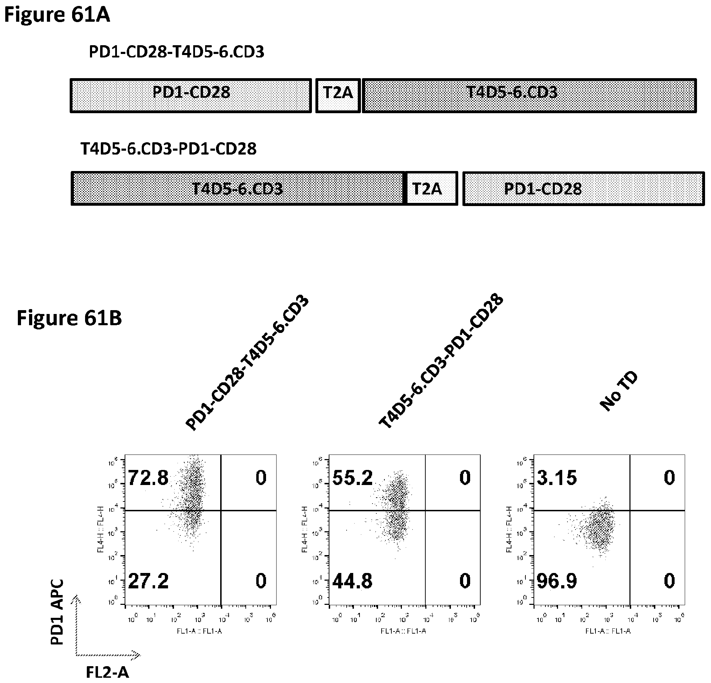

**Figure 61A** is an illustration of constructs for co-expressing PD1-CD28 switch receptor and affinity tuned T4D5-6.CD3 BiTEs. **Figure 61B** is a panel of graphs showing detection of PD 1-CD28 switch receptor of T cells lentivirally transduced with PD1-CD28 and T4D5-CD3 co-expression vectors.

**Figure 62A** is a graph showing increased lytic activity of T cells co-expressing both PD1-CD28 switch receptor and T4D5-6.CD3 affinity tuned BiTEs.

**Figures 62B-62G** are a panel of images showing Bis-RNA electroporated T cells generated by rapid expansion protocol (REP) of Dudley et al., J. Immunol., 26(4):332-342, 2003 modified for T cells directly isolated from normal donors. Bis-RNA electroporated T cells further improved in vivo anti-leukemia activity. Under REP conditions, the T cells are cultured with one or more factors, such as flt3-L, IL-1, IL-2, IL-3 and c-kit ligand. The phenotype of T cells expanded by REP or anti-CD3/anti-CD28 beads (Beads) was assessed **(****Figure 62B****).** REP T cells or anti-CD3/anti-CD28 bead T cells were electroporated with different amounts of CAR RNA or Bis-RNA and stimulated with different cell lines for 18 hr. CD137 up-regulation was analyzed by flow cytometry (gated on CD3+ T cells) **(****Figure 62C****).** Lytic activity was measured in REP T cells **(****Figure 62D****,** left panel) or anti-CD3/anti-CD28 beads T cells **(****Figure 62D****,** right panel) electroporated with different amounts of CAR RNA or Blinatumomab Bis-RNA. NSG mice were injected with 1x10⁶ Nalm6-CBG intravenously and 5 days later treated with 30x10⁶ CAR RNA or Blinatumomab Bis-RNA (Blina) T cells for the first treatment, followed by 5x10⁶ each, twice a week for three weeks starting day at 8 after Nalm6-CBG injection. Bioluminescence imaging (BLI) was conducted at the indicated times **(****Figure 62E****)** and the results of BLI and survival were plotted in **Figure 62F** and **Figure 62G****,** respectively.

**Figures 62H-62I** are a panel of images showing the generation of K562 based artificial antigen presenting cells (aAPC) expressing membrane bound OKT3. Lentiviral vectors (pLENS) expressing chimeric protein for membrane forms of OKT3 with either CD8 hinge and transmembrane (OKT3.8) or with CD8 hinge and CD28 transmembrane (OKT3.8.28) are illustrated in **Figure 62H****.** K562 based aAPC, K562-CD86-CD137L (KT) or K562-CD137L (2D11) cell lines were transduced with lentiviral OKT3.8 or OKT3.8.28 and the expression of the membrane bound OKT3 was detected using an antibody against murine IgG Fab **(Figure 62I).**

**Figure 62J** is a panel of images showing characterization of membrane bound OKT3 transduced K562 aAPC clones. Selection of the clones by limiting dilution was based on expression of membrane band OKT3 from OKT3.8.28 transduced KT aAPC.

**Figures 62K-62M** are a panel of graphs showing REP with K562 based artificial aAPC. **Figure 62K** is a graph showing OKT3 loaded K562-CD86-CD137L (KT) or K562-CD137L (2D11), or KT expressing membrane bound OKT3 (KT.OKT) were irradiated and cultured for one day (D1) or two days (D2) before being used to stimulated T cells at T cell:aAPC ratio of 1:250. **Figure 62L** is a panel of graphs showing the expanded T cells, independently expanded in the REP, stained for CD62L and CD28. **Figure 62M** is a graph showing different B cell lines in a REP experiment, as compared with KT cells.

### Example 5: T cells expressing modified TCRs.

Cancer patients treated with anti-tumor antigen TCR re-directed T lymphocytes by lentiviral or retroviral vectors show promising results. In this study, RNA was electroporated into T cells to determine if an efficient therapy for cancer adoptive immunotherapy could be developed. The T cells were compared to assess the in vivo potency of 1) T lymphocytes that expressed wildtype (wt) TCR or high affinity TCR against tumor antigen (NY-ESO-1) and 2) lentiviral vector transduced T cells as compared to RNA electroporated T cells that expressed wildtype (wt) TCR or high affinity TCR in Naml6 leukemia and A549 lung cancer mouse models.

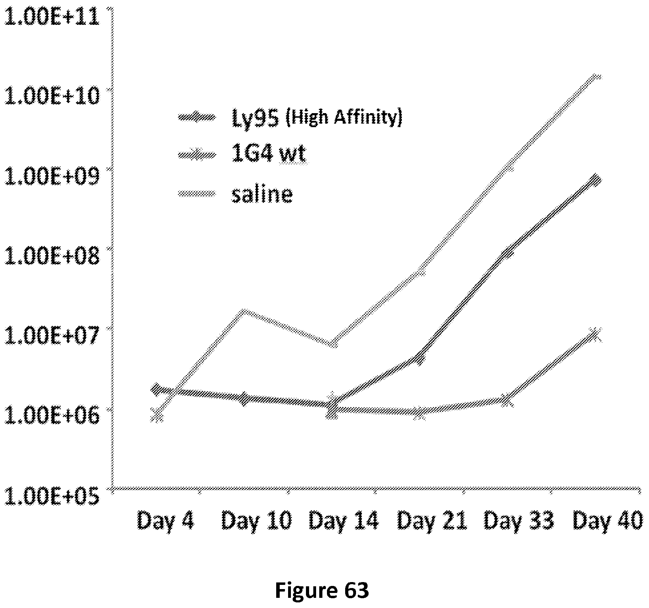

To improve TCR redirected T cell adoptive immunotherapy, T cells were electroporated with TCR RNA and the cells were injected into a leukemia mouse model. In NOD/SCID (NSG) mouse models, both lenti-virally transduced and RNA electroporated T cells expressing NY-EOS-1 wildtype TCR have similar treatment efficacies as higher affinity TCR **(****Figures 63****,** **64** and **65****).** The HLA-A2+, CD19+ leukemia cell line, Naml6, was transduced with NY-ESO-1 to generate Naml6-ESO tumor cells. One million of Naml6-ESO cells were injected into NSG mice and the mice were injected with T cells on day 5 or 7 (as indicated) after tumor inoculation. The tumor bearing mice were treated with T cells electroporated with either NY-ESO-1(1G4) wildtype TCR RNA (wt1G4), or its high affinity form (LY95a). It was found that T cells electroporated with NY-ESO-1 wildtype TCR RNA showed potent anti-tumor activity, while T cells with high affinity NY-ESO-1 TCR were much less potent **(****Figure 63****).**

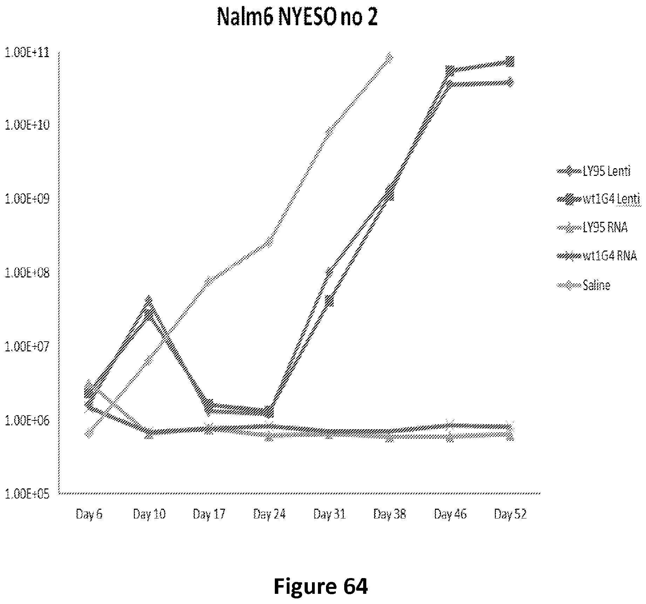

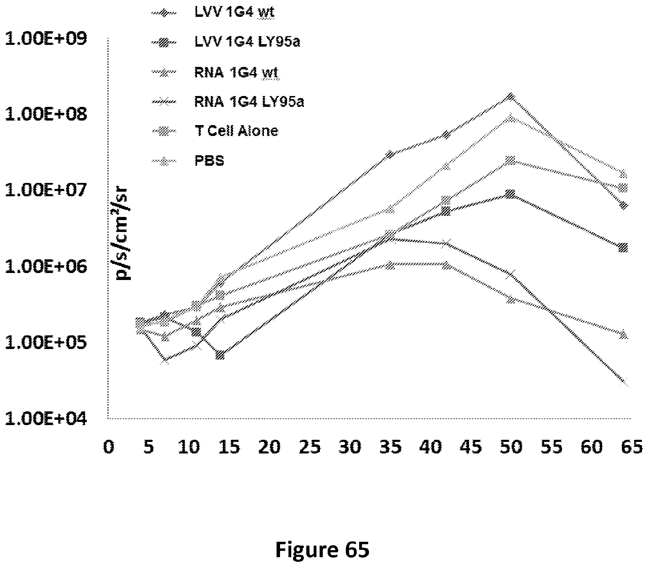

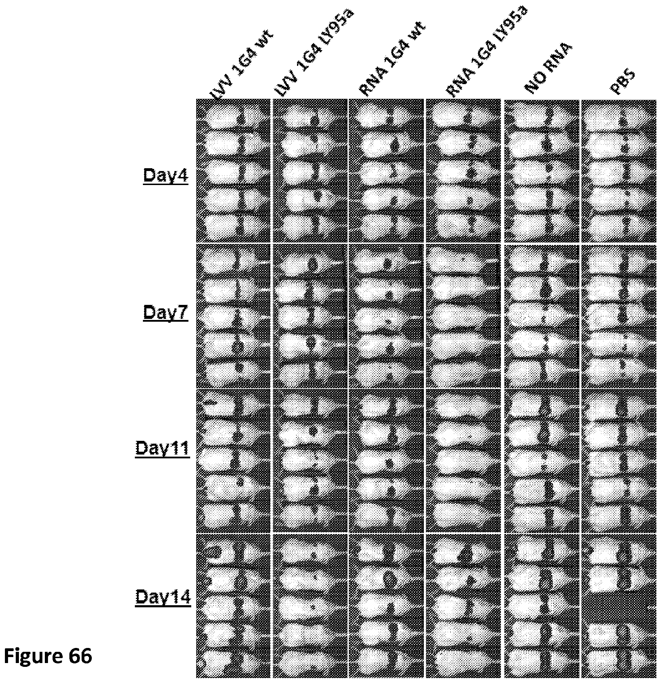

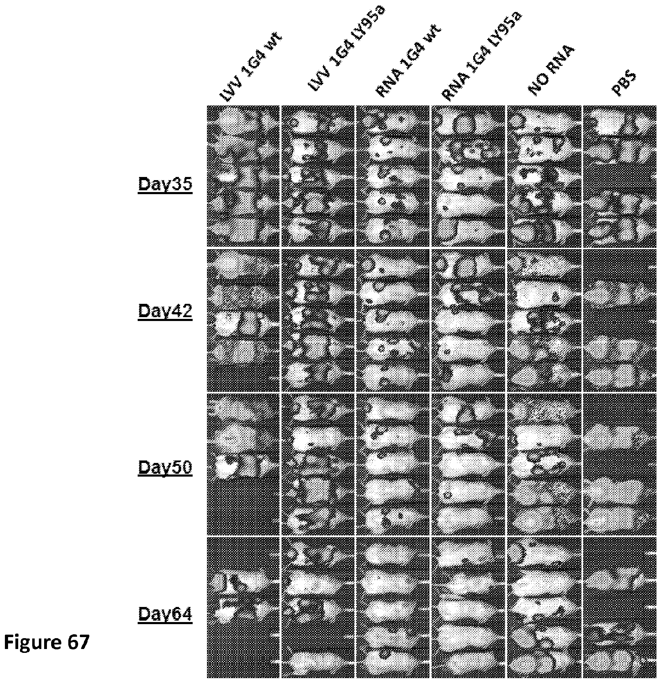

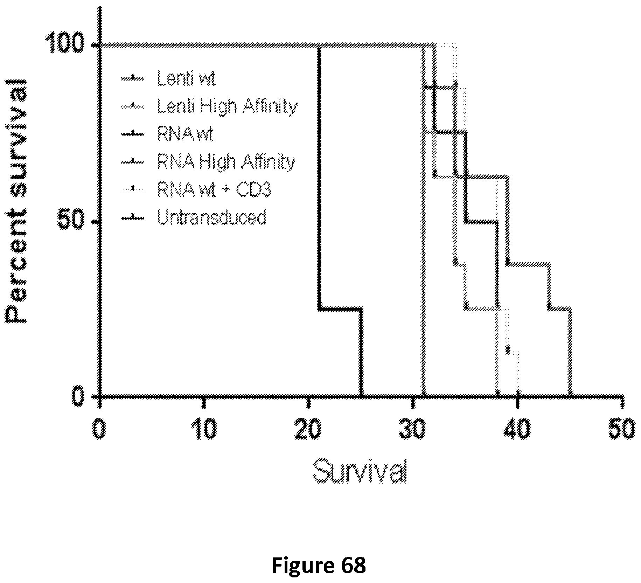

In both a Naml6-ESO leukemia and a A549 lung cancer NSG model, TCR (high affinity or wildtype) RNA electroporated T cells showed significantly better cancer treatment efficacy than lenti-transduced T cells with either the high affinity or wildtype TCRs **(****Figures 64** and **65****).** Multiple infusions of TCR RNA electroporated T cells controlled tumor growth longer than a single dose of lentiviral transduced T cells expressing either the high affinity TCR or wildtype TCR. This indicated that the electroporated T cells only transient treatment effect **(****Figure 64****).** T cells transferred with either wildtype TCR or high affinity TCR by either lenti-transduction or RNA electroporation were tested in the A549 lung model. It was found that both wildtype and high affinity TCR RNA T cells controlled tumor, but T cells with lentivirally transduced wildtype or high affinity TCR only showed slight and minimal treatment, as compared with TCR RNA T cells **(****Figure 65****).** **Figures 66-68** further show that TCR RNA electroporated T cells controlled tumor cell growth more efficiently than lenti-transduced T cells.

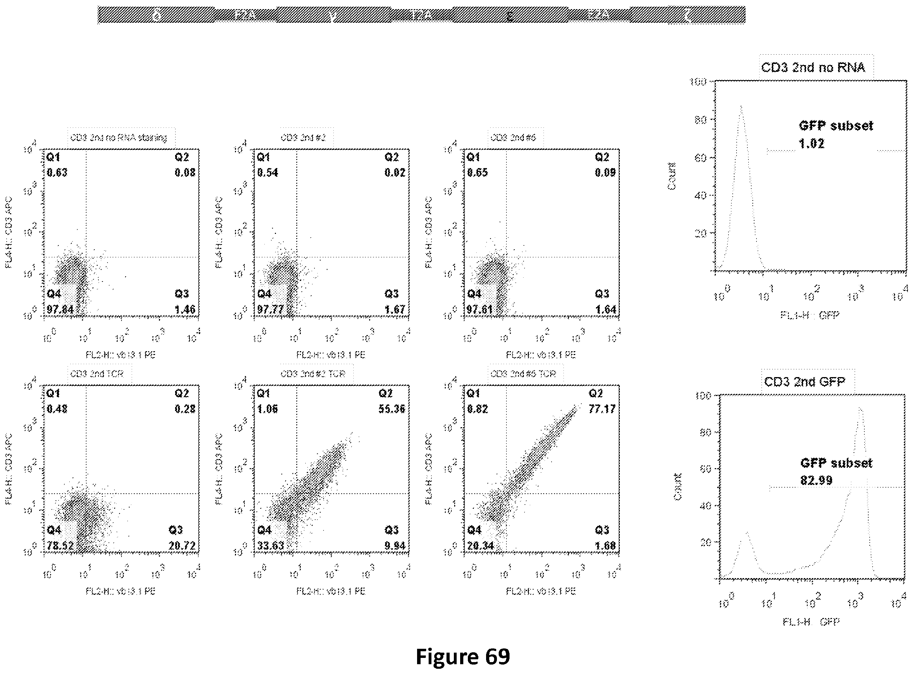

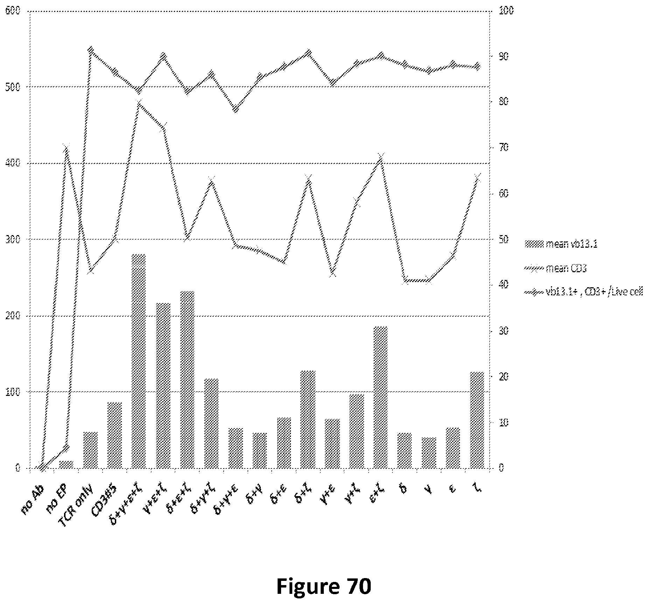

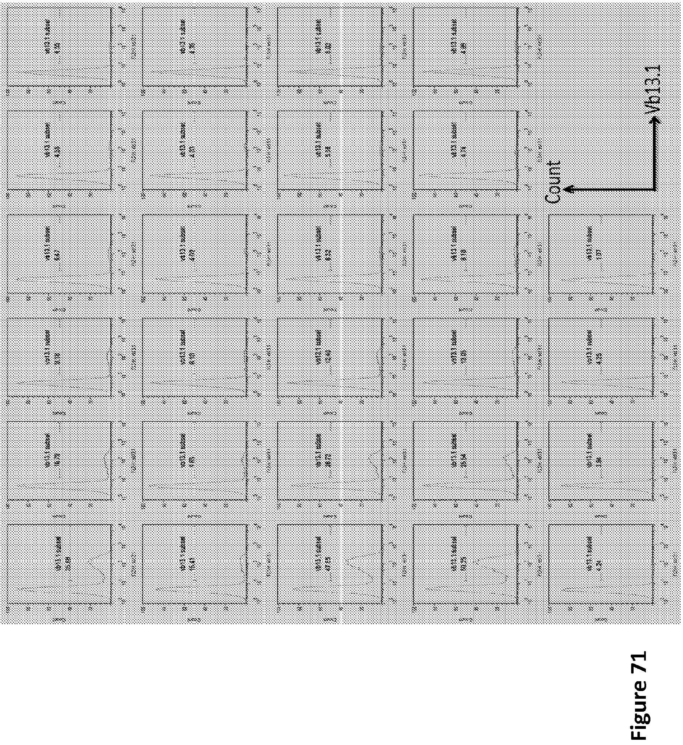

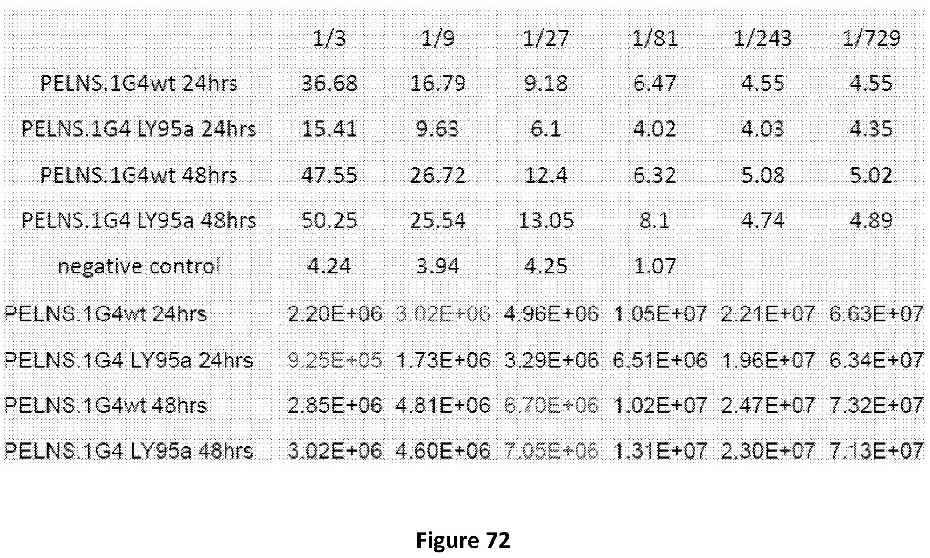

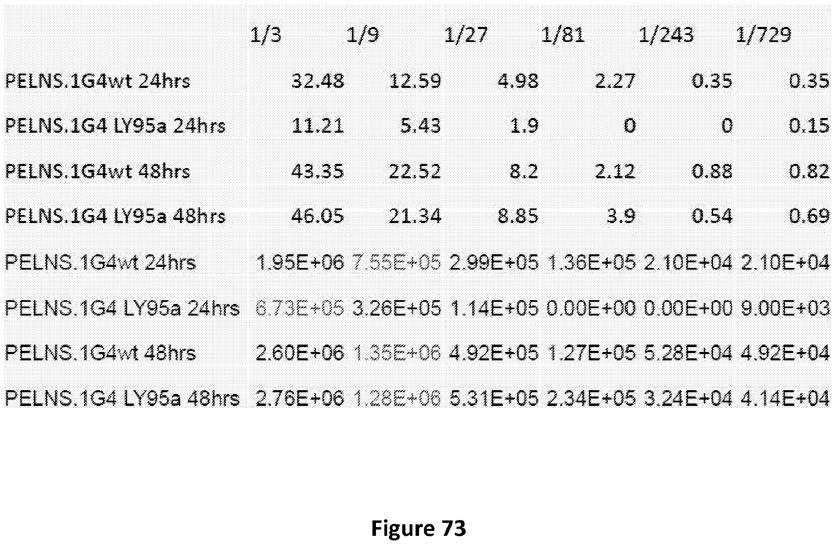

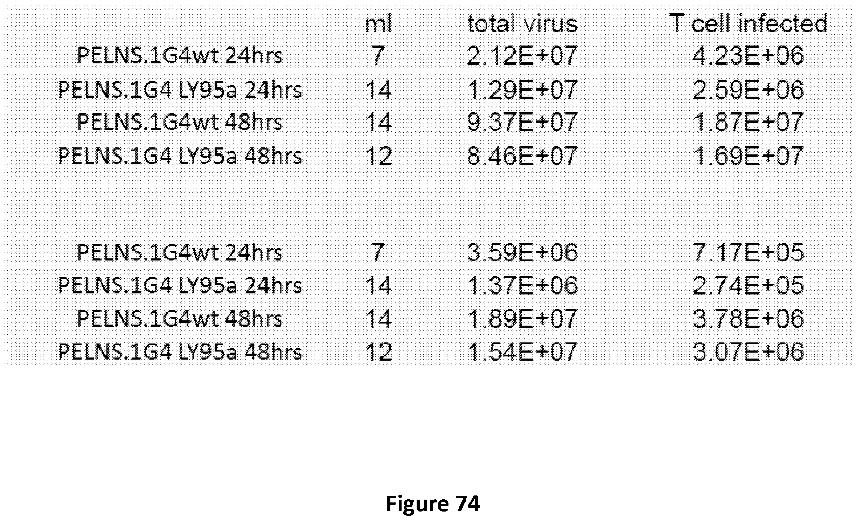

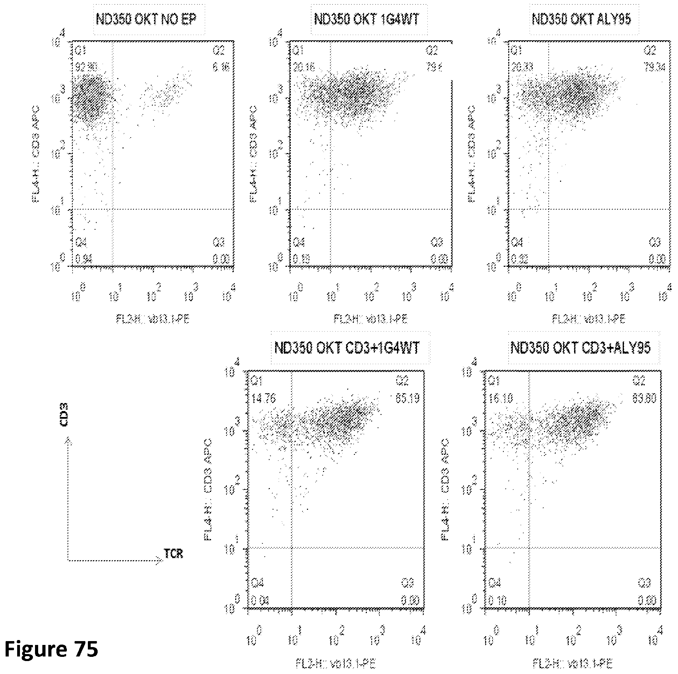

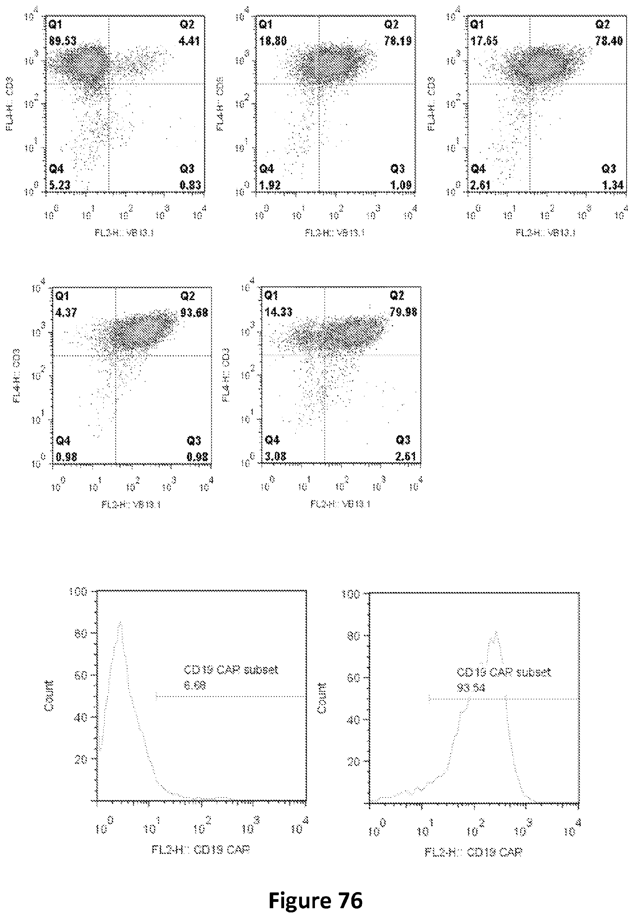

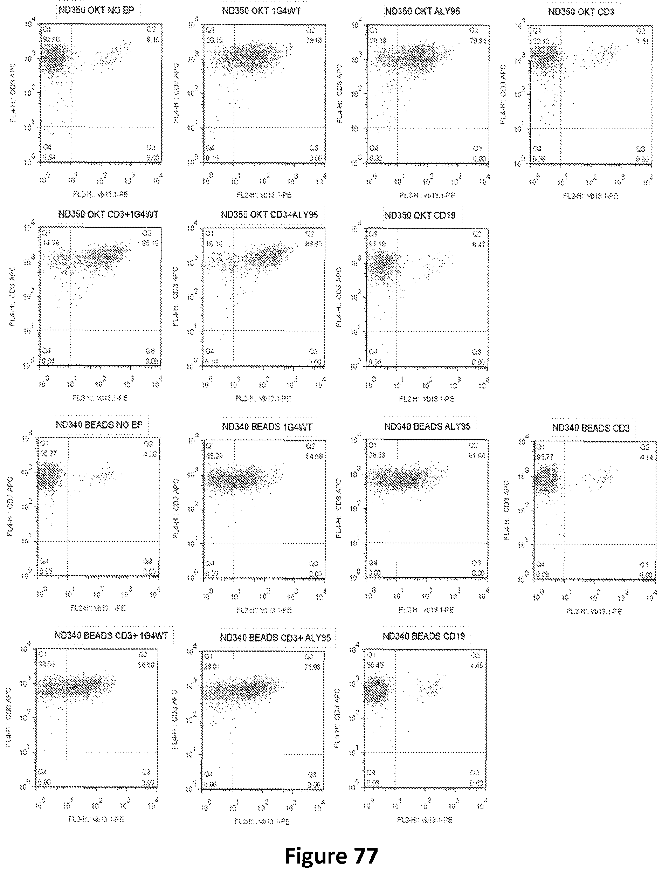

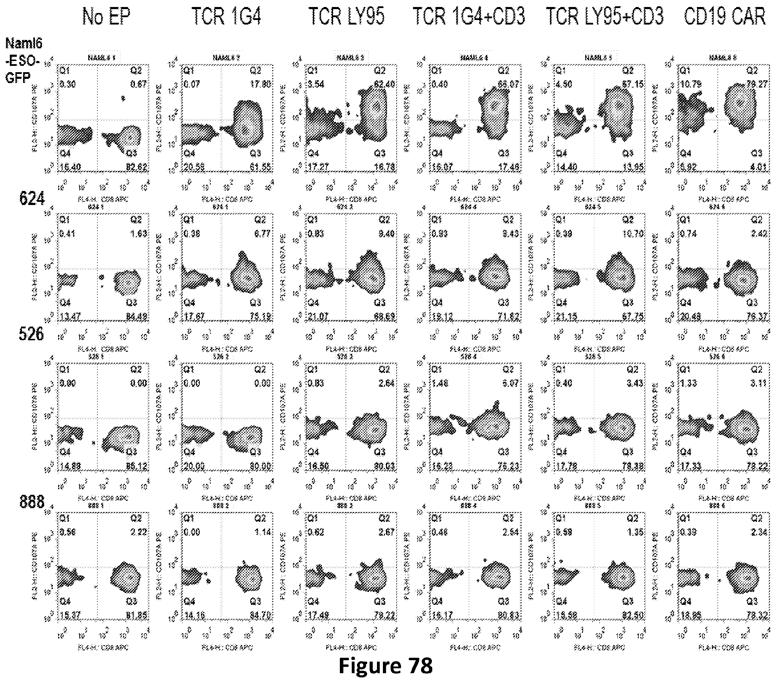

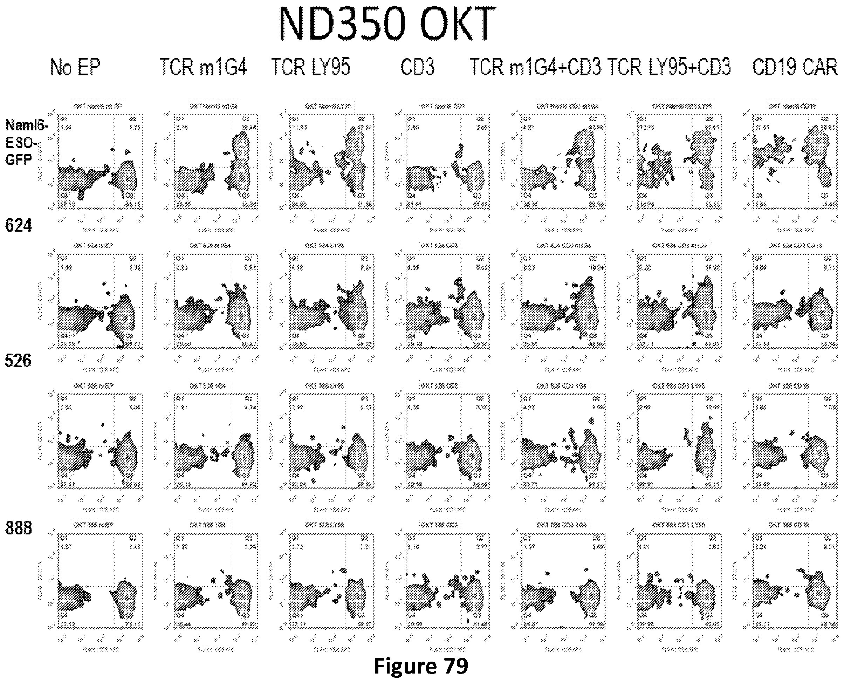

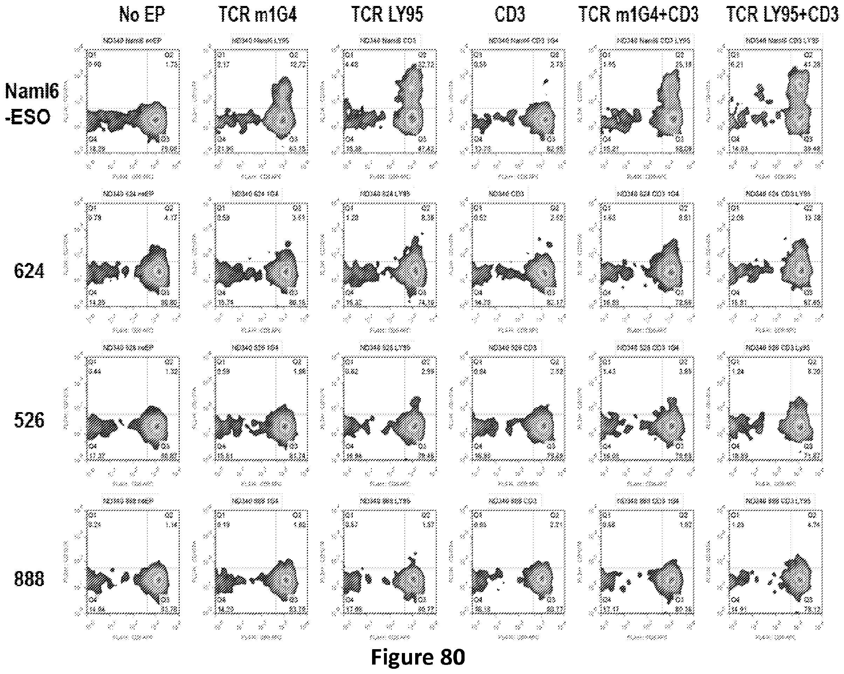

Based on pre-clinical animal data, lenti-TCR transduced T cells, both wildtype and high affinity, expressed in a single dose of RNA **(****Figures 64** and **65****),** indicating that lenti-TCR RNA T cells cannot be expended and persisted as efficiently as lenti-CAR T cells. This may be due to the lack of proper co-stimulation signals. Co-electroporating TCR with CD3 RNA further enhanced T cell anti-tumor activities as shown in **Figures 69-80****.** **Figure 69** is a panel of images showing an illustration of a CD3 construct and graphs showing TCR and CD3 expression in TCR and CD3 RNA electroporated T cells. **Figures 70** and **71** are graphs showing expression levels of TCR (vb13.1), CD3 and TCR (vb13.1)/CD3 were detected in electroporated T cells. **Figures 72-74** show tables showing infection efficiency. **Figures 75-80** show T cells electroporated with TCR RNA (y-axis) with or without CD3 and incubated with different tumor cell lines (x-axis).

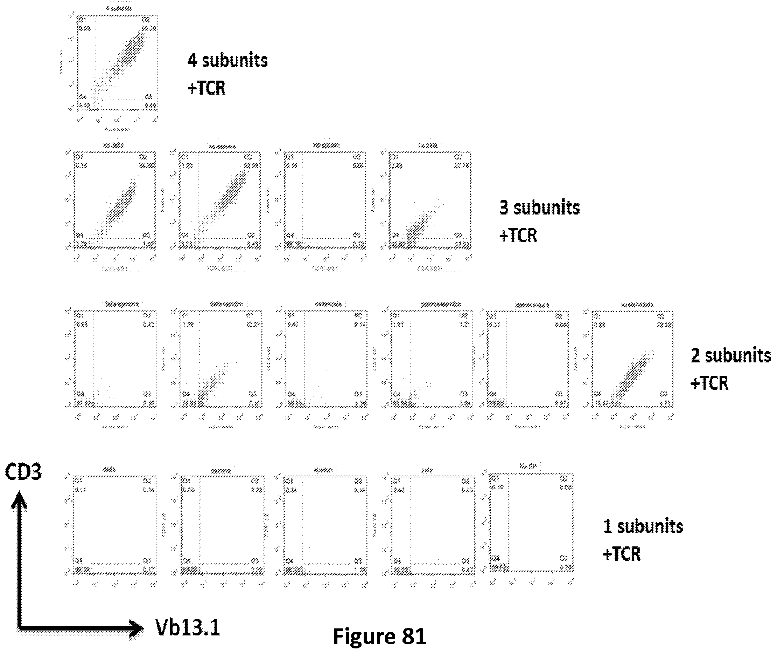

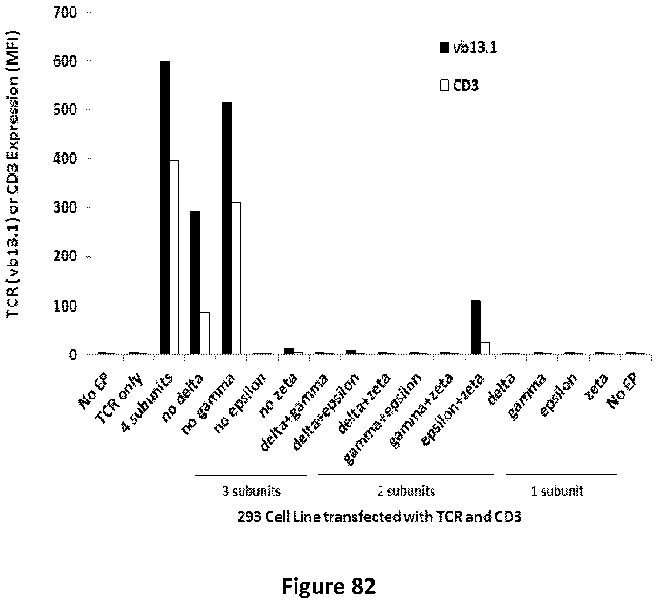

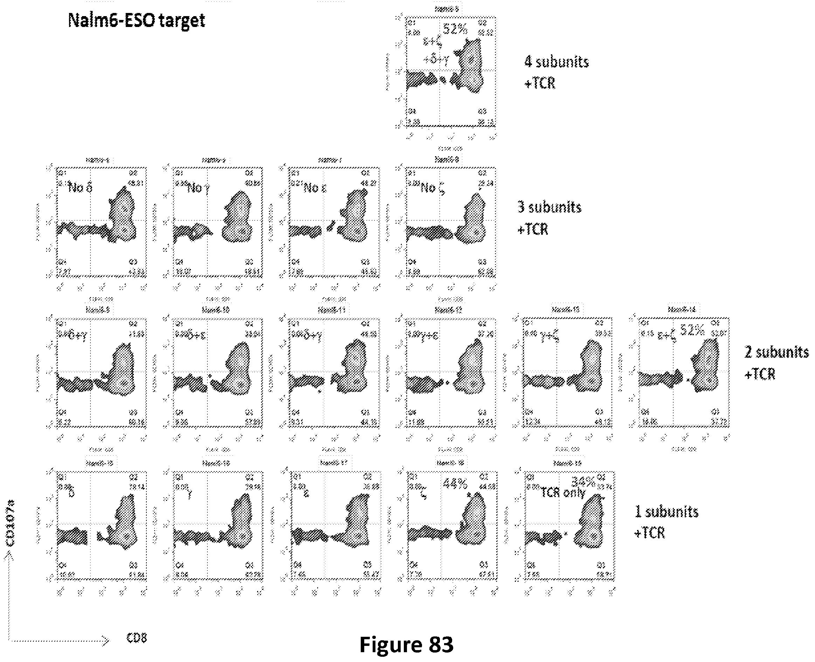

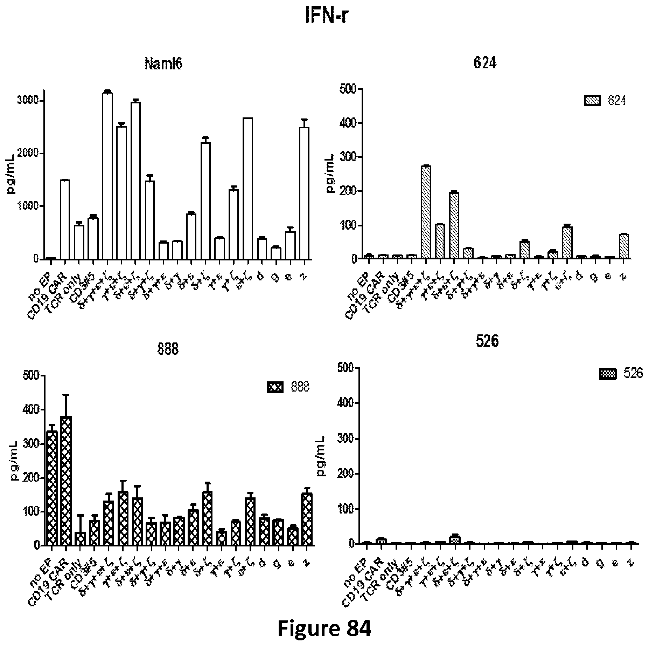

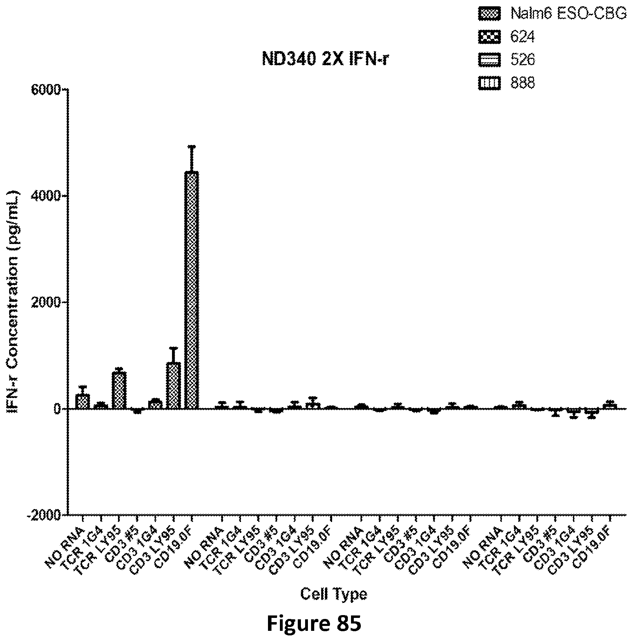

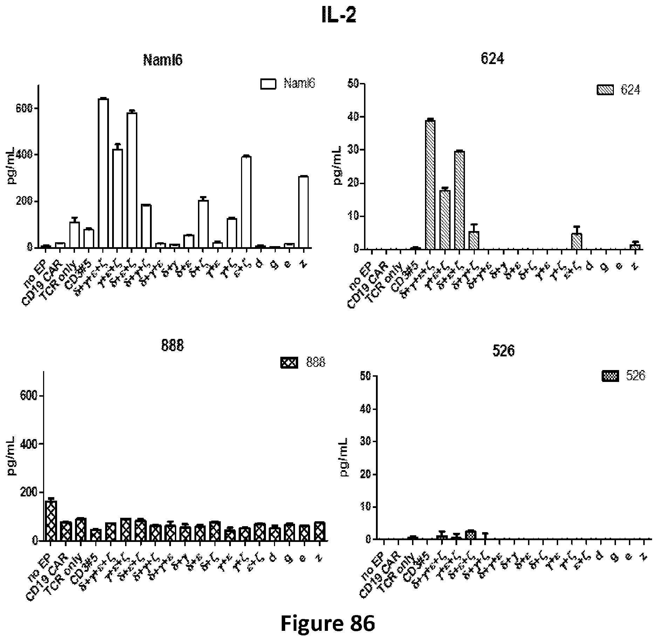

CD3 is a very important component for TCR mediated T cell function. The CD3/TCR complex is composed of six different molecules. In addition to the TCR alpha and beta chains, there are four CD3 units: gamma, delta, epsilon and zeta. By co-electroporation of RNA encoding the TCR alpha and beta chains from an anti-NY-ESO-1 TCR (1G4) with different combination of CD3 subunits into 293 cells, it was found that maximal TCR/CD3 expression was achieved by introducing all four CD3 subunits. As shown in **Figures 81** and **82****,** maximal T cell function was achieved by co-introducing all four subunits of CD3 with TCR RNA. Various combinations of CD3 and TCR subunits showed more or less T cell function enhancement with epsilon and zeta having the highest functionality. CD3 zeta with TCR RNA showed significant functional enhancement, compared with TCR alone, or TCR with other subunits. The CD3/TCR was expressed when only three or two subunits were co-electroporated, such as delta+epsilon+zeta, gamma+epsilon+zeta, and epsilon+zeta, indicating epsilon and zeta are relatively essential for TCR/CD3 expression **(****Figures 81** and **82****).** Tumor specific T cell function was tested by co-introducing 1G4 alpha/beta TCR with different combination of CD3 subunits. **Figure 83** is a panel of flow graphs showing CD107a up-regulation in TCR RNA and CD3 RNA co-electroporated T cells stimulated with tumor cells. **Figures 84****,** **85** and **86** show IFN-gamma and IL-2 expression in T cells electroporated with TCR RNA and different combinations of CD3 RNA after incubation with different tumor cell lines.

Thus, at a minimum co-introducing CD3 epsilon and zeta, or zeta alone, instead of all four subunits, may be a potential therapeutic if co-electroporation of multiple RNA strands poses technical challenges.

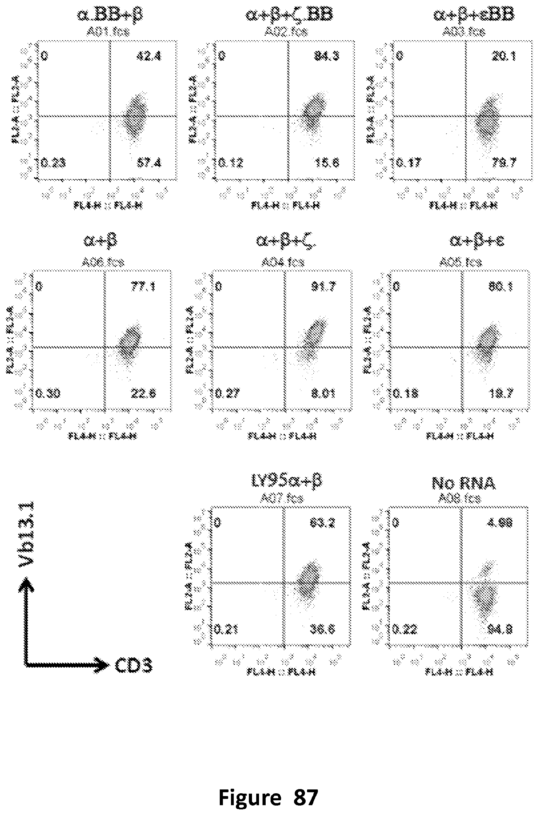

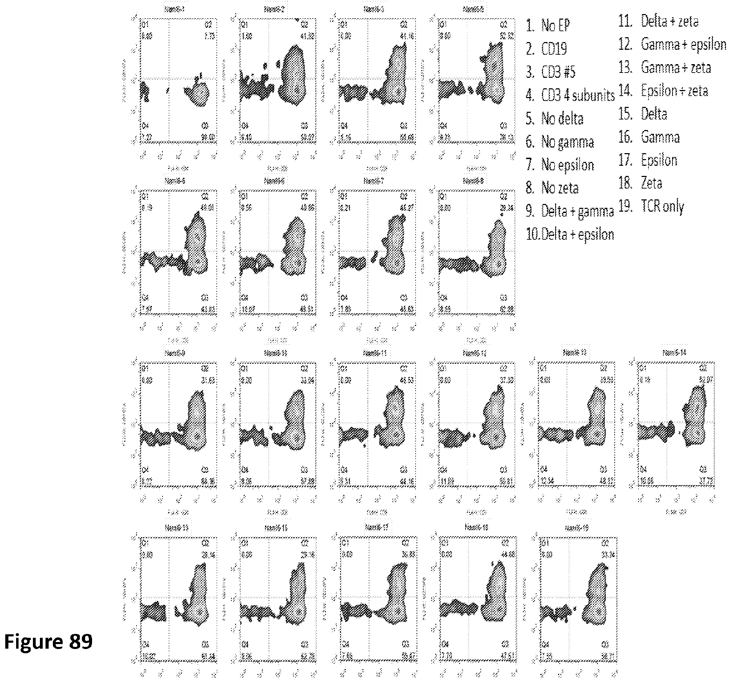

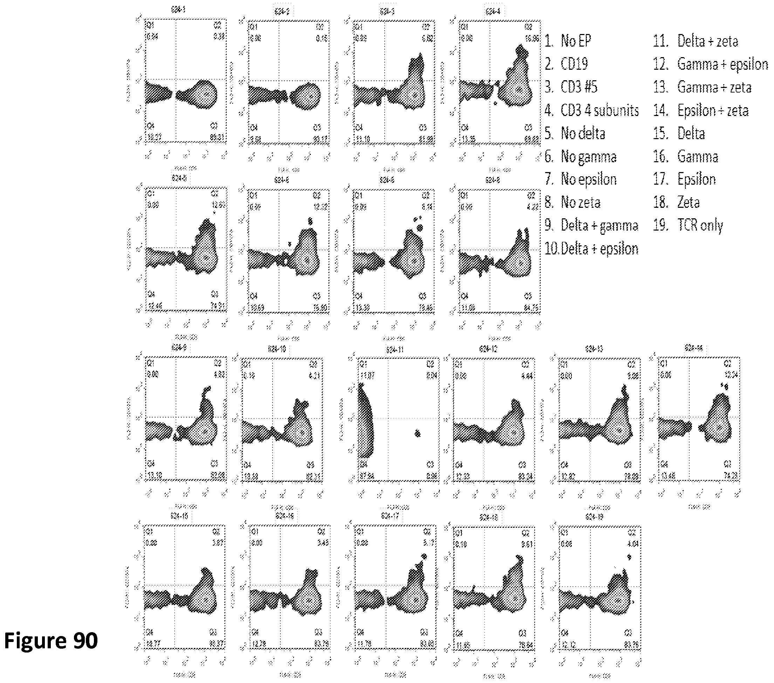

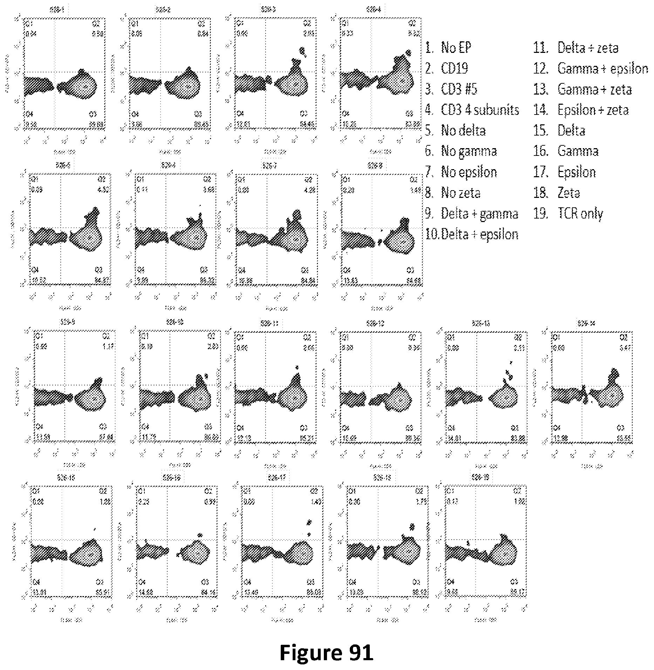

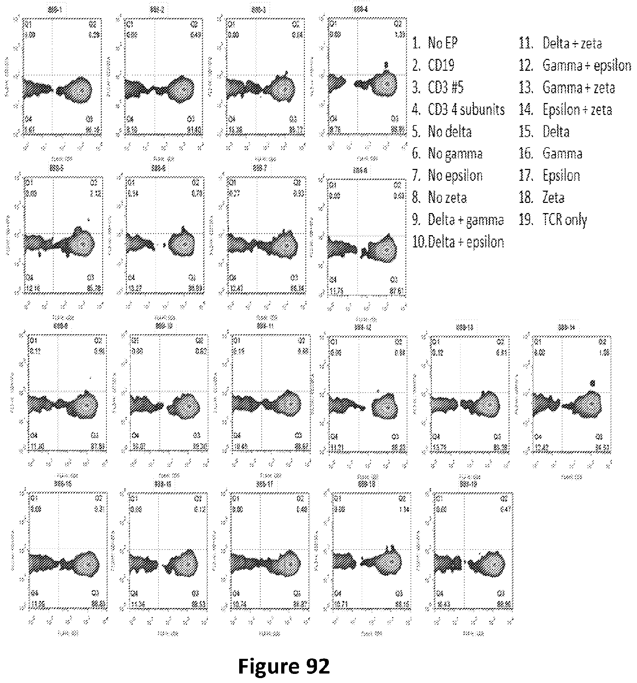

1G4 TCR alpha-41BB, CD3 zeta-41-BB, and CD3 epsilon-4-1BB constructs were generated by directly fusing a 4-1BB intracellular moiety to the C' terminus of the 1G4 TCR alpha, CD3 zeta or CD3 epsilon. RNA electroporation of these modified TCR or CD3 constructs showed that incorporation of 4-1BB into the TCR complex allowed TCR expression and functionality **(****Figures 87** and **88****).** Adding co-stimulatory signals to the C' terminus of either the TCR apha and/or beta chains or CD3 zeta and/or CD3 epsilon still maintained T cell function **(****Figures 89-92****)** and potentially provided T cells with a direct co-stimulatory signal.

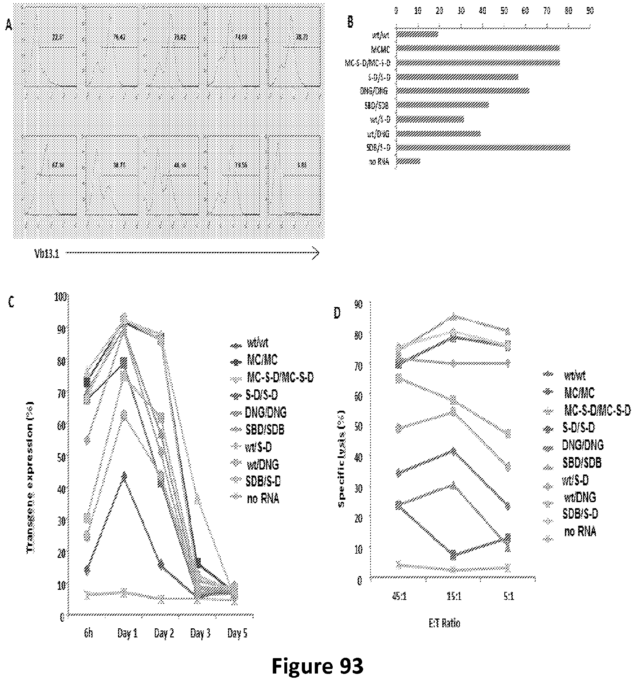

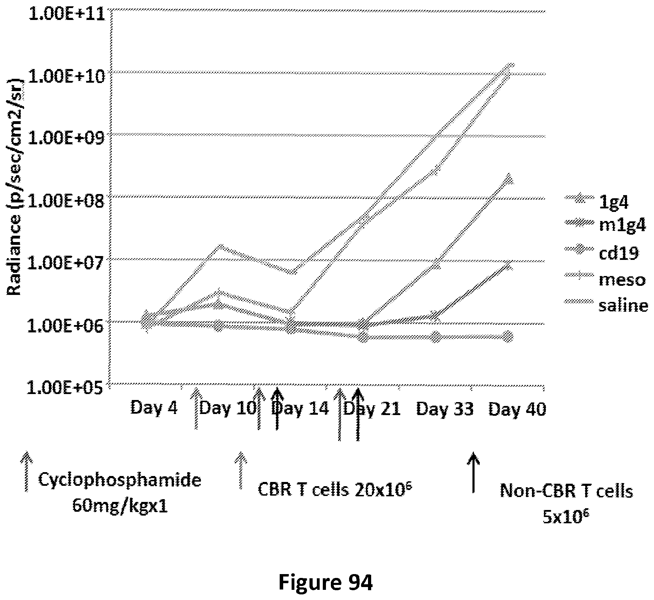

Adding a second disulfide bond to the TCR and removing N-glycosylation sites from TCR beta chain further enhanced both in vitro and in vivo functions of TCR RNA electroporated T cells **(****Figures 93** and **94****).** As shown in **Figure 93****,** mutations were made in the TCR alpha and/or beta chains and different combinations of alpha and beta were electroporated into T cells. It was found that maximal transgene expression **(****Figure 93A****)** and functionality (lytic ability) **(****Figure 93C** and **93D****)** was found by adding a second disulfide bond and removing defined N-glycosylation sites from the beta chain (Sa/S-Db) **(****Figure 93B****).** In an animal experiment, T cells with second disulfide bond to either the alpha or beta chains (m1G4) were compared with T cells electroporated with wildtype TCR (wt1G4) or CD19 CART cells **(****Figure 94****).** It was found that T cells with modified TCRs (m1G4) showed better treatment than the T cells with wildtype TCR (wt1G4), indicating that treatment may be improved by modifying the TCR with either a second disulfide bond and/or N-deglycosylation.

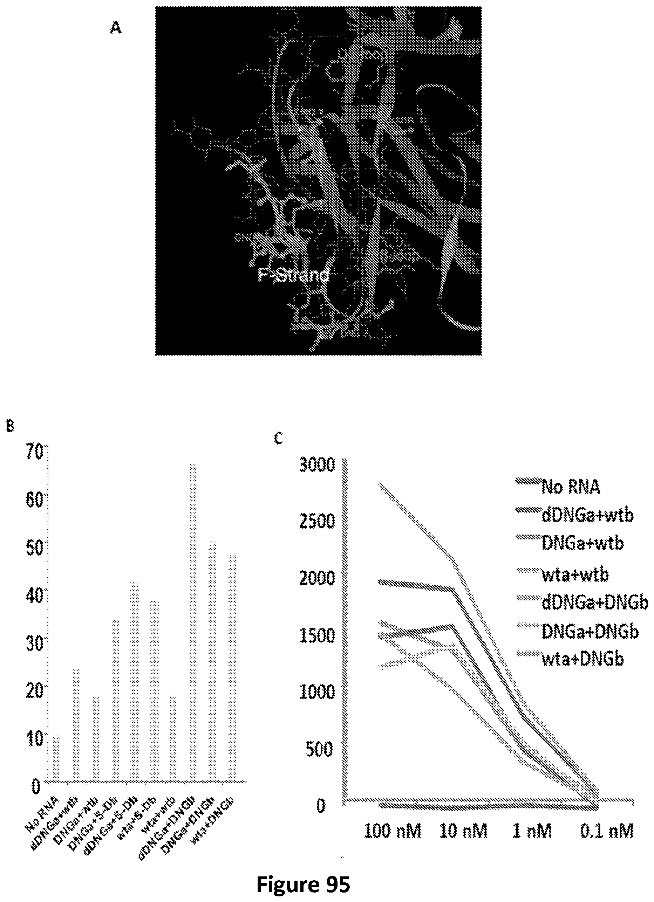

Interestingly, adding disulfide bonds to the TCR alpha chain impaired T cell function. **Figure 95** is a panel of images showing N-deglycosylation in the TCR alpha chain impaired function of RNA electroporated T cells as compared to adding disulfide bonds to the TCR or hybrid TCRs.

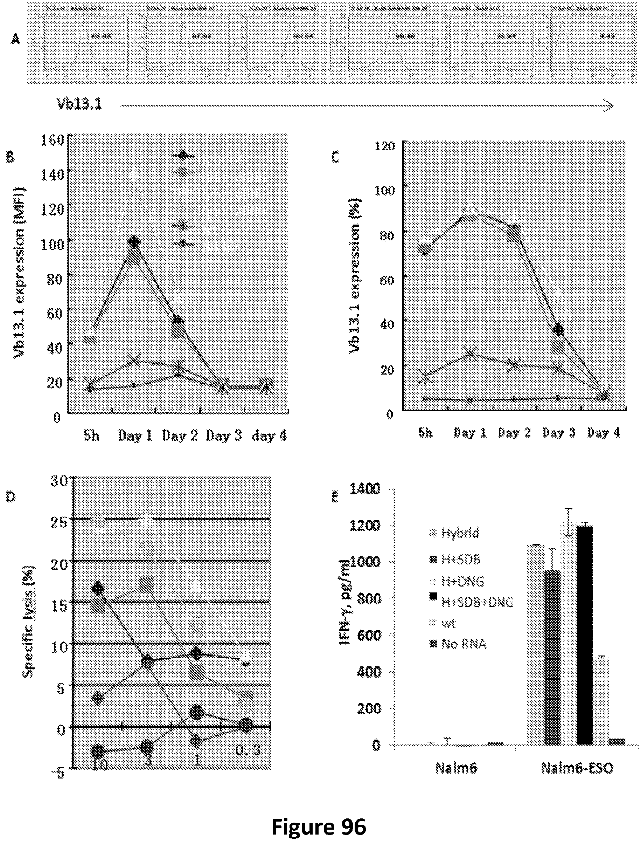

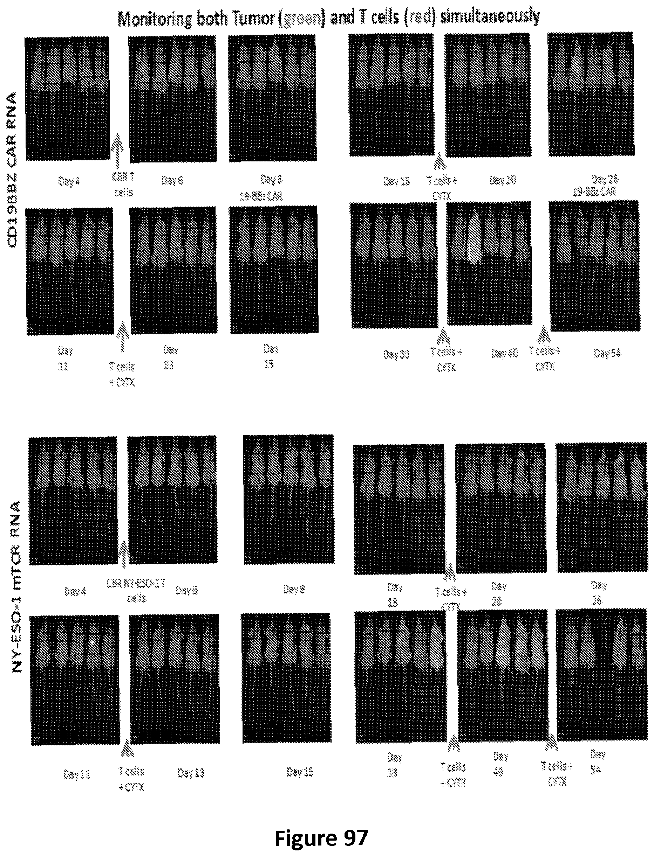

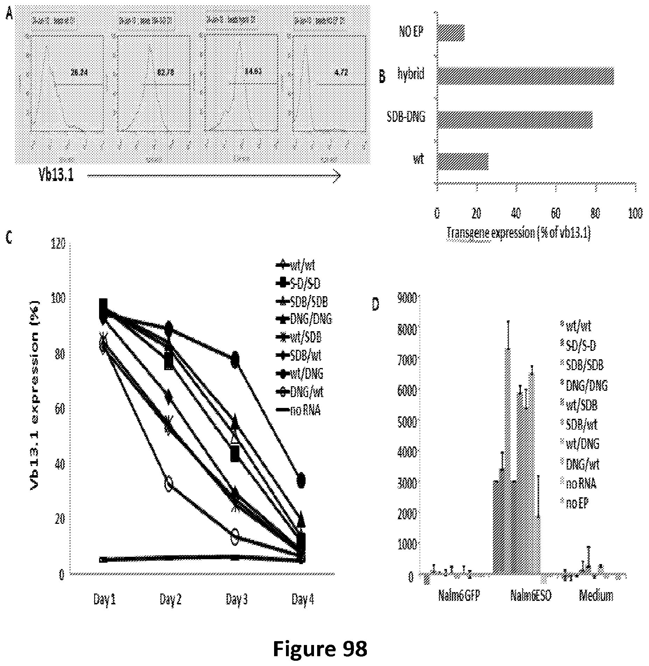

To determine if hybrid TCR with constant regions derived from mouse TCR constant regions affect functionality, hybrid TCRs containing murine constant regions were electroporated into T cells. **Figure 96** is a panel of images showing transgene expression and functionality of T cells electroporated with RNA encoding hybrid TCR containing murine constant regions. **Figure 97** is a panel of images showing fluorescence of injected tumor and hybrid TCR T cells in mouse models over time. **Figure 98** is a panel of images showing the addition of disulfide bonds to the alpha and beta chains, or N-deglycosylation of the beta chain of the TCR enhanced transgene expression and function of electroporated T cells as compared to adding disulfide bonds to the TCR or hybrid TCRs.

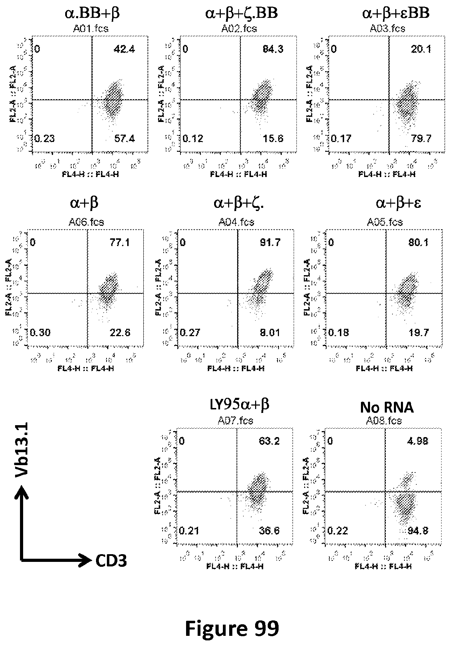

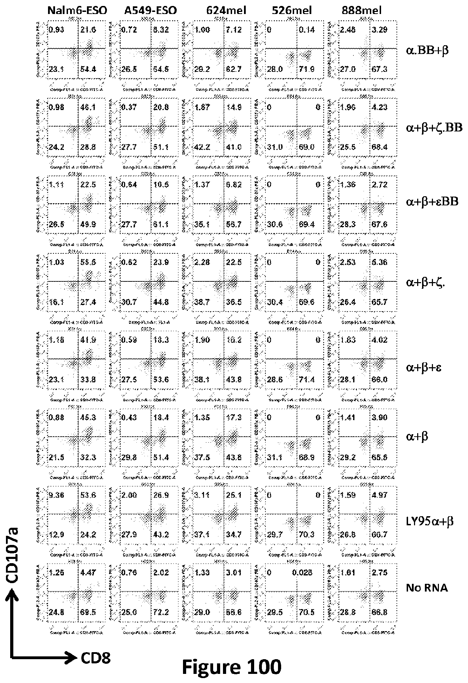

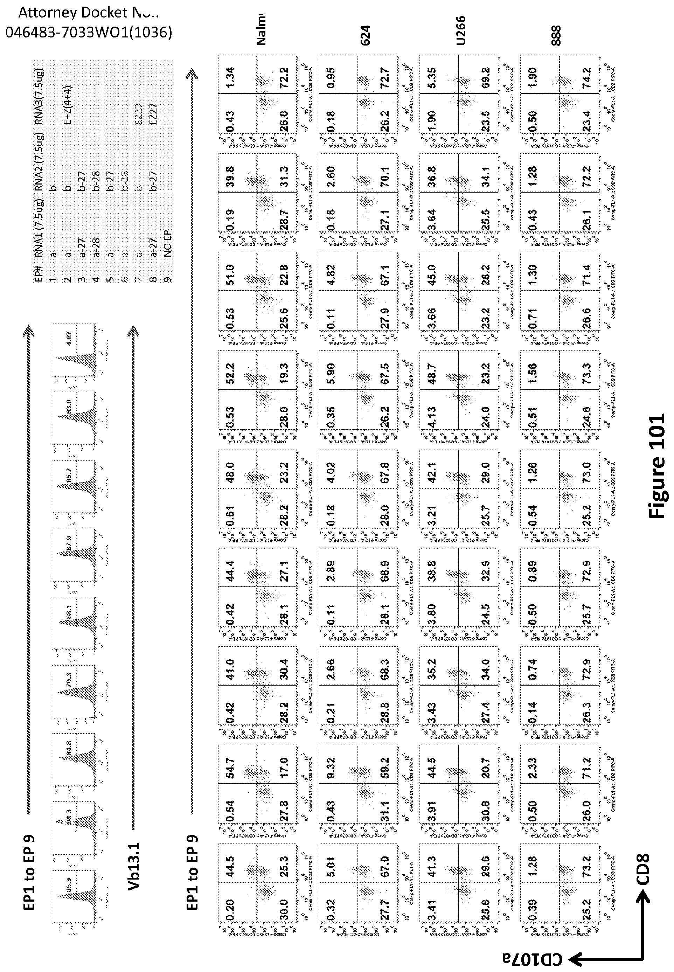

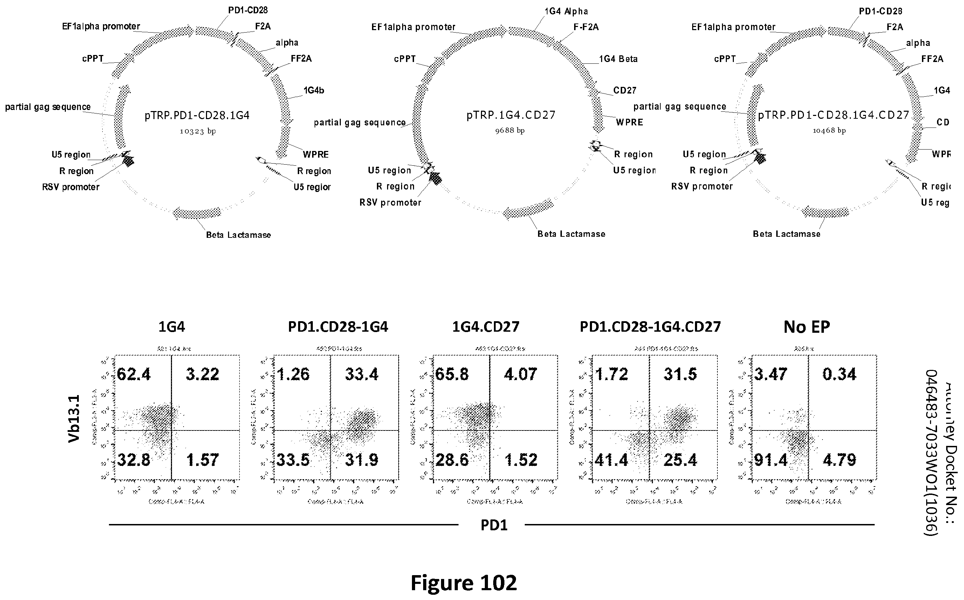

Adding co-stimulatory signals to the C' terminus of either TCR or CD3 chain maintained T cell function and provided the T cells with a co-stimulatory signal, similar to a CAR **(****Figures 99-101****).** **Figure 102** shows PD1 and vb13.1 were detected in T cells lentivirally transduced with PD1-CD28 RNA and 1G4 TCR RNA with or without CD27.

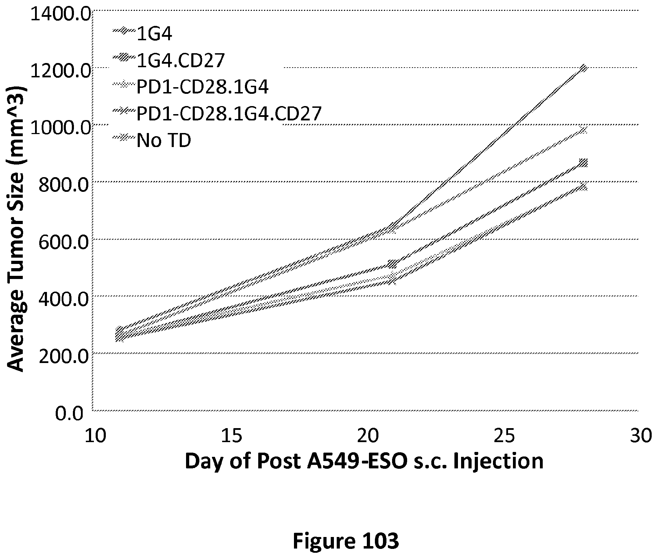

**Figure 103** is a graph showing 5E6 A549-ESO (HLA-A2 and NY-ESO-1 transduced A549) were injected subcutaneously at day 0. 1x10⁶ transduced T cells were injected at day 14 and the tumor size was measured. The preliminary results indicated no effect for TCR alone (1G4), while tumor growth was delayed for CD27 co-stimulatory signal (1G4.CD27), or PD1-CD28 switch receptor (PD1-CD28.1G4), or both CD27 co-stimulatory signal and PD1-CD28 switch receptor (PD1-CD28.1G4.CD27).

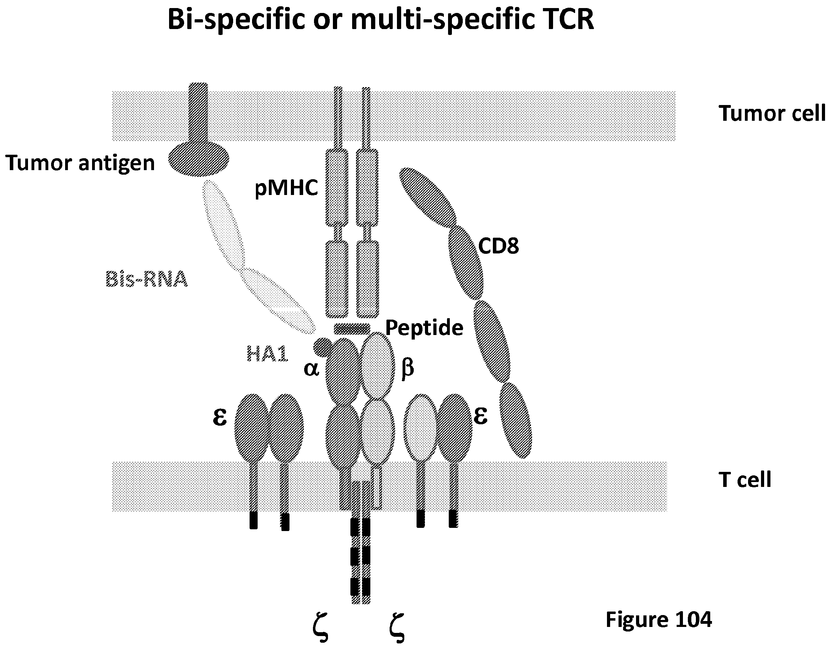

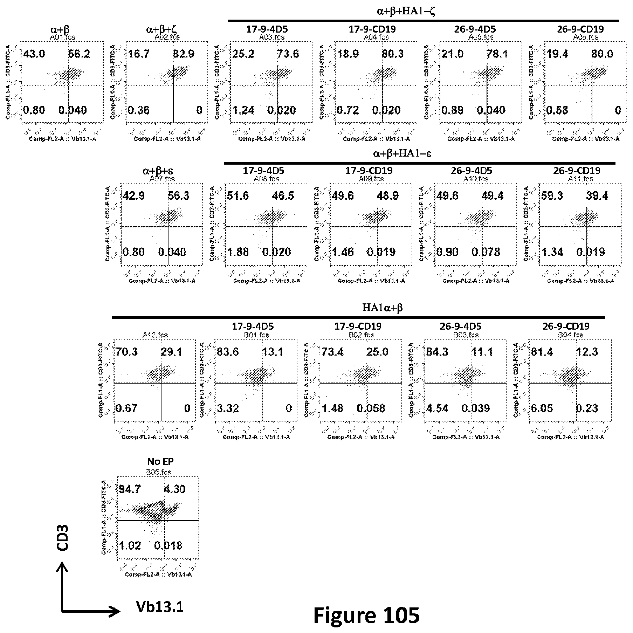

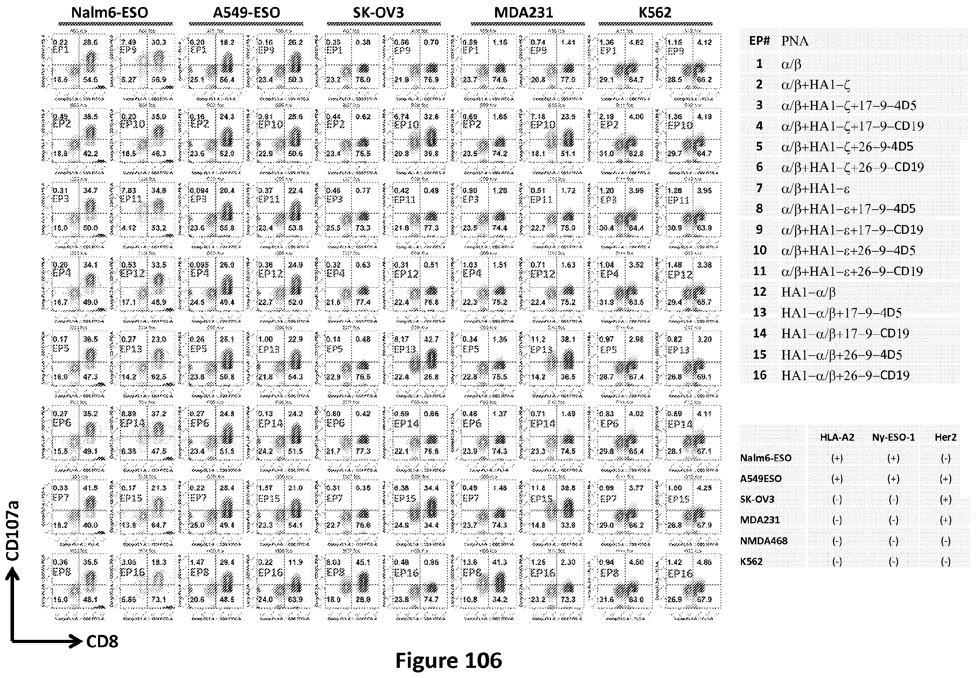

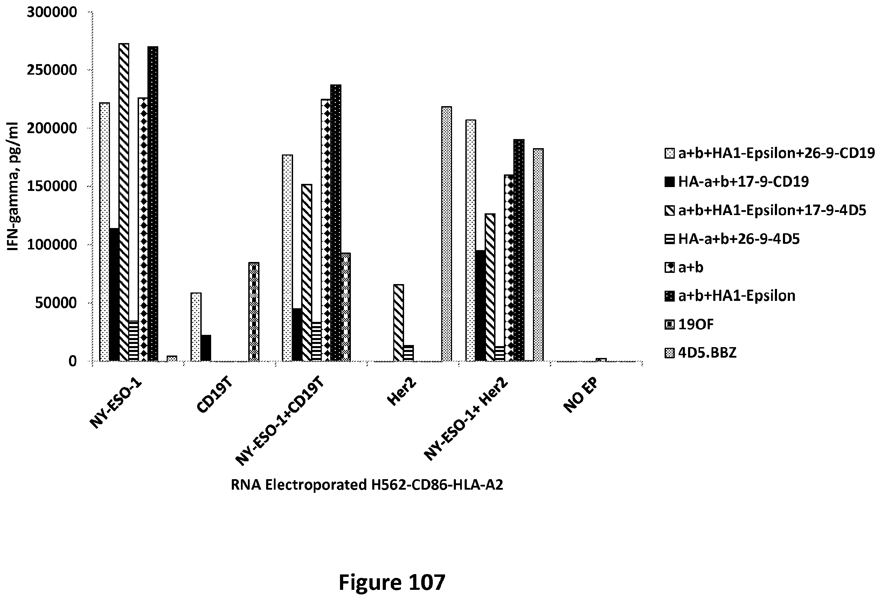

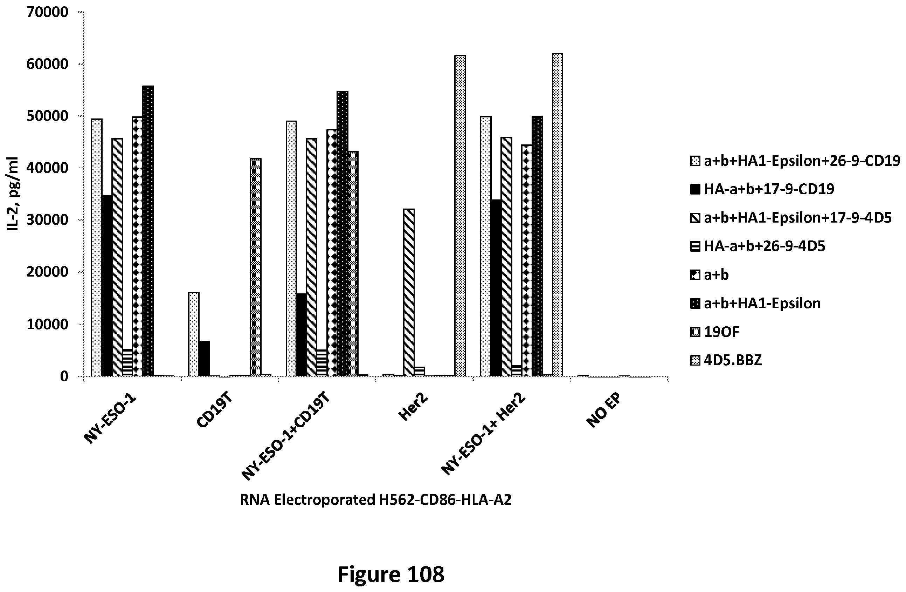

A schematic diagram is shown in **Figure 104** of a modified TCR capable of non-MHC restricted tumor antigen recognition with functional cognate antigen recognition. **Figures 105** and **106** show the TCR recognized both cognate MHC/peptide and surface tumor antigens (like CAR molecules) without HLA restriction when combined with Bis-RNA technology. In addition, IFN-gamma **(****Figure 107****)** and IL-2 **(****Figure 108****)** secretion was elevated in T cells modified with TCR co-introduced with bis-RNA.

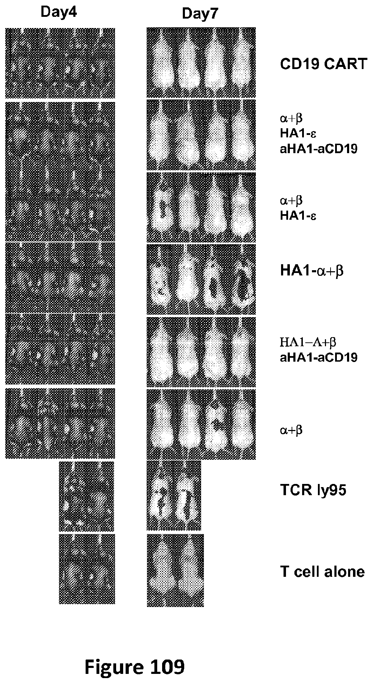

Mice injected with Nalm6-ESO (i.v.) and treated with T cells electroporated with modified TCRs show improved efficacy when the modified TCRs were co-delivered with bis-RNA against HA1 and CD19 (a+b/HA1-e/aHA1-aCD19, or HA1-A+b/aHA1-aCD19) as compared with the wild type TCR (a+b), wild type TCR plus HA1 modified CD3 epsilon (a+b HA1-e) or high affinity TCR (TCR ly95) **(****Figure 109****).**

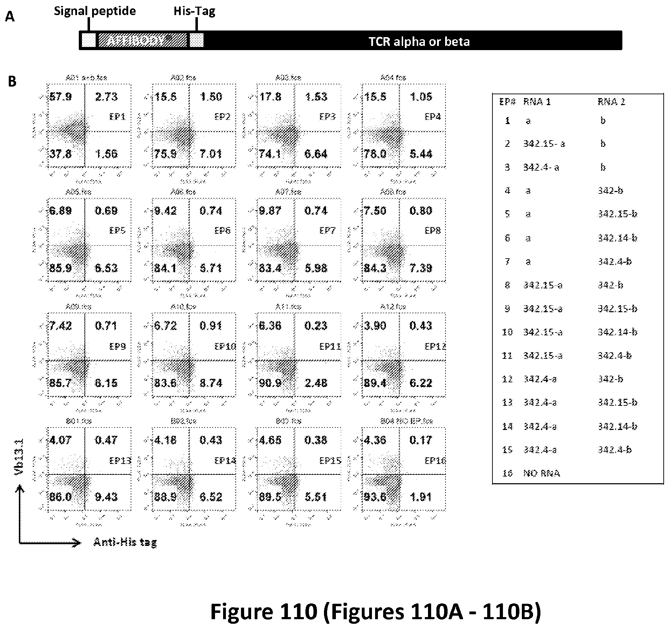

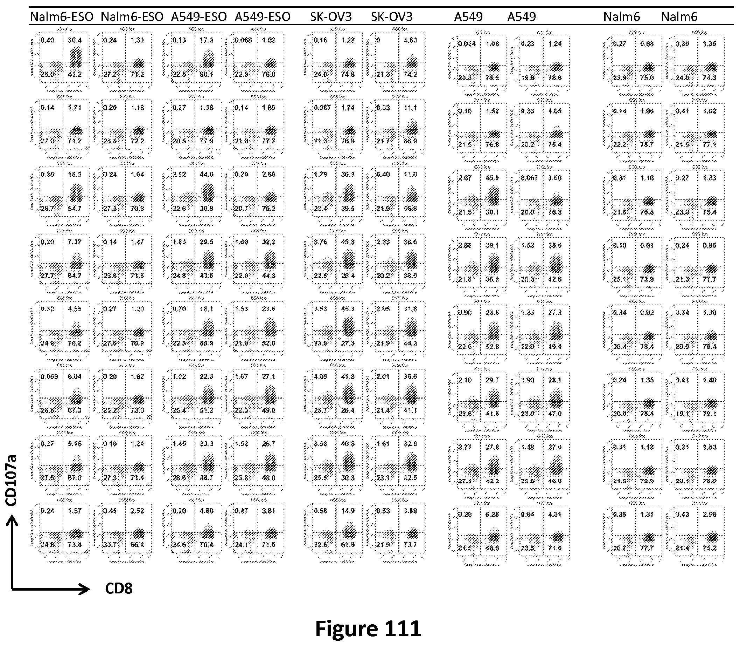

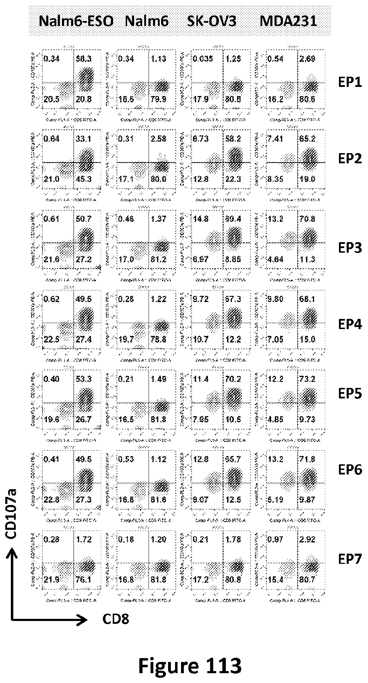

T cells were co-electroporated with TCR alpha (a) and beta (b), or an ErbB2 small molecule (342, 342.15, 342 or 342.4). As shown in **Figure 110****,** Vb13.1 TCR and His-tag were detected in the small molecule redirected TCR (Affi-TCR) RNA electroporated T cells. The electroporated T cells were stimulated with the tumor cell lines, Nalm-6-ESO (NY-eso-1+, ErbB2-), A549-ESO (NY-eso-1+, ErbB2+), SK-OV3 (NY-eso-1-, ErbB2+), A549 (NY-eso-1-, ErbB2+), or Nalm6 (NY-eso-1-, ErbB2-). Then the cells were assessed for CD107a expression. The results show that T cells with ErbB2 Affi-TCR specifically recognized both Ny-ESO-1 and ErbB2 positive tumors **(****Figure 111****).** Adding small molecules specific for CD3 epsilon minimally influenced NY-ESO-1 TCR expression, as shown in **Figure 112****.** Affi-CD3 epsilon co-electroporated T cells were found to be 76.5% to 82.9% CD8/vb13.1 double positive (EP3 to EP6), as compared with 44.5% for small molecule modified TCR T cells (EP2). Adding small molecules specific for CD3 epsilon minimally influenced NY-ESO-1 TCR ability to recognize NY-ESO-1 single positive tumor Nalm6-ESO, as shown in **Figure 113****.** Affi-CD3 epsilon co-electroporated T cells were found to be 49.5% to 53.3% CD8/CD107a double positive (EP3 to EP6), as compared with 33.1% for small molecule modified TCR T cells (EP2).

Moreover, the Affi-CD3 epsilon co-electroporated T cells (EP3 to EP6) showed higher antitumor activity than Affibody modified TCR (EP2) against ErbB2 positive tumor SK-OV3 and MDA231 **(****Figure 113****).**

## Claims

1. A modified T cell comprising an exogenous nucleic acid encoding a T cell receptor (TCR) comprising affinity for an antigen on a target cell and a nucleic acid encoding a bispecific antibody, wherein the T cell expresses the TCR and bispecific antibody on a surface of the T cell,
wherein the bispecific antibody comprises a bispecific antigen binding domain wherein the bispecific antigen binding domain comprises a first and a second single chain variable fragment (scFv) molecules, and
wherein the first scFv molecule is specific for at least one antigen on a target cell and the second scFv molecule is specific for an antigen on an activating T cell, and
wherein the activating T cell antigen is CD3.

2. The modified T cell of claim 1, wherein the TCR comprises at least one disulfide bond.

3. The modified T cell of claim 1, wherein the TCR comprises TCR alpha and beta chains, wherein the alpha and/or beta chain preferably comprises at least one N-deglycosylation; and wherein the TCR preferably comprises a co-stimulatory signaling domain at a C' terminal of at least one of the chains, preferably wherein the co-stimulatory signaling domain is 4-1BB.

4. The modified T cell of claim 1, wherein the TCR comprises at least one murine constant region.

5. The modified T cell of claim 1, wherein the TCR has higher affinity for the target cell antigen than the affinity of a wild type TCR for the target cell antigen.

6. The modified T cell of claim 1, wherein the target cell antigen is selected from the group consisting of a viral antigen, bacterial antigen, parasitic antigen, tumor cell associated antigen (TAA), disease cell associated antigen, and any fragment thereof.

7. The modified T cell of claim 1 further comprising a nucleic acid encoding a costimulatory molecule; preferably selected from the group consisting of CD3, CD27, CD28, CD83, CD86, CD127, 4-1BB, 4-1BBL, PD1 and PD1L, preferably the CD3 comprises at least two different CD3 chains; more preferably the different CD3 chains are CD3 zeta and epsilon chains.

8. A method for generating a modified T cell comprising introducing into a T cell a nucleic acid encoding a modified T cell receptor (TCR) comprising affinity for an antigen on a target cell and a nucleic acid encoding a bispecific antibody, wherein the T cell is capable of expressing the TCR and bispecific antibody, preferably wherein nucleic acid encoding the TCR comprises a nucleic acid encoding a TCR alpha and a TCR beta chain,
wherein the bispecific antibody comprises a bispecific antigen binding domain wherein the bispecific antigen binding domain comprises a first and a second single chain variable fragment (scFv) molecules, and
wherein the first scFv molecule is specific for at least one antigen on a target cell and the second scFv molecule is specific for an antigen on an activating T cell, and
wherein the activating T cell antigen is CD3.

9. The method of claim 8, wherein the T cell is obtained from the group consisting of peripheral blood mononuclear cells, cord blood cells, a purified population of T cells, and a T cell line.

10. The method of claim 8, wherein the nucleic acids comprise in vitro transcribed RNA or synthetic RNA.

11. The method of claim 8 further comprising expanding the T cell, preferably by culturing the T cell with a factor selected from the group consisting of flt3-L, IL-1, IL-2, IL-3 and c-kit ligand; preferably expanding comprises electroporating the T cell with RNA encoding a chimeric membrane protein and culturing the electroporated T cell, preferably the chimeric membrane protein comprises a single chain variable fragment (scFv) against CD3 and an intracellular domain comprising a fragment of an intracellular domain of CD28 and 4-1BB.

12. The method of claim 8 further comprising one or more of the following steps:
(i) cryopreserving the T cell prior to or after introducing the nucleic acids;
(ii) thawing the cryopreserved T cell prior to or after introducing the nucleic acids,
(iii) co-electroporating a RNA encoding a TCR alpha chain and a separate RNA encoding a TCR beta chain;
(iv) electroporating a RNA encoding CD3 into the T cells; preferably the CD3 RNA is co-electroporated with a TCR nucleic acid;
(v) expressing the bispecific antibody as a membrane protein.

13. The modified T cell of claim 1 for use in stimulating a T cell-mediated immune response to a target cell or tissue in a subject; optionally further comprising inducing lysis of the target cell or tissue; preferably wherein the induced lysis is antibody-dependent cell-mediated cytotoxicity (ADCC).

14. The modified T cell of claim 1 for use in adoptive cell transfer therapy for preventing or treating an immune reaction that is adverse to the subject.

15. The modified T cell of claim 1 for use in treating an autoimmune disease or cancer, preferably wherein the autoimmune disease is selected from the group consisting of Acquired Immunodeficiency Syndrome (AIDS), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barre syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Meniere's disease, mixed connective tissue disease, multiple sclerosis, myasthenia gravis, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjogren's syndrome, stiff-man syndrome, systemic lupus erythematosus, Takayasu arteritis, temporal arteritis/giant cell arteritis, ulcerative colitis, uveitis, vitiligo, Wegener's granulomatosis, and any combination thereof; and the cancer is selected from the group consisting of breast cancer, prostate cancer, ovarian cancer, cervical cancer, skin cancer, pancreatic cancer, colorectal cancer, renal cancer, liver cancer, brain cancer, lymphoma, leukemia, lung cancer, and any combination thereof.

16. A pharmaceutical composition comprising the modified T cell of claim 1 and optionally a pharmaceutically acceptable carrier.

17. The modified T cell of claim 1, wherein the nucleic acid encoding a bispecific antibody is an electroporated RNA.

18. The modified T cell of claim 1, wherein the TCR is a wild type TCR.

## Patentansprüche

1. Modifizierte T-Zelle, umfassend eine exogene Nukleinsäure, die für einen T-Zell-Rezeptor (TCR) codiert, welcher Affinität für ein Antigen auf einer Zielzelle umfasst, und eine Nukleinsäure, die für einen bispezifischen Antikörper codiert, wobei die T-Zelle den TCR und den bispezifischen Antikörper auf einer Oberfläche der T-Zelle exprimiert,
wobei der bispezifische Antikörper eine bispezifische Antigenbindungsdomäne umfasst, wobei die bispezifische Antigenbindungsdomäne Moleküle eines ersten und eines zweiten einzelkettigen variablen Fragments (scFv) umfasst und
wobei das erste scFv-Molekül für mindestens ein Antigen auf einer Zielzelle spezifisch ist und das zweite scFv-Molekül für ein Antigen auf einer aktivierenden T-Zelle spezifisch ist und
wobei das Antigen der aktivierenden T-Zelle CD3 ist.

2. Modifizierte T-Zelle nach Anspruch 1, wobei der TCR mindestens eine Disulfidbindung umfasst.

3. Modifizierte T-Zelle nach Anspruch 1, wobei der TCR TCR-Alpha- und Beta-Ketten umfasst, wobei die Alpha- und/oder Beta-Kette vorzugsweise mindestens eine N-Deglykosylierung umfasst und wobei der TCR vorzugsweise eine kostimulatorische Signaldomäne an einem C'-Terminus von mindestens einer der Ketten umfasst, wobei die kostimulatorische Signaldomäne vorzugsweise 4-1BB ist.

4. Modifizierte T-Zelle nach Anspruch 1, wobei der TCR mindestens eine konstante Region von der Maus umfasst.

5. Modifizierte T-Zelle nach Anspruch 1, wobei der TCR eine höhere Affinität für das Zielzellantigen als die Affinität eines Wildtyp-TCR für das Zielzellantigen aufweist.

6. Modifizierte T-Zelle nach Anspruch 1, wobei das Zielzellantigen ausgewählt ist aus der Gruppe bestehend aus einem viralen Antigen, einem bakteriellen Antigen, einem parasitären Antigen, einem tumorzellassoziierten Antigen (TAA), einem krankheitszellassoziierten Antigen und einem beliebigen Fragment davon.

7. Modifizierte T-Zelle nach Anspruch 1, die ferner eine Nukleinsäure umfasst, die für ein kostimulatorisches Molekül codiert, welches vorzugsweise ausgewählt ist aus der Gruppe bestehend aus CD3, CD27, CD28, CD83, CD86, CD127, 4-1BB, 4-1BBL, PD1 und PD1L, wobei das CD3 vorzugsweise mindestens zwei verschiedene CD3-Ketten umfasst; bevorzugter sind die verschiedenen CD3-Ketten CD3-Zeta- und Epsilon-Ketten.

8. Verfahren zum Erzeugen einer modifizierten T-Zelle, umfassend das Einführen einer Nukleinsäure, die für einen modifizierten T-Zell-Rezeptor (TCR) mit Affinität für ein Antigen auf einer Zielzelle codiert, und einer Nukleinsäure, die für einen bispezifischen Antikörper codiert, in eine T-Zelle, wobei die T-Zelle in der Lage ist, den TCR und den bispezifischen Antikörper zu exprimieren, wobei vorzugsweise die Nukleinsäure, die für den TCR codiert, eine Nukleinsäure umfasst, die für eine TCR-Alpha- und eine TCR-Beta-Kette codiert,
wobei der bispezifische Antikörper eine bispezifische Antigenbindungsdomäne umfasst, wobei die bispezifische Antigenbindungsdomäne Moleküle eines ersten und eines zweiten einzelkettigen variablen Fragments (scFv) umfasst und
wobei das erste scFv-Molekül für mindestens ein Antigen auf einer Zielzelle spezifisch ist und das zweite scFv-Molekül für ein Antigen auf einer aktivierenden T-Zelle spezifisch ist und
wobei das aktivierende T-Zell-Antigen CD3 ist.

9. Verfahren nach Anspruch 8, wobei die T-Zelle aus der Gruppe bestehend aus peripheren mononukleären Blutzellen, Nabelschnurblutzellen, einer gereinigten Population von T-Zellen und einer T-Zelllinie erhalten wird.

10. Verfahren nach Anspruch 8, wobei die Nukleinsäuren in vitro transkribierte RNA oder synthetische RNA umfassen.

11. Verfahren nach Anspruch 8, ferner umfassend das Expandieren der T-Zelle, vorzugsweise durch Kultivieren der T-Zelle mit einem Faktor, der aus der Gruppe bestehend aus Flt3-L, IL-1, IL-2, IL-3 und c-Kit-Ligand ausgewählt ist, wobei das Expandieren vorzugsweise das Elektroporieren der T-Zelle mit RNA, die für ein chimäres Membranprotein codiert, und das Kultivieren der elektroporierten T-Zelle umfasst, wobei das chimäre Membranprotein vorzugsweise ein einzelkettiges variables Fragment (scFv) gegen CD3 und eine intrazelluläre Domäne umfasst, welche ein Fragment einer intrazellulären Domäne von CD28 und 4-1 BB umfasst.

12. Verfahren nach Anspruch 8, das ferner einen oder mehrere der folgenden Schritte umfasst:
(i) Kryokonservieren der T-Zelle vor oder nach dem Einbringen der Nukleinsäuren,
(ii) Auftauen der kryokonservierten T-Zelle vor oder nach dem Einbringen der Nukleinsäuren,
(iii) Co-Elektroporieren einer RNA, die für eine TCR-Alpha-Kette codiert, und einer separaten RNA, die für eine TCR-Beta-Kette codiert,
(iv) Elektroporieren einer RNA, die für CD3 codiert, in die T-Zellen; vorzugsweise wird die CD3-RNA zusammen mit einer TCR-Nukleinsäure elektroporiert,
(v) Exprimieren des bispezifischen Antikörpers als Membranprotein.

13. Modifizierte T-Zelle nach Anspruch 1 zur Verwendung beim Stimulieren einer T-Zell-vermittelten Immunantwort auf eine Zielzelle oder ein Zielgewebe in einem Probanden, gegebenenfalls ferner umfassend das Induzieren der Lyse der Zielzelle oder des Zielgewebes, wobei vorzugsweise die induzierte Lyse eine Antikörperabhängige, zellvermittelte Zytotoxizität (ADCC) ist.

14. Modifizierte T-Zelle nach Anspruch 1 zur Verwendung in einer adoptiven Zelltransfertherapie zur Verhinderung oder Behandlung einer für den Probanden nachteiligen Immunreaktion.

15. Modifizierte T-Zelle nach Anspruch 1 zur Verwendung bei der Behandlung einer Autoimmunerkrankung oder von Krebs, wobei die Autoimmunerkrankung vorzugsweise ausgewählt ist aus der Gruppe bestehend aus erworbenem Immundefektsyndrom (AIDS), Alopecia areata, ankylosierender Spondylitis, Antiphospholipid-Syndrom, autoimmunologischer Form des Morbus Addison, autoimmunhämolytischer Anämie, Autoimmunhepatitis, autoimmuner Innenohrerkrankung (AIED), autoimmunem lymphoproliferativem Syndrom (ALPS), autoimmuner thrombozytopenischer Purpura (ATP), Morbus Behçet, Kardiomyopathie, Dermatitis herpetiformis bei Zöliakie, chronischem Erschöpfungssyndrom (CFIDS), chronischinflammatorischer demyelinisierender Polyneuropathie (CIPD), narbigem Pemphigoid, Kälteagglutininkrankheit, CREST-Syndrom, Morbus Crohn, Degos-Syndrom, juveniler Dermatomyositis, diskoidem Lupus, essentieller gemischter Kryoglobulinämie, Fibromyalgie/Fibromyositis, Morbus Basedow, Guillain-Barré-Syndrom, Hashimoto-Thyreoiditis, idiopathischer Lungenfibrose, idiopathischer thrombozytopenischer Purpura (ITP), IgA-Nephropathie, insulinabhängigem Diabetes mellitus, juveniler chronischer Arthritis (Morbus Still), juveniler rheumatoider Arthritis, Morbus Menière, gemischter Bindegewebserkrankung, Multipler Sklerose, Myasthenia gravis, perniziöser Anämie, Polyarteritis nodosa, Polychondritis, polyendokrinen Syndromen, Polymyalgia rheumatica, Polymyositis und Dermatomyositis, primärer Agammaglobulinämie, primärer biliärer Cholangitis, Psoriasis, Psoriasis-Arthritis, Raynaud-Phänomen, Reiter-Krankheit, rheumatischem Fieber, rheumatoider Arthritis, Sarkoidose, Sklerodermie (progressive systemische Sklerose (PSS), auch bekannt als systemische Sklerose (SS)), Sjögren-Syndrom, Stiff-Man-Syndrom, systemischem Lupus erythematodes, Takayasu-Arteriitis, Arteriitis temporalis/Riesenzellarteriitis, Colitis ulcerosa, Uveitis, Vitiligo, Wegener-Granulomatose und jeder Kombination davon, und der Krebs ausgewählt ist aus der Gruppe bestehend aus Brustkrebs, Prostatakrebs, Eierstockkrebs, Gebärmutterhalskrebs, Hautkrebs, Bauchspeicheldrüsenkrebs, Darmkrebs, Nierenkrebs, Leberkrebs, Hirntumor, Lymphom, Leukämie, Lungenkrebs und jeder Kombination davon.

16. Pharmazeutische Zusammensetzung, umfassend die modifizierte T-Zelle nach Anspruch 1 und gegebenenfalls einen pharmazeutisch akzeptablen Träger.

17. Modifizierte T-Zelle nach Anspruch 1, wobei die Nukleinsäure, die für einen bispezifischen Antikörper codiert, eine elektroporierte RNA ist.

18. Modifizierte T-Zelle nach Anspruch 1, wobei der TCR ein Wildtyp-TCR ist.

## Revendications

1. Cellule T modifiée comprenant un acide nucléique exogène codant pour un récepteur de cellule T (TCR) comprenant une affinité pour un antigène sur une cellule cible et un acide nucléique codant pour un anticorps bispécifique, dans laquelle la cellule T exprime le TCR et l'anticorps bispécifique sur une surface de la cellule T,
dans laquelle l'anticorps bispécifique comprend un domaine de liaison à l'antigène bispécifique dans laquelle le domaine de liaison à l'antigène bispécifique comprend une première et une deuxième molécules de fragment variable à chaîne unique (scFv), et
dans laquelle la première molécule scFv est spécifique d'au moins un antigène sur une cellule cible et la deuxième molécule scFv est spécifique d'un antigène sur une cellule T activatrice, et
dans laquelle l'antigène du lymphocyte T activateur est CD3.

2. Cellule T modifiée selon la revendication 1, dans laquelle le TCR comprend au moins une liaison disulfure.

3. Cellule T modifiée selon la revendication 1, dans laquelle le TCR comprend des chaînes alpha et bêta du TCR, dans laquelle la chaîne alpha et/ou bêta comprend de préférence au moins une N-déglycosylation ; et dans laquelle le TCR comprend de préférence un domaine de signalisation co-stimulatoire à un terminal C' d'au moins une des chaînes, de préférence dans laquelle le domaine de signalisation co-stimulatoire est 4-1BB.

4. Cellule T modifiée selon la revendication 1, dans laquelle le TCR comprend au moins une région constante murine.

5. Cellule T modifiée selon la revendication 1, dans laquelle le TCR a une affinité plus élevée pour l'antigène de la cellule cible que l'affinité d'un TCR de type sauvage pour l'antigène de la cellule cible.

6. Cellule T modifiée selon la revendication 1, dans laquelle l'antigène de la cellule cible est sélectionné dans le groupe constitué d'un antigène viral, d'un antigène bactérien, d'un antigène parasitaire, d'un antigène associé à une cellule tumorale (TAA), d'un antigène associé à une cellule malade, et de n'importe quel fragment de ceux-ci.

7. Cellule T modifiée selon la revendication 1 comprenant en outre un acide nucléique codant pour une molécule costimulatrice ; de préférence sélectionnée dans le groupe constitué de CD3, CD27, CD28, CD83, CD86, CD127, 4-1BB, 4-1BBL, PD1 et PD1L, de préférence le CD3 comprend au moins deux chaînes CD3 différentes ; plus préférentiellement les chaînes CD3 différentes sont les chaînes CD3 zêta et epsilon.

8. Procédé pour générer une cellule T modifiée comprenant l'introduction dans une cellule T d'un acide nucléique codant pour un récepteur de cellule T modifié (TCR) comprenant une affinité pour un antigène sur une cellule cible et un acide nucléique codant pour un anticorps bispécifique, dans lequel la cellule T est capable d'exprimer le TCR et l'anticorps bispécifique, de préférence dans lequel l'acide nucléique codant pour le TCR comprend un acide nucléique codant pour une chaîne alpha de TCR et une chaîne bêta de TCR,
dans lequel l'anticorps bispécifique comprend un domaine de liaison à l'antigène bispécifique dans lequel le domaine de liaison à l'antigène bispécifique comprend une première et une deuxième molécules de fragment variable à chaîne unique (scFv), et
dans lequel la première molécule scFv est spécifique d'au moins un antigène sur une cellule cible et la deuxième molécule scFv est spécifique d'un antigène sur une cellule T activatrice, et
dans lequel l'antigène du lymphocyte T activateur est CD3.

9. Procédé selon la revendication 8, dans lequel la cellule T est obtenue à partir du groupe constitué par les cellules mononucléaires du sang périphérique, les cellules du sang de cordon, une population purifiée de cellules T, et une lignée de cellules T.

10. Procédé selon la revendication 8, dans lequel les acides nucléiques comprennent un ARN transcrit *in vitro* ou un ARN synthétique.

11. Procédé selon la revendication 8 comprenant en outre l'expansion de la cellule T, de préférence en cultivant la cellule T avec un facteur sélectionné dans le groupe constitué de flt3-L, IL-1, IL-2, IL-3 et c-kit ligand ; de préférence, l'expansion comprend l'électroporisation de la cellule T avec un ARN codant pour une protéine membranaire chimérique et la culture de la cellule T électroporée, de préférence la protéine membranaire chimérique comprend un fragment variable à chaîne unique (scFv) contre CD3 et un domaine intracellulaire comprenant un fragment d'un domaine intracellulaire de CD28 et de 4-1BB.

12. Procédé selon la revendication 8 comprenant en outre une ou plusieurs des étapes supplémentaires suivantes :
(i) cryoconservation de la cellule T avant ou après l'introduction des acides nucléiques ;
(ii) décongélation de la cellule T cryoconservée avant ou après l'introduction des acides nucléiques,
(iii) électroporer un ARN codant pour une chaîne alpha du TCR et un ARN distinct codant pour une chaîne bêta du TCR ;
(iv) électroporer un ARN codant pour le CD3 dans les cellules T ; de préférence, l'ARN CD3 est coélectroporé avec un acide nucléique TCR ;
(v) exprimer l'anticorps bispécifique sous la forme d'une protéine membranaire.

13. Cellule T modifiée selon la revendication 1, utilisée pour stimuler une réponse immunitaire à médiation cellulaire T contre une cellule ou un tissu cible chez un sujet ; comprenant éventuellement en outre l'induction de la lyse de la cellule ou du tissu cible ; de préférence, dans laquelle la lyse induite est une cytotoxicité à médiation cellulaire dépendante des anticorps (ADCC).

14. Cellule T modifiée selon la revendication 1, destinée à être utilisée dans une thérapie par transfert cellulaire adoptif pour prévenir ou traiter une réaction immunitaire néfaste pour le sujet.

15. Cellule T modifiée selon la revendication 1 pour le traitement d'une maladie auto-immune ou d'un cancer, de préférence dans laquelle la maladie auto-immune est sélectionnée dans le groupe constitué par le syndrome d'immunodéficience acquise (SIDA), l'alopécie areata, la spondylarthrite ankylosante, le syndrome des antiphospholipides, la maladie d'Addison auto-immune, l'anémie hémolytique auto-immune, l'hépatite auto-immune, la maladie auto-immune de l'oreille interne (AIED), le syndrome lymphoprolifératif auto-immun (ALPS), le purpura thrombocytopénique auto-immun (ATP), la maladie de Behcet, la cardiomyopathie, le sprue cœliaque-dermatite hepetiformis ; le syndrome de fatigue chronique et de dysfonctionnement immunitaire (CFIDS), la polyneuropathie inflammatoire démyélinisante chronique (CIPD), la pemphigoïde cicatricielle, la maladie de l'agglutinine froide, le syndrome de Crest, la maladie de Crohn, la maladie de Degos, la dermatomyosite juvénile, le lupus discoïde, la cryoglobulinémie mixte essentielle, la fibromyalgie-fibromyosite, la maladie de Basedow, le syndrome de Guillain-Barre, la thyroïdite d'Hashimoto, la fibrose pulmonaire idiopathique, le purpura thrombocytopénique idiopathique (PTI), la néphropathie à IgA, le diabète sucré insulinodépendant, l'arthrite chronique juvénile (maladie de Still), la polyarthrite rhumatoïde juvénile, la maladie de Ménière, la maladie du tissu conjonctif mixte, la sclérose en plaques, la myasthénie grave, l'anémie pernicieuse, la polyartérite noueuse, la polychondrite, les syndromes polyglandulaires, la polymyalgie rhumatismale, la polymyosite et la dermatomyosite, l'agammaglobulinémie primaire, la cirrhose biliaire primitive, le psoriasis, l'arthrite psoriasique, le phénomène de Raynaud, le syndrome de Reiter, le rhumatisme articulaire aigu, la polyarthrite rhumatoïde, la sarcoïdose, la sclérodermie [sclérose systémique progressive (SSP), également appelée sclérose systémique (SS)], le syndrome de Sjögren, le syndrome de l'homme raide, le lupus érythémateux disséminé, l'artérite de Takayasu, l'artérite temporale/artérite à cellules géantes, la colite ulcéreuse, l'uvéite, le vitiligo, la granulomatose de Wegener, et toute combinaison de ces maladies ; et le cancer est sélectionné dans le groupe constitué par le cancer du sein, le cancer de la prostate, le cancer de l'ovaire, le cancer du col de l'utérus, le cancer de la peau, le cancer du pancréas, le cancer colorectal, le cancer du rein, le cancer du foie, le cancer du cerveau, le lymphome, la leucémie, le cancer du poumon, et toute combinaison de ceux-ci.

16. Composition pharmaceutique comprenant la cellule T modifiée selon la revendication 1 et éventuellement un support pharmaceutiquement acceptable.

17. Cellule T modifiée selon la revendication 1, dans laquelle l'acide nucléique codant pour un anticorps bispécifique est un ARN électroporé.

18. Cellule T modifiée selon la revendication 1, dans laquelle le TCR est un TCR de type sauvage.
